# EUROPEAN PATENT APPLICATION

(11) **EP 2 147 944 A1**
(43) Date of publication of application: **27.01.2010**
(21) Application number: 08740979.3
(22) Date of filing: 23.04.2008
(51) Int. Cl.: C08G 61/12, C07C 211/61, C07C 255/58, C09K 11/06, H01L 51/50

(54) **PYRENE POLYMER AND LUMINESCENT ELEMENT MADE WITH THE SAME**

(30) Priority: 27.04.2007 JP 2007118650; 28.09.2007 JP 2007253864
(71) Applicant: Sumitomo Chemical Company, Limited, Tokyo 104-8260 (JP); Sumation Co., Ltd., Chuo-ku Tokyo 104-8260 (JP)
(72) Inventor: FUKUSHIMA, Daisuke, Tsukuba-shi Ibaraki 305-0045 (JP); TSUBATA, Yoshiaki, Tsukuba-shi Ibaraki 305-0045 (JP); SEKINE, Chizu, Tsukuba-shi Ibaraki 300-4249 (JP)
(74) Representative: Duckworth, Timothy John
(86) International application number: PCT/JP2008/058311
(87) International publication number: WO 2008/136492

(57) **Abstract**

A polymer compound comprising a constitutional unit of the formula (1), and a repeating unit of any of the formulae (2) to (4): 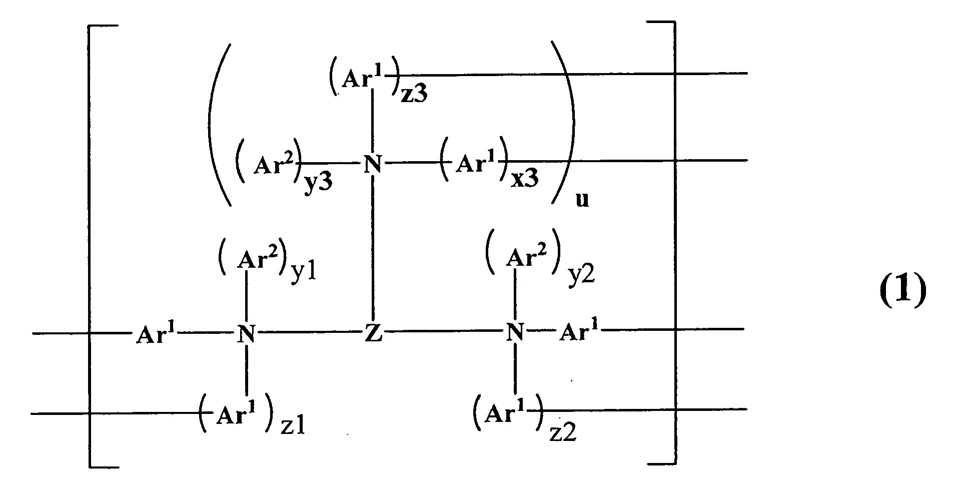 (wherein, x3, y1, y2, z1 to z3 are 0 or 1. y3 represents an integer of 0 to 2. u represents an integer of 0 to 8. Z represents a pyrene residue, Ar¹ represents an arylene group or di-valent heterocyclic group, and Ar² represents an aryl group or mono-valent heterocyclic group.)

⁅Ar³-⁆ (2)

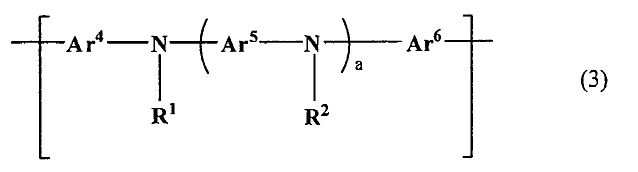

⁅Ar⁷-X¹⁆ (4)

## Description

### Technical Field

The present invention relates to a pyrene polymer compound and a light emitting device using the same.

### Background Art

Light emitting devices such as organic electroluminescence devices and the like are capable of manifesting multi-color light emission easily in addition to properties such as driving at low voltage, high luminance and the like, thus, suitable for applications such as displays and the like and recently paid to attention. For production of this light emitting device, light emitting materials and hole transporting materials are used.

As light emitting materials and charge transporting materials, investigated are polymer compounds which can be dissolved in a solvent and form an organic layer according to an application method. As such polymer compounds, polymer compounds are suggested containing a diphenylaminoanthracenediyl group (International Publication WO. 2005/49546).

### Disclosure of the Invention

However, the above-described polymer compounds do not necessarily show a sufficient balance between "fluorescence intensity" and "light emission efficiency of a light emitting device obtained when used for manufacturing of a light emitting device".

Thus, an object of the present invention is to provide a polymer compound showing an excellent balance between "fluorescence intensity" and "light emission efficiency of a light emitting device obtained when used for manufacturing of a light emitting device".

The present invention provides, in a first aspect, a polymer compound comprising a constitutional unit of the following formula (1), and at least one repeating unit selected from the group consisting of a repeating unit of the following formula (2), a repeating unit of the following formula (3) and a repeating unit of the following formula (4): 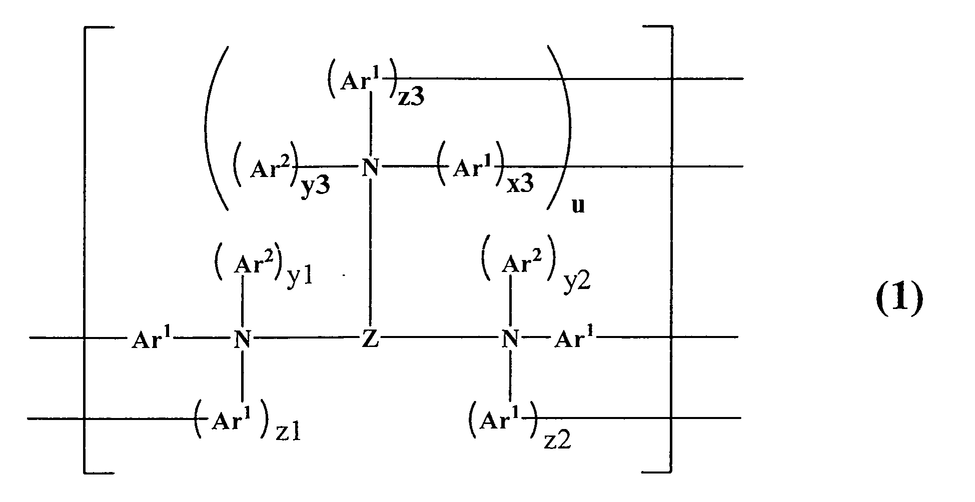 (wherein, x3, y1, y2, z1, z2 and z3 represent each independently 0 or 1. y3 represents an integer of 0 to 2. Here, y1+z1 and y2+z2 are 1, and x3+y3+z3 is 2. u represents an integer of 0 to 8. When u is 2 or more, x3's, y3's and z3's may each be the same or different. Z represents an unsubstituted or substituted (2+u)-valent pyrene residue, Ar¹ represents an unsubstituted or substituted arylene group or an unsubstituted or substituted di-valent heterocyclic group, and Ar² represents an unsubstituted or substituted aryl group or an unsubstituted or substituted mono-valent heterocyclic group. A plurality of Ar¹'s may be the same or different. When there exist a plurality of Ar²'s, they may be the same or different.)

⁅Ar³⁆ (2)

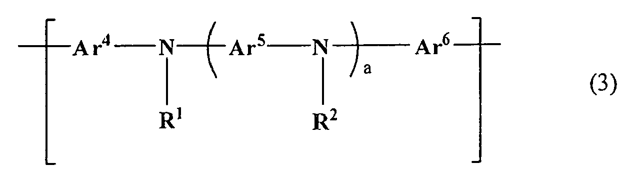

⁅Ar⁷-X¹⁆ (4)

(wherein, Ar³ and Ar⁷ represent each independently an unsubstituted or substituted arylene group, an unsubstituted or substituted di-valent heterocyclic group or a di-valent group having a metal complex structure. Ar⁹, Ar⁵ and Ar⁶ represent each independently an unsubstituted or substituted arylene group, an unsubstituted or substituted di-valent heterocyclic group or an unsubstituted or substituted di-valent group having two aromatic rings connected via a single bond. R¹ and R² represent each independently a hydrogen atom, alkyl group, aryl group, mono-valent heterocyclic group or arylalkyl group. X¹ represents -CR³=CR⁴- or -C≡C-. Here, R³ and R⁴ represent each independently a hydrogen atom, alkyl group, aryl group, mono-valent heterocyclic group, carboxyl group, substituted carboxyl group or cyano group. a represents 0 or 1.).

The present invention provides, in a second aspect, a method of producing a polymer compound comprising at least one constitutional unit of the above-described formula (1), and at least one selected from the group consisting of a repeating unit of the above-described formula (2), a repeating unit of the above-described formula (3) and a repeating unit of the above-described formula (4), comprising condensation-polymerizing at least one compound of the following formula (a), and at least one selected from the group consisting of a compound of the following formula (b-1), a compound of the following formula (b-2) and a compound of the following formula (b-3): 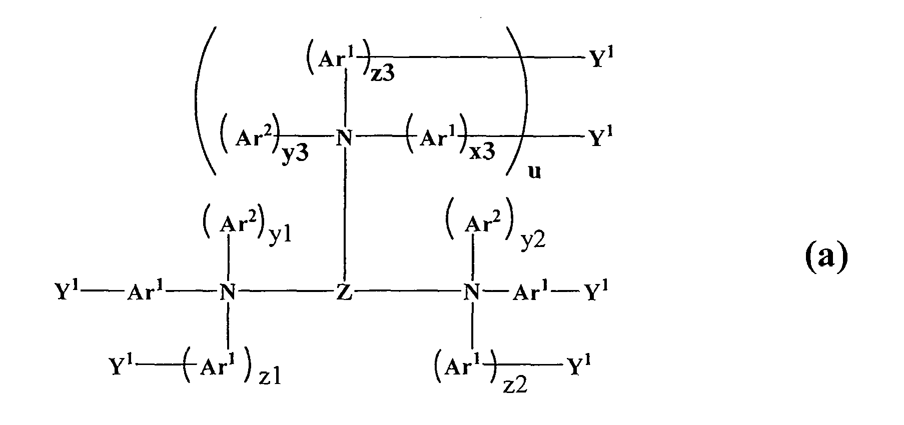 (wherein, x3, y1, y2, y3, z1, z2, z3, u, Z, Ar¹ and Ar² have the same meanings as described above. Y¹ represents a halogen atom, sulfonate group of the following formula (a-1), methoxy group, borate residue, boric acid residue, group of the following formula (a-2), group of the following formula (a-3), or group of the following formula (a-4). A plurality of Y¹'s may be the same or different. A plurality of Ar¹'s may be the same or different. When there exist a plurality of Ar²'s, they may be the same or different.)

**Y¹-Ar³-Y¹** **(b-1)**

(wherein, Ar³ and Y¹ have the same meanings as described above. A plurality of Y¹'s may be the same or different.) 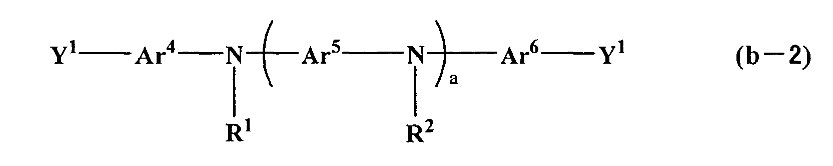 (wherein, Ar⁴, Ar⁵, Ar⁶, R¹, R² and a have the same meanings as described above. A plurality of Y¹'s may be the same or different.)

**Y¹-Ar⁷-X¹-Y¹** (b-3))

(wherein, Ar⁷, X¹ and Y¹ have the same meanings as described above. A plurality of Y¹'s may be the same or different.) 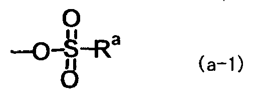 (wherein, R^{a} represents an unsubstituted or substituted alkyl group or an unsubstituted or substituted aryl group.)

**-MgX_{A}** (a-2)

(wherein, X_{A} represents a halogen atom.)

**-ZnX_{A}** (a-3)

(wherein, X_{A} has the same meaning as described above.)

**-Sn(R^{a})₃** **(a-4)**

(wherein, R^{a} has the same meaning as described above. A plurality of R^{a}'s may be mutually the same or different.).

The present invention provides, in a third aspect, a compound of the following formula (11): 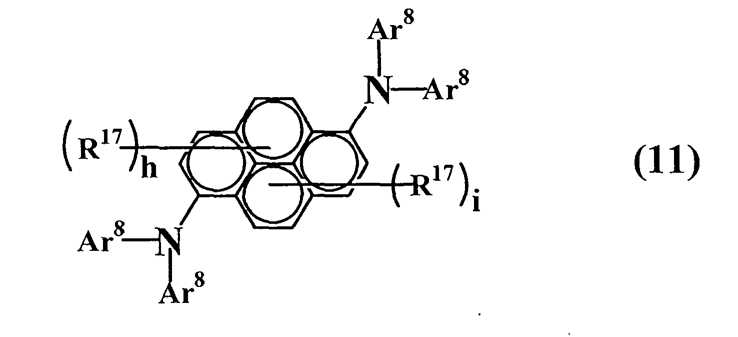 (wherein, R¹⁷ represents a halogen atom. h represents an integer of 1 to 4, and i represents an integer of 0 to 4. Ar⁸ represents an unsubstituted or substituted alkyl group, an unsubstituted or substituted aryl group, or an unsubstituted or substituted mono-valent heterocyclic group. A plurality of Ar⁸'s may be the same or different. When there exist a plurality of R¹⁷'s, they may be mutually the same or different.).

The present invention provides, in a fourth aspect, a compound of the following formula (15): 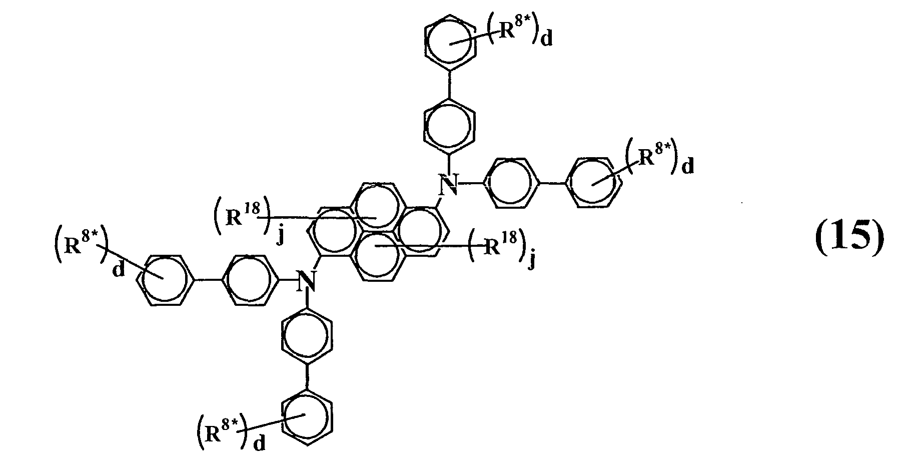 (wherein, R¹⁸ represents an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, acyl group, acyloxy group, imine residue, amide group, acid imide group, mono-valent heterocyclic group, carboxyl group, substituted carboxyl group, nitro group or cyano group. j represents an integer of 0 to 4. R^{8*} represents an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group, cyano group, boric acid residue or borate residue. d represents an integer of 0 to 5. When there exist a plurality of R^{8*}'s and R¹⁸'S respectively, they may be mutually the same or different. A plurality of d's and j's may be respectively the same or different.).

The present invention provides, in a fifth aspect, a method of producing a compound of the following formula (12): 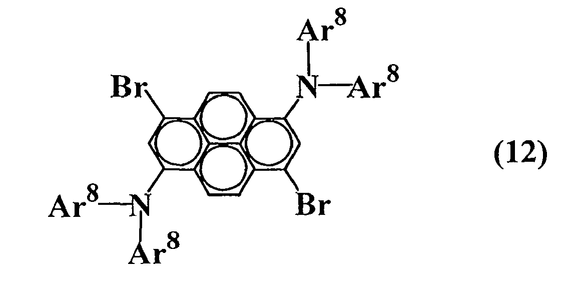 (wherein, Ar⁸ has the same meaning as described above. A plurality of Ar⁸'s may be the same or different.) comprising reacting a compound of the following formula (19) : 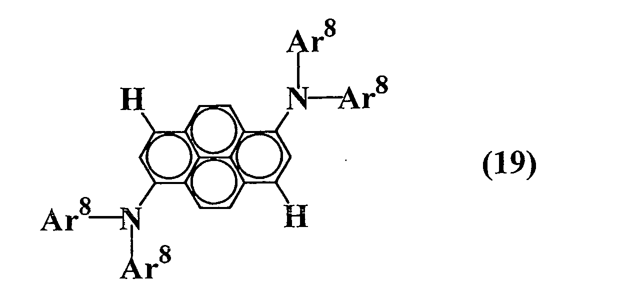 (wherein, Ar⁸ has the same meaning as described above. A plurality of Ar⁸'s may be mutually the same or different.)
with a brominating agent.

The present invention provides, in a sixth aspect, a composition comprising at least one material selected from the group consisting of hole transporting materials, electron transporting materials and light emitting materials, and the above-described polymer compound.

The present invention provides, in a seventh aspect, a solution comprising the above-described polymer compound, and a solvent.

The present invention provides, in an eighth aspect, a thin film comprising the above-described polymer compound.

The present invention provides, in a ninth aspect, a light emitting device having electrodes composed of an anode and a cathode, and an organic layer provided between the electrodes and containing the above-described polymer compound.

### Modes for Carrying Out the Invention

The present invention will be described in detail below.

In the present specification, "constitutional unit" denotes one or more units present in a polymer compound, and "repeating unit" denotes two or more units present in a polymer compound. "n-valent heterocyclic group" (n is 1 or 2) means a group obtained by removing n hydrogen atoms from a heterocyclic compound (particularly , heterocyclic compound having an aromatic property). "heterocyclic compound" includes organic compounds having a cyclic structure in which elements constituting the ring include not only a carbon atom, but also hetero atoms such as an oxygen atom, sulfur atom, nitrogen atom, phosphorus atom, boron atom and the like contained in the ring.

### <Polymer Compound>

### -- Constitutional unit of the formula (1) --

The polymer compound of the present invention comprises one kind or two or more kinds of constitutional units of the formula (1). The above-described constitutional unit of the formula (1) is preferably a repeating unit (namely, two or more constitutional units of the same kind are present).

In the above-described formula (1), Z represents an unsubstituted or substituted (2+u)-valent pyrene residue. In the present specification, "(2+u)-valent pyrene residue" means a group obtained by removing (2+u) hydrogen atoms from pyrene.

When Z has a substituent, the substituent is usually selected from the group consisting of an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group and cyano group, preferably selected from the group consisting of an alkyl group, alkoxy group, aryl group, aryloxy group, arylalkyl group, arylalkoxy group, arylalkenyl group, arylalkynyl group, substituted amino group, halogen atom, acyl group, mono-valent heterocyclic group, substituted carboxyl group and cyano group, more preferably selected from the group consisting of an alkyl group, alkoxy group, aryl group, aryloxy group, substituted amino group, acyl group, mono-valent heterocyclic group and substituted carboxyl group, further preferably selected from the group consisting of an alkyl group, alkoxy group, aryl group, substituted amino group and mono-valent heterocyclic group, and especially preferably is an aryl group or substituted amino group and particularly preferably an aryl group.

The above-described alkyl group may be any of linear, branched or cyclic, and has a carbon number of usually about 1 to 20, preferably 1 to 15, more preferably 1 to 10. The above-described alkyl group includes a methyl group, ethyl group, n-propyl group, i-propyl group, n-butyl group, i-butyl group, t-butyl group, n-pentyl group, isoamyl group, n-hexyl group, cyclohexyl group, n-heptyl group, n-octyl group, 2-ethylhexyl group, n-nonyl group, n-decyl group, 3,7-dimethyloctyl group, lauryl group, trifluoromethyl group, pentafluoroethyl group, perfluorobutyl group, perfluorohexyl group, perfluorooctyl group and the like, and preferable from the standpoint of a balance between the solubility of the resultant polymer compound in an organic solvent and the heat resistance thereof, and the like are a methyl group, ethyl group, n-propyl group, i-propyl group, n-butyl group, i-butyl group, t-butyl group, n-pentyl group, isoamyl group, n-hexyl group, n-octyl group, 2-ethylhexyl group, n-decyl group and 3,7-dimethyloctyl group.

The above-described alkoxy group may be any of linear, branched or cyclic, and has a carbon number of usually about 1 to 20, preferably 1 to 15. The above-described alkoxy group includes a methoxy group, ethoxy group, n-propyloxy group, i-propyloxy group, n-butoxy group, i-butoxy group, t-butoxy group, n-pentyloxy group, n-hexyloxy group, cyclohexyloxy group, n-heptyloxy group, n-octyloxy group, 2-ethylhexyloxy group, n-nonyloxy group, n-decyloxy group, 3,7-dimethyloctyloxy group, lauryloxy group, trifluoromethoxy group, pentafluoroethoxy group, perfluorobutoxy group, perfluorohexyloxy group, perfluorooctyloxy group, methoxymethyloxy group, 2-methoxyethyloxy group, 2-ethoxyethyloxy group and the like, and preferable from the standpoint of a balance between the solubility of the resultant polymer compound in an organic solvent and the heat resistance thereof, and the like are an n-butyloxy group, n-pentyloxy group, n-hexyloxy group, n-octyloxy group, 2-ethylhexyloxy group, n-decyloxy group and 3,7-dimethyloctyloxy group.

The above-described alkylthio group may be any of linear, branched or cyclic, and has a carbon number of usually about 1 to 20, preferably 3 to 20. The above-described alkylthio group includes a methylthio group, ethylthio group, n-propylthio group, i-propylthio group, n-butylthio group, i-butylthio group, t-butylthio group, n-pentylthio group, n-hexylthio group, cyclohexylthio group, n-heptylthio group, n-octylthio group, 2-ethylhexylthio group, n-nonylthio group, n-decylthio group, 3,7-dimethyloctylthio group, laurylthio group, trifluoromethylthio group and the like, and preferable from the standpoint of a balance between the solubility of the resultant polymer compound in an organic solvent and the heat resistance thereof, and the like are an n-pentylthio group, n-hexylthio group, n-octylthio group, 2-ethylhexylthio group, n-decylthio group and 3,7-dimethyloctylthio group.

The above-described aryl group is an atomic group obtained by removing one hydrogen atom from an aromatic hydrocarbon, and includes those having a condensed ring, and those having two or more independent benzene rings or condensed rings connected directly or via a group such as vinylene and the like. The above-described aryl group has a carbon number of usually about 6 to 60, preferably 6 to 48, more preferably 6 to 20, further preferably 6 to 10. This carbon number does not include the carbon number of the substituent. The above-described aryl group includes a phenyl group, 1-naphthyl group, 2-naphthyl group, 1-anthracenyl group, 2-anthracenyl group, 9-anthracenyl group, 1-tetracenyl group, 2-tetracenyl group, 5-tetracenyl group, 1-pyrenyl group, 2-pyrenyl group, 4-pyrenyl group, 2-perylenyl group, 3-perylenyl group, 2-fluorenyl group, 3-fluorenyl group, 4-fluorenyl group, 1-biphenylenyl group, 2-biphenylenyl group, 2-phenanthrenyl group, 9-phenanthrenyl group, 6-chrysenyl group, 1-coronenyl group, 2-phenylphenyl group, 3-phenylphenyl group, 4-phenylphenyl group, 4-(anthracen-9-yl)phenyl group, [1,1']binaphthalen-4-yl group, 10-phenylanthracen-9-yl group, [9,9']bianthracen-10-yl group and the like, and these may be further substituted with an alkyl group, alkoxy group, alkyloxycarbonyl group, acyl group, N,N-dialkylamino group, N,N-diarylamino group, cyano group, nitro group, chlorine atom, fluorine atom or the like.

The above-described aryloxy group has a carbon number of usually about 6 to 60, preferably 7 to 48. The above-described aryloxy group includes a phenoxy group, C₁ to C₁₂ alkoxyphenoxy groups ("C₁ to C₁₂ alkoxy" means that the alkoxy portion has a carbon number of 1 to 12, being applicable also in the following descriptions), C₁ to C₁₂ alkylphenoxy groups ("C₁ to C₁₂ alkyl" means that the alkyl portion has a carbon number of 1 to 12, being applicable also in the following descriptions), 1-naphthyloxy group, 2-naphthyloxy group, pentafluorophenyloxy group and the like, and preferable from the standpoint of a balance between the solubility of the resultant polymer compound in an organic solvent and the heat resistance thereof, and the like are C₁ to C₁₂ alkoxyphenoxy groups and C₁ to C₁₂ alkylphenoxy groups. As the above-described C₁ to C₁₂ alkoxyphenoxy group, exemplified are a methoxyphenoxy group, ethoxyphenoxy group, n-propyloxyphenoxy group, i-propyloxyphenoxy group, n-butoxyphenoxy group, i-butoxyphenoxy group, t-butoxyphenoxy group, n-pentyloxyphenoxy group, n-hexyloxyphenoxy group, cyclohexyloxyphenoxy group, n-heptyloxyphenoxy group, n-octyloxyphenoxy group, 2-ethylhexyloxyphenoxy group, n-nonyloxyphenoxy group, n-decyloxyphenoxy group, 3,7-dimethyloctyloxyphenoxy group, lauryloxyphenoxy group and the like. As the above-described C₁ to C₁₂ alkylphenoxy group, exemplified are a methylphenoxy group, ethylphenoxy group, dimethylphenoxy group, n-propylphenoxy group, 1,3,5-trimethylphenoxy group, methylethylphenoxy group, i-propylphenoxy group, n-butylphenoxy group, i-butylphenoxy group, t-butylphenoxy group, n-pentylphenoxy group, isoamylphenoxy group, n-hexylphenoxy group, n-heptylphenoxy group, n-octylphenoxy group, n-nonylphenoxy group, n-decylphenoxy group, n-dodecylphenoxy group and the like.

The above-described arylthio group has a carbon number of usually about 3 to 60. The above-described arylthio group includes a phenylthio group, C₁ to C₁₂ alkoxyphenylthio group, C₁ to C₁₂ alkylphenylthio group, 1-naphthylthio group, 2-naphthylthio group, pentafluorophenylthio group and the like, and preferable from the standpoint of a balance between the solubility of the resultant polymer compound in an organic solvent and the heat resistance thereof, and the like are C₁ to C₁₂ alkoxyphenylthio groups and C₁ to C₁₂ alkylphenylthio groups.

The above-described arylalkyl group has a carbon number of usually about 7 to 60, preferably 7 to 48. The above-described arylalkyl group includes phenyl C₁ to C₁₂ alkyl groups, C₁ to C₁₂ alkoxyphenyl C₁ to C₁₂ alkyl groups, C₁ to C₁₂ alkylphenyl C₁ to C₁₂ alkyl groups, 1-naphthyl C₁ to C₁₂ alkyl groups, 2-naphthyl C₁ to C₁₂ alkyl groups and the like, and preferable from the standpoint of a balance between the solubility of the resultant polymer compound in an organic solvent and the heat resistance thereof, and the like are C₁ to C₁₂ alkoxyphenyl C₁ to C₁₂ alkyl groups and C₁ to C₁₂ alkylphenyl C₁ to C₁₂ alkyl groups.

The above-described arylalkoxy group has a carbon number of usually about 7 to 60, preferably 7 to 48. The above-described arylalkoxy group includes phenyl C₁ to C₁₂ alkoxy groups such as a phenylmethoxy group, phenylethoxy group, phenylbutoxy group, phenylpentyloxy group, phenylhexyloxy group, phenylheptyloxy group, phenyloctyloxy group and the like, C₁ to C₁₂ alkoxyphenyl C₁ to C₁₂ alkoxy groups, C₁ to C₁₂ alkylphenyl C₁ to C₁₂ alkoxy groups, 1-naphthyl C₁ to C₁₂ alkoxy groups, 2-naphthyl C₁ to C₁₂ alkoxy groups and the like, and preferable from the standpoint of a balance between the solubility of the resultant polymer compound in an organic solvent and the heat resistance thereof, and the like are C₁ to C₁₂ alkoxyphenyl C₁ to C₁₂ alkoxy groups and C₁ to C₁₂ alkylphenyl C₁ to C₁₂ alkoxy groups.

The above-described arylalkylthio group has a carbon number of usually about 7 to 60, preferably 7 to 48. The above-described arylalkylthio group includes phenyl C₁ to C₁₂ alkylthio groups, C₁ to C₁₂ alkoxyphenyl C₁ to C₁₂ alkylthio groups, C₁ to C₁₂ alkylphenyl C₁ to C₁₂ alkylthio groups, 1-naphthyl C₁ to C₁₂ alkylthio groups, 2-naphthyl C₁ to C₁₂ alkylthio groups and the like, and preferable from the standpoint of a balance between the solubility of the resultant polymer compound in an organic solvent and the heat resistance thereof, and the like are C₁ to C₁₂ alkoxyphenyl C₁ to C₁₂ alkylthio groups and C₁ to C₁₂ alkylphenyl C₁ to C₁₂ alkylthio groups.

The above-described arylalkenyl group has a carbon number of usually about 8 to 60. The above-described arylalkenyl group includes phenyl C₂ to C₁₂ alkenyl groups ("C₂ to C₁₂ alkenyl" means that the alkenyl portion has a carbon number of 2 to 12, being applicable also in the following descriptions), C₁ to C₁₂ alkoxyphenyl C₂ to C₁₂ alkenyl groups, C₁ to C₁₂ alkylphenyl C₂ to C₁₂ alkenyl groups, 1-naphthyl C₂ to C₁₂ alkenyl groups, 2-naphthyl C₂ to C₁₂ alkenyl groups and the like, and preferable from the standpoint of a balance between the solubility of the resultant polymer compound in an organic solvent and the heat resistance thereof, and the like are C₁ to C₁₂ alkoxyphenyl C₂ to C₁₂ alkenyl groups and C₂ to C₁₂ alkylphenyl C₁ to C₁₂ alkenyl groups.

The above-described arylalkynyl group has a carbon number of usually about 8 to 60. The above-described arylalkynyl group includes phenyl C₂ to C₁₂ alkynyl groups ("C₂ to C₁₂ alkynyl" means that the alkynyl portion has a carbon number of 2 to 12, being applicable also in the following descriptions), C₁ to C₁₂ alkoxyphenyl C₂ to C₁₂ alkynyl groups, C₁ to C₁₂ alkylphenyl C₂ to C₁₂ alkynyl groups, 1-naphthyl C₂ to C₁₂ alkynyl groups, 2-naphthyl C₂ to C₁₂ alkynyl groups and the like, and preferable from the standpoint of a balance between the solubility of the resultant polymer compound in an organic solvent and the heat resistance thereof, and the like are C₁ to C₁₂ alkoxyphenyl C₂ to C₁₂ alkynyl groups and C₁ to C₁₂ alkylphenyl C₂ to C₁₂ alkynyl groups.

The above-described substituted amino group includes amino groups obtained by substitution with one or two groups selected from the group consisting of an alkyl group, aryl group, arylalkyl group and mono-valent heterocyclic group, and the definitions and examples of the alkyl group, aryl group, arylalkyl group or mono-valent heterocyclic group are the same as the definitions and examples explained in the section of a substituent when the above-described Z has a substituent (those for the mono-valent heterocyclic group are described later). The above-described substituted amino group has a carbon number of usually about 1 to 200, preferably 2 to 150, more preferably 2 to 100, further preferably 12 to 72. This carbon number does not include the carbon number of the substituent. The above-described substituted amino group includes a methylamino group, dimethylamino group, ethylamino group, diethylamino group, n-propylamino group, di-n-propylamino group, i-propylamino group, diisopropylamino group, di-n-butylamino group, di-i-butylamino group, di-t-butylamino group, di-n-pentylamino group, di-n-hexylamino group, dicyclohexylamino group, di-n-octylamino group, di-2-ethylhexylamino group, di-n-decylamino group, di-3,7-dimethyloctylamino group, dipyrrolidylamino group, dipiperidylamino group, ditrifluoromethylamino group, phenylamino group, diphenylamino group, di-1-naphthylamino group, di-2-naphthylamino group, N-9-anthracenyl-9-anthraceneamine group, di-1-pyrenylamino group, dipyridylamino group, dipyridazinylamino group, dipyrimidinylamino group, dipyrazylamino group, di(triazyl)amino group, N,N-bis(4-phenylphenyl)amino group and the like, and these may be further substituted with an alkyl group (preferably, an alkyl group having 1 to 20 carbon atoms, more preferably, an alkyl group having 1 to 8 carbon atoms, including, for example, a methyl group, n-butyl group, t-butyl group, n-octyl group and the like), alkoxy group, aryl group, alkyloxycarbonyl group, acyl group, N,N-dialkylamino group, N,N-diarylamino group, cyano group, nitro group, chlorine atom, fluorine atom or the like.

The above-described substituted silyl group includes silyl groups obtained by substitution with one, two or three groups selected from the group consisting of an alkyl group, aryl group, arylalkyl group and mono-valent heterocyclic group. The above-described substituted silyl group has a carbon number of usually about 1 to 60, preferably 3 to 48. The alkyl group, aryl group, arylalkyl group and mono-valent heterocyclic group may have a substituent. As the above-described substituted silyl group, exemplified are a trimethylsilyl group, triethylsilyl group, tri-n-propylsilyl group, tri-i-propylsilyl group, dimethyl-i-propylsilyl group, diethyl-i-propylsilyl group, t-butyldimethylsilyl group, n-pentyldimethylsilyl group, n-hexyldimethylsilyl group, n-heptyldimethylsilyl group, n-octyldimethylsilyl group, 2-ethylhexyldimethylsilyl group, n-nonyldimethylsilyl group, n-decyldimethylsilyl group, 3,7-dimethyloctyldimethylsilyl group, lauryldimethylsilyl group, phenyl C₁ to C₁₂ alkylsilyl groups, C₁ to C₁₂ alkoxyphenyl C₁ to C₁₂ alkylsilyl groups, C₁ to C₁₂ alkylphenyl C₁ to C₁₂ alkylsilyl groups, 1-naphthyl C₁ to C₁₂ alkylsilyl groups, 2-naphthyl C₁ to C₁₂ alkylsilyl groups, phenyl C₁ to C₁₂ alkyldimethylsilyl groups, triphenylsilyl group, tri-p-xylylsilyl group, tribenzylsilyl group, diphenylmethylsilyl group, t-butyldiphenylsilyl group, dimethylphenylsilyl group and the like.

As the above-described halogen atom, exemplified are a fluorine atom, chlorine atom, bromine atom and iodine atom.

The above-described acyl group has a carbon number of usually about 2 to 20, preferably 2 to 18. The above-described acyl group includes an acetyl group, propionyl group, butyryl group, isobutyryl group, pivaloyl group, benzoyl group, trifluoroacetyl group, pentafluorobenzoyl group and the like.

The above-described acyloxy group has a carbon number of usually about 2 to 20, preferably 2 to 18. The above-described acyloxy group includes an acetoxy group, propionyloxy group, butyryloxy group, isobutyryloxy group, pivaloyloxy group, benzoyloxy group, trifluoroacetyloxy group, pentafluorobenzoyloxy group and the like.

The above-described imine residue has a carbon number of about 2 to 20, preferably 2 to 18. The above-described imine residue includes groups of the following structural formulae, and the like. 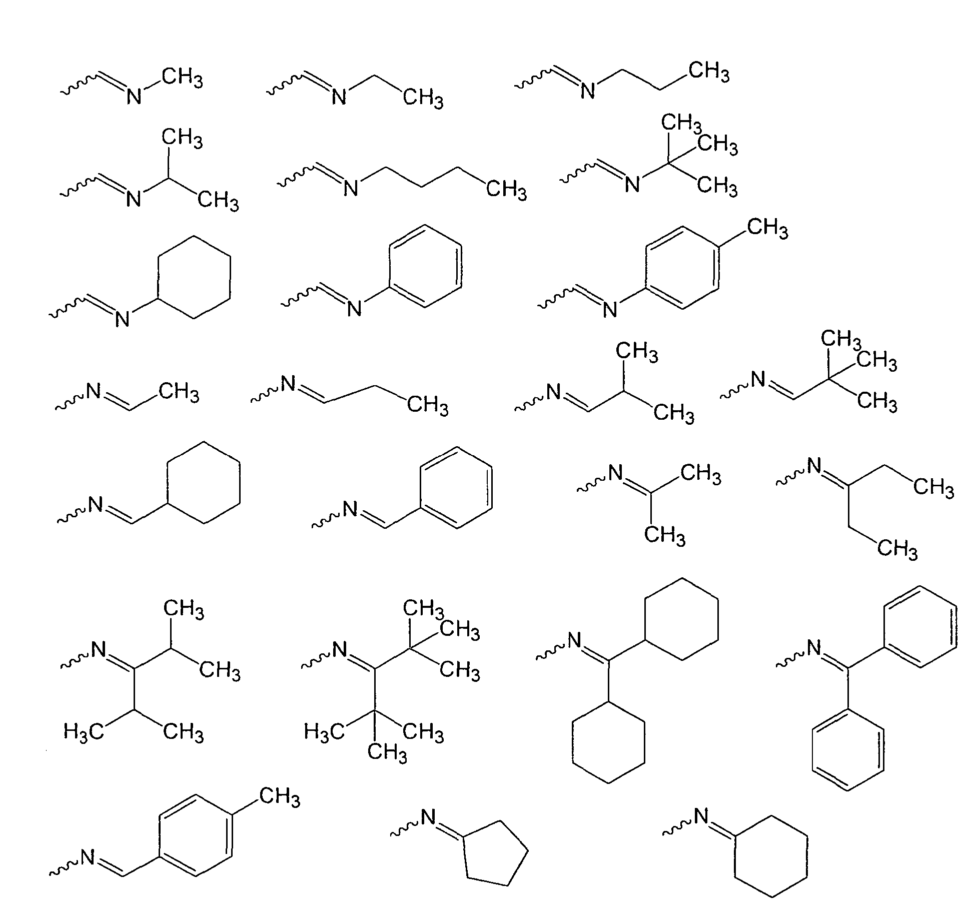 (in the formulae, the wavy line represents a free bond, and means a possibility of a geometric isomer such as a cis body, trans body or the like depending on the kind of the imine residue.).

The above-described amide group has a carbon number of usually about 2 to 20, preferably 2 to 18. The above-described amide group includes a formamide group, acetamide group, propioamide group, butyroamide group, benzamide group, trifluoroacetamide group, pentafluorobenzamide group, diformamide group, diacetamide group, dipropioamide group, dibutyroamide group, dibenzamide group, ditrifluoroacetamide group, dipentafluorobenzamide group and the like.

The above-described acid imide group means a residue obtained by removing from an acid imide a hydrogen atom connected to its nitrogen atom, and has a carbon number of about 4 to 20, and the following groups and the like are exemplified.

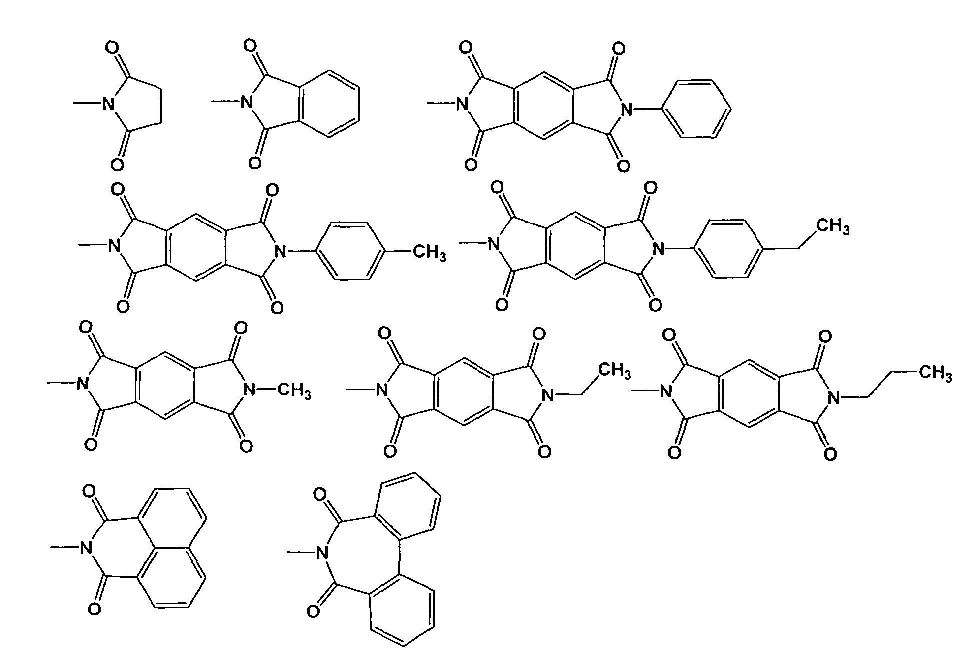

The above-described mono-valent heterocyclic group has a carbon number of usually about 4 to 60, preferably 4 to 20. The carbon number of the mono-valent heterocyclic group does not include the carbon number of the substituent. As the above-described mono-valent heterocyclic group, exemplified are a thienyl group, pyrrolyl group, furyl group, pyridyl group, piperidyl group, quinolyl group, isoquinolyl group, pyrimidyl group, triazinyl group and the like, and preferable are a thienyl group, pyridyl group, quinolyl group, isoquinolyl group, pyrimidyl group and triazinyl group, more preferable are a thienyl group, pyridyl group, pyrimidyl group and triazinyl group. The above-described mono-valent heterocyclic group may further have a substituent such as an alkyl group, alkoxy group and the like.

The above-described substituted carboxyl group includes carboxyl groups substituted with an alkyl group, aryl group, arylalkyl group or mono-valent heterocyclic group, and the carbon number thereof is usually about 2 to 60, preferably 2 to 48. The above-described substituted carboxyl group includes a methoxycarbonyl group, ethoxycarbonyl group, n-propoxycarbonyl group, i-propoxycarbonyl group, n-butoxycarbonyl group, i-butoxycarbonyl group, t-butoxycarbonyl group, n-pentyloxycarbonyl group, n-hexyloxycarbonyl group, cyclohexyloxycarbonyl group, n-heptyloxycarbonyl group, n-octyloxycarbonyl group, 2-ethylhexyloxycarbonyl group, n-nonyloxycarbonyl group, n-decyloxycarbonyl group, 3,7-dimethyloctyloxycarbonyl group, n-dodecyloxycarbonyl group, trifluoromethoxycarbonyl group, pentafluoroethoxycarbonyl group, perfluorobutoxycarbonyl group, perfluorohexyloxycarbonyl group, perfluorooctyloxycarbonyl group, phenoxycarbonyl group, naphthoxycarbonyl group, pyridyloxycarbonyl group and the like The alkyl group, aryl group, arylalkyl group or mono-valent heterocyclic group may have a substituent. The carbon number of the substituted carboxyl group does not include the carbon number of the substituent.

In the above-described formula (1), Ar¹ represents an unsubstituted or substituted arylene group or an unsubstituted or substituted di-valent heterocyclic group, and preferably an unsubstituted or substituted arylene group, more preferably an unsubstituted or substituted phenylene group. It is preferable that at least one of a plurality of Ar¹s is an unsubstituted or substituted arylene group.

The above-described arylene group means an atomic group obtained by removing two hydrogen atoms from an aromatic hydrocarbon, and includes those having an independent benzene ring or condensed ring. The above-described arylene group has a carbon number of usually about 6 to 60, preferably 6 to 48, more preferably 6 to 30, further preferably 6 to 18, especially preferably 6 to 10, particularly preferably 6. This carbon number does not include the carbon number of the substituent. The above-described arylene group includes a 1,4-phenylene group, 1,3-phenylene group, 1,2-phenylene group, 1,4-naphthalenediyl group, 1,5-naphthalenediyl group, 2,6-naphthalenediyl group, 1,4-anthracenediyl group, 1,5-anthracenediyl group, 2,6-anthracenediyl group, 9,10-anthracenediyl group, 2,7-phenanthrenediyl group, 1,7-naphthacenediyl group, 2,8-naphthacenediyl group, 2,7-fluorenediyl group and the like, preferably a 1,4-phenylene group, 1,3-phenylene group, 1,2-phenylene group, 1,4-naphthalenediyl group, 2,6-naphthalenediyl group, 1,4-anthranediyl group, 1,5-anthranediyl group, 2,6-anthranediyl group, 9,10-anthranediyl group, more preferably a 1,4-phenylene group, 1,3-phenylene group, 1,2-phenylene group, 1, 4-naphthalenediyl group, 2,6-naphthalenediyl group, further preferably a 1,4-phenylene group, 1,3-phenylene group, 1, 4-naphthalenediyl group, particularly preferably a 1,4-phenylene group.

The above-described di-valent heterocyclic group has a carbon number of usually about 4 to 60, preferably 4 to 20, more preferably 4 to 9, further preferably 4 to 5. The above-described di-valent heterocyclic group includes a 2,5-thiophenediyl group, N-methyl-2,5-pyrrolediyl group, 2,5-furandiyl group, 2,5-pyridinediyl group, 2,6-pyridinediyl group, 2,4-quinolinediyl group, 2,6-quinolinediyl group, 1,4-isoquinolinediyl group, 1,5-isoquinolinediyl group, 5,8-quinoxalinediyl group and the like, preferably a 2,5-thiophenediyl group, 2,5-pyridinediyl group, 2,6-pyridinediyl group, 2,4-quinolinediyl group, 2,6-quinolinediyl group, 1,4-isoquinolinediyl group, 1,5-isoquinolinediyl group, more preferably a 2,5-pyridinediyl group, 2,6-pyridinediyl group, 1,4-isoquinolinediyl group, further preferably a 2,5-pyridinediyl group, 2,6-pyridinediyl group.

When Ar¹ has a substituent, examples thereof include preferably those selected from the group consisting of an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group and cyano group, more preferably those selected from the group consisting of an alkyl group, alkoxy group, aryl group, aryloxy group, arylalkyl group, arylalkoxy group, substituted amino group, substituted silyl group, acyl group, substituted carboxyl group and cyano group, further preferably those selected from the group consisting of an alkyl group, alkoxy group, aryl group, aryloxy group, arylalkyl group, arylalkoxy group and substituted carboxyl group, especially preferably those selected from the group consisting of an alkyl group, alkoxy group and aryl group, and particularly preferably an alkyl group.

The definitions and examples of the above-described alkyl group, alkoxy group, alkylthio group, aryl group, aryloxy group, arylthio group, arylalkyl group, arylalkoxy group, arylalkylthio group, arylalkenyl group, arylalkynyl group, substituted amino group, substituted silyl group, halogen atom, acyl group, acyloxy group, imine residue, amide group, acid imide group, mono-valent heterocyclic group and substituted carboxyl group are the same as the definitions and examples explained in the section of a substituent when the above-described Z has a substituent.

In the above-described formula (1), Ar² represents an unsubstituted or substituted aryl group or an unsubstituted or substituted mono-valent heterocyclic group, and preferably an unsubstituted or substituted aryl group. It is preferable that at least one of a plurality of Ar²s is an unsubstituted or substituted aryl group.

The above-described aryl group means an atomic group obtained by removing one hydrogen atom from an aromatic hydrocarbon, and includes those having an independent benzene ring or condensed ring. The above-described aryl group has a carbon number of usually about 6 to 60, preferably 6 to 48, more preferably 6 to 30, further preferably 6 to 18, especially preferably 6 to 10, particularly preferably 6. This carbon number does not include the carbon number of the substituent. The above-described aryl group includes a phenyl group, 1-naphthyl group, 2-naphthyl group, 1-anthracenyl group, 2-anthracenyl group, 9-anthracenyl group, 1-phenanthryl group, 9-phenanthryl group, 1-naphthacenyl group, 5-naphthacenyl group, 1-pyrenyl group, 3-perylenyl group, 2-fluorenyl group and the like, preferably a phenyl group, 1-naphthyl group, 2-naphthyl group, 1-anthracenyl group, 2-anthracenyl group, 9-anthracenyl group, 2-fluorenyl group, more preferably a phenyl group, 1-naphthyl group, 2-naphthyl group, further preferably a phenyl group.

The above-described mono-valent heterocyclic group has a carbon number of usually about 4 to 60, preferably 4 to 20, more preferably 4 to 9, further preferably 4 to 5. The carbon number of the mono-valent heterocyclic group does not include the carbon number of the substituent. The above-described mono-valent heterocyclic group includes a 2-thienyl group, 3-thienyl group, 2-pyrrolyl group, 3-pyrrolyl group, N-methyl-2-pyrrolyl group, 2-furyl group, 3-furyl group, 2-pyridyl group, 3-pyridyl group, 4-pyridyl group, 2-quinolyl group, 4-quinolyl group, 5-quinolyl group, 1-isoquinolyl group, 3-isoquinolyl group, 6-isoquinolyl group, 5-quinoxalyl group and the like, preferably a 2-thienyl group, 3-thienyl group, 2-pyridyl group, 3-pyridyl group, 4-pyridyl group, 2-quinolyl group, 4-quinolyl group, 5-quinolyl group, 1-isoquinolyl group, 3-isoquinolyl group, 6-isoquinolyl group, more preferably a 2-thienyl group, 3-thienyl group, 2-pyridyl group, 3-pyridyl group, 4-pyridyl group, further preferably a 2-pyridyl group, 3-pyridyl group, 4-pyridyl group.

When Ar² has a substituent, examples thereof include preferably those selected from the group consisting of an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group and cyano group, more preferably those selected from the group consisting of an alkyl group, alkoxy group, aryl group, aryloxy group, arylalkyl group, arylalkoxy group, substituted amino group, substituted silyl group, acyl group, substituted carboxyl group and cyano group, further preferably those selected from the group consisting of an alkyl group, alkoxy group, aryl group, aryloxy group, arylalkyl group, arylalkoxy group and substituted carboxyl group, especially preferably those selected from the group consisting of an alkyl group, alkoxy group, aryl group, particularly preferably an alkyl group.

The definitions and examples of the above-described alkyl group, alkoxy group, alkylthio group, aryl group, aryloxy group, arylthio group, arylalkyl group, arylalkoxy group, arylalkylthio group, arylalkenyl group, arylalkynyl group, substituted amino group, substituted silyl group, halogen atom, acyl group, acyloxy group, imine residue, amide group, acid imide group, mono-valent heterocyclic group and substituted carboxyl group are the same as the definitions and examples explained in the section of a substituent when the above-described Z has a substituent

In the above-described formula (1), x3, y1, y2, z1, z2 and z3 represent each independently 0 or 1. y3 represents an integer of 0 to 2. Here, y1+z1 and y2+z2 are 1, and x3+y3+z3 is 2. In the above-described formula (1), it is preferable that x3, y1, y2 and y3 are 1, and z1, z2 and z3 are 0. When there exist a plurality of x3's, y3's and z3's, these may be the same or different.

In the above-described formula (1), u represents an integer of 0 to 8, preferably an integer of 0 to 4, more preferably an integer of 0 to 2, further preferably 0 or 2, particularly preferably 0. When u is 0, Z has two bonds.

In the above-described formula (1), when x3 is 0, the nitrogen atom directly connected to Ar² appended with a suffix y3 and/or Ar¹ appended with a suffix z3 has three bonds connecting to the Ar², Ar¹ and Z.

In the above-described formula (1), when z1 is 0, the nitrogen atom directly connected to Ar² appended with a suffix y1 has three bonds connecting to the Ar², the Ar¹ and Z.

In the above-described formula (1), when z2 is 0, the nitrogen atom directly connected to Ar² appended with a suffix y2 has three bonds connecting to the Ar², the Ar¹ and Z.

In the above-described formula (1), when z3 is 0, the nitrogen atom directly connected to Ar² appended with a suffix y3 has three bonds connecting to the Ar², the Ar¹ and Z.

Each of the above-described constitutional units of the formula (1) has 2 to (4+2u) free bonds for connecting to adjacent constitutional units or repeating units.

The above-described constitutional units of the formula (1) include the following units (A-1) to (A-15), (B-1) to (B-23), (C-1) to (C-24), (D-1) to (D-12), (E-1) to (E-18), (F-1) to (F-9), (G-1) to (G-9), (H-1) to (H-9), (I-1) to (I-9), (J-1) to (J-3), (K-1) to (K-3), (L-1) to (L-3), (M-1) to (M-21), (N-1) to (N-3), (P-1) to (P-3), (Q-1) to (Q-3), (R-1), (S-1); and these following units having a substituent such as an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group, cyano group and the like; etc. In the following units (A-1) to (A-15), (B-1) to (B-23), (C-1) to (C-24), (D-1) to (D-12), (E-1) to (E-18), (F-1) to (F-9), (G-1) to (G-9), (H-1) to (H-9), (I-1) to (I-9), (J-1) to (J-3), (K-1) to (K-3), (L-1) to (L-3), (M-1) to (M-21), (N-1) to (N-3), (P-1) to (P-3), (Q-1) to (Q-3), (R-1), (S-1), the free bond in an aromatic ring may take any position.

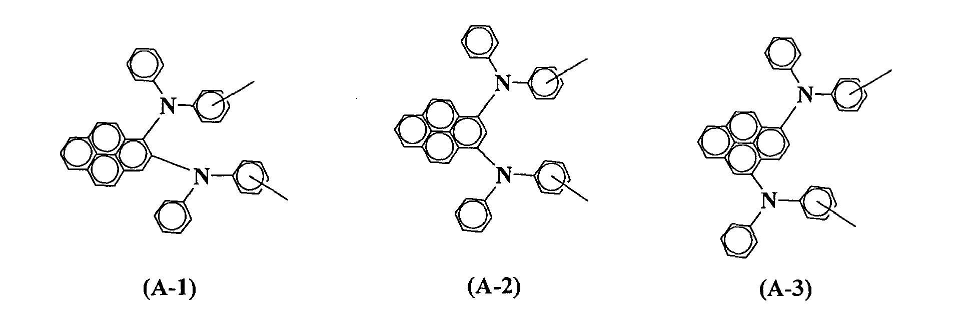

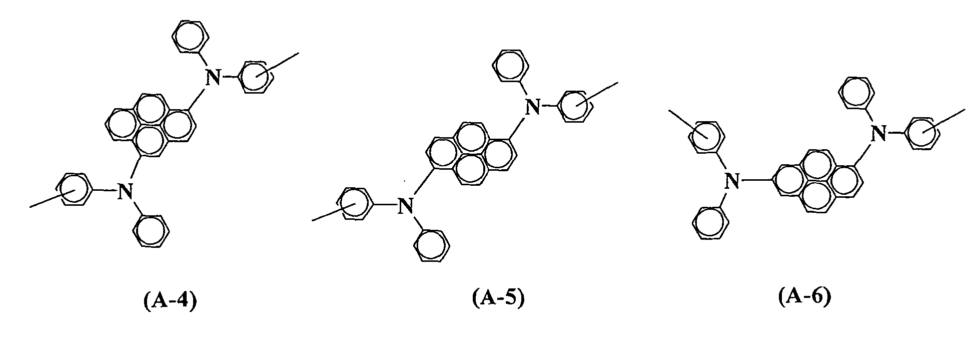

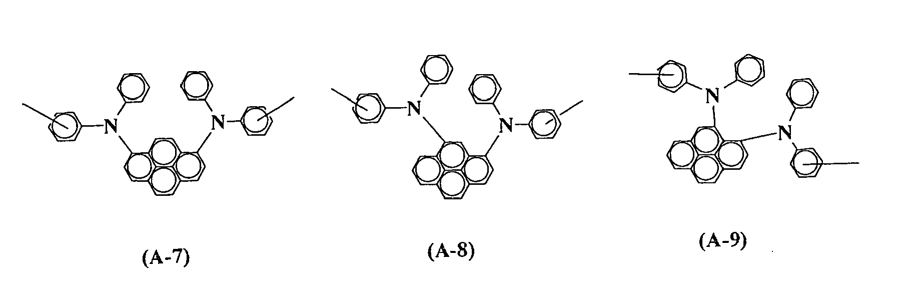

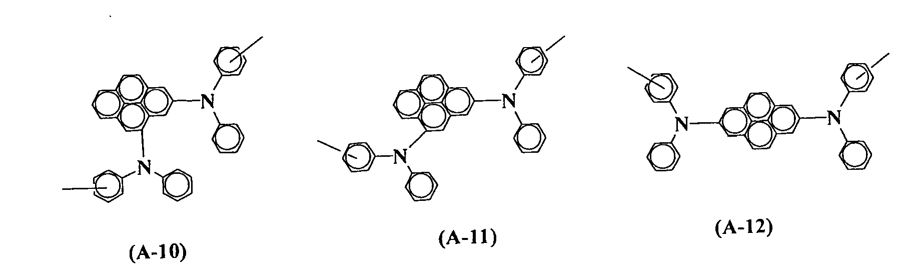

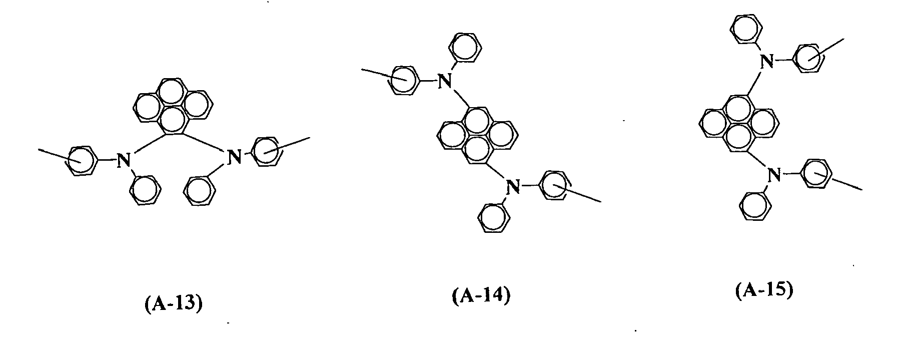

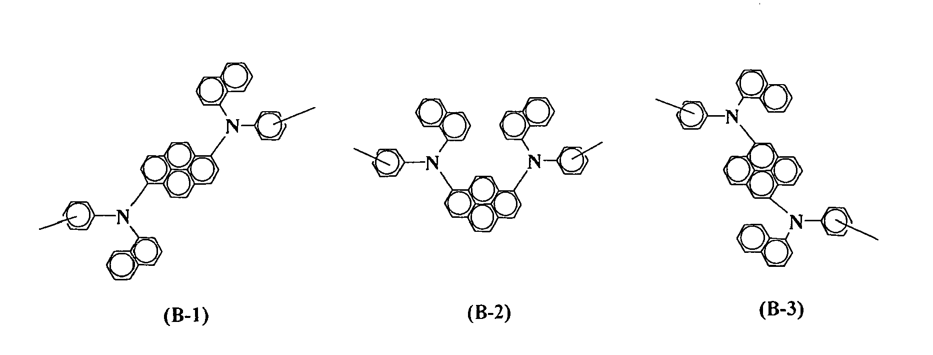

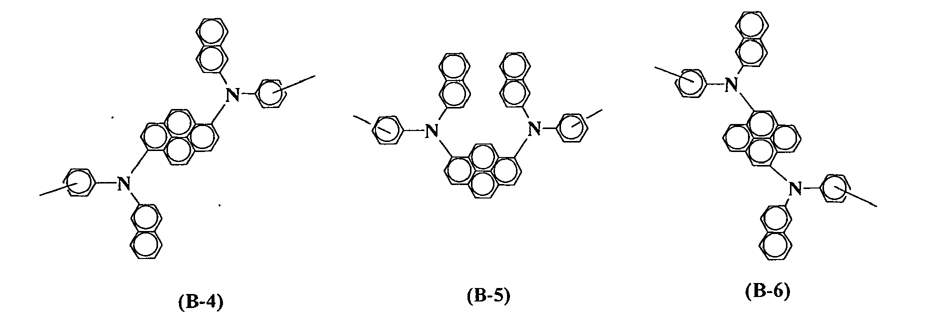

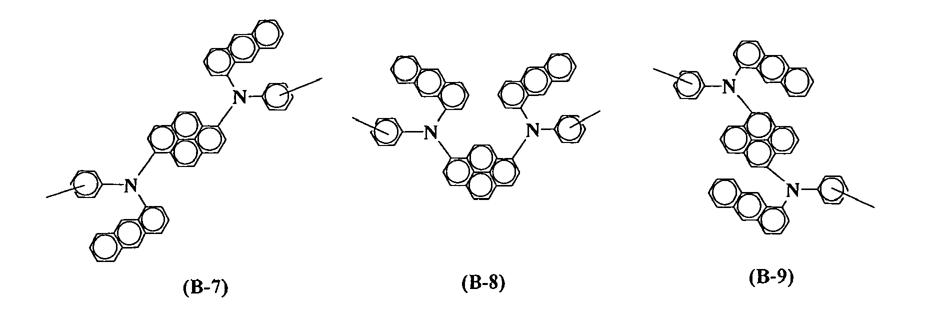

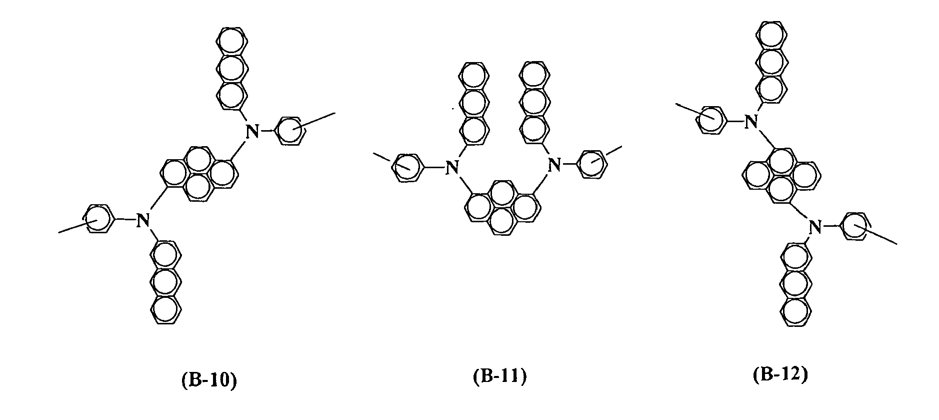

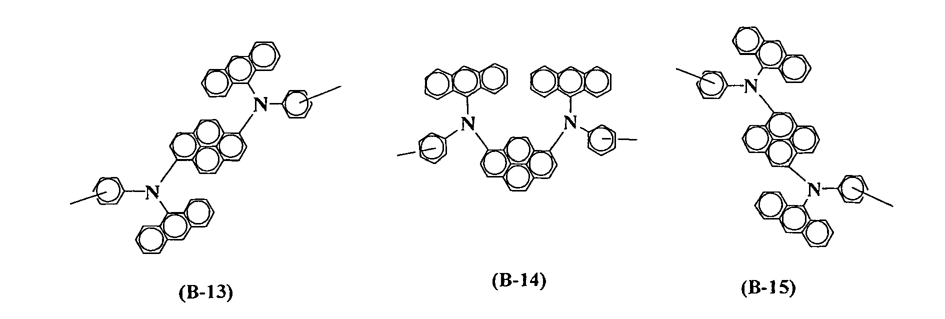

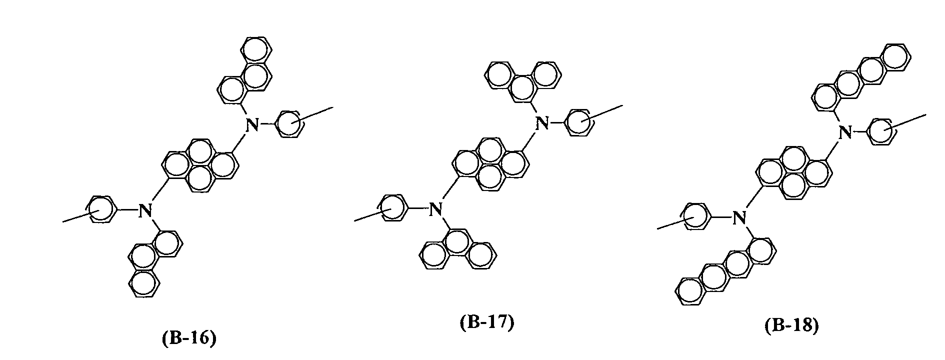

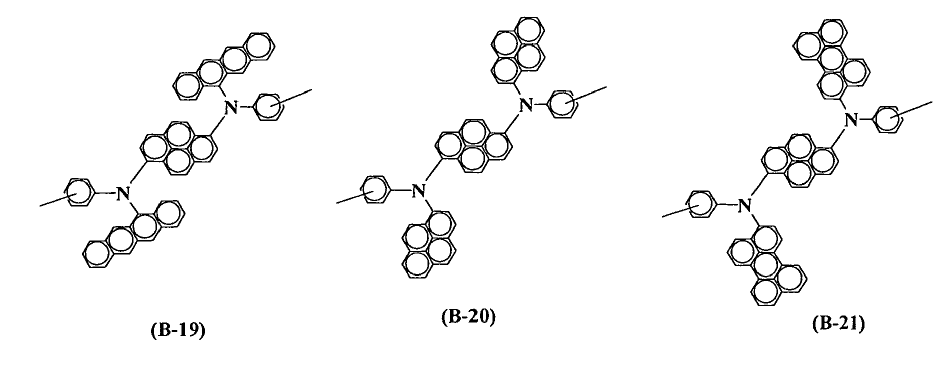

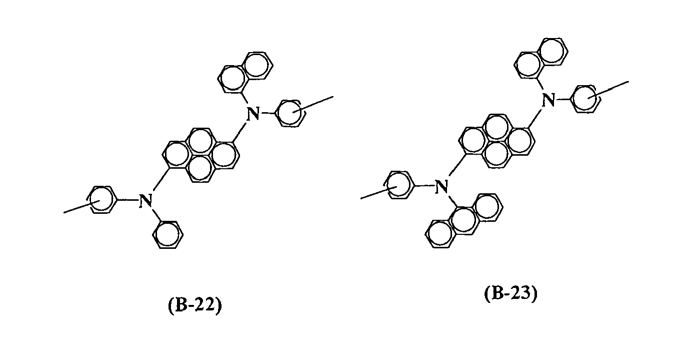

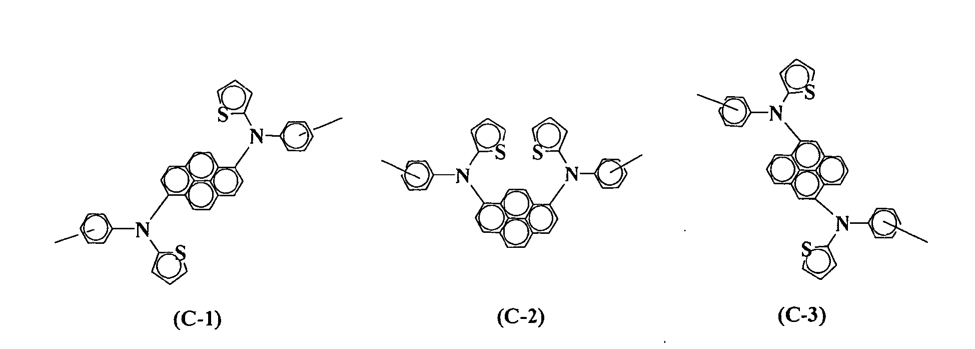

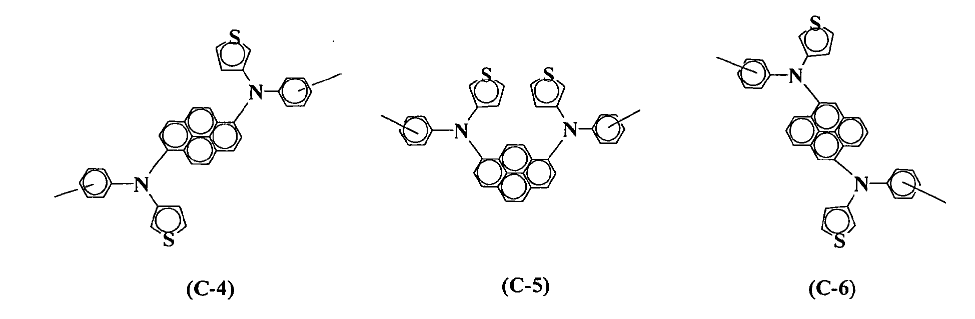

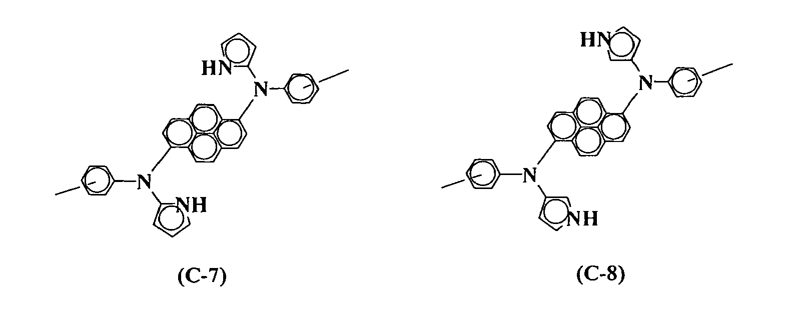

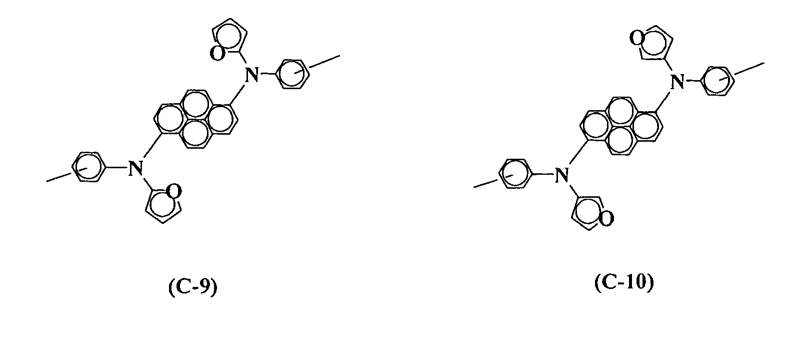

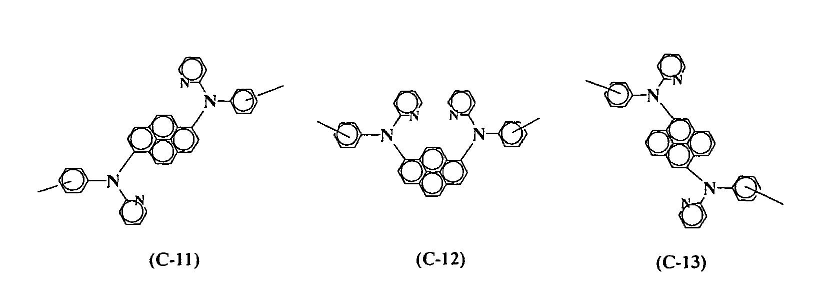

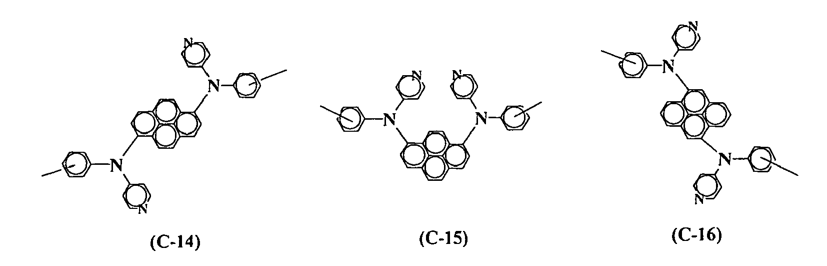

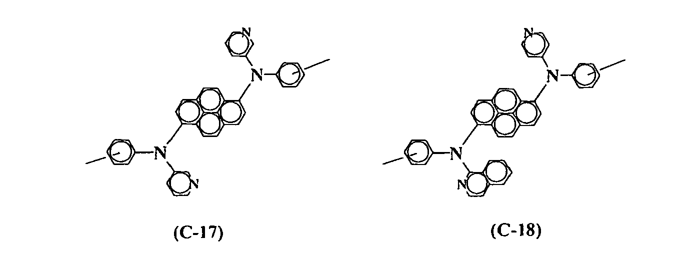

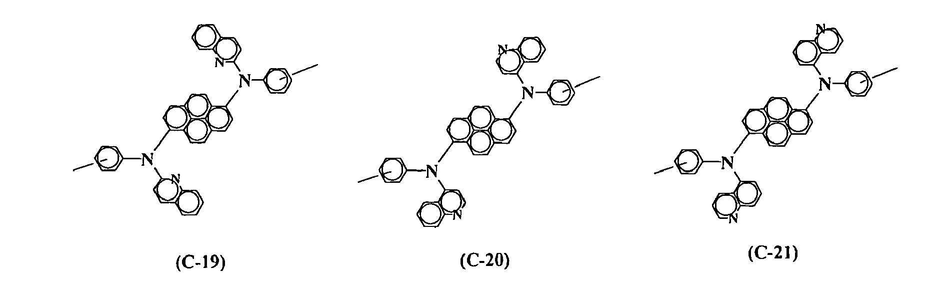

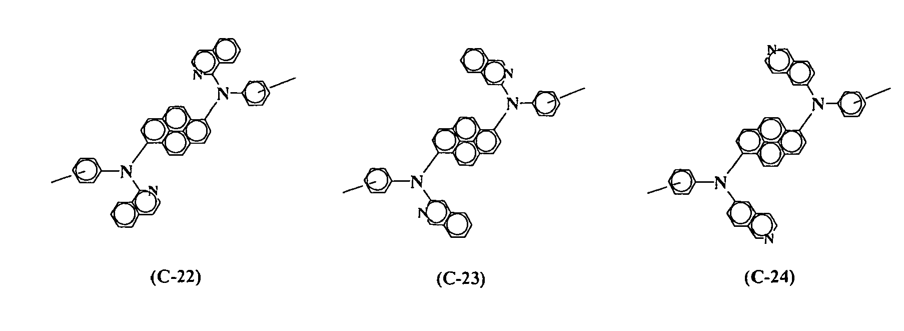

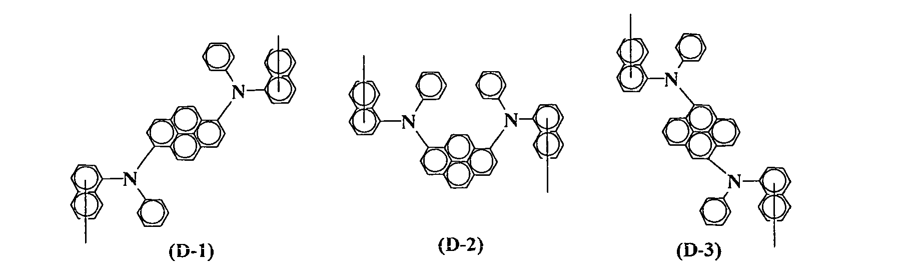

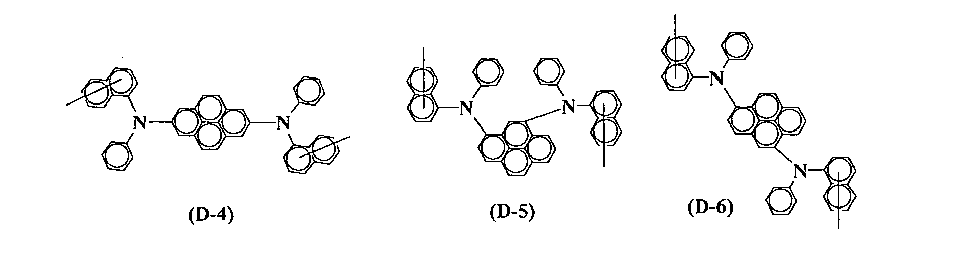

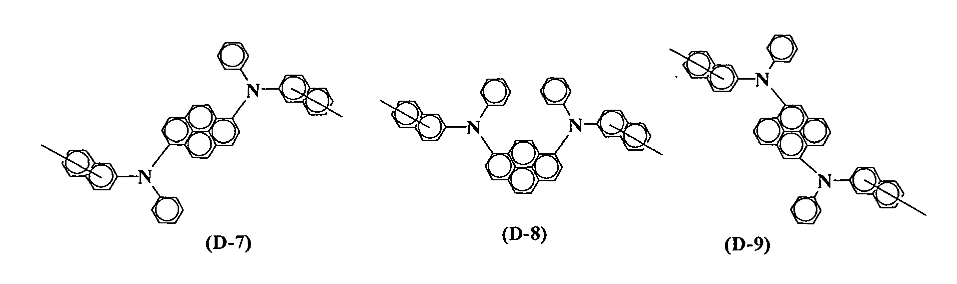

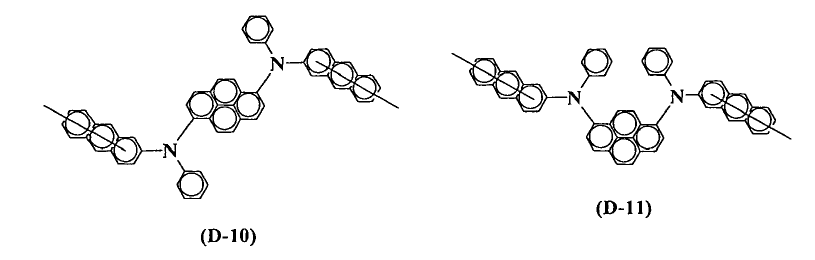

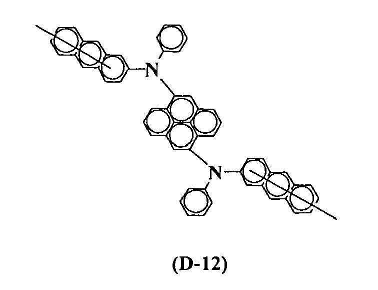

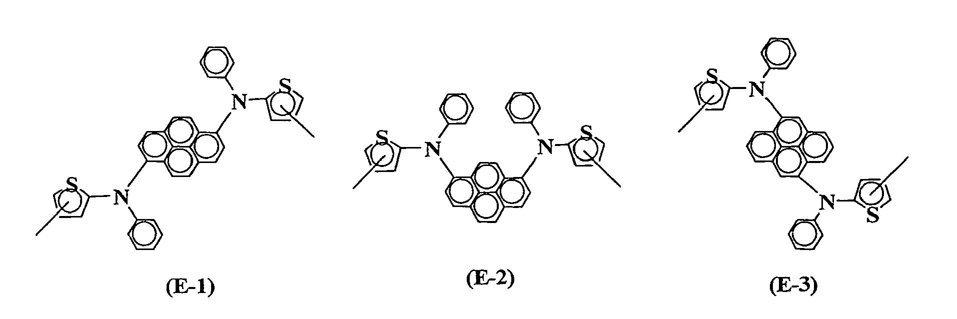

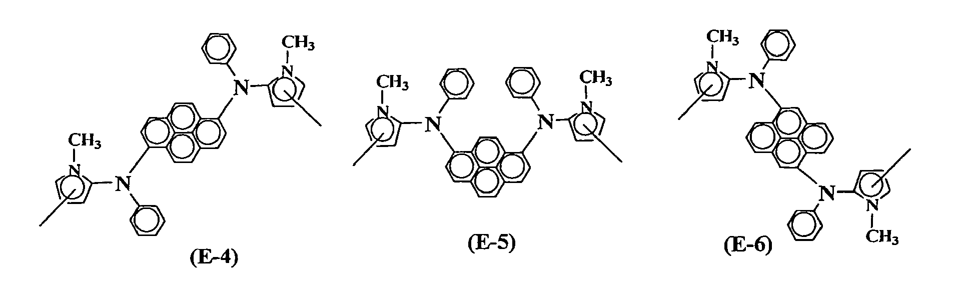

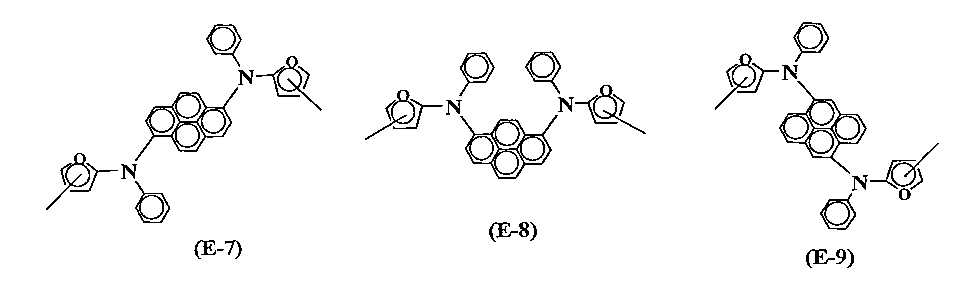

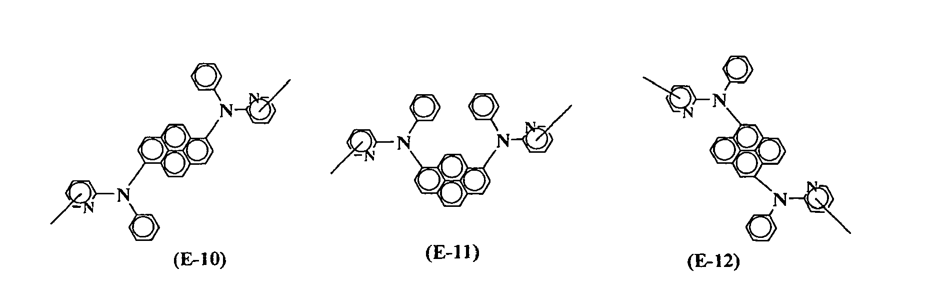

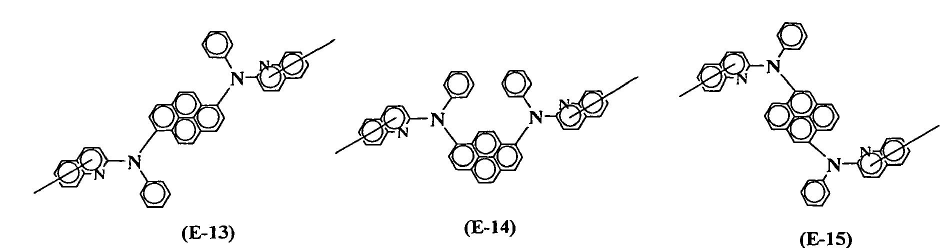

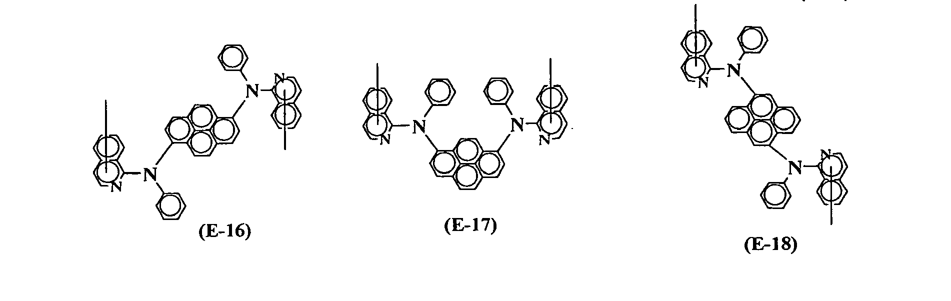

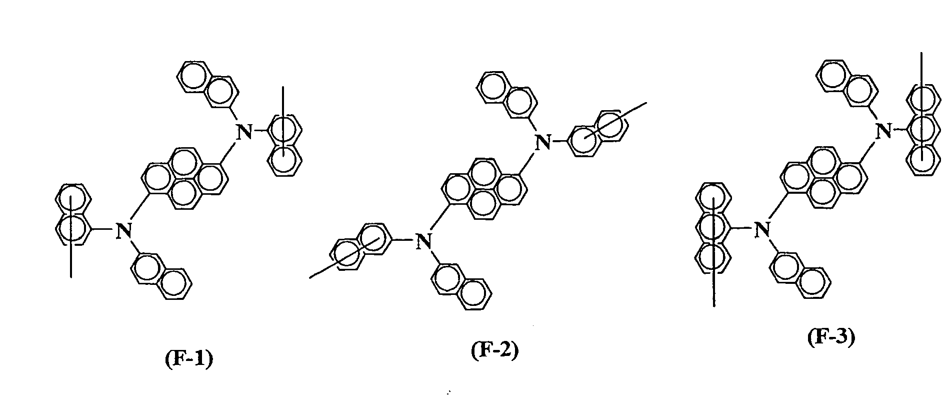

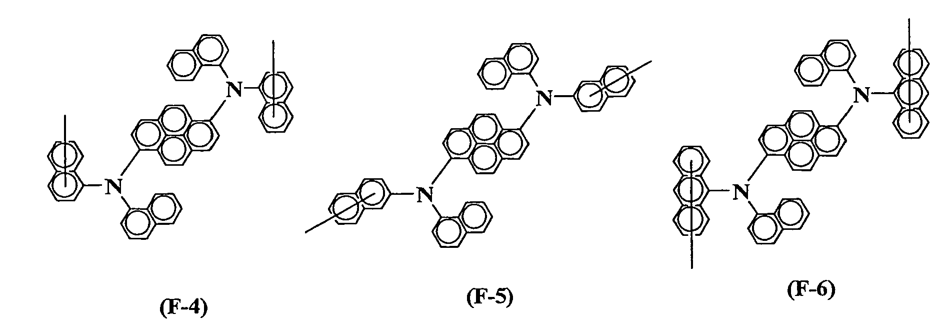

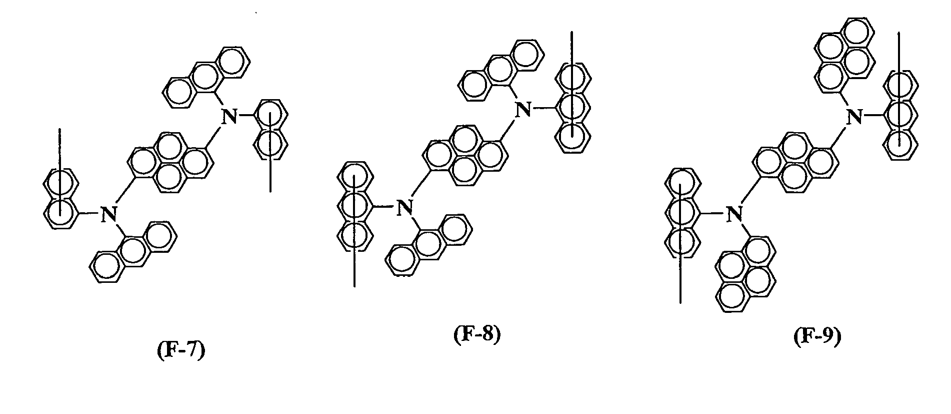

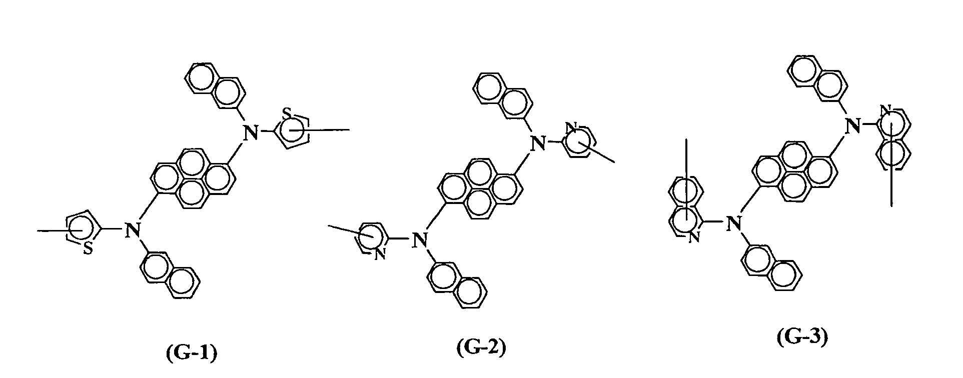

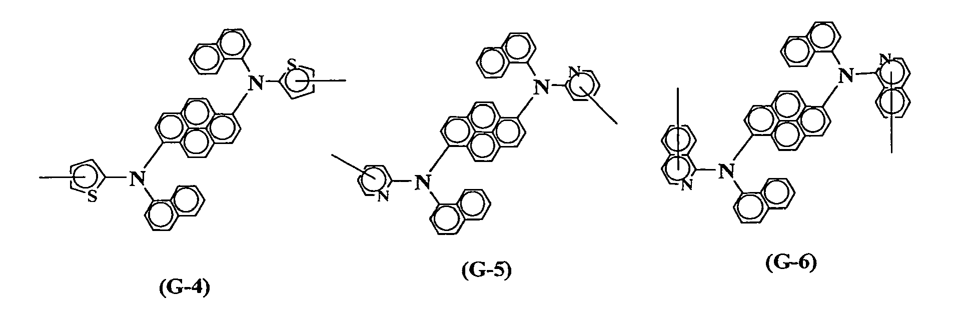

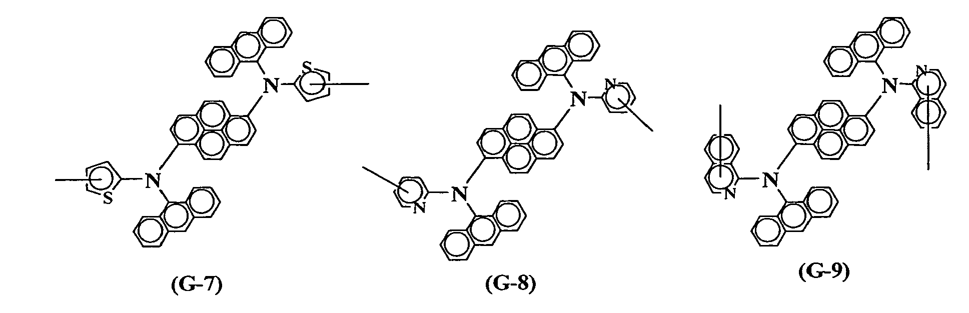

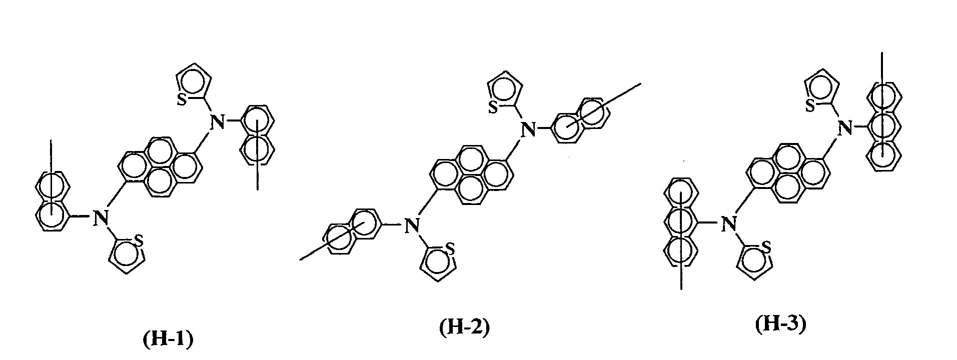

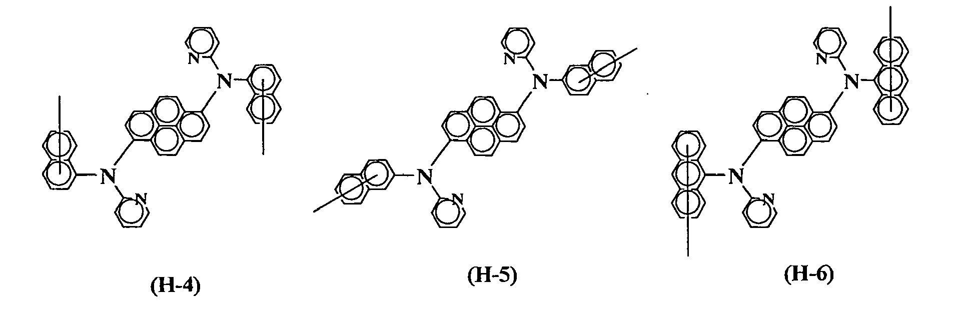

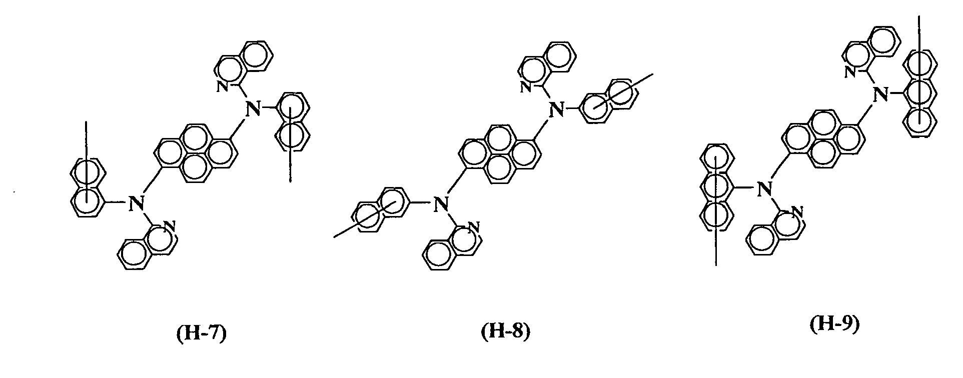

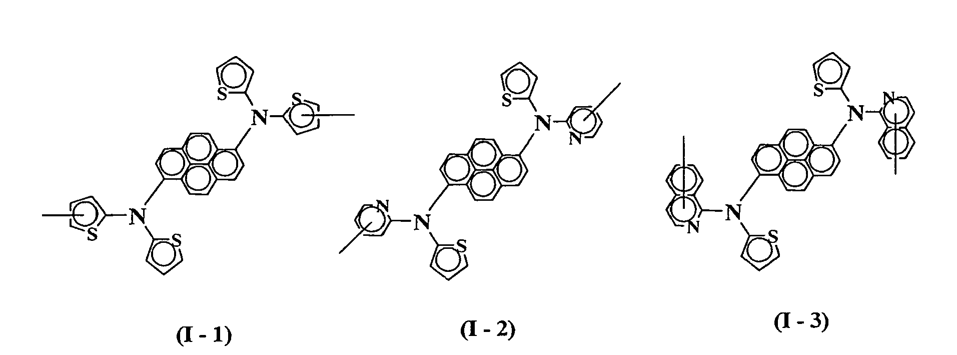

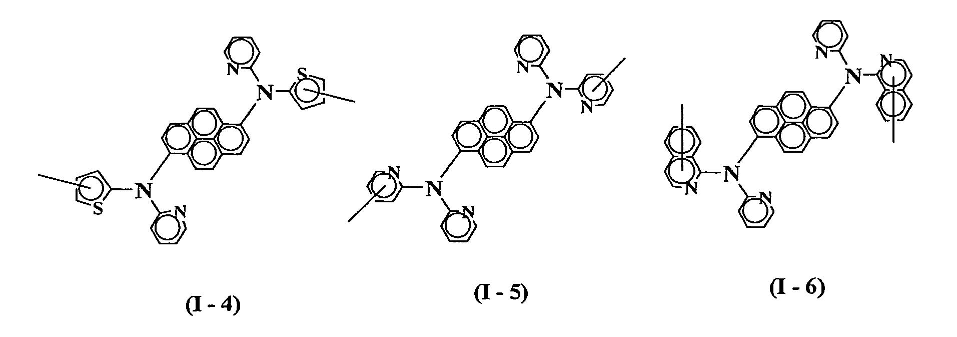

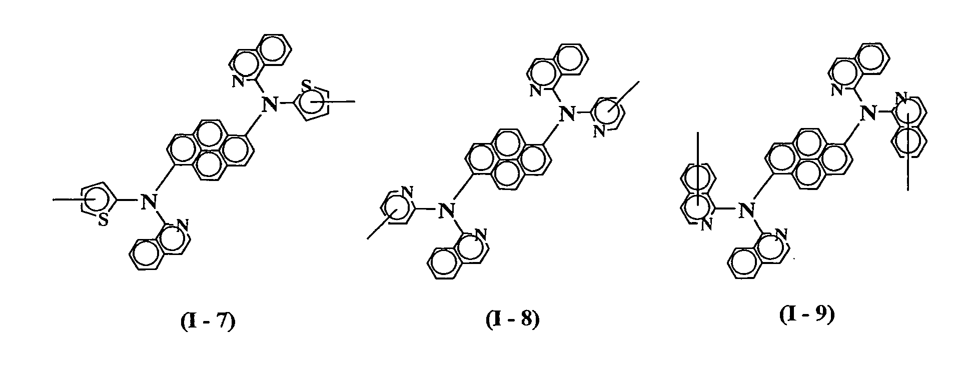

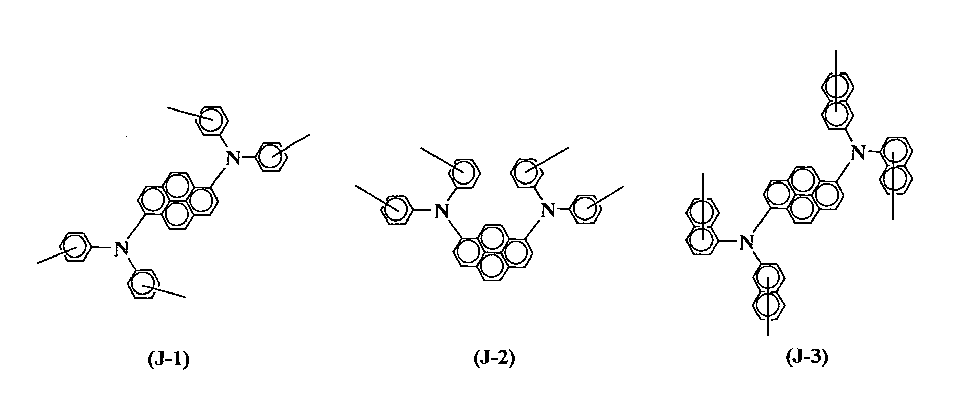

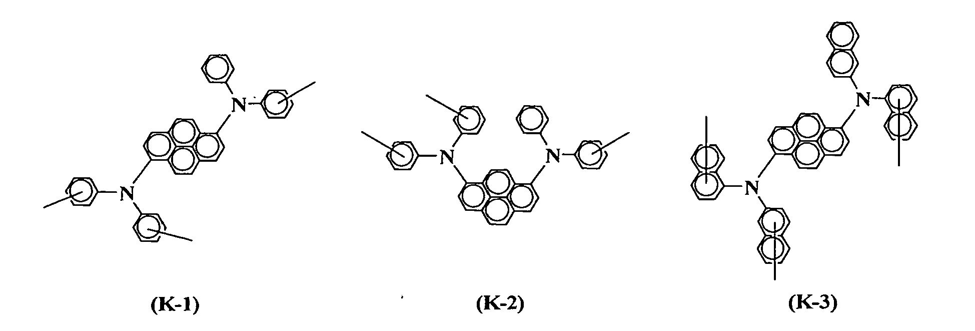

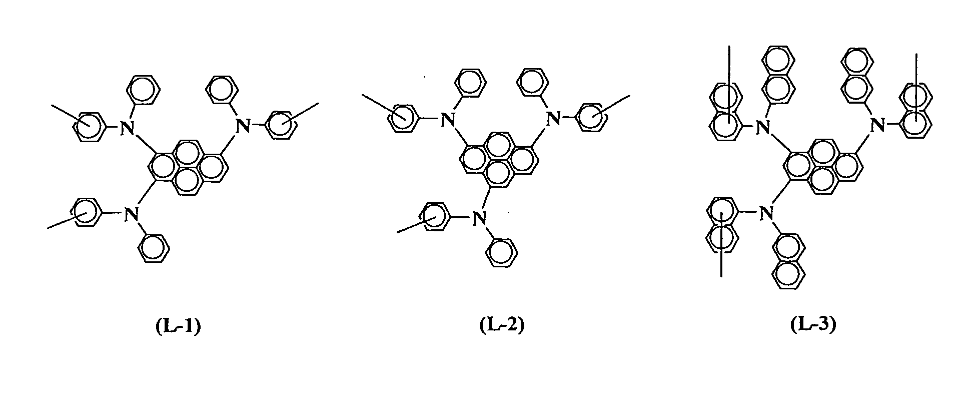

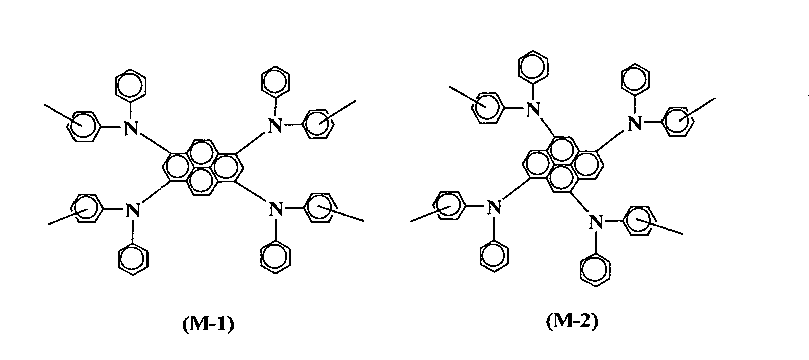

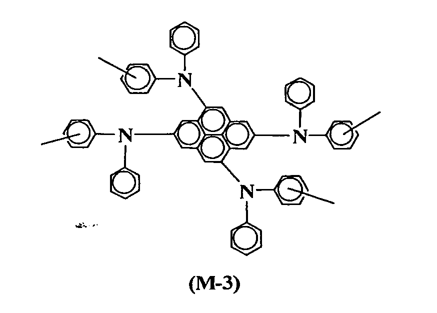

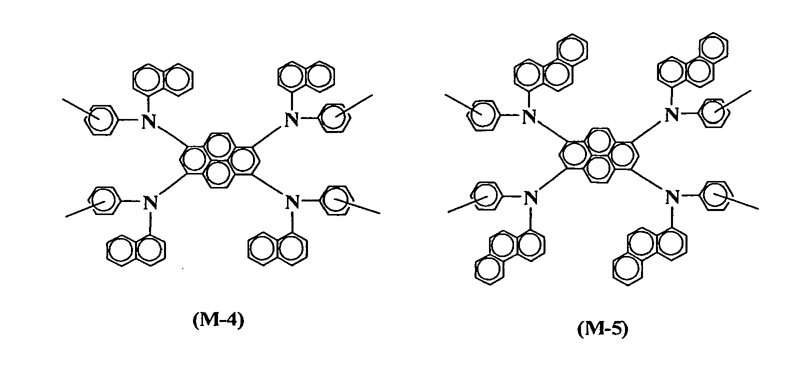

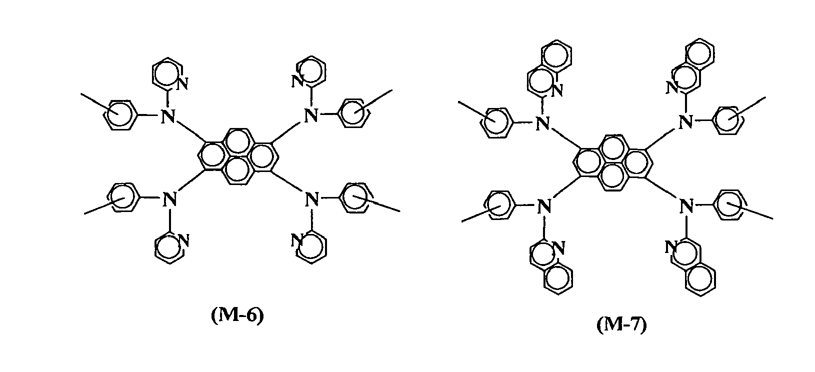

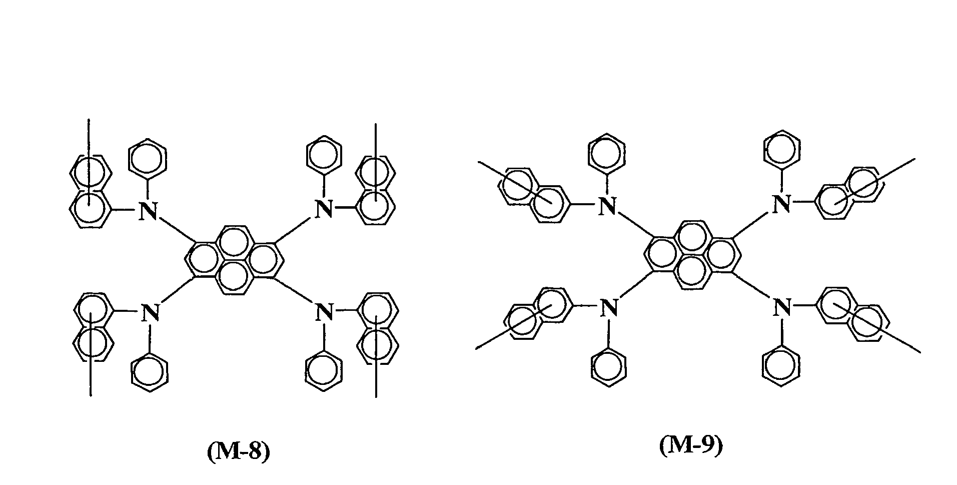

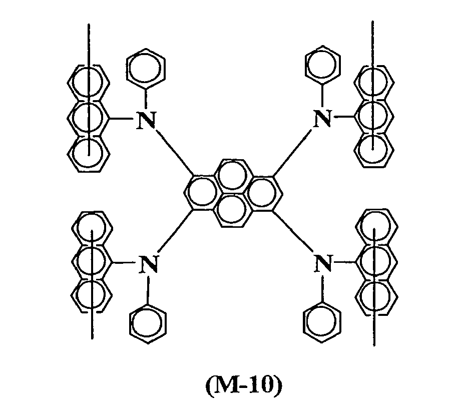

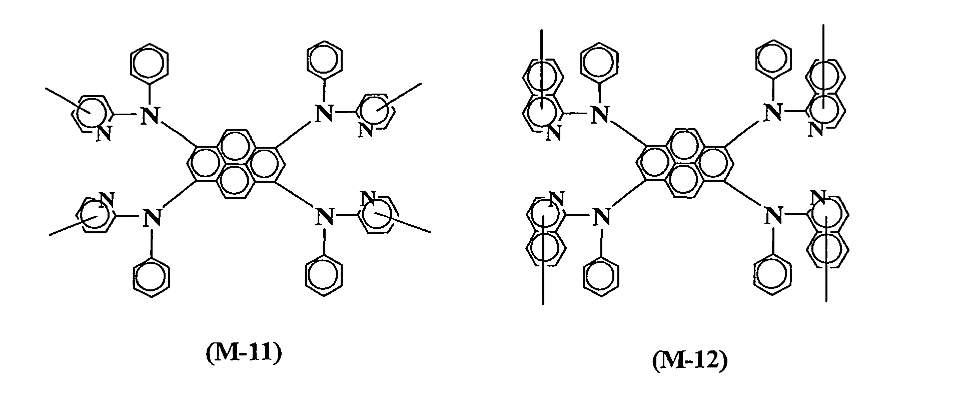

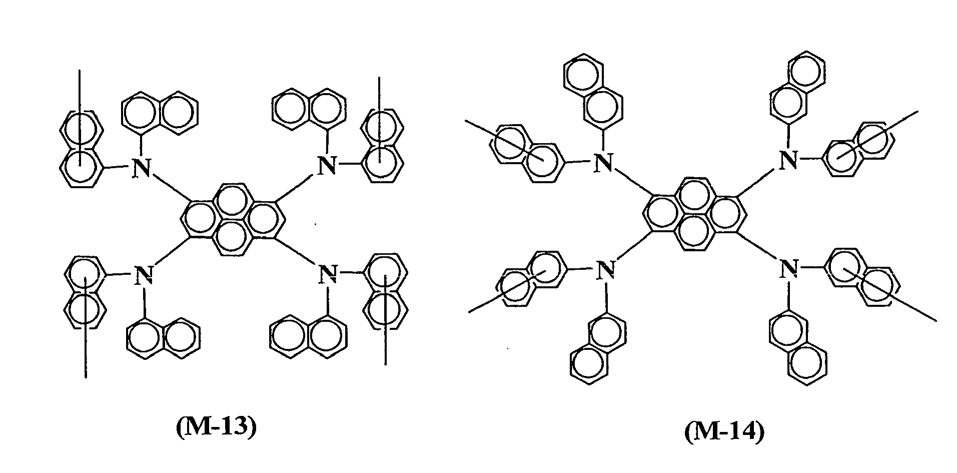

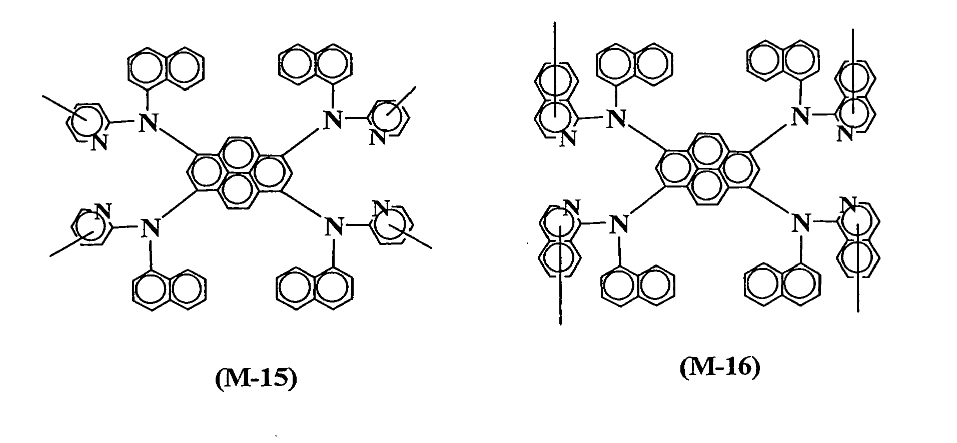

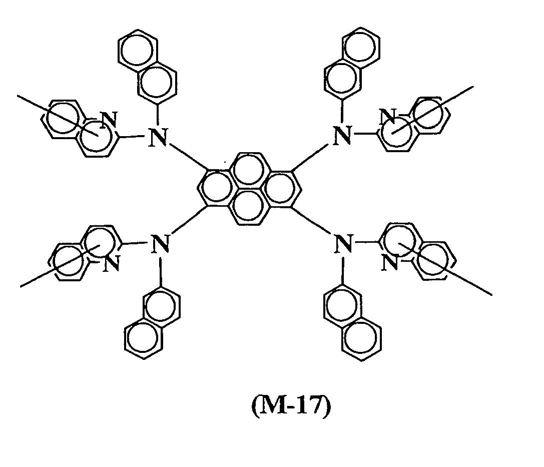

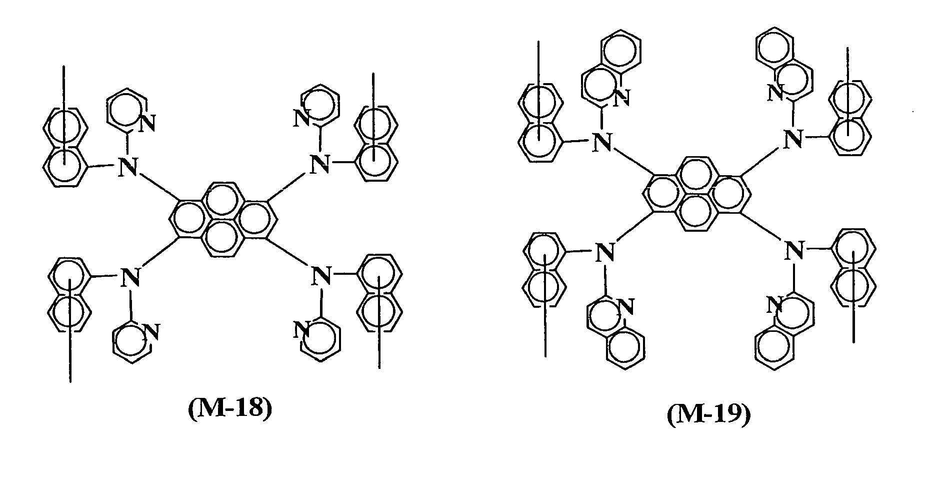

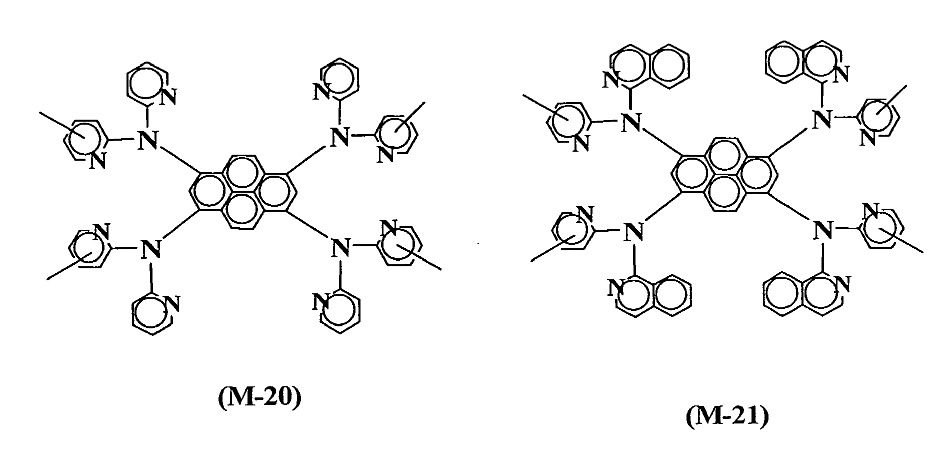

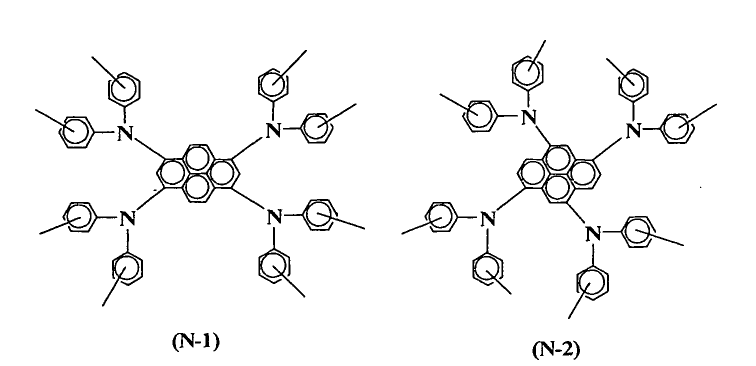

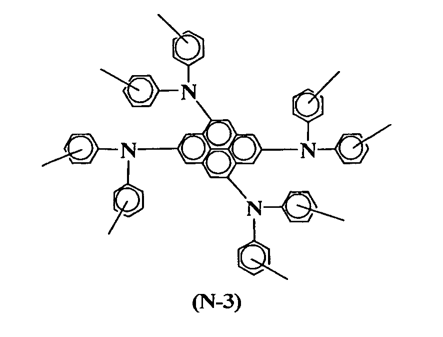

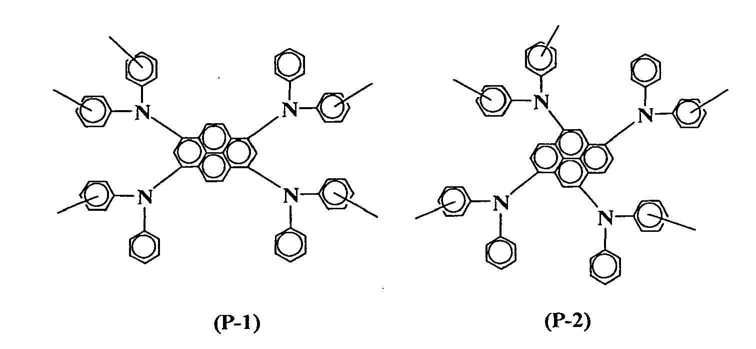

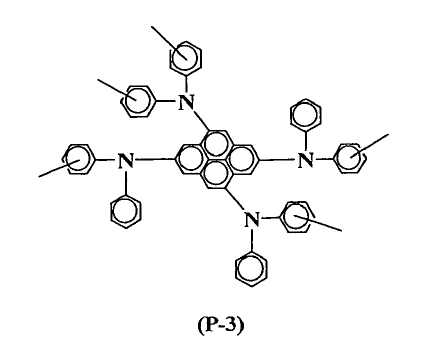

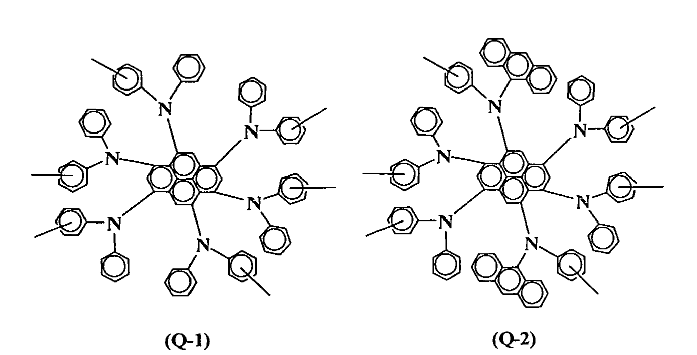

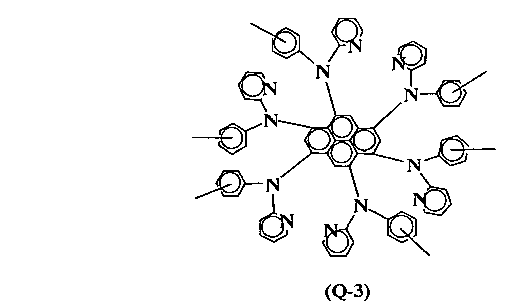

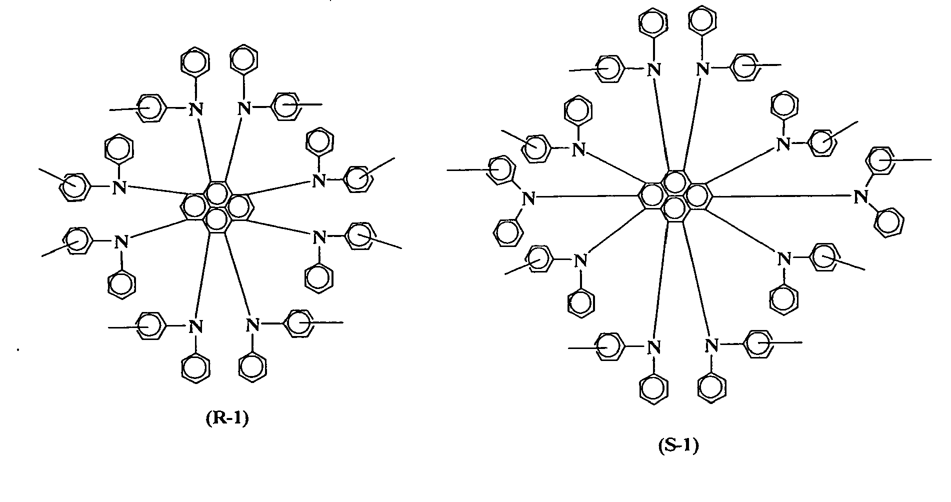

From the standpoint of the fluorescence intensity of a polymer compound, the life of a light emitting device or the efficiency of a light emitting device, the above-described the constitutional unit of the formula (1) includes preferably those of the formulae (A-1) to (A-15), (C-1) to (C-17), (D-1) to (D-12), (E-1) to (E-18), (H-1) to (H-6), (I-1) to (I-6), (M-1) to (M-3), (M-8) to (M-12) and (A-1) to (A-15), (C-1) to (C-17), (D-1) to (D-12), (E-1) to (E-18), (H-1) to (H-6), (I-1) to (I-6), (M-1) to (M-3), (M-8) to (M-12) each having a substituent such as an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group, cyano group and the like, more preferably those of the formulae (A-1) to (A-15), (D-1) to (D-12), (E-1) to (E-18), (M-1) to (M-3), (M-8) to (M-12), and (A-1) to (A-15), (D-1) to (D-12), (E-1) to (E-18), (M-1) to (M-3), (M-8) to (M-12) each having a substituent such as an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group, cyano group and the like, further preferably those of the formulae (A-1) to (A-15), (M-1) to (M-3), (M-8) to (M-12), and (A-1) to (A-15), (M-1) to (M-3), (M-8) to (M-12) each having a substituent such as an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group, cyano group and the like, especially preferably those of the formulae (A-5), (A-7), (A-12), (A-14), (M-1) to (M-3), and (A-5), (A-7), (A-12), (A-14), (M-1) to (M-3) each having a substituent such as an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group, cyano group and the like, particularly preferably those of the formulae (A-5) and (A-5) each having a substituent such as an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group, cyano group and the like.

From the standpoint of the fluorescence intensity of a polymer compound, the life of a light emitting device or the efficiency of a light emitting device, the above-described the constitutional unit of the formula (1) includes preferably those of the following formula (1A): 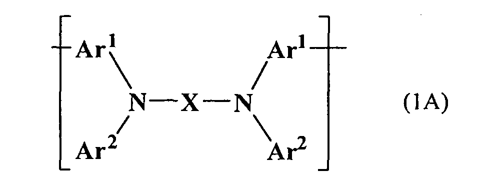 (wherein, X represents an unsubstituted or substituted pyrenediyl group. Ar¹ and Ar² are as described above. Two Ar¹'s may be the same or different. Two Ar²'s may be the same or different.)(that is, those of the formula (1) in which y1 and y2 are 1, z1 and z2 are 0, and u is 0), or those of the following formula (1B): 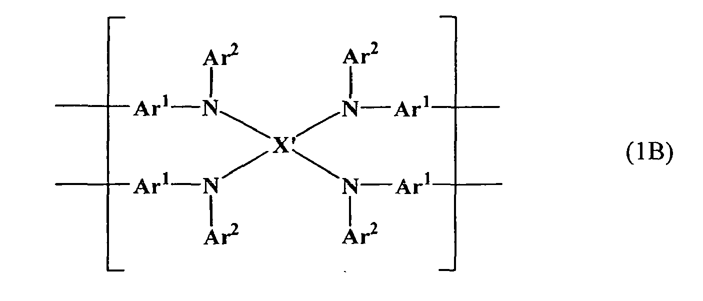 (wherein, X' represents an unsubstituted or substituted tetra-valent pyrene residue (that is, a group obtained by removing four hydrogen atoms from pyrene). Ar¹ and Ar² are as described above. Four Ar¹'s may be the same or different. Four Ar²'s may be the same or different.)(that is, those of the formula (1) in which x3, y1, y2 and y3 are 1, z1, z2 and z3 are 0, and u is 2).

The above-described the constitutional unit of the formula (1A) includes more preferably those of the following formula (5): 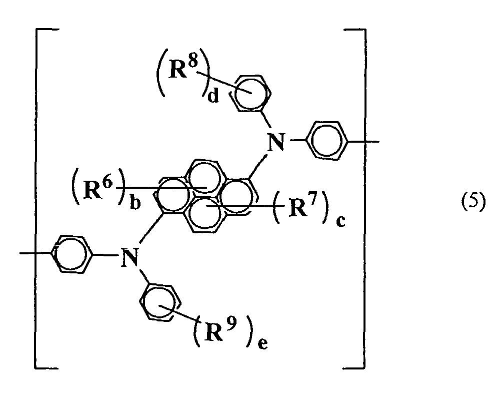 (wherein, R⁶, R⁷, R⁸ and R⁹ represent each independently an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group or cyano group. b and c represent each independently an integer of 0 to 4, and d and e represent each independently an integer of 0 to 5. When there exist a plurality of R⁶'s, R^{7's}s, R⁸'s and R⁹'s respectively, they may mutually be the same or different.), further preferably those of the following formula (5A): 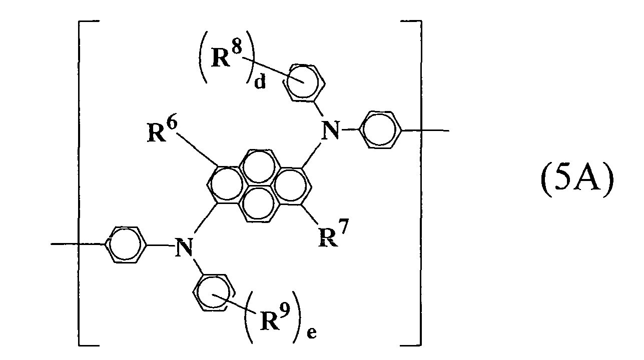 (wherein, R⁶, R⁷, R⁸, R⁹, d and e are as described above. When there exist a plurality of R⁸'s and R⁹'s respectively, they may mutually be the same or different.).

In the formula (5), when b is 1 or more, R⁶ is connected to a site ^{*} of a pyrenediyl group in the formula (5), and when c is 1 or more, R⁷ is connected to a site #.

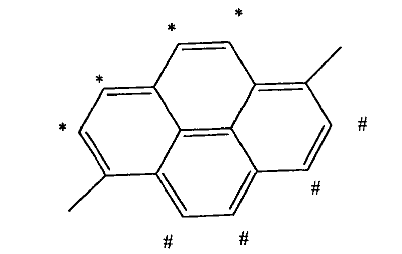

The above-described constitutional unit of the formula (1B) includes more preferably those of the following formula (5'): 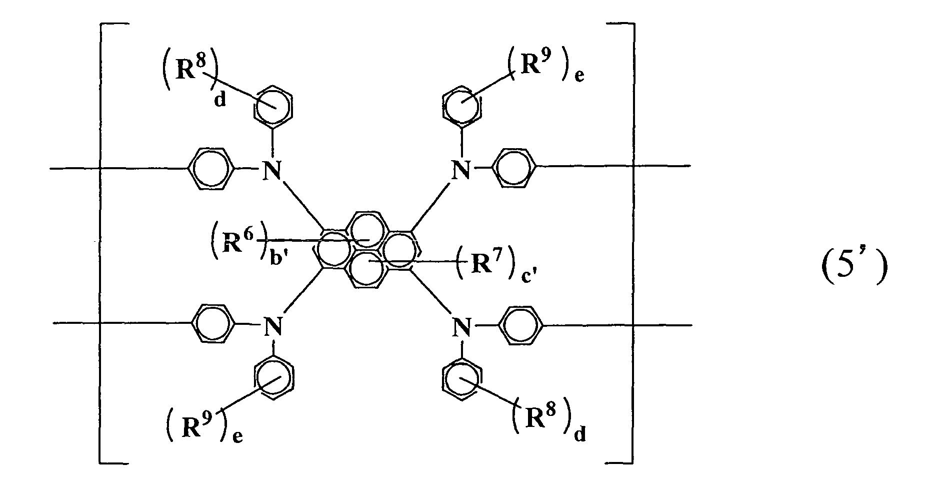 (wherein, R⁶, R⁷, R⁸, R⁹, d and e are as described above. b' and c' represent each independently an integer of 0 to 3. When there exist a plurality of R⁶'s, R⁷'s, R⁸'s and R⁹'s respectively, they may mutually be the same or different. Two d's and two e's may each be the same or different.).

The definitions and examples of the alkyl group, alkoxy group, alkylthio group, aryl group, aryloxy group, arylthio group, arylalkyl group, arylalkoxy group, arylalkylthio group, arylalkenyl group, arylalkynyl group, substituted amino group, substituted silyl group, halogen atom, acyl group, acyloxy group, imine residue, amide group, acid imide group, mono-valent heterocyclic group and substituted carboxyl group represented by R⁶, R⁷, R⁸ and R⁹ in the above-described formulae (5), (5A), (5') are the same as the definitions and examples explained in the section of a substituent when the above-described Z has a substituent.

From the standpoint of improvement in the solubility of the resultant polymer compound, easiness of synthesis of a polymer compound, attainment of blue light emission and the like, R⁶ and R⁷ in the above-described formulae (5), (5A), (5') represent preferably an alkyl group, alkoxy group, alkylthio group, aryloxy group, arylalkyl group, arylalkoxy group, more preferably an alkyl group, alkoxy group, further preferably an alkyl group.

From the standpoint of attainment of green light emission and the like, R⁶ and R⁷ in the above-described formulae (5), (5A), (5') represent preferably an aryl group, arylalkenyl group, arylalkynyl group, amino group, substituted amino group, halogen atom, acyl group, acyloxy group, imine residue, mono-valent heterocyclic group, carboxyl group, substituted carboxyl group, nitro group, cyano group, or aryl group substituted with an alkyl group, more preferably an aryl group, arylalkenyl group, arylalkynyl group, substituted amino group, acyl group, or mono-valent heterocyclic group, further preferably an aryl group, substituted amino group, acyl group, or mono-valent heterocyclic group, especially preferably an aryl group, or substituted amino group, particularly preferably an aryl group.

In the above-described formula (5), b and c represent each independently preferably an integer of 0 to 2, more preferably 2. In the above-described formula (5'), b' and c' represent each independently preferably 0 or 1, more preferably 0.

From the standpoint of improvement in the solubility of the resultant polymer compound, easiness of synthesis of a polymer compound, and the like, R⁸ and R⁹ in the above-described formulae (5), (5A), (5') represent preferably an alkyl group, alkoxy group, aryl group, aryloxy group, arylalkyl group or arylalkoxy group, more preferably an alkyl group, alkoxy group, arylalkyl group or arylalkoxy group, further preferably an alkyl group, alkoxy group, particularly preferably an alkyl group.

In the above-described formulae (5), (5A), (5'), d and e represent each independently an integer of 0 to 5, preferably an integer of 0 to 3, more preferably an integer of 1 to 3, further preferably 1.

In the above-described formulae (5), (5A), (5'), R⁶ and R⁷ may be the same or different, and preferably the same.

In the above-described formulae (5), (5A), (5'), it is preferable that R⁶ and R⁷ represent each independently an aryl group, R⁸ and R⁹ represent each independently an alkyl group; R⁶ and R⁷ represent each independently a substituted amino group, and R⁸ and R⁹ represent each independently an alkyl group.

Examples of the above-described di-valent group of the formula (5) (including those of the formula (5A)) include the following groups (6A-1), (6B-1) to (6B-6), (6C-1) to (6C-5), (6D-1) to (6D-5), (6E-1) to (6E-3), (6F-1) to (6F-3), (6G-1) to (6G-3), (6H-1) to (6H-5), (6I-1), (6J-1) to (6J-3), (6K-1) to (6K-9), (6L-1) to (6L-9), (6M-1) to (6M-9), (6N-1) to (6N-9), (6P-1) to (6P-3), (6Q-1) to (6Q-33), (6R-1) to (6R-7), (6S-1) to (6S-9), (6T-1) to (6T-18), (6U-1) to (6U-3), (6V-1) to (6V-10), and the like.

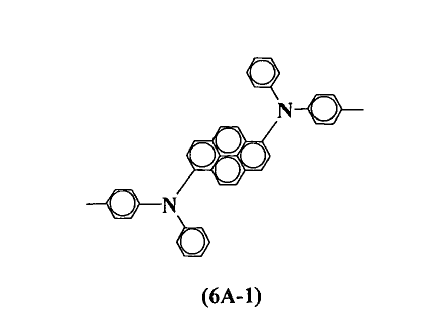

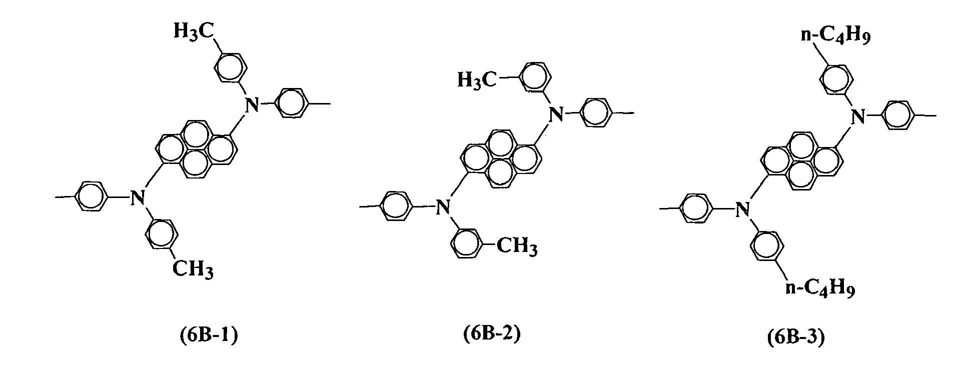

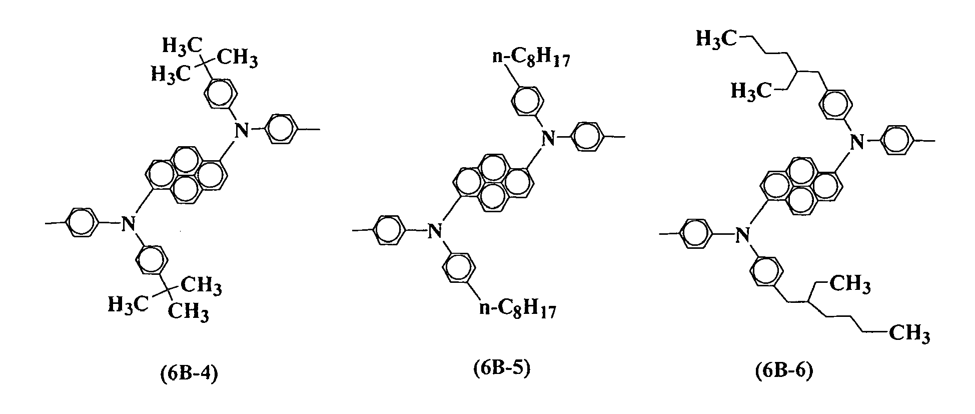

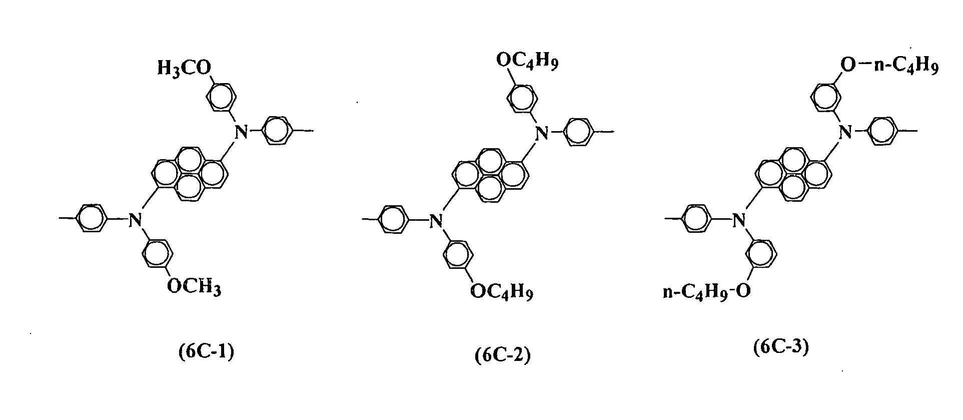

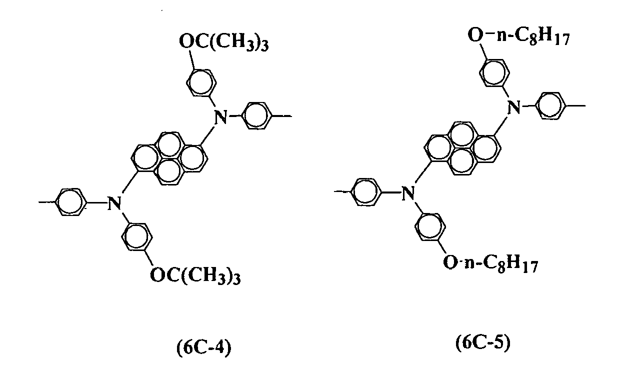

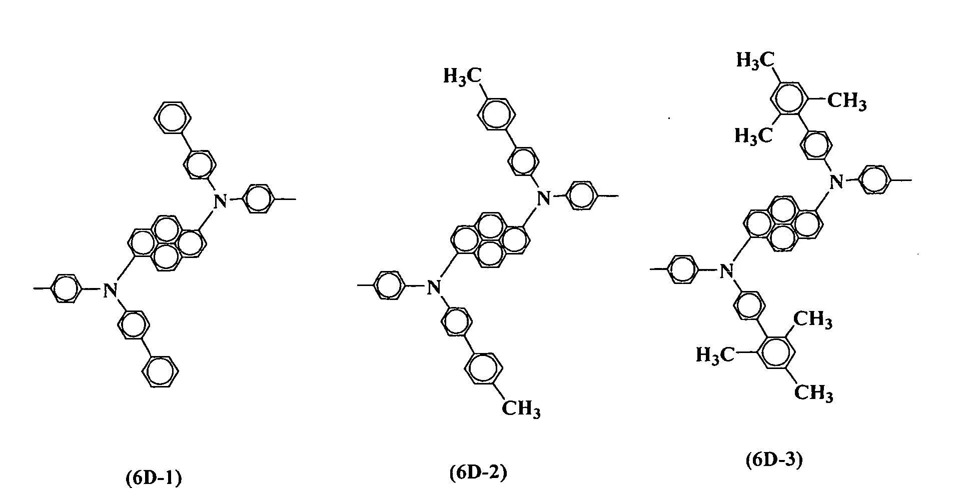

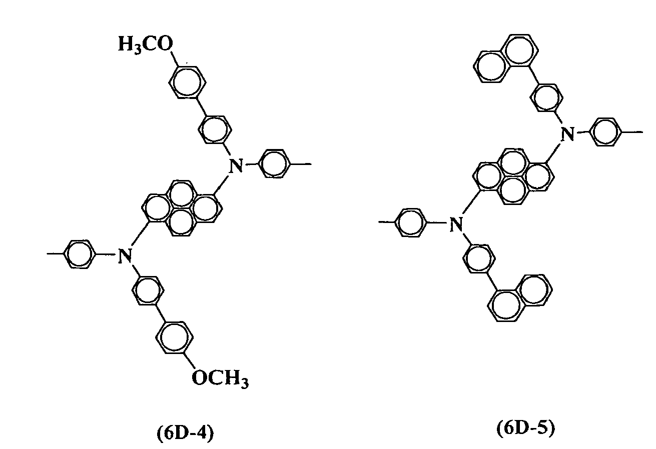

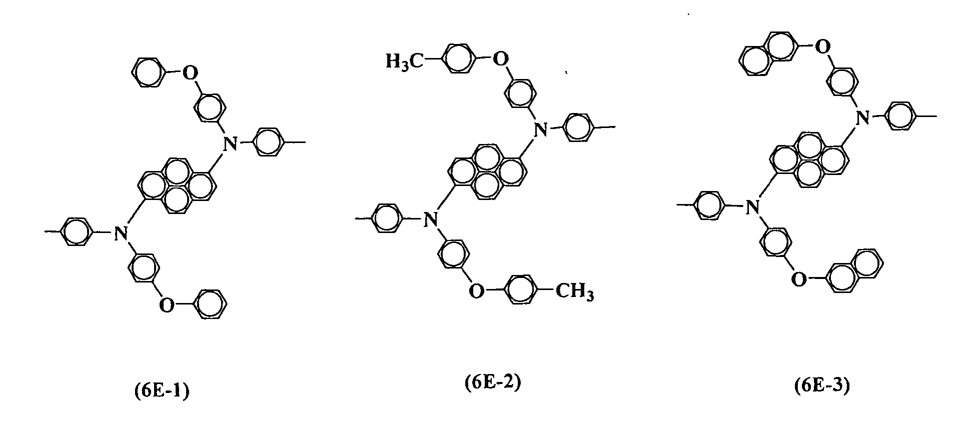

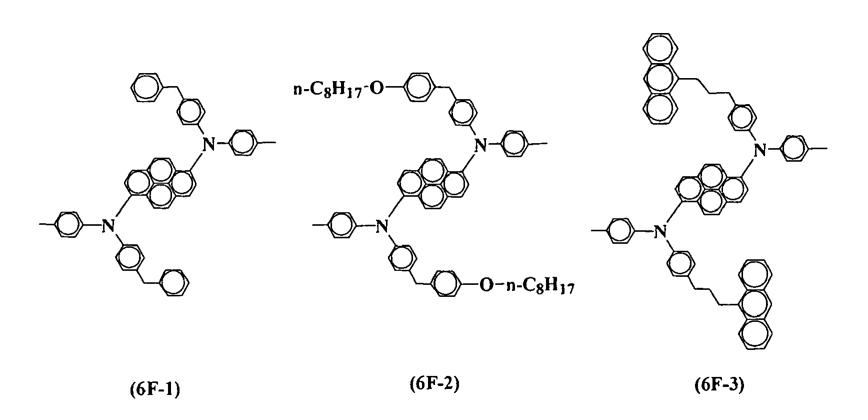

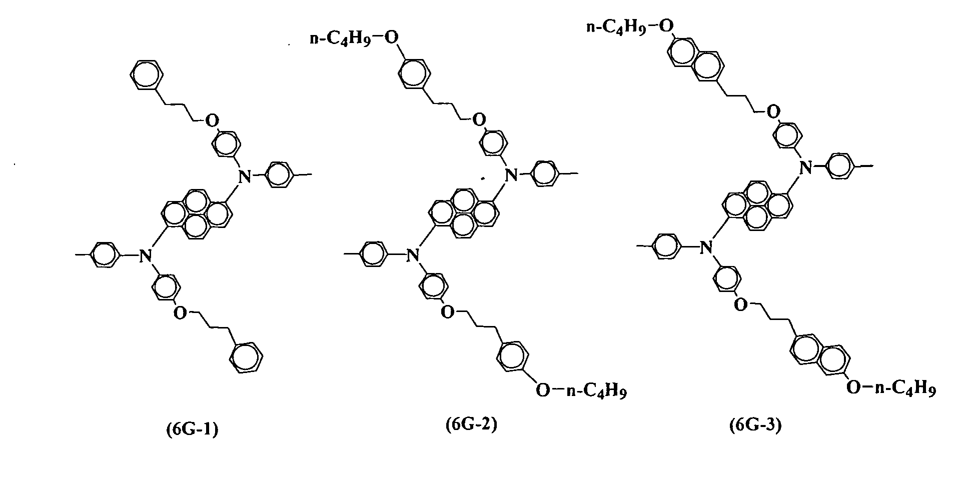

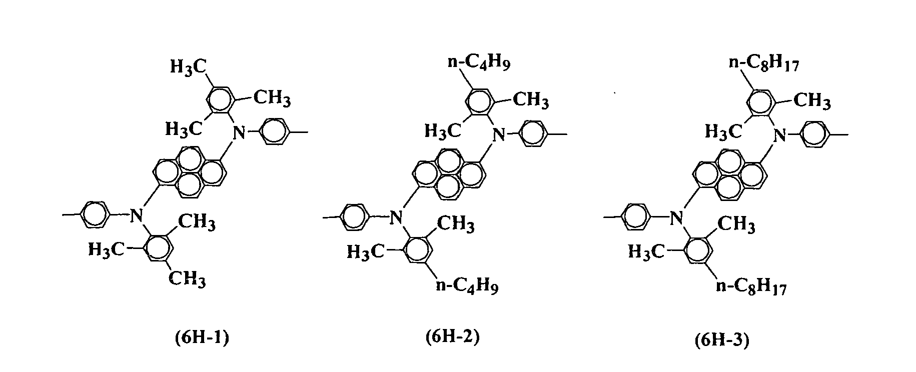

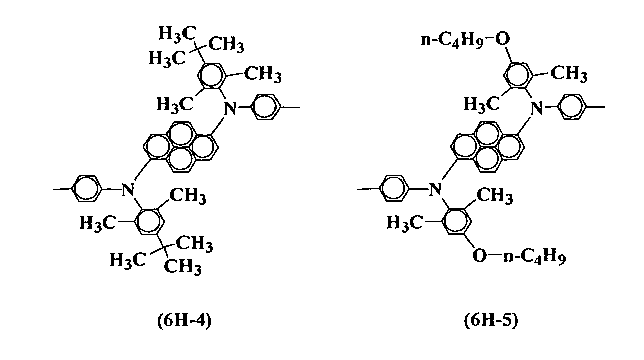

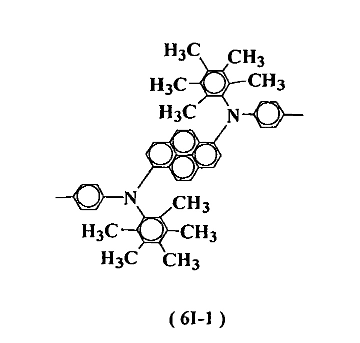

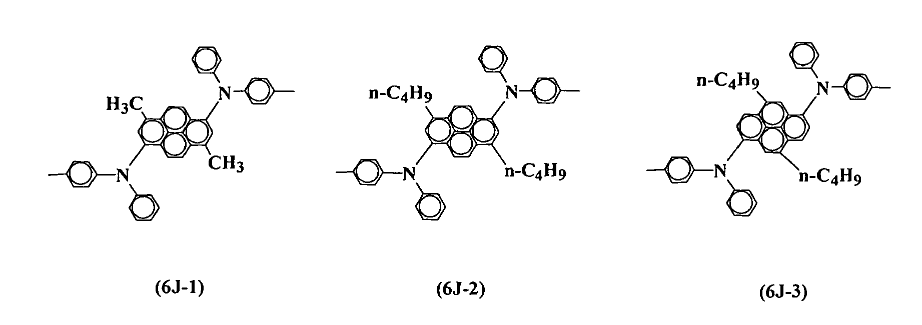

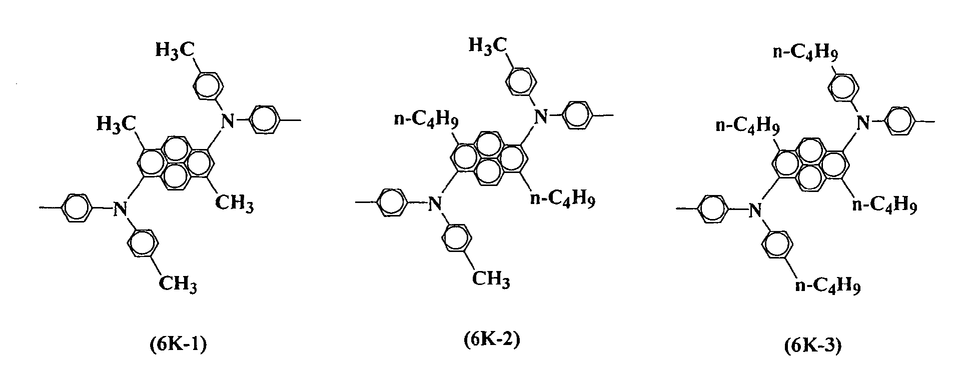

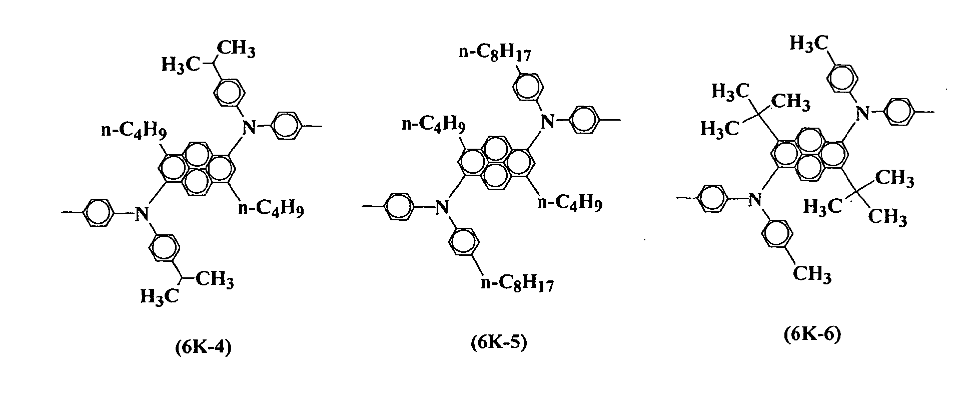

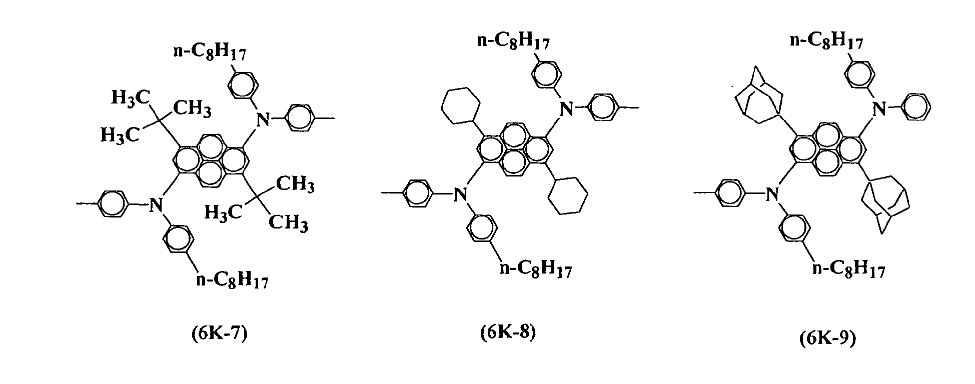

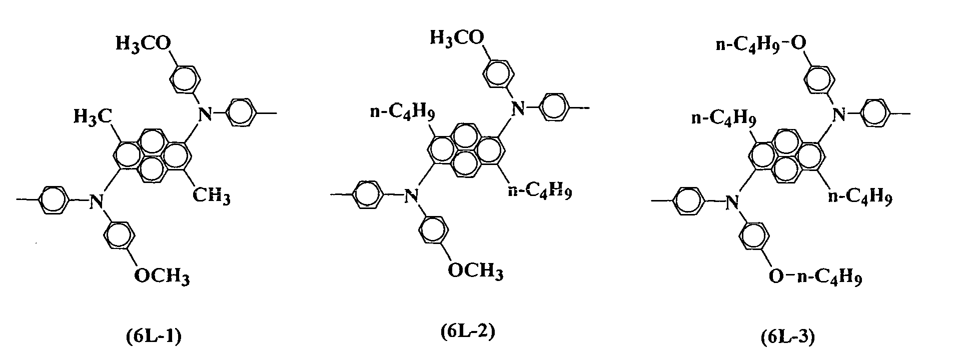

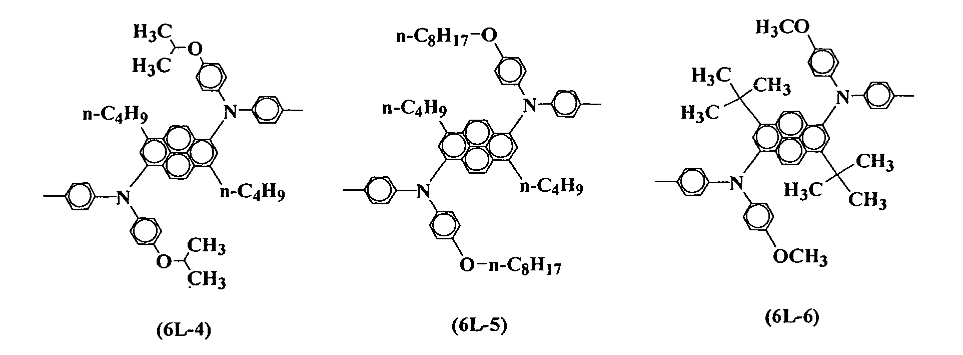

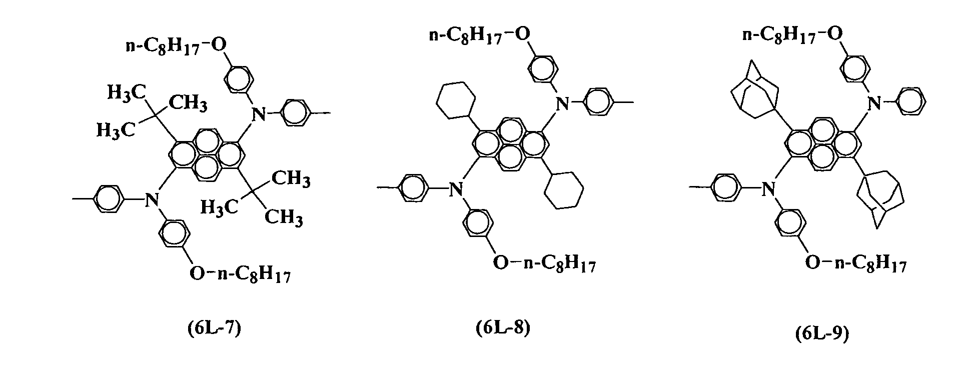

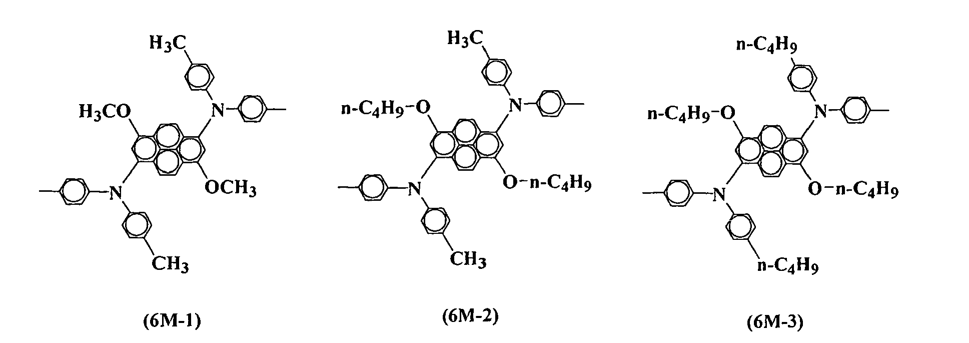

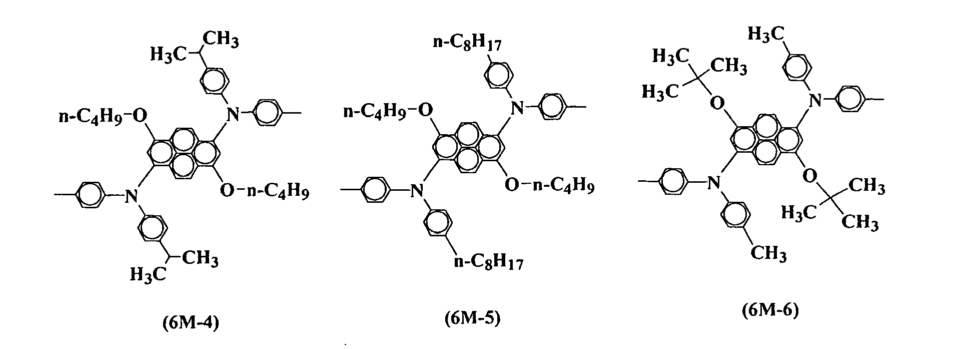

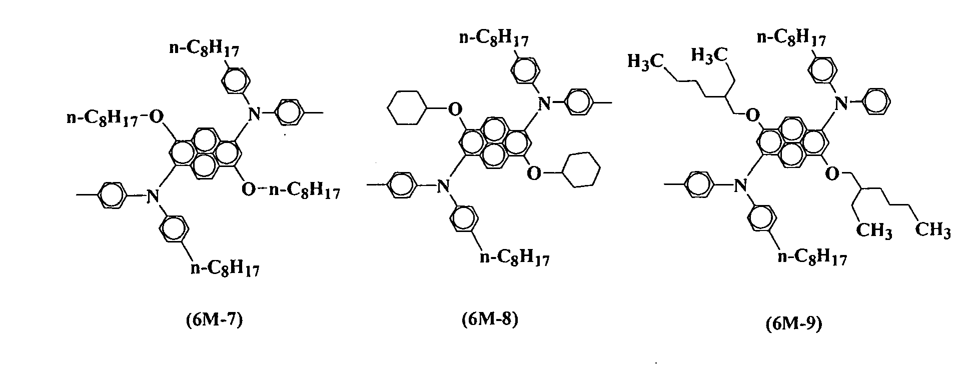

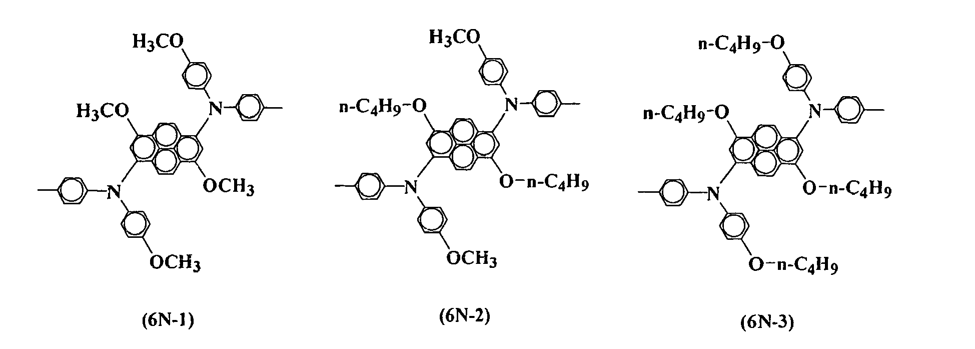

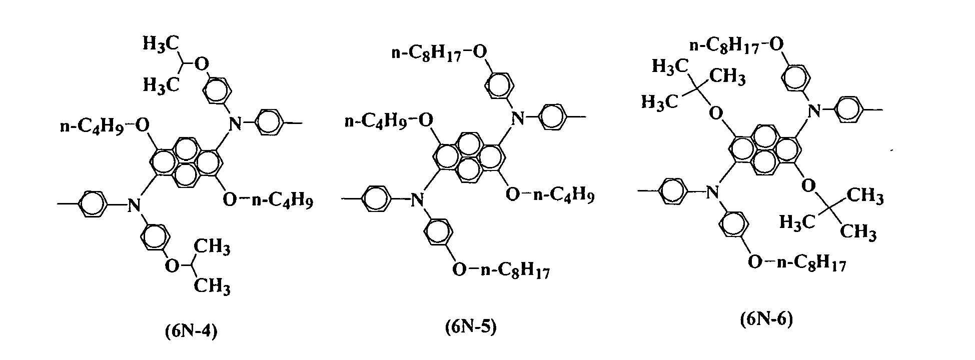

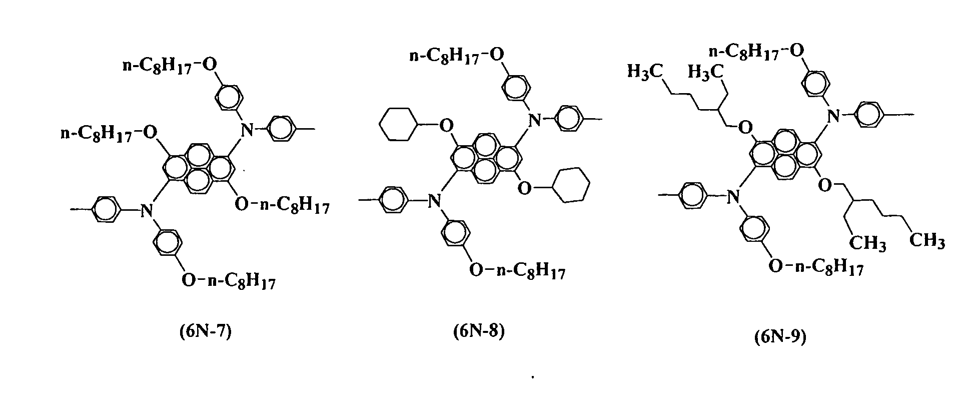

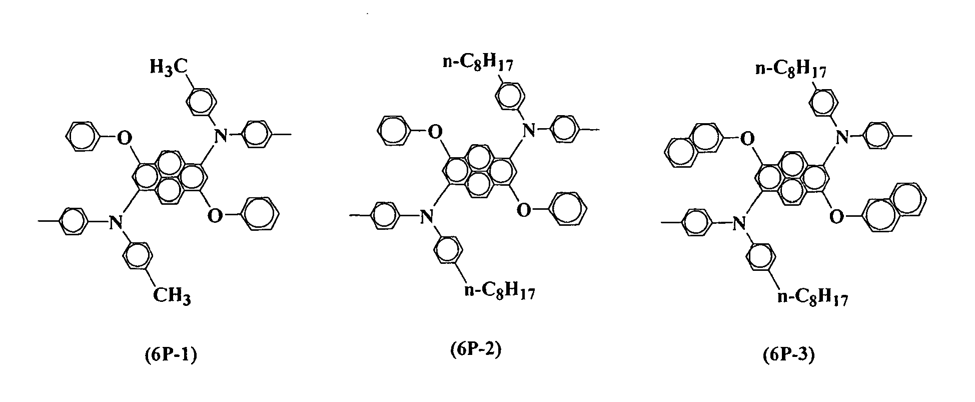

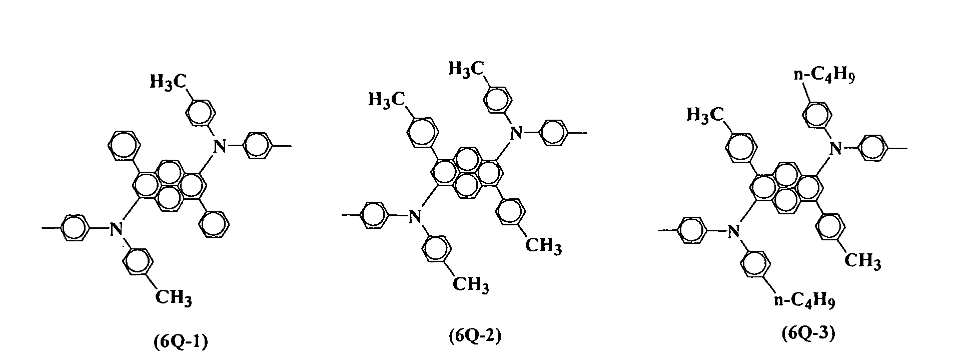

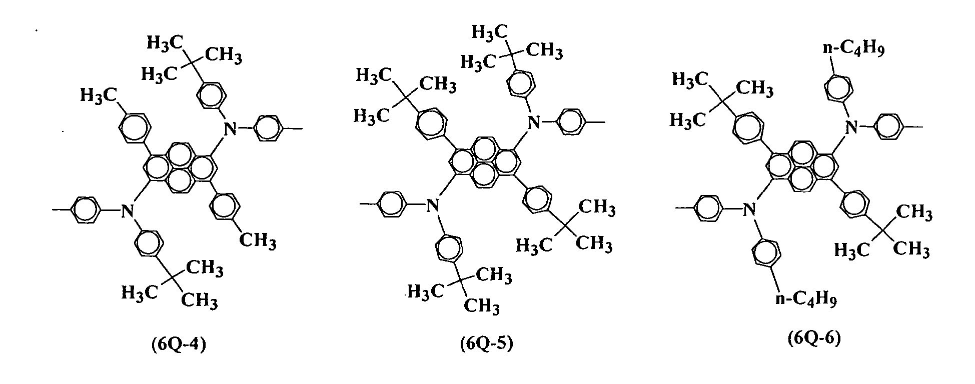

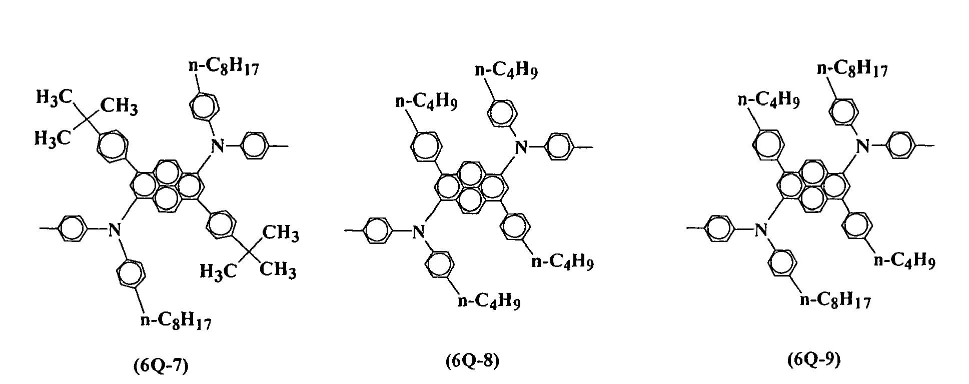

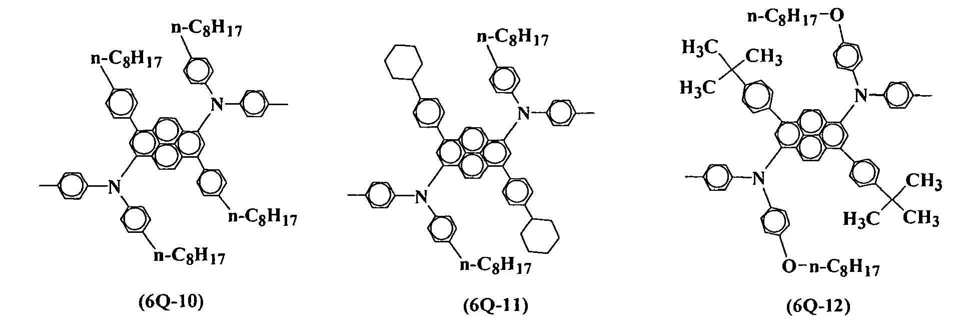

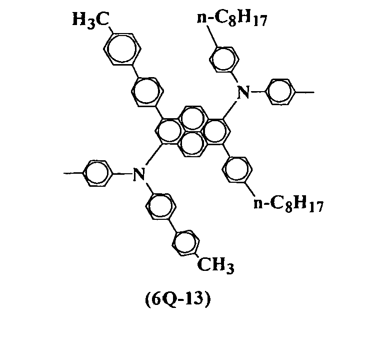

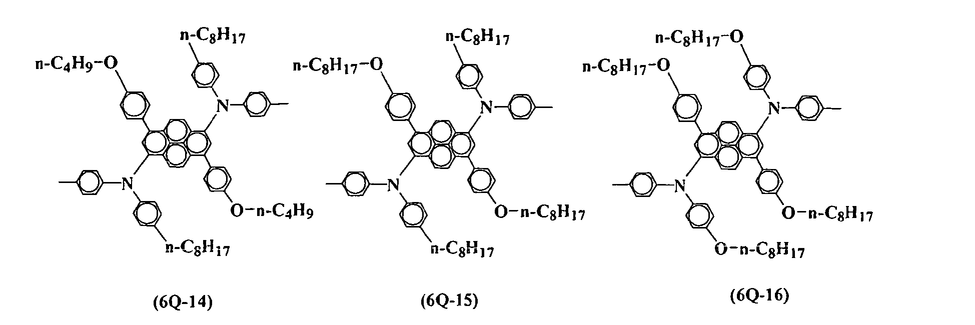

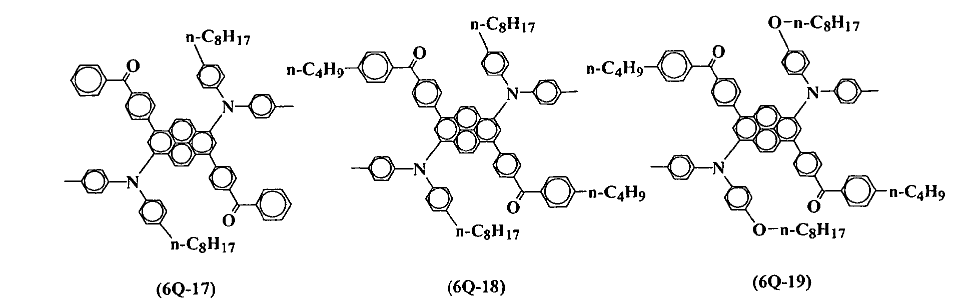

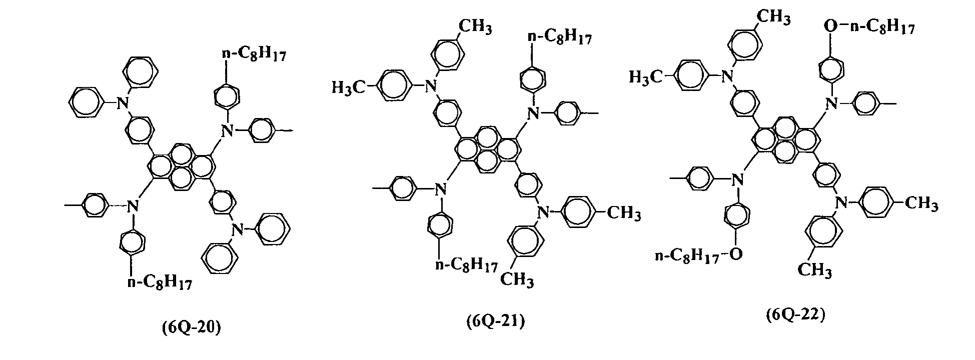

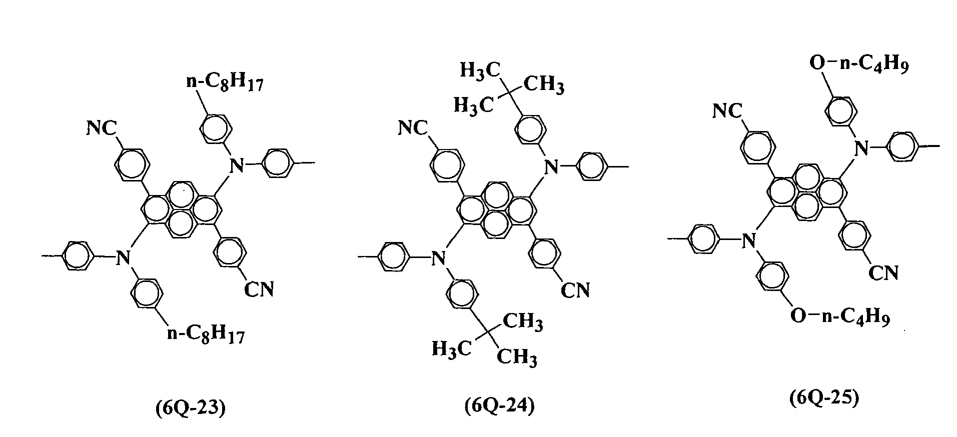

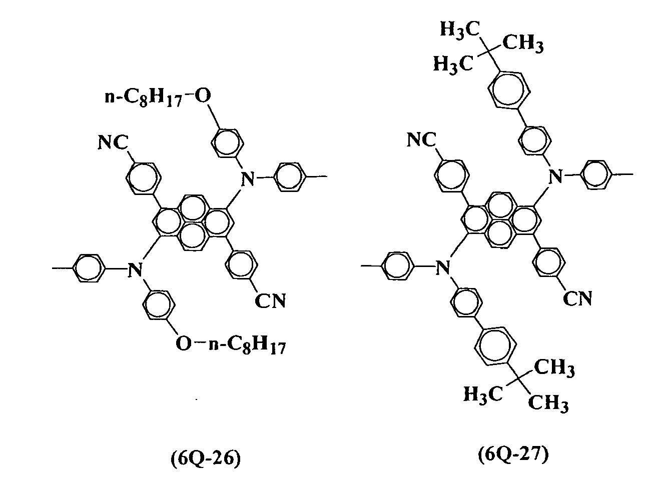

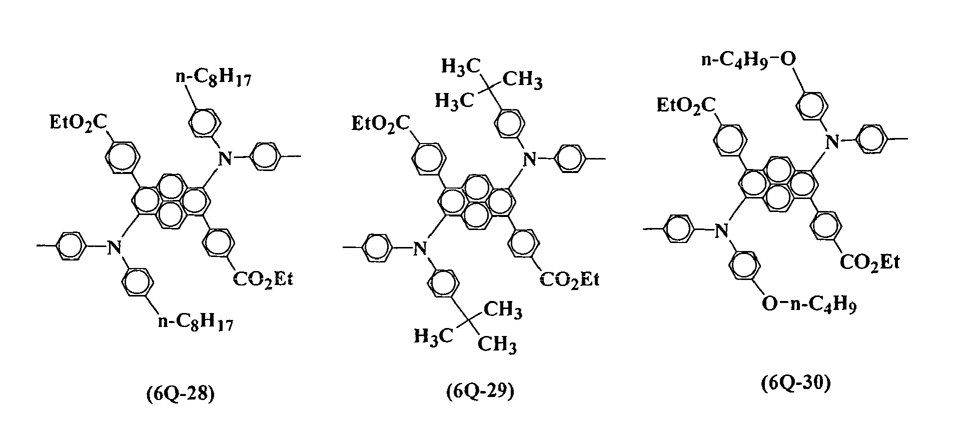

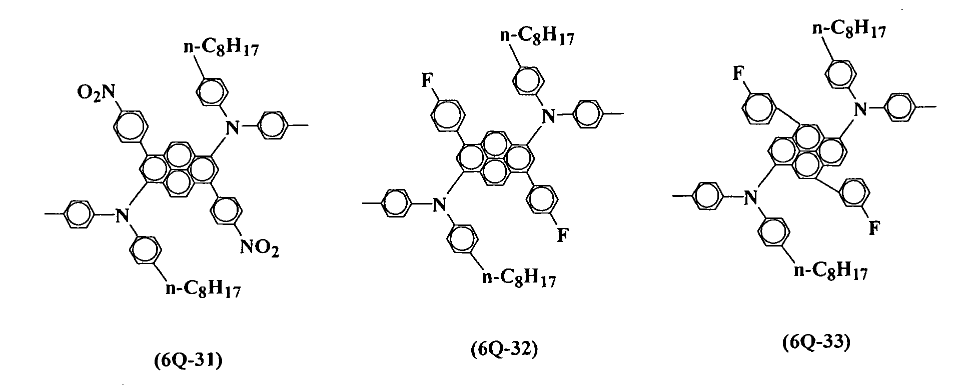

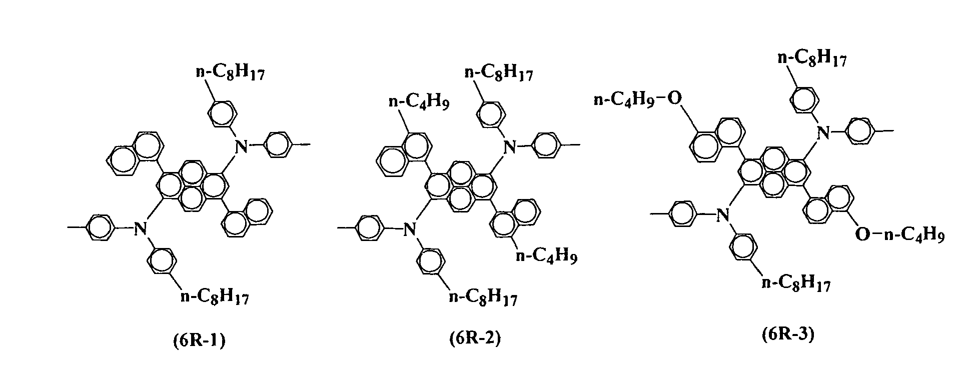

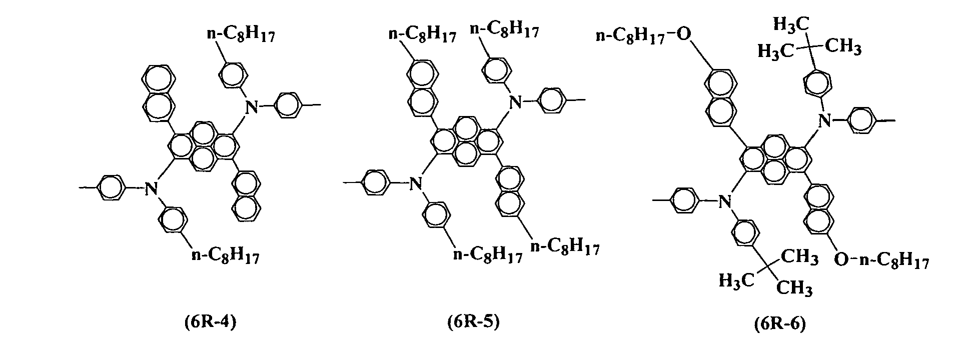

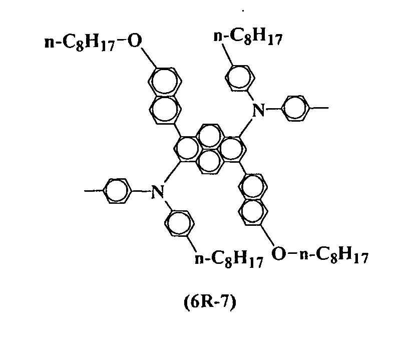

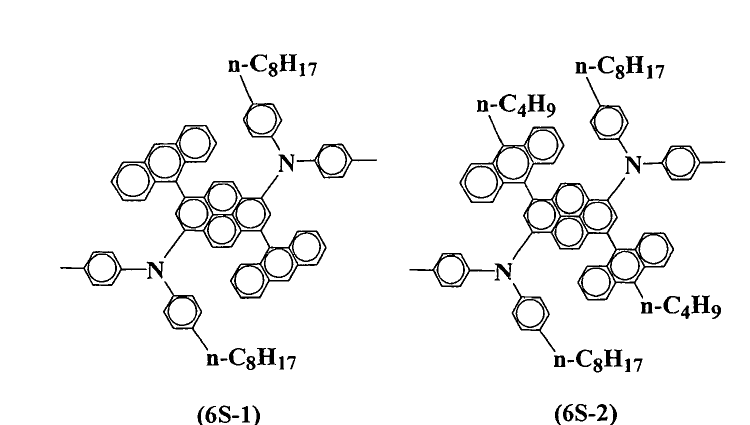

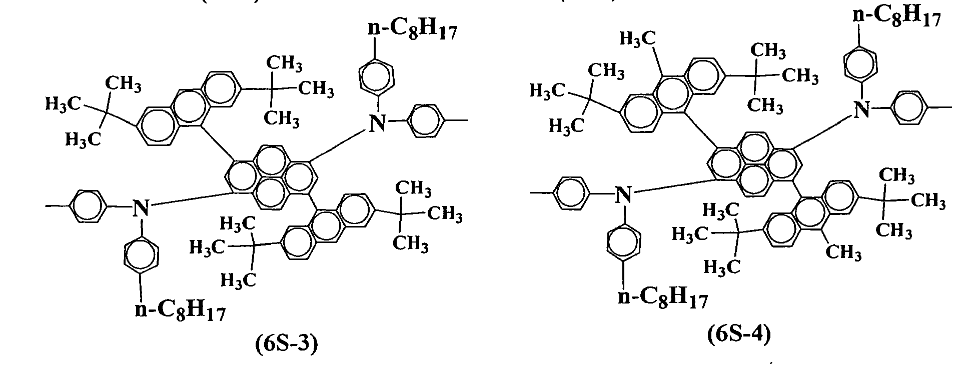

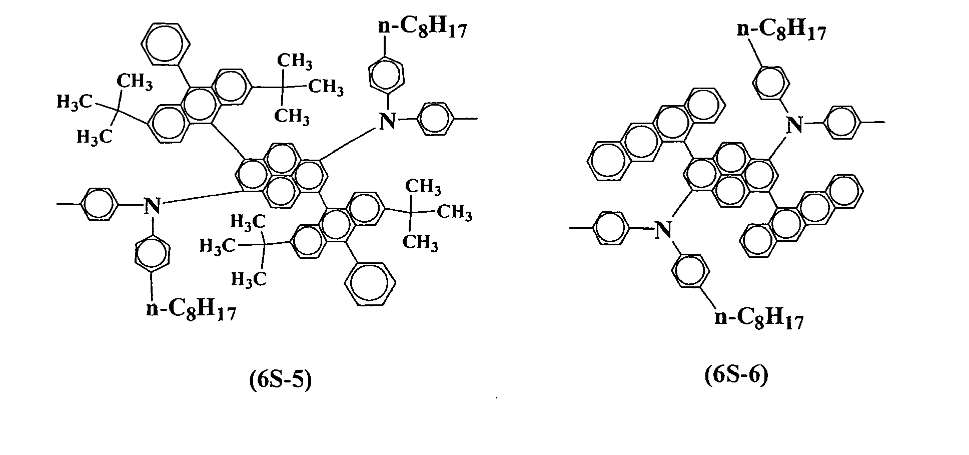

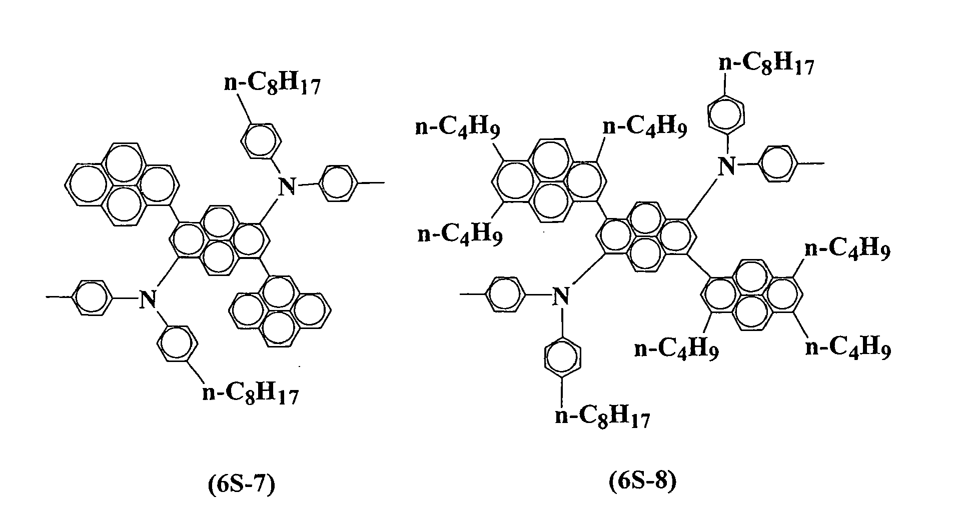

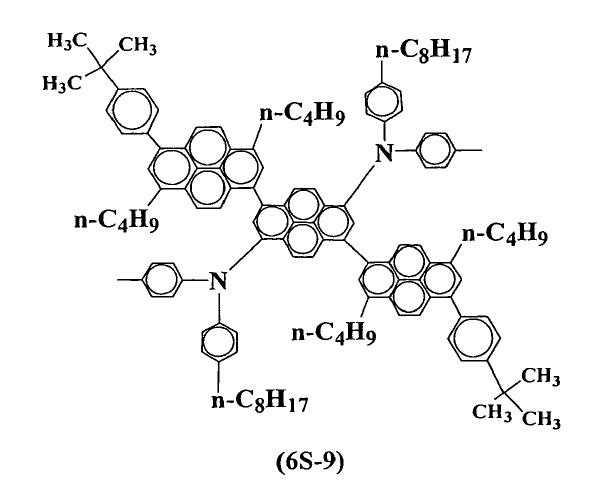

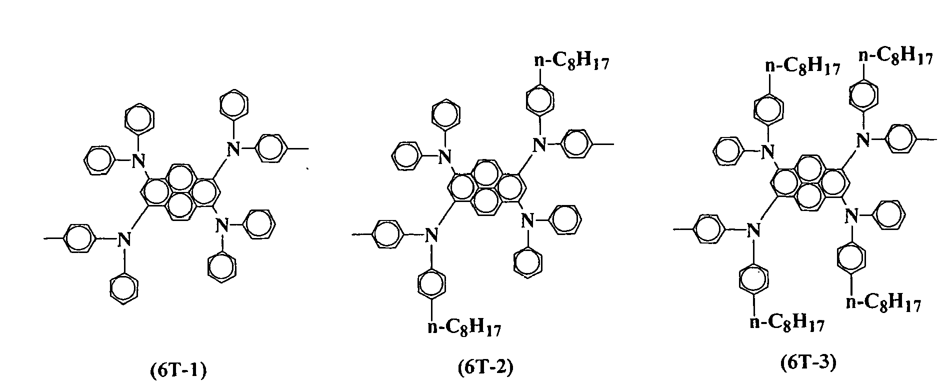

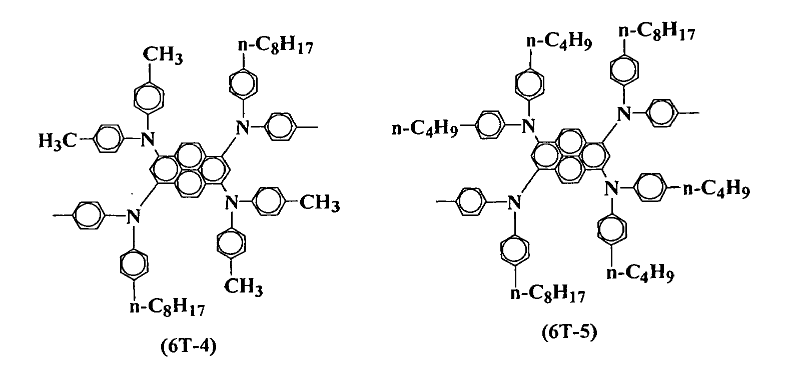

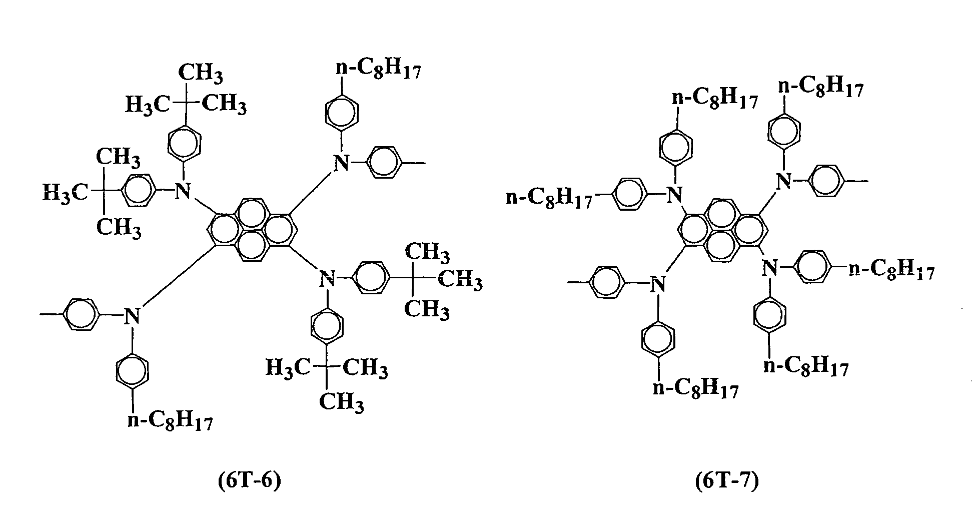

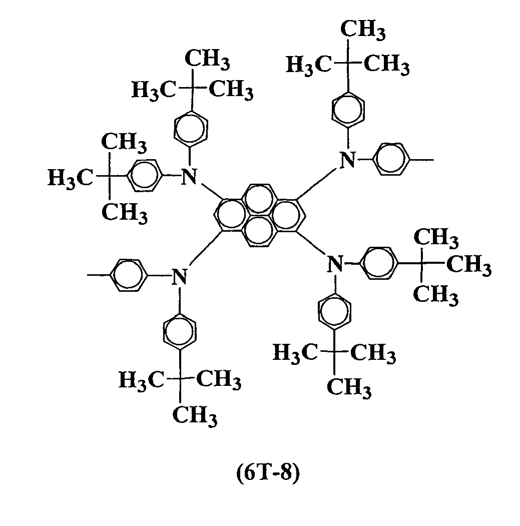

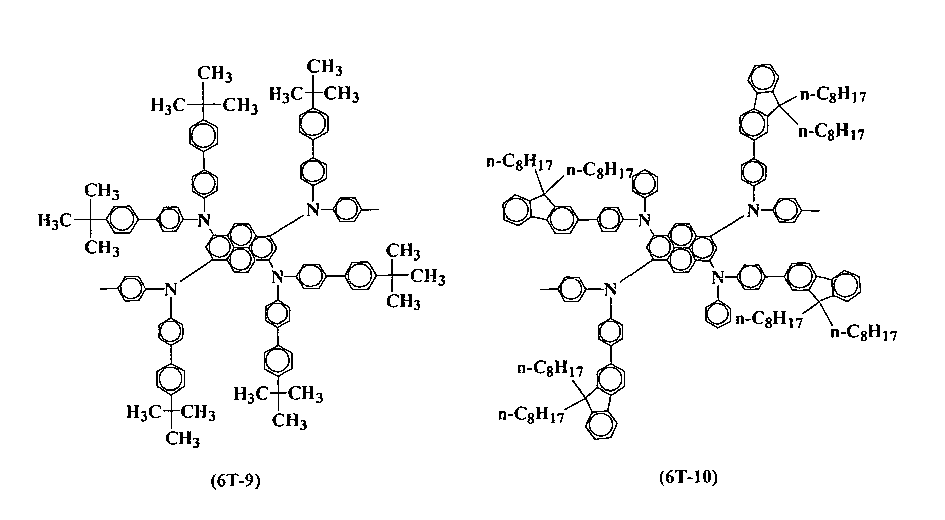

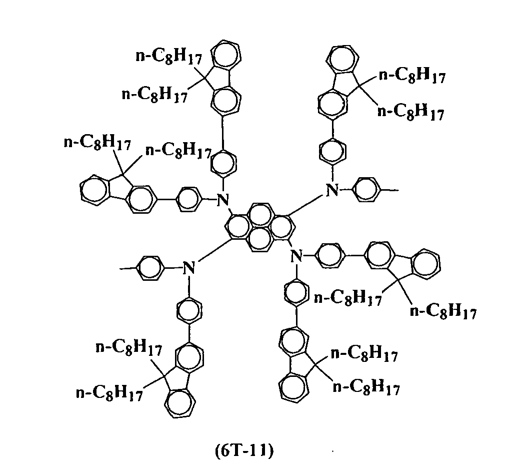

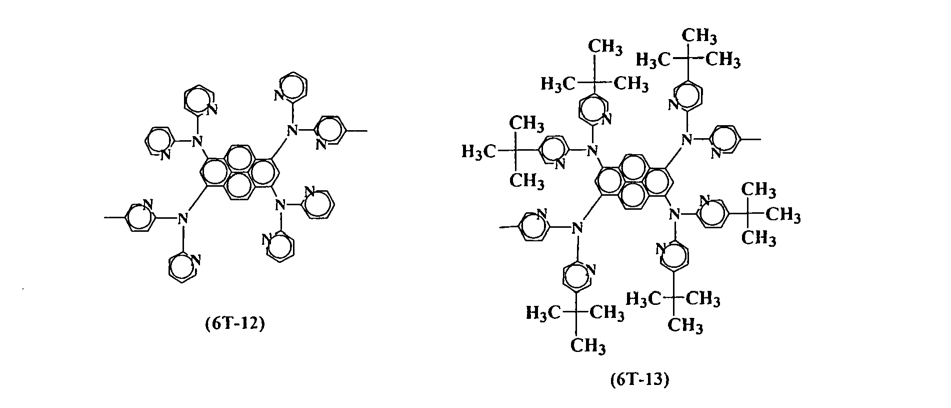

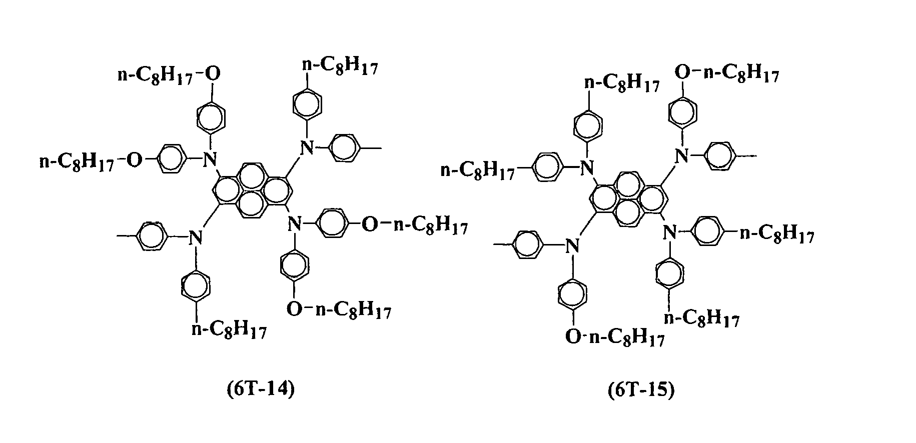

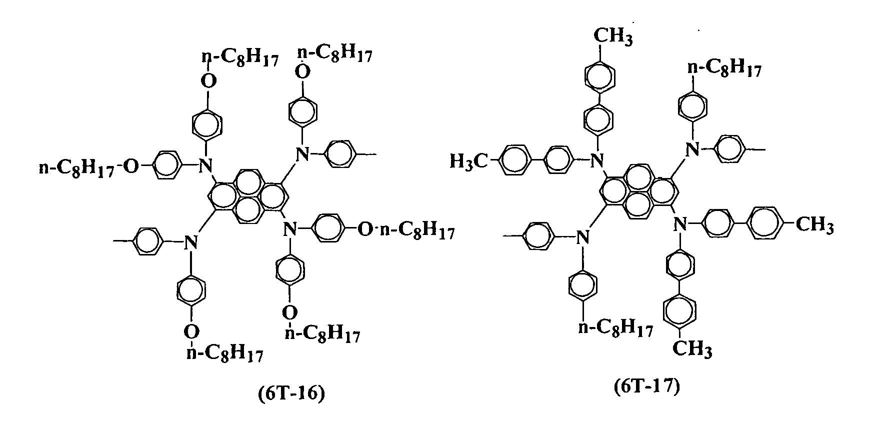

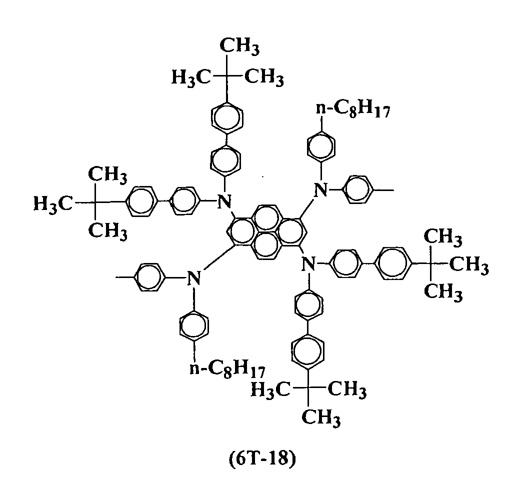

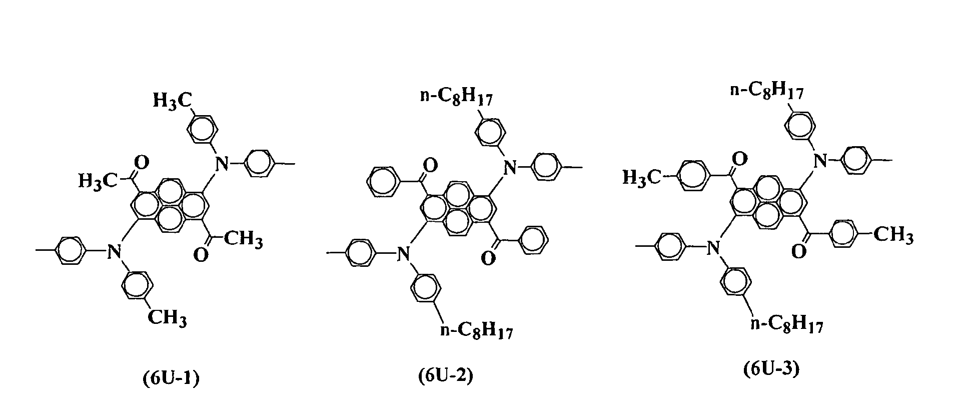

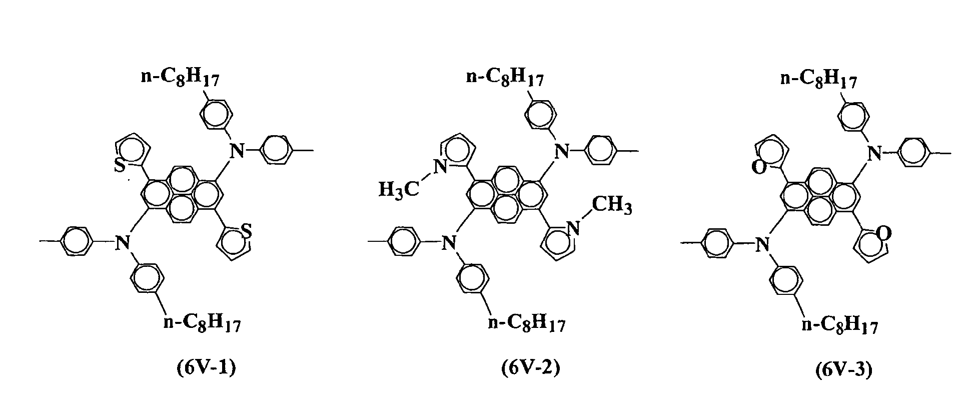

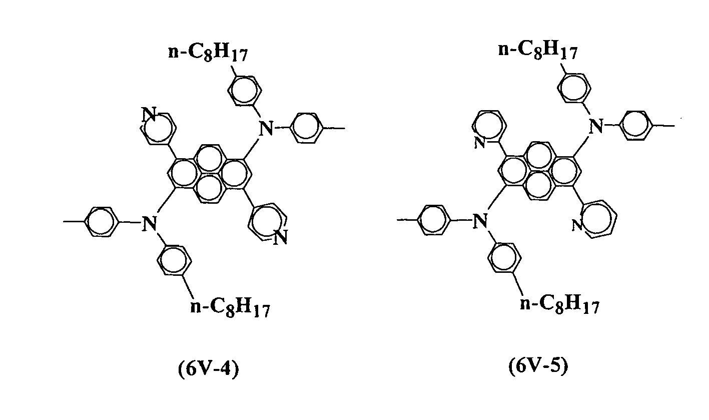

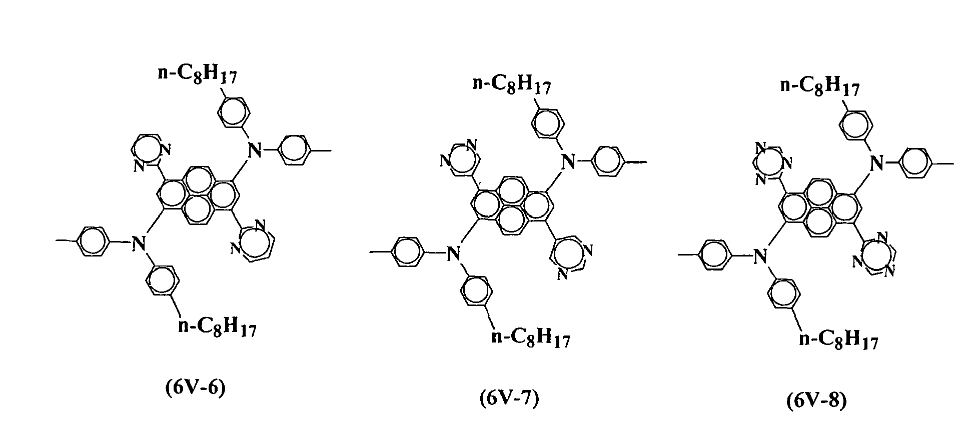

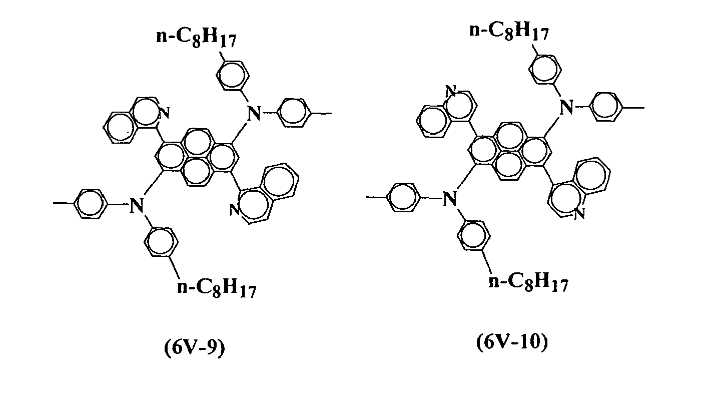

Examples of the above-described di-valent group of the formula (5') include the following groups (5'A-1), (5'B-1) to (5'B-5), (5'C-1) to (5'C-5), (5'D-1) to (5'D-6), (5'E-1) to (5'E-2), (5'F-1) to (5'F-5), (5'G-1) to (5'G-5), (5'H-1) to (5'H-5), (5'J-1) to (5'J-4), (5'K-1) to (5'K-6), (5'L-1) to (5'L-6), and the like.

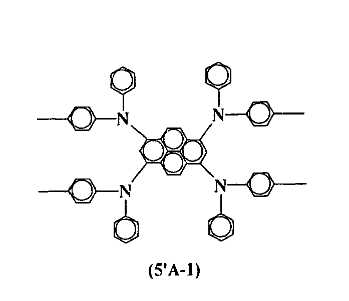

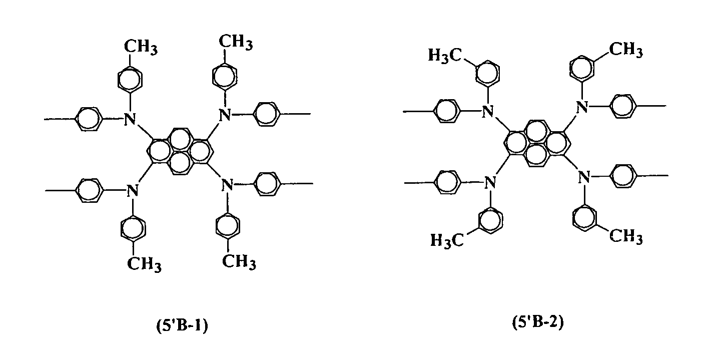

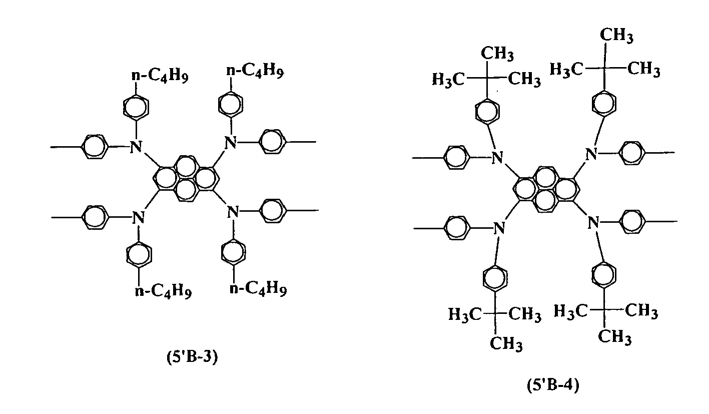

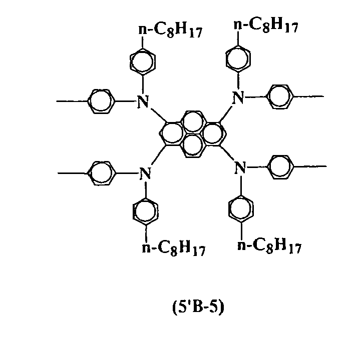

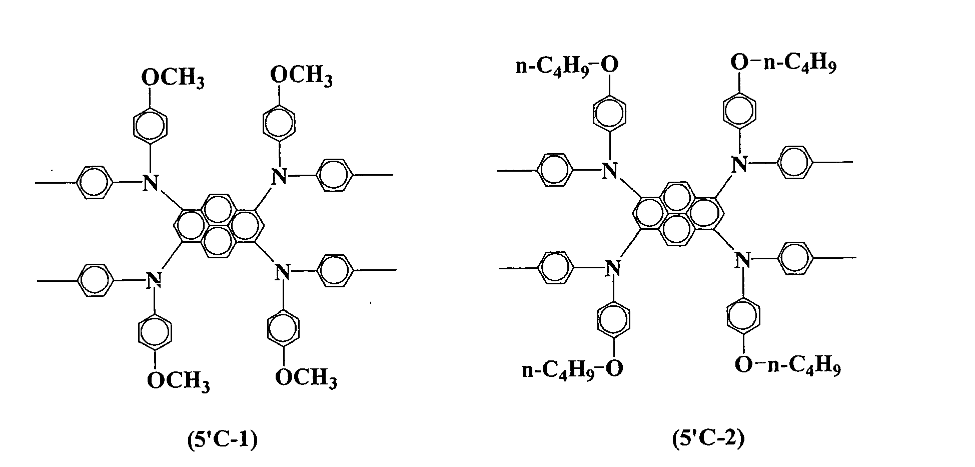

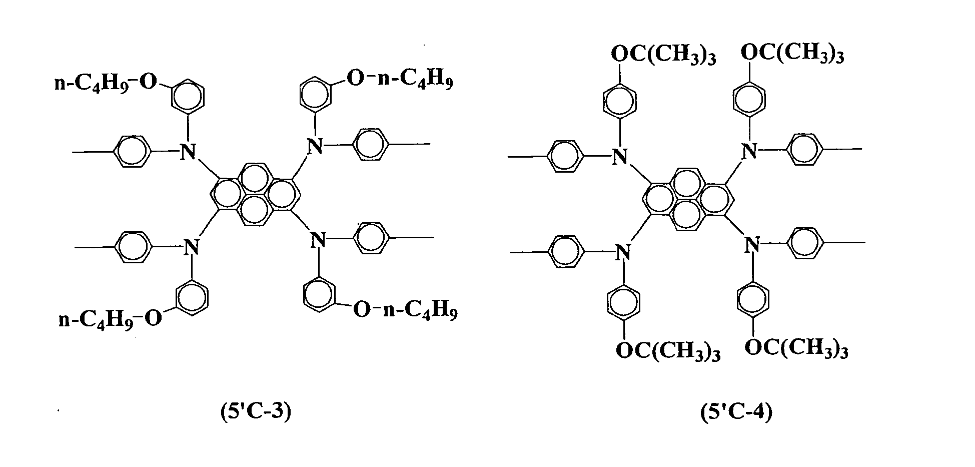

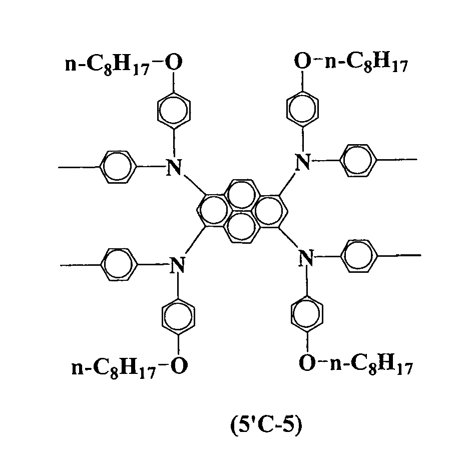

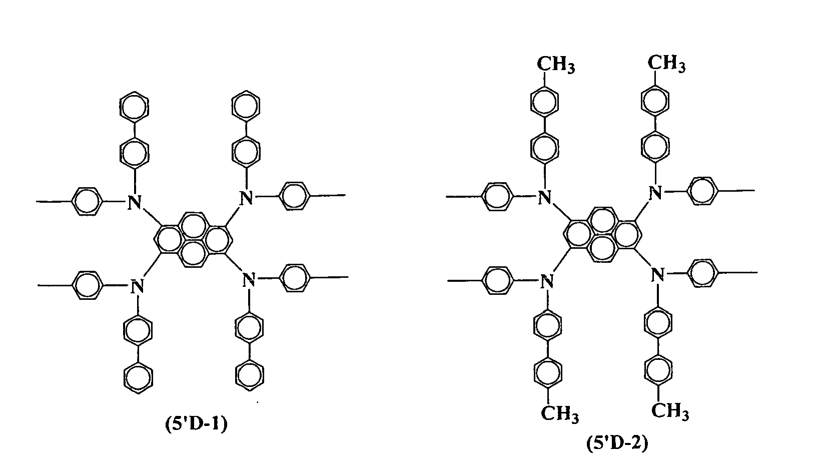

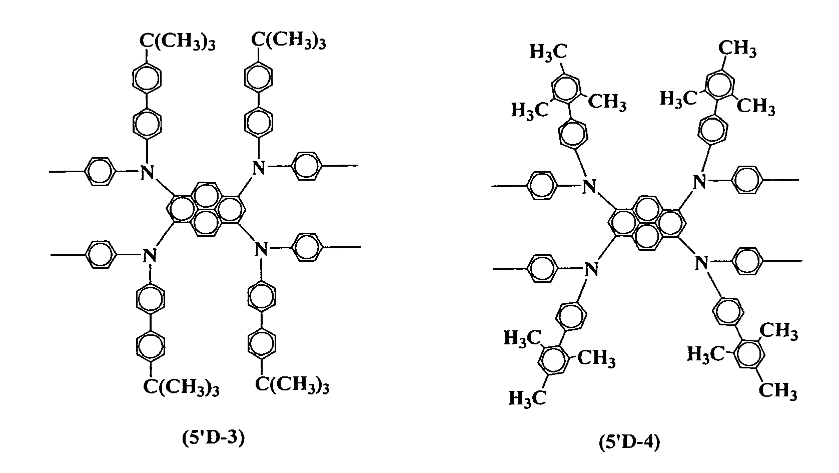

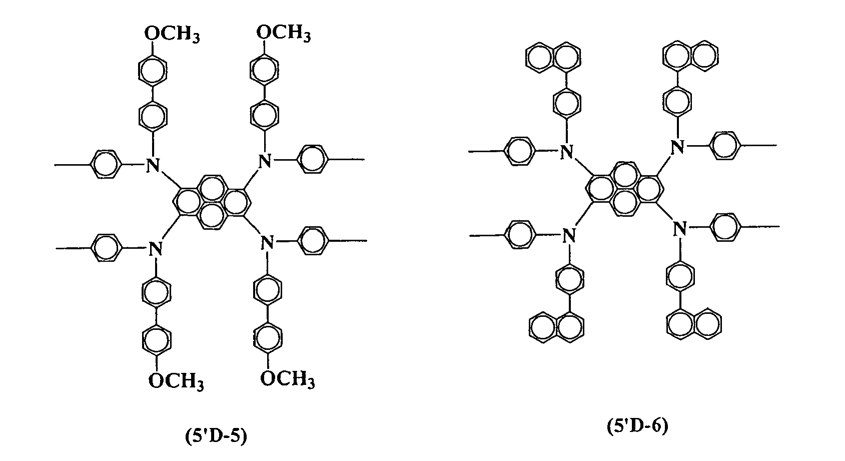

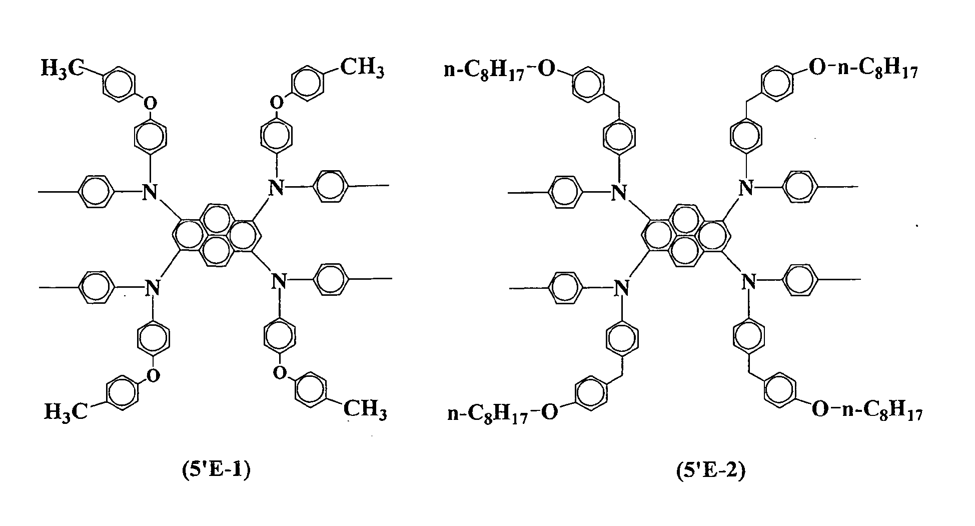

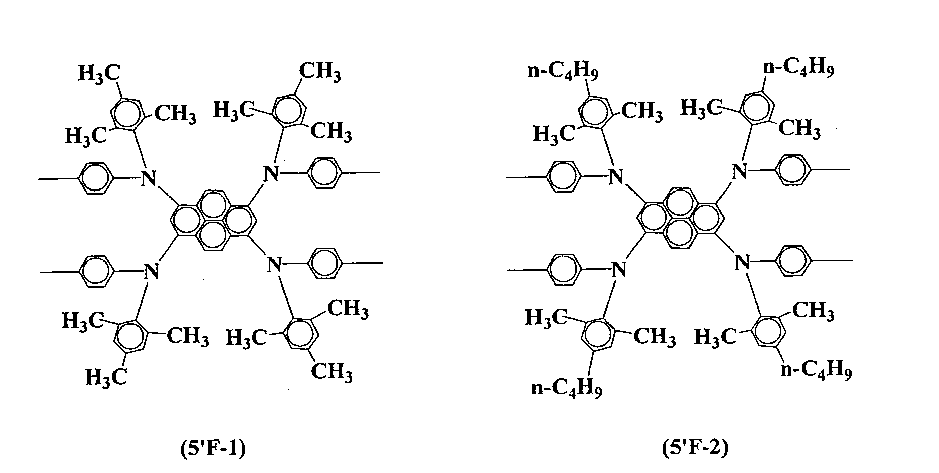

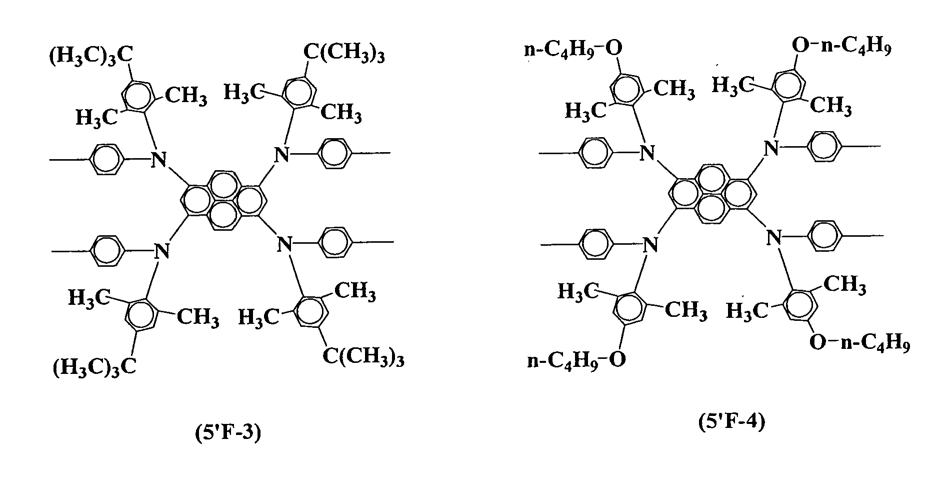

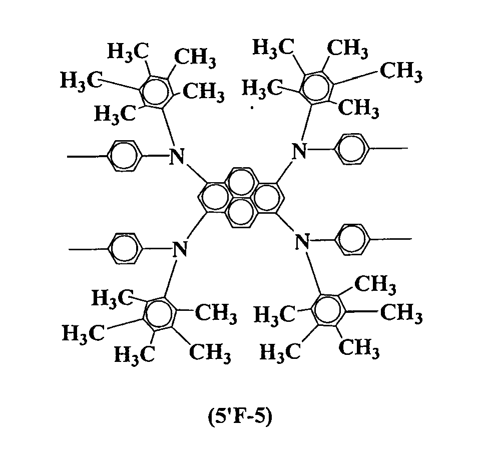

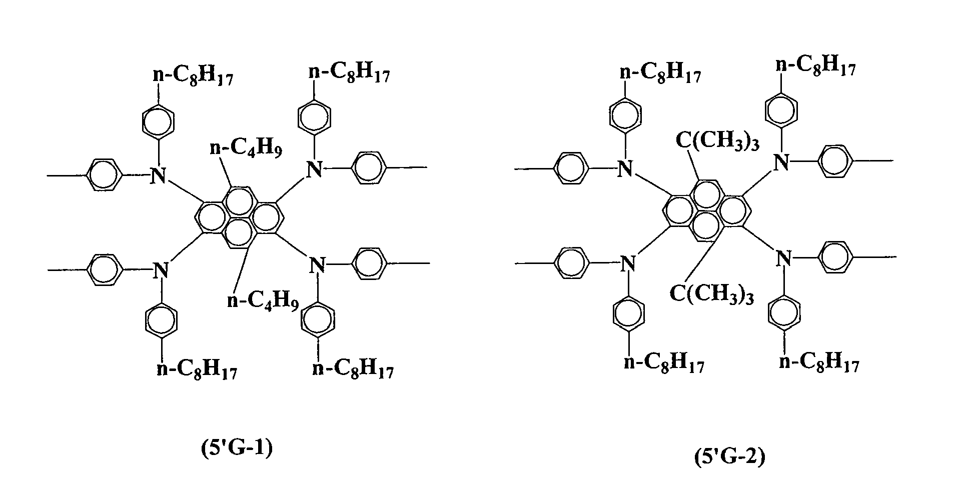

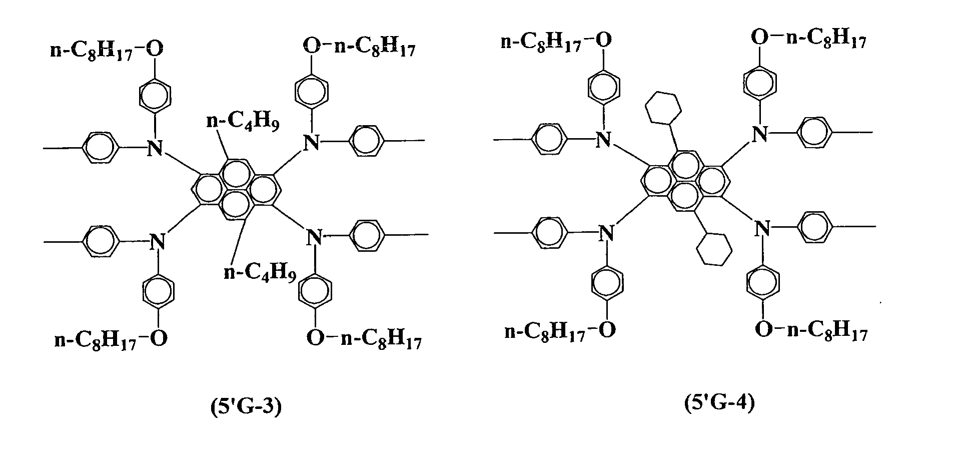

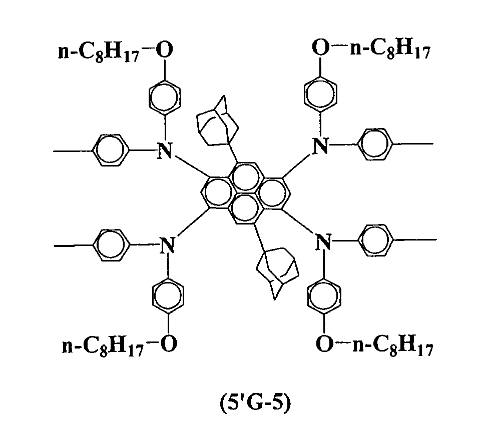

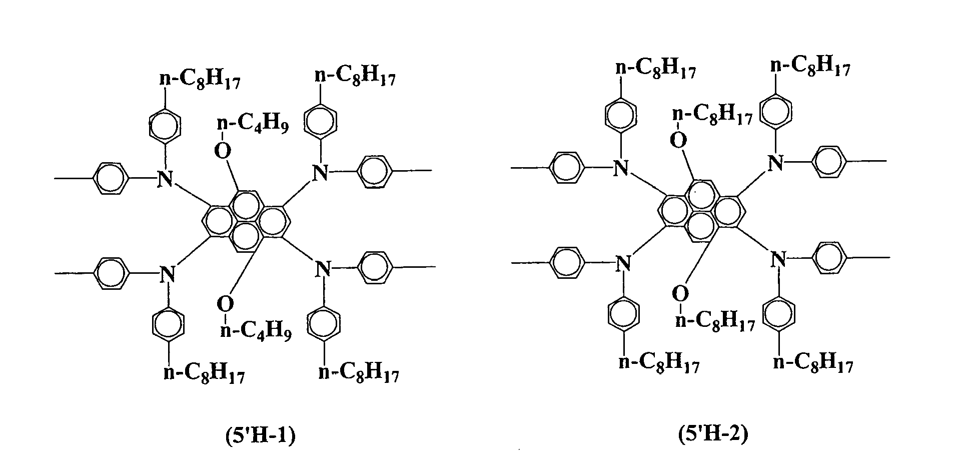

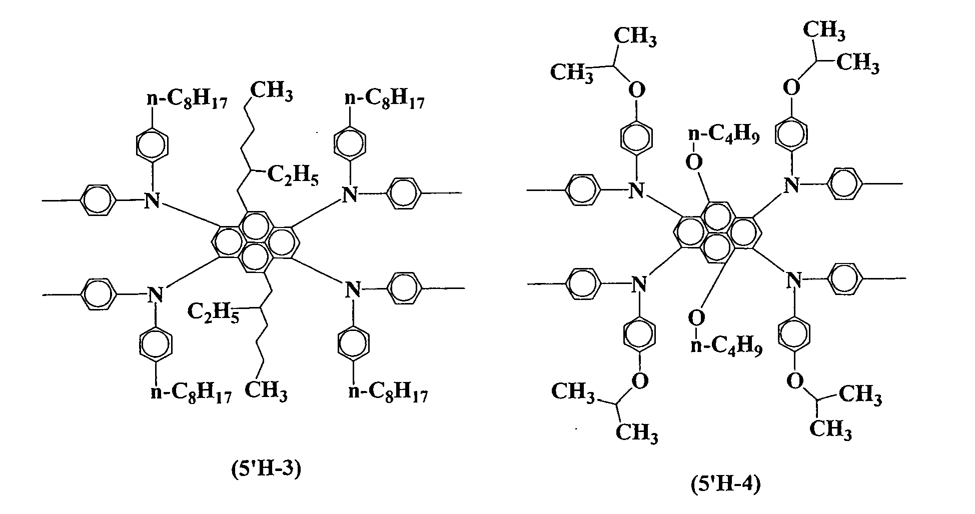

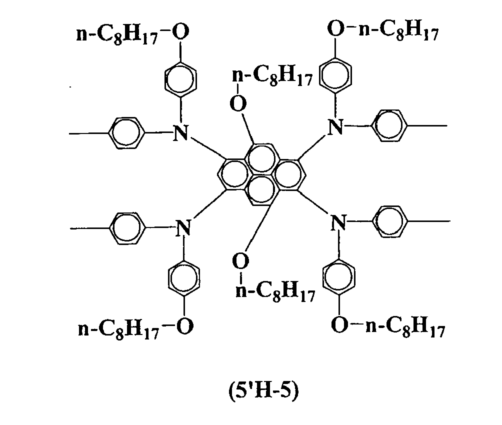

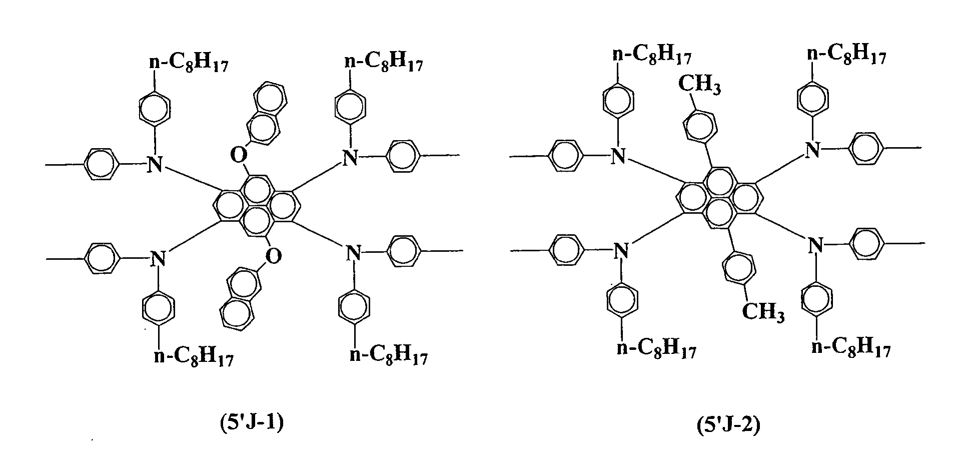

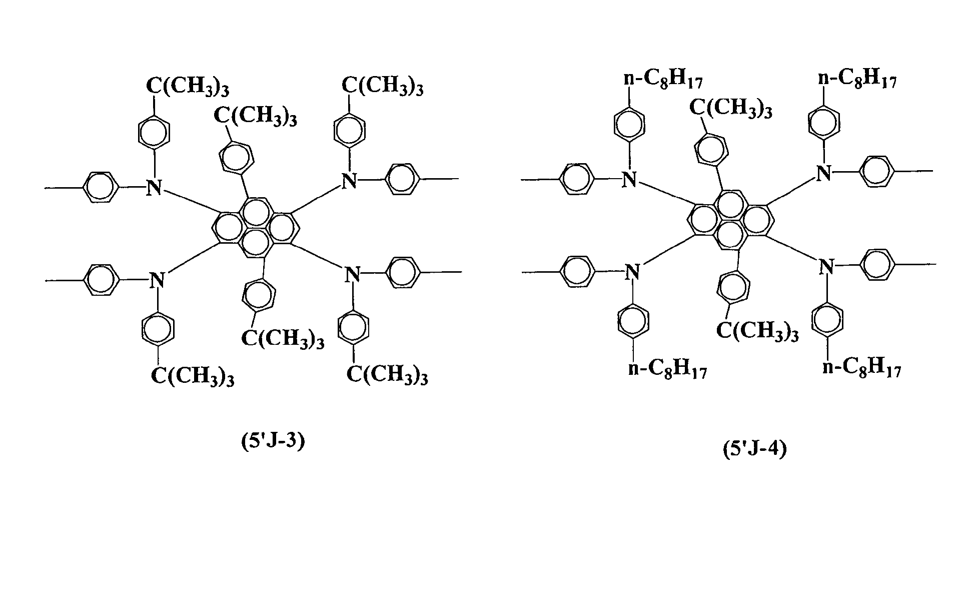

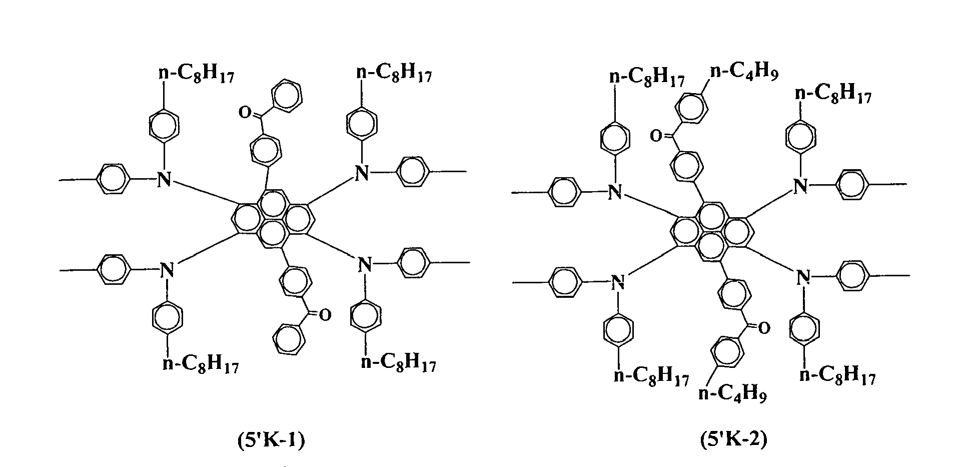

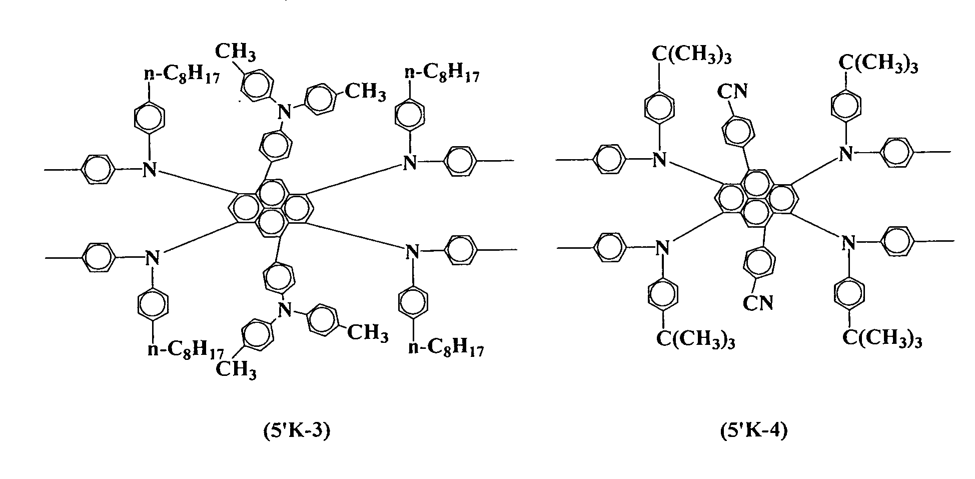

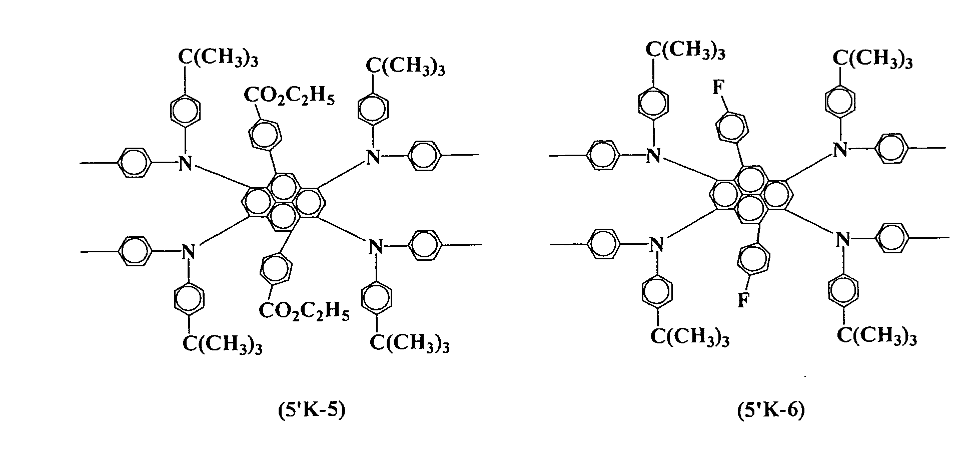

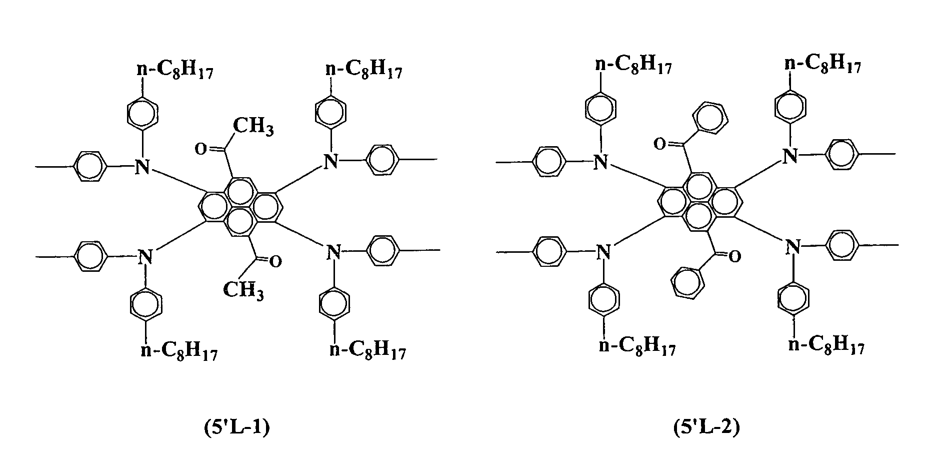

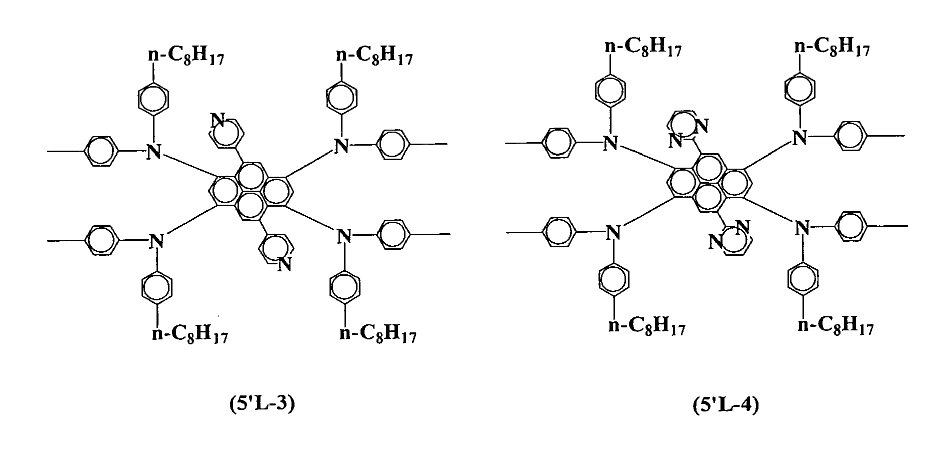

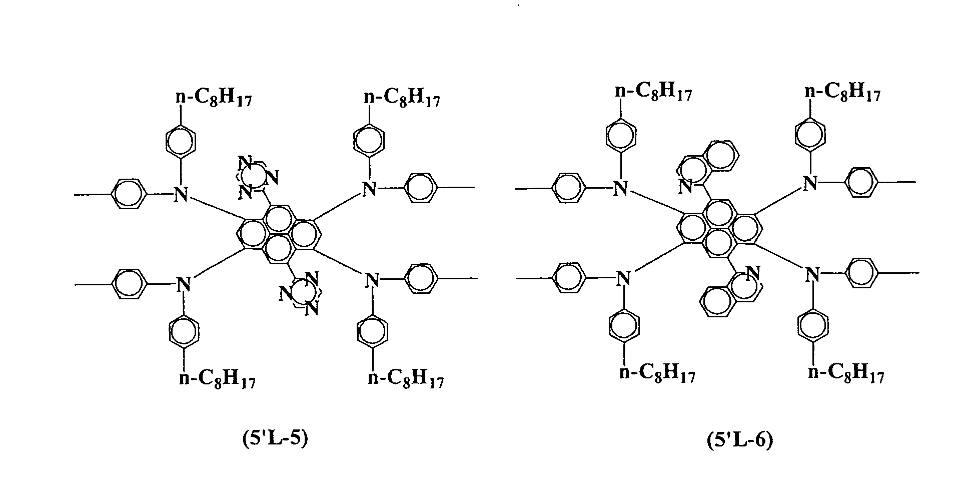

### -- Repeating unit of the formula (2) --

The above-described repeating unit of the formula (2) will be described.

In the above-described formula (2), Ar³'s represent each independently an unsubstituted or substituted arylene group, an unsubstituted or substituted di-valent heterocyclic group or a di-valent group having a metal complex structure.

The above-described arylene group means an atomic group obtained by removing two hydrogen atoms from an aromatic hydrocarbon, and includes those having an independent benzene ring or condensed ring. The above-described arylene group has a carbon number of usually about 6 to 60, preferably 6 to 48, more preferably 6 to 30, further preferably 6 to 18, especially preferably 10 to 14, particularly preferably 7. This carbon number does not include the carbon number of the substituent.

The above-described arylene group includes unsubstituted or substituted phenylene groups such as a 1,4-phenylene group, 1,3-phenylene group, 1,2-phenylene group and the like; unsubstituted or substituted naphthalenediyl groups such as a 1,4-naphthalenediyl group, 1,5-naphthalenediyl group, 2,6-naphthalenediyl group and the like; unsubstituted or substituted anthracenediyl groups such as a 1,4-anthracenediyl group, 1,5-anthracenediyl group, 2,6-anthracenediyl group, 9,10-anthracenediyl group and the like; unsubstituted or substituted phenanthrenediyl groups such as a 2,7-phenanthrenediyl group and the like; unsubstituted or substituted naphthacenediyl groups such as a 1,7-naphthacenediyl group, 2,8-naphthacenediyl group, 5,12-naphthacenediyl group and the like; unsubstituted or substituted fluorenediyl groups such as a 2,7-fluorenediyl group, 3,6-fluorenediyl group and the like; unsubstituted or substituted pyrenediyl groups such as a 1,6-pyrenediyl group, 1,8-pyrenediyl group, 2,7-pyrenediyl group, 4,9-pyrenediyl group and the like; unsubstituted or substituted perylenediyl groups such as a 3,9-perylenediyl group, 3,10-perylenediyl group and the like; etc., and preferably unsubstituted or substituted phenylene groups and unsubstituted or substituted fluorenediyl groups.

When the above-described arylene group has a substituent, examples thereof include preferably those selected from the group consisting of an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group and cyano group, more preferably an alkyl group, alkoxy group, aryl group, aryloxy group, substituted amino group or mono-valent heterocyclic group, further preferably an alkyl group, alkoxy group or aryl group. The definitions and examples of the above-described alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group and cyano group are the same as the definitions and examples explained in the section of a substituent when the above-described Z has a substituent.

The above-described di-valentheterocyclic group has a carbon number of usually about 4 to 60, preferably 4 to 48, more preferably 4 to 30, further preferably 6 to 22, especially preferably 6 to 12, particularly preferably 12. This carbon number does not include the carbon number of the substituent.. The above-described di-valentheterocyclic group includes unsubstituted or substituted pyridinediyl groups such as a 2,5-pyridinediyl group, 2,6-pyridinediyl group and the like; unsubstituted or substituted thiophenediyl groups such as a 2,5-thiophenediyl group and the like; unsubstituted or substituted furandiyl groups such as a 2,5-furandiyl group and the like; unsubstituted or substituted quinolinediyl groups such as a 2,6-quinolinediyl group and the like; unsubstituted or substituted isoquinolinediyl groups such as a 1,4-isoquinolinediyl group, 1,5-isoquinolinediyl group and the like; unsubstituted or substituted quinoxalinediyl groups such as a 5,8-quinoxalinediyl group and the like; unsubstituted or substituted benzo[1,2,5]thiadiazolediyl groups such as a 4,7-benzo[1,2,5]thiadiazolediyl group and the like; unsubstituted or substituted benzothiazolediyl groups such as a 4,7-benzothiazolediyl group and the like; unsubstituted or substituted carbazolediyl groups such as a 2,7-carbazolediyl group, 3,6-carbazolediyl group and the like; unsubstituted or substituted phenoxazinediyl groups such as a 3,7-phenoxazinediyl group and the like; unsubstituted or substituted phenothiazinediyl groups such as a 3,7-phenothiazinediyl group and the like; unsubstituted or substituted dibenzosilolediyl groups such as a 2,7-dibenzosilolediyl group and the like; etc., and preferably an unsubstituted or substituted benzo[1,2,5]thiadiazolediyl group, an unsubstituted or substituted phenoxazinediyl group, an unsubstituted or substituted phenothiazinediyl group.

When the above-described di-valent heterocyclic group has a substituent, examples thereof include preferably those selected from the group consisting of an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group and cyano group, more preferably an alkyl group, alkoxy group, aryl group, aryloxy group, substituted amino group or mono-valent heterocyclic group, further preferably an alkyl group, alkoxy group or aryl group. The definitions and examples of the above-described alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group and cyano group are the same as the definitions and examples explained in the section of a substituent when the above-described Z has a substituent.

The above-described di-valent group having a metal complex structure means an atomic group remaining after removal of two hydrogen atoms from an organic ligand of a metal complex having the organic ligand and a center metal. The organic ligand has a carbon number of usually about 4 to 60. The above-described organic ligand includes 8-quinolinol and derivatives thereof, benzoquinolinol and derivatives thereof, 2-phenylpyridine and derivatives thereof, 2-phenylbenzothiazole and derivatives thereof, 2-phenylbenzoxazole and derivatives thereof, porphyrin and derivatives thereof, and the like.

Examples of the center metal of the above-described metal complex include aluminum, zinc, beryllium, iridium, platinum, gold, europium, terbium and the like.

The above-described metal complex includes metal complexes known as low molecular weight fluorescent materials and phosphorescent materials, and triplet light emitting complexes, and the like.

As the above-described di-valent group having a metal complex structure, the following formulae 126 to 132 are exemplified.

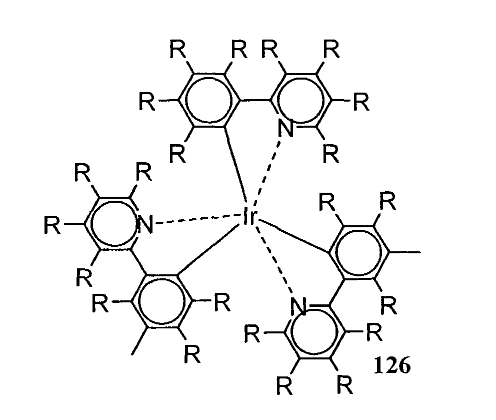

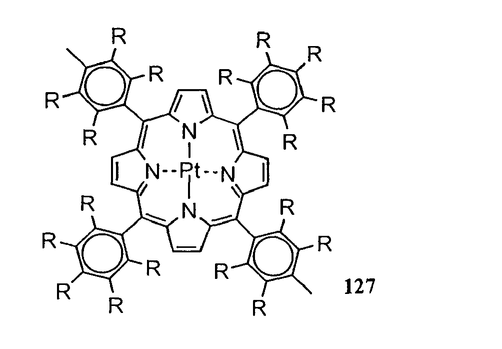

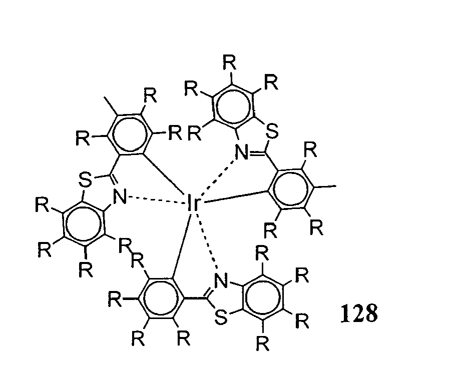

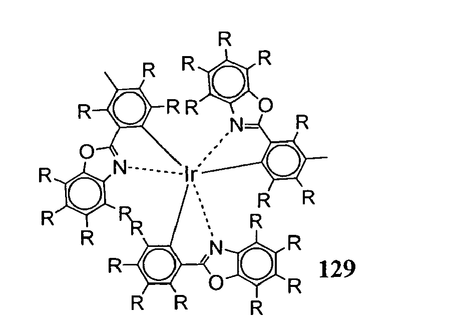

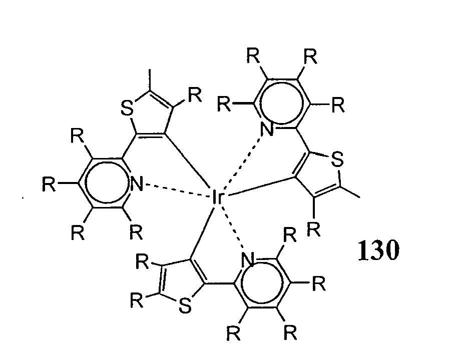

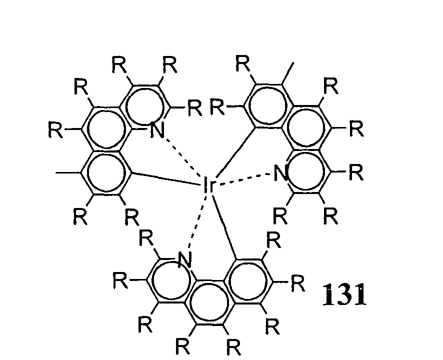

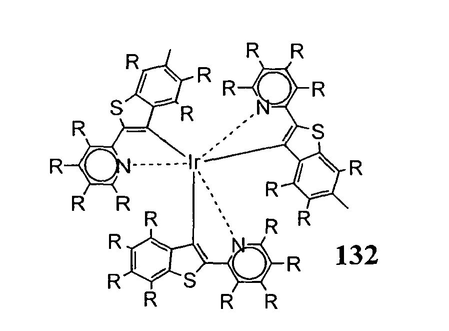

In the above-described formulae 126 to 132, R's represent each independently a hydrogen atom, alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group or cyano group. The carbon atom in the above-described di-valent groups of the formulae 126 to 132 may be substituted with a nitrogen atom, oxygen atom or sulfur atom, and the hydrogen atom in them may be substituted with a fluorine atom.

The definitions and examples of the above-described alkyl group, alkoxy group, alkylthio group, aryl group, aryloxy group, arylthio group, arylalkyl group, arylalkoxy group, arylalkylthio group, arylalkenyl group, arylalkynyl group, substituted amino group, substituted silyl group, halogen atom, acyl group, acyloxy group, imine residue, amide group, acid imide group, mono-valent heterocyclic group, substituted carboxyl group are the same as the definitions and examples explained in the section of a substituent when the above-described Z has a substituent.

In the above-described formula (2), Ar³ represents preferably an unsubstituted or substituted 1,4-phenylene group, an unsubstituted or substituted 1,3-phenylene group, an unsubstituted or substituted 1,4-naphthalenediyl group, an unsubstituted or substituted 1,5-naphthalenediyl group, an unsubstituted or substituted 2,6-naphthalenediyl group, an unsubstituted or substituted 9,10-anthracenediyl group, an unsubstituted or substituted 2,7-phenanthrylene group, an unsubstituted or substituted 5,12-naphthacenylene group, an unsubstituted or substituted 2,7-fluorenediyl group, an unsubstituted or substituted 3,6-fluorenediyl group, an unsubstituted or substituted 1,6-pyrenediyl group, an unsubstituted or substituted 1,8-pyrenediyl group, an unsubstituted or substituted 3,9-perylenediyl group, an unsubstituted or substituted 3,10-perylenediyl group, an unsubstituted or substituted 2,6-quinolinediyl group, an unsubstituted or substituted 1,4-isoquinolinediyl group, an unsubstituted or substituted 1,5-isoquinolinediyl group, an unsubstituted or substituted 5,8-quinoxalinediyl group, an unsubstituted or substituted 4,7-benzo[1,2,5]thiadiazolediyl group, an unsubstituted or substituted 3,7-phenoxazinediyl group or an unsubstituted or substituted 3,7-phenothiazinediyl group, more preferably an unsubstituted or substituted 1,4-phenylene group, an unsubstituted or substituted 1,4-naphthalenediyl group, an unsubstituted or substituted 1,5-naphthalenediyl group, an unsubstituted or substituted 2,6-naphthalenediyl group, an unsubstituted or substituted 9,10-anthracenediyl group, an unsubstituted or substituted 2,7-fluorenediyl group, an unsubstituted or substituted 1,6-pyrenediyl group, an unsubstituted or substituted 3,9-perylenediyl group, an unsubstituted or substituted 3,10-perylenediyl group, an unsubstituted or substituted 2,6-quinolinediyl group, an unsubstituted or substituted 1,4-isoquinolinediyl group, an unsubstituted or substituted 5,8-quinoxalinediyl group, an unsubstituted or substituted 4,7-benzo[1,2,5]thiadiazolediyl group, an unsubstituted or substituted 3,7-phenoxazinediyl group or an unsubstituted or substituted 3,7-phenothiazinediyl group, further preferably an unsubstituted or substituted 1,4-phenylene group, an unsubstituted or substituted 1,4-naphthalenediyl group, an unsubstituted or substituted 1,5-naphthalenediyl group, an unsubstituted or substituted 2,6-naphthalenediyl group, an unsubstituted or substituted 9,10-anthracenediyl group, an unsubstituted or substituted 2,7-fluorenediyl group, an unsubstituted or substituted 5,8-quinoxalinediyl group, an unsubstituted or substituted 4,7-benzo[1,2,5]thiadiazolediyl group, an unsubstituted or substituted 3,7-phenoxazinediyl group or an unsubstituted or substituted 3,7-phenothiazinediyl group, especially preferably an unsubstituted or substituted 1,4-phenylene group, an unsubstituted or substituted 2,7-fluorenediyl group, an unsubstituted or substituted 4,7-benzo[1,2,5]thiadiazolediyl group, an unsubstituted or substituted 3,7-phenoxazinediyl group or an unsubstituted or substituted 3,7-phenothiazinediyl group, particularly preferably a di-valent group of the following formulae (6) to (10). 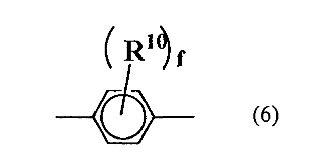 (wherein, R¹⁰ represents an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group or cyano group. f represents an integer of 0 to 4. When there exist a plurality of R¹⁰'s, they may mutually be the same or different.) 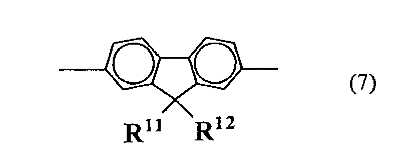 (wherein, R¹¹ and R¹² represent each independently a hydrogen atom, alkyl group, aryl group, arylalkyl group or mono-valent heterocyclic group.) 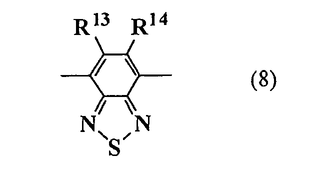 (wherein, R¹³ and R¹⁴ represent each independently a hydrogen atom, alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group or cyano group.) 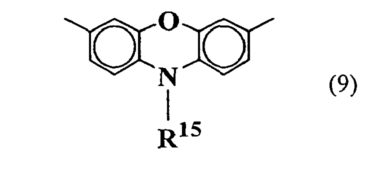 (wherein, R¹⁵ represents a hydrogen atom, alkyl group, aryl group, mono-valent heterocyclic group or arylalkyl group.) 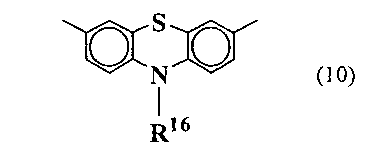 (wherein, R¹⁶ represents a hydrogen atom, alkyl group, aryl group,, mono-valent heterocyclic group or arylalkyl group.)

In the above-described formula (6), R¹⁰ represents preferably an alkyl group, alkoxy group, aryl group, aryloxy group, arylalkyl group, arylalkoxy group, arylalkenyl group, arylalkynyl group, substituted amino group, acyl group or mono-valent heterocyclic group, more preferably an alkyl group, alkoxy group, aryl group, aryloxy group, substituted amino group, acyl group or mono-valent heterocyclic group, further preferably an alkyl group, alkoxy group, aryl group or mono-valent heterocyclic group, particularly preferably an alkyl group, alkoxy group or aryl group. The definitions and examples of the above-described alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group and cyano group are the same as the definitions and examples explained in the section of a substituent when the above-described Z has a substituent.

In the above-described formula (6), f represents preferably an integer of 1 to 4, more preferably an integer of 1 to 3, further preferably 1 or 2, particularly preferably 2.

In the above-described formula (7), R¹¹ and R¹² represent each independently preferably an alkyl group, aryl group or mono-valent heterocyclic group, more preferably an alkyl group or aryl group. The definitions and examples of the alkyl group, aryl group, arylalkyl group and mono-valent heterocyclic group are the same as the definitions and examples explained in the section of a substituent when the above-described Z has a substituent

In the above-described formula (8), R¹³ and R¹⁴ represent each independently preferably a hydrogen atom, alkyl group, alkoxy group, aryl group, aryloxy group, arylalkyl group, arylalkoxy group, substituted amino group, acyl group or mono-valent heterocyclic group, more preferably a hydrogen atom, alkyl group, alkoxy group, aryl group, aryloxy group or mono-valent heterocyclic group, further preferably a hydrogen atom or alkyl group, particularly preferably a hydrogen atom. The definitions and examples of the above-described alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group and cyano group are the same as the definitions and examples explained in the section of a substituent when the above-described Z has a substituent.

In the above-described formula (9), R¹⁵ represents preferably an alkyl group, aryl group or mono-valent heterocyclic group, more preferably an alkyl group or aryl group, further preferably an aryl group. The definitions and examples of the above-described alkyl group, aryl group, mono-valent heterocyclic group and arylalkyl group are the same as the definitions and examples explained in the section of a substituent when the above-described Z has a substituent.

In the above-described formula (10), R¹⁶ represents preferably an alkyl group, aryl group or mono-valent heterocyclic group, more preferably an alkyl group or aryl group, further preferably an aryl group. The definitions and examples of the above-described alkyl group, aryl group, mono-valent heterocyclic group and arylalkyl group are the same as the definitions and examples explained in the section of a substituent when the above-described Z has a substituent.

### -- Repeating unit of the formula (3) --

The above-described repeating unit of the formula (3) will be described.

In the above-described formula (3), Ar⁴, Ar⁵ and Ar⁶ represent each independently an unsubstituted or substituted arylene group, an unsubstituted or substituted di-valent heterocyclic group or an unsubstituted or substituted di-valent group having two aromatic rings connected via a single bond.

The arylene group represented by Ar⁴, Ar⁵ and Ar⁶ means an atomic group obtained by removing two hydrogen atoms from an aromatic hydrocarbon, and includes those having an independent benzene ring or condensed ring. Here, Ar⁵ represents usually a group other than "unsubstituted or substituted pyrenediyl group". The above-described arylene group has a carbon number of usually about 6 to 60, preferably 6 to 48, more preferably 6 to 30, further preferably 6 to 18, especially preferably 6 to 10, particularly preferably 6. This carbon number does not include the carbon number of the substituent. The above-described arylene group includes a 1,3-phenylene group, 1,4-phenylene group, 1,4-naphthalenediyl group, 2,6-naphthalenediyl group, 9,10-anthracenediyl group, 2,7-phenanthrenediyl group, 5,12-naphthacenediyl group, 2,7-fluorenediyl group, 3,8-perylenediyl group and the like.

The di-valent heterocyclic group represented by Ar⁴, Ar⁵ and Ar⁶ has a carbon number of usually about 4 to 60, preferably 4 to 20, more preferably 4 to 9, further preferably 4 to 5. The above-described di-valent heterocyclic group includes a 2,5-thiophenediyl group, N-methyl-2,5-pyrrolediyl group, 2,5-furandiyl group, 2,5-pyridinediyl group, 2,6-pyridinediyl group, 2,4-quinolinediyl group, 2,6-quinolinediyl group, 1,4-isoquinolinediyl group, 1,5-isoquinolinediyl group, 4,7-benzo[1,2,5]thiadiazolediyl group, 3,7-phenoxazinediyl group, 3,6-carbazolediyl group and the like.

The di-valent group having two aromatic rings connected via a single bond represented by Ar⁴, Ar⁵ and Ar⁶ includes groups of the following formula (3A-1) to formula (3A-8).

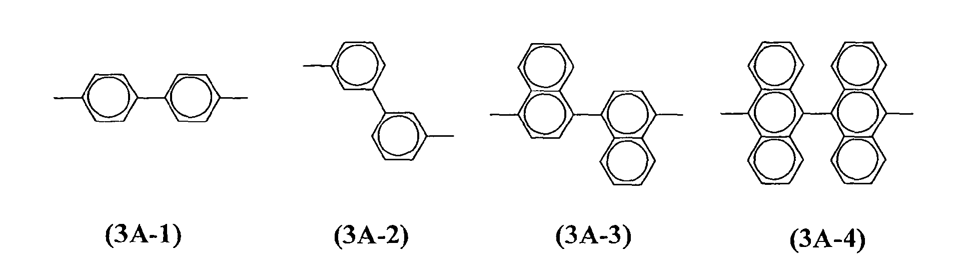

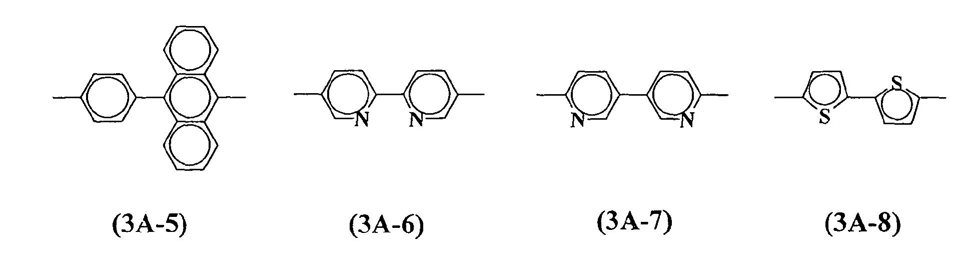

When Ar⁴, Ar⁵ and Ar⁶ have a substituent, examples thereof include preferably those selected from the group consisting of an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group and cyano group, more preferably those selected from the group consisting of an alkyl group, alkoxy group, aryl group, aryloxy group, arylalkyl group, arylalkoxy group, substituted amino group, substituted silyl group, acyl group, substituted carboxyl group and cyano group, further preferably those selected from the group consisting of an alkyl group, alkoxy group, aryl group, aryloxy group, arylalkyl group, arylalkoxy group and substituted carboxyl group, especially preferably those selected from the group consisting of an alky group, alkoxy group and aryl group, particularly preferably an alkyl group. The definitions and examples of the above-described alkyl group, alkoxy group, alkylthio group, aryl group, aryloxy group, arylthio group, arylalkyl group, arylalkoxy group, arylalkylthio group, arylalkenyl group, arylalkynyl group, substituted amino group, substituted silyl group, halogen atom, acyl group, acyloxy group, imine residue, amide group, acid imide group, mono-valent heterocyclic group and substituted carboxyl group are the same as the definitions and examples explained in the section of a substituent when the above-described Z has a substituent.

Ar⁴ and Ar⁶ represent preferably an unsubstituted or substituted arylene group, more preferably an unsubstituted or substituted 1,3-phenylene group, an unsubstituted or substituted 1,4-phenylene group, an unsubstituted or substituted 1,4-naphthalenediyl group, an unsubstituted or substituted 2,6-naphthalenediyl group, an unsubstituted or substituted 2,5-pyridinediyl group, an unsubstituted or substituted 1,4-isoquinolinediyl group or an unsubstituted or substituted group of the formula (3A-1), further preferably an unsubstituted or substituted 1,4-phenylene group or an unsubstituted or substituted 1,4-naphthalenediyl group, particularly preferably an unsubstituted or substituted 1,4-phenylene group.

Ar⁵ represents preferably an unsubstituted or substituted 1,3-phenylene group, an unsubstituted or substituted 1,4-phenylene group, an unsubstituted or substituted 1,4-naphthalenediyl group, an unsubstituted or substituted 2,7-fluorenediyl group, an unsubstituted or substituted 2,5-pyridinediyl group, an unsubstituted or substituted 1,4-isoquinolinediyl group, an unsubstituted or substituted 4,7-benzo[1,2,5]thiadiazolediyl group, an unsubstituted or substituted 3,7-phenoxazinediyl group, an unsubstituted or substituted group of the formula (3A-1) or an unsubstituted or substituted group of the formula (3A-4), more preferably an unsubstituted or substituted 1,4-phenylene group, an unsubstituted or substituted 1,4-naphthalenediyl group, an unsubstituted or substituted 2,7-fluorenediyl group, an unsubstituted or substituted 2,5-pyridinediyl group, an unsubstituted or substituted 1,4-isoquinolinediyl group or an unsubstituted or substituted group of the formula (3A-1), further preferably an unsubstituted or substituted 1,4-phenylene group or an unsubstituted or substituted group of the formula (3A-1).

R¹ and R² represent each independently preferably an alkyl group, aryl group or mono-valent heterocyclic group, more preferably an alkyl group or aryl group, further preferably an aryl group. The definitions and examples of the above-described alkyl group, aryl group, mono-valent heterocyclic group and arylalkyl group are the same as the definitions and examples explained in the section of a substituent when the above-described Z has a substituent.

As the above-described repeating unit of the formula (3), the following formulae (3B-1) to (3B-4) are preferable. In these formulae, R^{a} represents a hydrogen atom, alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group or cyano group. A plurality of R^{a}'s may be the same or different. The definitions and examples of the above-described alkyl group, alkoxy group, alkylthio group, aryl group, aryloxy group, arylthio group, arylalkyl group, arylalkoxy group, arylalkylthio group, arylalkenyl group, arylalkynyl group, substituted amino group, substituted silyl group, halogen atom, acyl group, acyloxy group, imine residue, amide group, acid imide group, mono-valent heterocyclic group and substituted carboxyl group are the same as the definitions and examples explained in the section of a substituent when the above-described Z has a substituent.

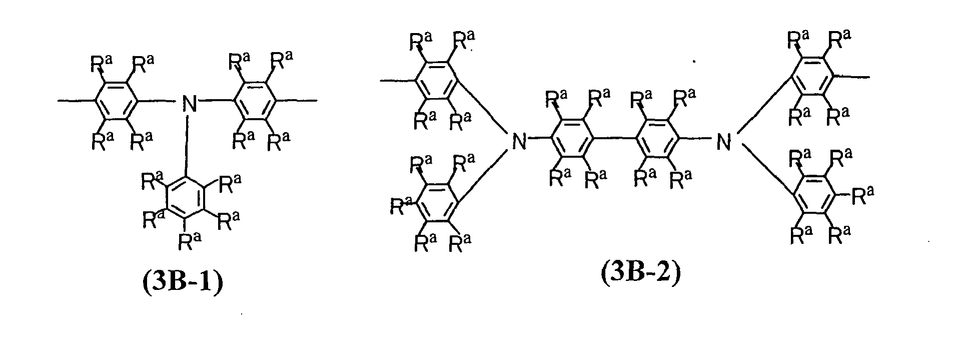

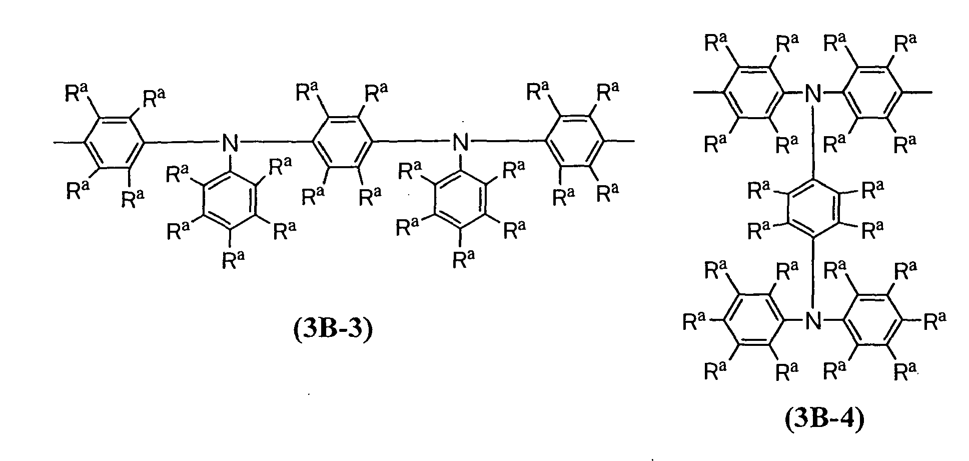

### -- Repeating unit of the formula (4) --

The above-described repeating unit of the formula (4) will be described.

Ar⁷ represents an unsubstituted or substituted arylene group, an unsubstituted or substituted di-valent heterocyclic group or a di-valent group having a metal complex structure.

Here, the definitions and examples of the unsubstituted or substituted arylene group, unsubstituted or substituted di-valent heterocyclic group or di-valent group having a metal complex structure are the same as the definitions and examples explained in the above-described section of Ar³.

X¹ represents -CR³=CR⁴- or -C=C-.

R³ and R⁴ represent each independently preferably a hydrogen atom, alkyl group or aryl group, more preferably a hydrogen atom or aryl group. The group represented by R³ and R⁴ may have a substituent. The definitions and examples of the above-described alkyl group, aryl group, mono-valent heterocyclic group and substituted carboxyl group are the same as the definitions and examples explained in the section of a substituent when the above-described Z has a substituent.

As preferable examples of the above-described repeating unit of the formula (4), the following formulae (4A-1) to (4A-11) are mentioned.

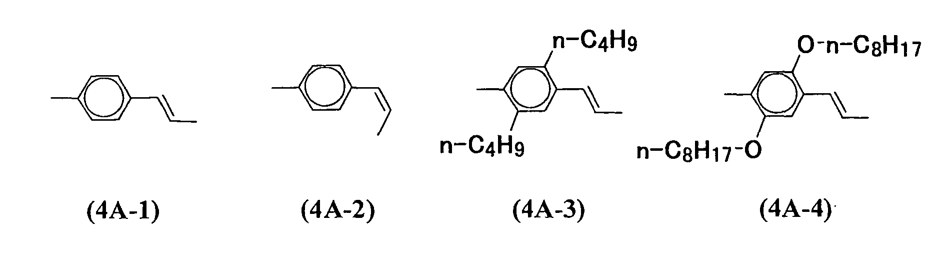

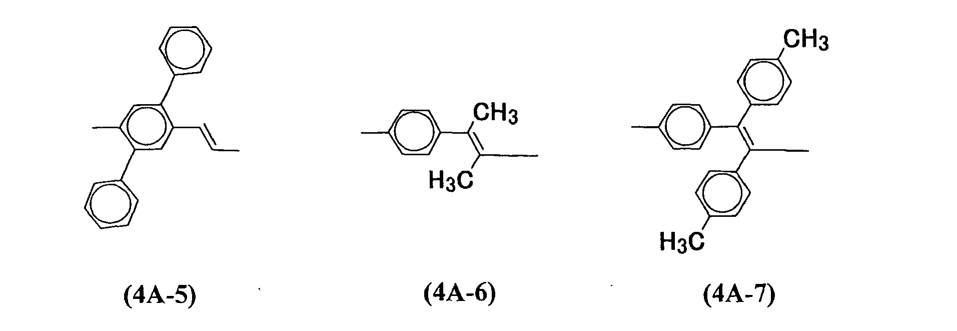

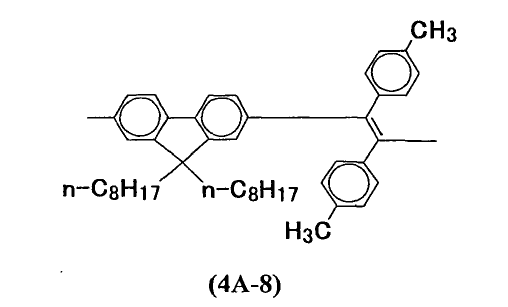

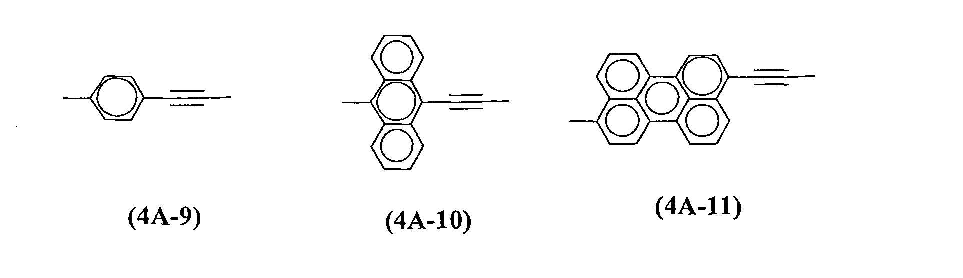

### -- Relation of constitutional unit/repeating unit --

In the polymer compound of the present invention, the total mole number of the constitutional unit of the formula (1), the repeating unit of the formula (2), the repeating unit of the formula (3) and the repeating unit of the formula (4) with respect to the total mole number of all constitutional units and all repeating units is preferably 90 to 100%, more preferably 95 to 100%, further preferably 98 to 100%, particularly preferably 100%, from the standpoint of the heat resistance of the polymer compound, device properties (light emission efficiency, life) and the like.

In the polymer compound of the present invention, the total mole number of the constitutional unit of the formula (1), the repeating unit of the formula (2) and the repeating unit of the formula (3) with respect to the total mole number of all constitutional units and all repeating units is preferably 90 to 100%, more preferably 95 to 100%, further preferably 98 to 100%, particularly preferably 100%, from the standpoint of the heat resistance of the polymer compound, device properties (light emission efficiency, life) and the like.

As the polymer compound of the present invention, preferable are those containing at least one repeating unit of the formula (1) and at least one selected from the group consisting of a repeating unit of the formula (2) and a repeating unit of the formula (3), more preferable are those containing at least one repeating unit of the formula (1) and at least one repeating unit of the formula (2), and those containing at least one repeating unit of the formula (1), at least one repeating unit of the formula (2) and at least one repeating unit of the formula (3), further preferable are those containing at least one repeating unit of the formula (1) and at least one selected from the group consisting of a repeating unit of the formula (7) and a repeating unit of the formula (9), and those containing at least one repeating unit of the formula (1), at least one repeating unit of the formula (3) and at least one selected from the group consisting of a repeating unit of the formula (7) and a repeating unit of the formula (9), and particularly preferable are those containing at least one repeating unit of the formula (1), at least one repeating unit of the formula (7) and at least one repeating unit of the formula (9), and those containing at least one repeating unit of the formula (1), at least one repeating unit of the formula (7) and at least one repeating unit of the formulae (3B-1) to (3B-4), from the standpoint of device properties (light emission efficiency) and the like.

The polymer compound of the present invention has a polystyrene-equivalent number average molecular weight (Mn) by gel permeation chromatography (hereinafter, referred to as "GPC") of usually about 1×10³ to 1×10⁸, preferably 1×10⁴ to 1×10⁶. The polystyrene-equivalent weight average molecular weight is usually about 1×10³ to 1×10⁸, and from the standpoint of film formability and from the standpoint of efficiency when made into a device, it is preferably 1×10⁴ to 5×10⁶, more preferably 3×10⁴ to 1×10⁶.

When a polymerization active group remains intact on the end of the polymer compound of the present invention, there is a possibility of lowering of light emission property and life when the polymer compound is used for manufacturing of a light emitting device, thus, it is preferable that the end group is a stable group. Those having a conjugated bond to the main chain are preferable, and for example, structures containing bonding to an aryl group or heterocyclic group via a carbon-carbon bond are mentioned, and substituents described in JP-A No. 9-45478, chemical formula 10, and the like are also mentioned.

In the polymer compound of the present invention, constitutional units of the formula (1), and repeating units of the formulae (2) to (4) may be contained singly or in combination with another or more.

The polymer compound of the present invention may be any copolymer, and for example, may be any of block copolymers, random copolymers, alternative copolymers, graft copolymers and the like.

The polymer compound of the present invention is useful as a light emitting material, charge transporting material or the like, and may be used together with other compound having high molecular weight in use (that is, may be used as a composition described later).

### <Method of producing polymer compound>

Next, preferable methods of producing a polymer compound of the present invention will be described.

The polymer compound of the present invention can be produced by, for example, condensation-polymerizing at least one compound of the above-described formula (a), and at least one selected from the group consisting of a compound of the above-described formula (b-1), a compound of the above-described formula (b-2) and a compound of the above-described formula (b-3).

In the above-described formulae (a), (b-1) to (b-3), (a-2) and (a-3), the halogen atoms represented by Y¹ and X_{A} include a chlorine atom, bromine atom and iodine atom.

In the above-described formulae (a), (b-1) to (b-3), the boronic acid ester residue residue represented by Y¹ includes, for example, groups of the following formulae.

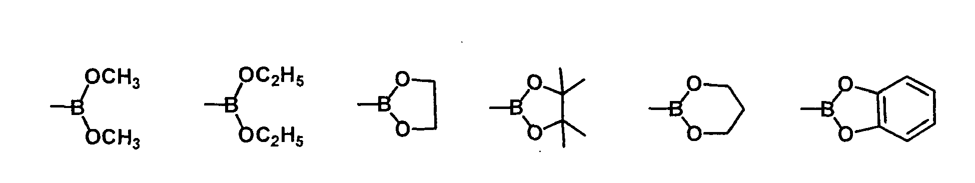

In the above-described formula (a-1), the definitions and examples of the alkyl group and aryl group represented by R^{a} are the same as the definitions and examples explained in the section of a substituent when the above-described Z has a substituent.

Examples of the sulfonate group of the above-described formula (a-1) include a methanesulfonate group, trifluoromethanesulfonate group, phenylsulfonate group, 4-methylphenylsulfonate group and the like.

In the above-described formula (a-4), the definitions and examples of the alkyl group and aryl group represented by R^{a} are the same as the definitions and examples explained in the section of a substituent when the above-described Z has a substituent. The above-described group of the formula (a-4) includes, for example, a trimethylstannanyl group, triethylstannanyl group, tributylstannanyl group and the like.

As the above-described compounds of the formulae (a), (b-1) to (b-3), those previously synthesized and isolated may be used, or they may be prepared in the reaction system and used as they are.

In the above-described formulae (a), (b-1) to (b-3), Y¹ represents preferably a halogen atom, borate residue or boric acid residue from the standpoint of simplicity of synthesis of the compounds of the formulae (a), (b-1) to (b-3) and handling thereof, and the like.

As the condensation polymerization method, there are methods of reacting the compounds of the formulae (a), (b-1) to (b-3), if necessary, using a suitable catalyst and a suitable base.

As the above-described catalyst, for example, catalysts are mentioned composed of a transition metal complex such as palladium complexes such as palladium[tetrakis(triphenylphosphine)], [tris(dibenzylideneacetone)]dipalladium, palladium acetate and the like, nickel complexes such as nickel[tetrakis(triphenylphosphine)], [1,3-bis(diphenylphosphino)propane]dichloronickel, [bis(1,4-cyclooctadiene)]nickel and the like, and if necessary, further of a ligand such as triphenylphosphine, tri(t-butylphosphine), tricyclohexylphosphine, diphenylphosphinopropane, bipyridyl and the like. As the above-described catalyst, those previously synthesized may be used, and those prepared in the reaction system may be used as they are. The above-described catalysts may be used singly or in combination with another or more.

In the case of use of the above-described catalyst, the amount of the transition metal compound with respect to the sum of the mole numbers of the compounds of the formulae (a), (b-1) to (b-3) is preferably 0.00001 to 3 mol equivalent, more preferably 0.00005 to 0.5 mol equivalent, further preferably 0.0001 to 0.2 mol equivalent.

Examples of the above-described base include inorganic bases such as sodium carbonate, potassium carbonate, cesium carbonate, potassium fluoride, cesium fluoride, tripotassium phosphate and the like, and organic bases such as tetrabutylammonium fluoride, tetrabutylammonium chloride, tetrabutylammonium bromide, tetrabutylammonium hydroxide and the like.

In the case of use of the above-described base, it is preferably 0.5 to 20 mol equivalent, more preferably 1 to 10 mol equivalent with respect to the sum of the mole numbers of the compounds of the formulae (a), (b-1) to (b-3).

The above-described condensation polymerization may be carried out in the absence of a solvent or in the presence of a solvent, and usually, it is carried out in the presence of an organic solvent.

The above-described organic solvent includes, for example, toluene, xylene, mesitylene, tetrahydrofuran, 1,4-dioxane, dimethoxyethane, N,N-dimethylacetamide, N,N-dimethylformamide and the like, though it varies depending on the kind and the reaction of the compounds of the formulae (a), (b-1) to (b-3). In general, a deoxidation treatment is desirably carried out for suppressing side reactions. These organic solvents may be used singly or in combination with another or more.

The use amount of the above-described organic solvents is so adjusted that the total concentration of the compounds of the formulae (a), (b-1) to (b-3) is usually 0.1 to 90 wt%, preferably 1 to 50 wt%, more preferably 2 to 30 wt%.

The reaction temperature of the above-described condensation polymerization is preferably -100 °C to 200 °C, more preferably -80 °C to 150 °C, further preferably 0 °C to 120°C.

The above-described reaction time is usually 1 hour or longer, preferably 2 to 500 hours, depending on conditions such as the reaction temperature and the like.

The above-described condensation polymerization is desirably carried out under anhydrous conditions in some cases. For example, when Y¹ in the above-described formulae (a), (b-1) to (b-3) is a group of the above-described formula (a-2), the condensation polymerization is carried out under anhydrous conditions.

The above-described condensation polymerization method includes, for example, a method of polymerization by the Suzuki reaction (Chem. Rev., vol. 95, p. 2457 (1995)), a method of polymerization by the Grignard reaction (Kyoritsu Publication, Polymer Functional Material Series (kobunshi kino zairyo series), vol. 2, Synthesis and Reaction of Polymer (2) (Kobunshi no Gosei to Hanno (2)), p. 432 to 433), a method of polymerization by the Yamamoto Polymerization method (Prog. Polym. Sci., vol. 17, p. 1153 to 1205,1992), and the like.

The post treatment of the above-described condensation polymerization can be carried out by known methods, and for example, there are mentioned methods in which the reaction solution obtained in the above-described condensation polymerization is added to a lower alcohol such as methanol and the like to deposit a precipitate which is then filtrated and dried.

A polymer compound of the present invention is obtained by the above-described post treatment, and when the purity of the polymer compound is low, it may be advantageously purified by usual methods such as re-crystallization, continuous extraction with a Soxlet extractor, column chromatography and the like.

### <Compound useful for production of polymer compound>

As compounds useful for production of the polymer compound, for example, compounds of the above-described formula (11) are mentioned.

In the above-described formula (11), R¹⁷ represents a halogen atom. The halogen atom represented by R¹⁷ includes a fluorine atom, chlorine atom, bromine atom and iodine atom, preferably a bromine atom or iodine atom, more preferably a bromine atom.

h represents an integer of 1 to 4, preferably 1.

i represents an integer of 0 to 4, preferably 1.

In the above-described formula (11), Ar⁸ represents an unsubstituted or substituted alkyl group, an unsubstituted or substituted aryl group or an unsubstituted or substituted mono-valent heterocyclic group. Ar⁸ represents preferably an unsubstituted or substituted aryl group, an unsubstituted or substituted mono-valent heterocyclic group, more preferably an unsubstituted or substituted aryl group.

The definitions and examples of the alkyl group, aryl group and mono-valent heterocyclic group represented by Ar⁸ are the same as the definitions and examples explained in the section of a substituent when the above-described Z has a substituent, and include preferably unsubstituted or substituted aryl groups, more preferably an unsubstituted or substituted phenyl group, an unsubstituted or substituted biphenyl group. When these groups are substituted, they can be substituted by the same substituents optionally carried on Z, and may be substituted with a boric acid residue, or borate residue.

The above-described compound of the formula (11) is preferably a compound of the following formula (12) or the following formula (13). 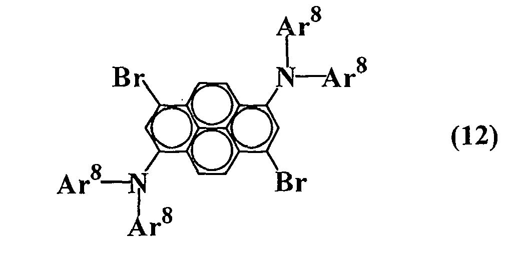 (wherein, Ar⁸ has the same meaning as described above. A plurality of Ar⁸'s may be the same or different.) 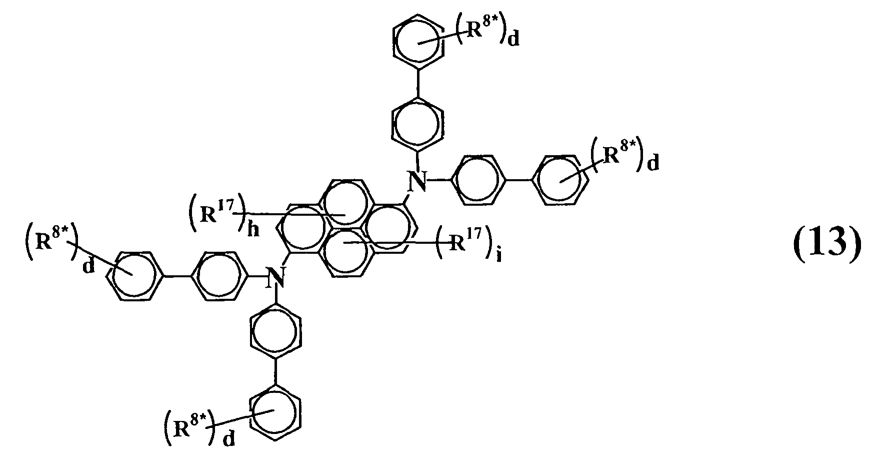 (wherein, R^{8*} represents an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group, cyano group, boric acid residue or borate residue. d represents an integer of 0 to 5. R¹⁷, h and i have the same meanings as described above. When there exist a plurality of R^{8*}_{,}s and R¹⁷'s respectively, they may be mutually the same or different. A plurality of d's may be the same or different.).

In the above-described formula (13), the alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group and cyano group represented by R^{8*} are the same groups as explained and exemplified as R^{8*}

In the above-described formula (13), the boric acid residue or borate residue represented by R^{8*} is the same as explained and exemplified as Y¹.

The above-described compound of the formula (13) is preferably a compound of the following formula (14). 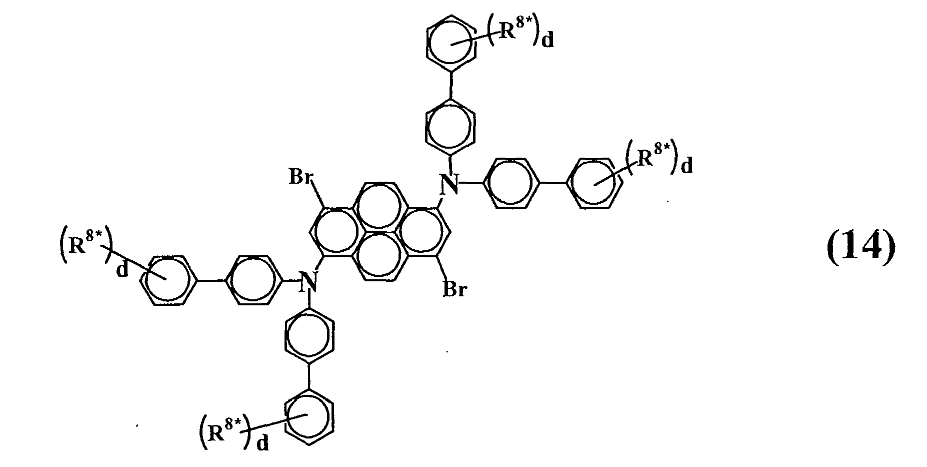 (wherein, R^{8*} and d have the same meanings as described above. When there exist a plurality of R^{8*}'s, they may be mutually the same or different).

As the above-described compound of the formula (14), for example, compounds of the following formulae (14-1) to (14-4) are mentioned.

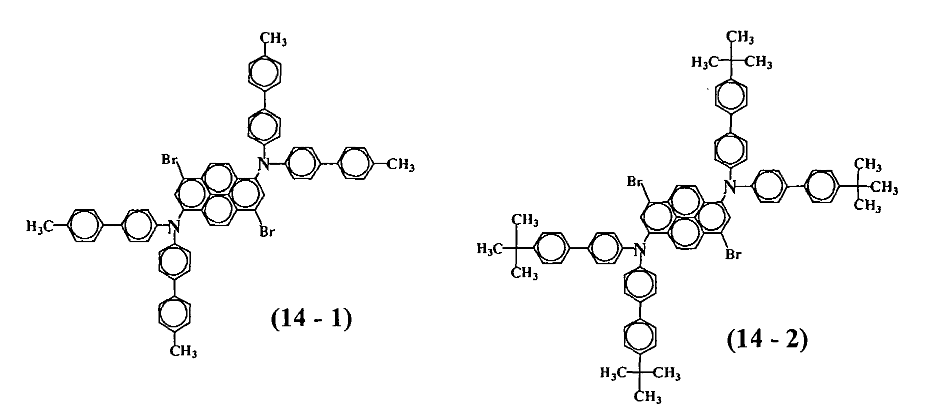

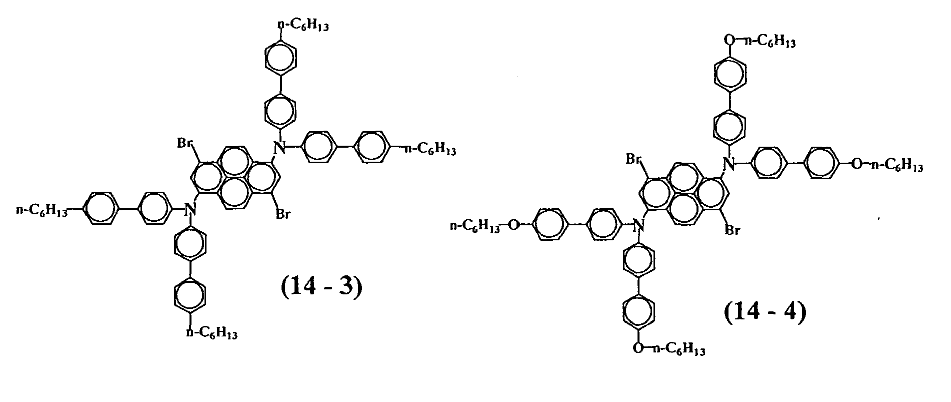

As compounds useful for production of the above-described polymer compound, compounds of the formula (15) are also mentioned. The above-described compound of the formula (15) is particularly useful for production of a compound of the formula (13).

In the above-described formula (15), the definitions and examples of the alkyl group, alkoxy group, alkylthio group, aryl group, aryloxy group, arylthio group, arylalkyl group, arylalkoxy group, arylalkylthio group, arylalkenyl group, arylalkynyl group, substituted amino group, substituted silyl group, acyl group, acyloxy group, imine residue, amide group, acid imide group, mono-valent heterocyclic group, substituted carboxyl group represented by R¹⁸ are the same as the definitions and examples explained in the section of a substituent when the above-described Z has a substituent. R¹⁸ represents preferably an alkyl group, alkoxy group, aryl group or mono-valent heterocyclic group, more preferably an alkyl group or aryl group, further preferably an alkyl group.

j represents an integer of 0 to 4, preferably an integer of 0 to 2, more preferably 0 or 1, particularly preferably 1.

The above-described compound of the formula (15) is preferably a compound of the following formula (16). 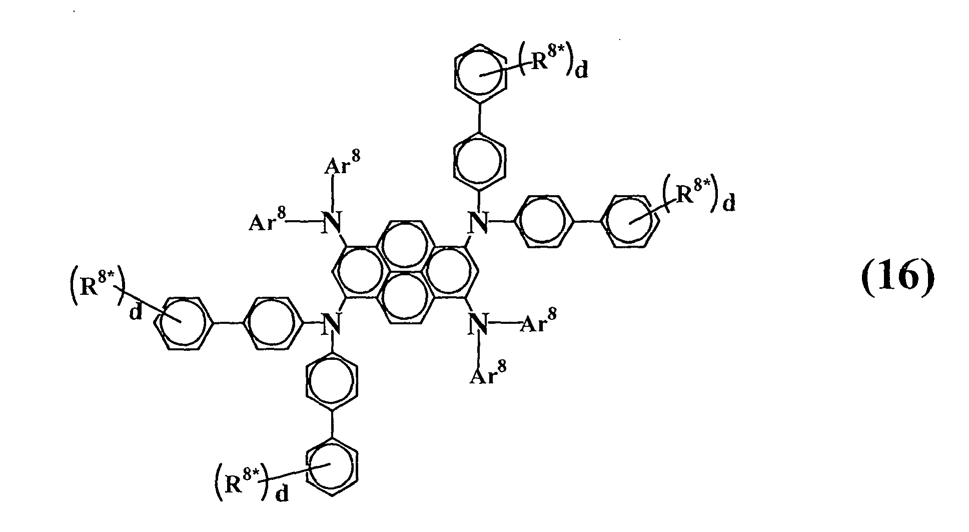 (wherein, Are⁸ R^{8*} and d are as described above. A plurality of Ar⁸'s and d's may each be the same or different. When there exist a plurality of R^{8*}'s, they may be mutually the same or different.).

The above-described compound of the formula (16) is preferably a compound of the following formula (17): 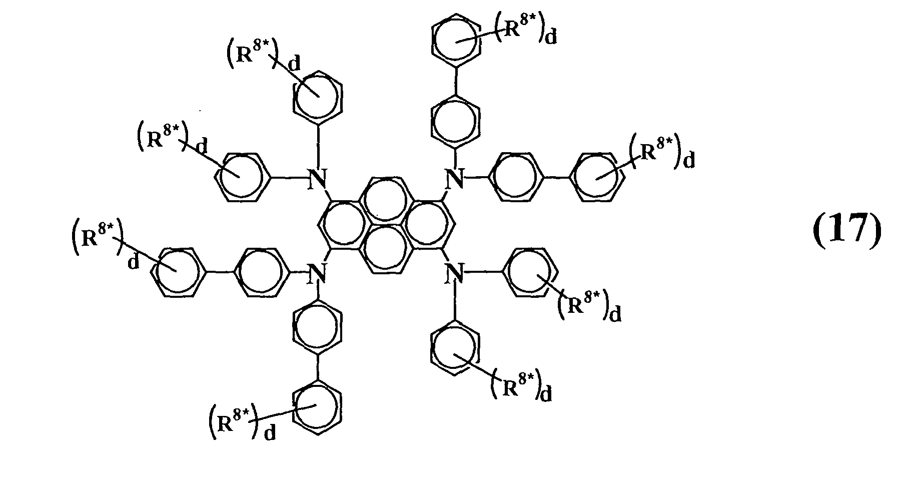 (wherein, R^{8*} and d are as described above. When there exist a plurality of R^{8*}'s, they may be mutually the same or different. A plurality of d's may be the same or different.).

The above-described compound of the formula (17) is preferably a compound of the following formula (18): 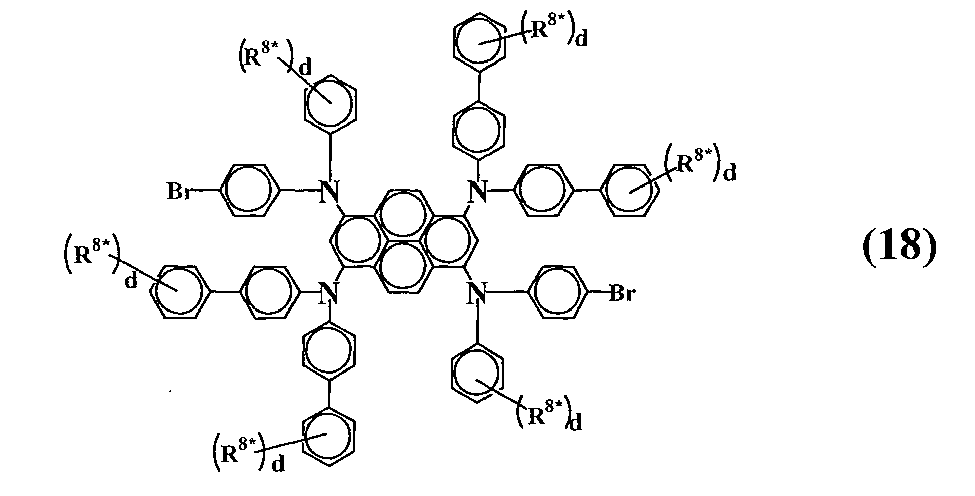 (wherein, R^{8*} and d are as described above. When there exist a plurality of R^{8*}'s, they may be mutually the same or different. A plurality of d's may be the same or different.).

As the above-described compound of the formula (18), for example, compounds of the following formulae (18-1) to (18-4) are mentioned.

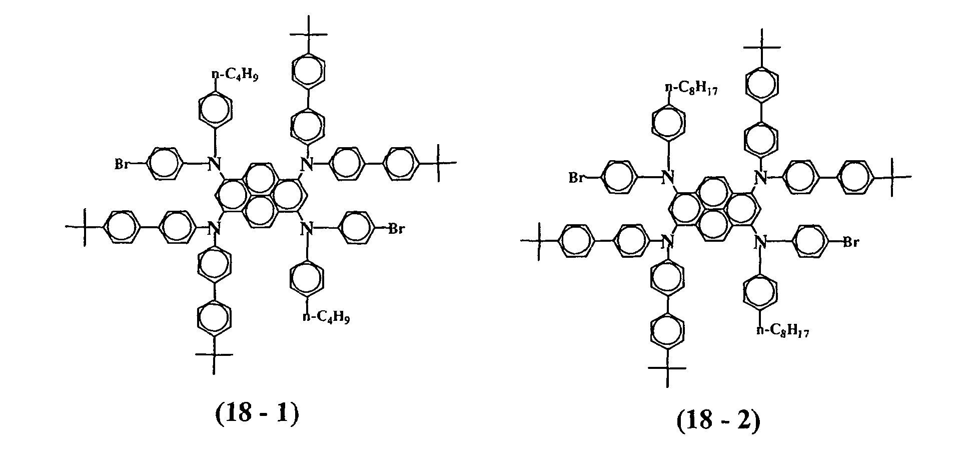

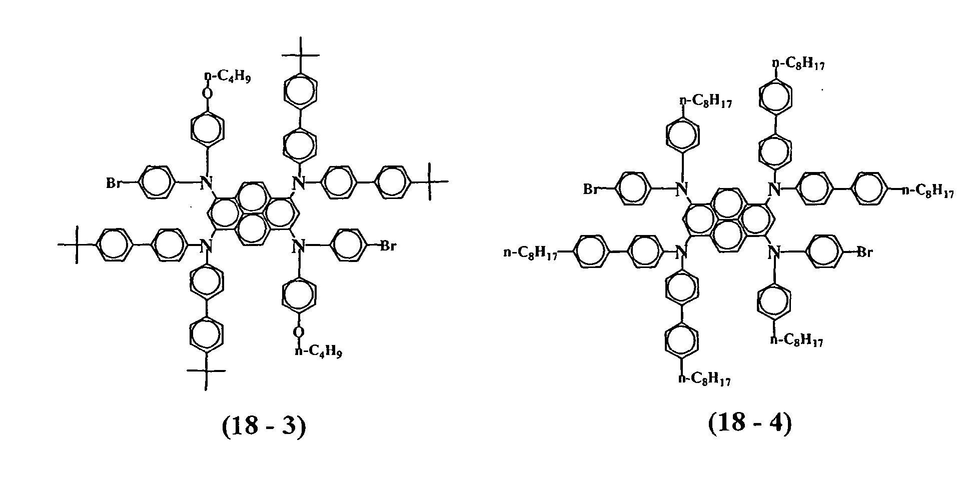

Methods of producing compounds useful for production of the polymer compound of the present invention will be illustrated using a method for producing a compound of the formula (12) as one example.

The above-described compound of the formula (12) is obtained by a production method comprising reaction of a compound of the formula (19) and a brominating agent. In the above-described reaction, the compounds of the formula (19) and brominating agents may each be used singly or in combination with another or more.

As the above-described brominating agent, preferable are bromine, N-bromosuccinimide , tetrabutylammonium tribromide, benzyltrimethylammonium tribromide, and more preferable is tetrabutylammonium tribromide.

### <Composition>

The composition of the present invention comprises at least one material selected from the group consisting of hole transporting material, electron transporting materials and light emitting materials, and a polymer compound of the present invention. This composition can be used, for example, as a light emitting material or charge transporting material.

The content ratio of the above-described at least one material selected from the group consisting of hole transporting material, electron transporting materials and light emitting materials to the polymer compound of the present invention may be determined according to its application, and in the case of application of a light emitting material, the weight of the polymer compound of the present invention is usually 20 to 99 parts by weight, preferably 40 to 95 parts by weight with respect to 100 parts by weight of the weight of the whole composition.

The composition of the present invention can also be prepared in the form of solution (sometimes referred to also as "ink composition") by allowing the composition to contain a solvent such as an organic solvent and the like, as described later. The details will be described later.

The composition of the present invention has a polystyrene-equivalent number average molecular weight of usually about 1×10³ to 1×10⁸, preferably 1×10⁴ to 1×10⁶. The polystyrene-equivalent weight average molecular weight is usually about 1×10³ to 1×10⁸, and from the standpoint of film formability and from the standpoint of light emission efficiency of the resultant device, it is preferably 1×10⁴ to 5×10⁶. Here, the average molecular weight of the composition of the present invention is a value obtained by analyzing the composition by GPC.

### <Solution (ink composition)>

The solution of the present invention comprises a polymer compound of the present invention, and a solvent. In another embodiment, the solution of the present invention comprises a composition of the present invention containing a solvent. This solution is useful for a printing method and the like. The solution of the present invention may also contain a hole transporting material, electron transporting material, light emitting material, stabilizer, thickener (compound of high molecular weight and poor solvent for increasing viscosity), compound of low molecular weight for decreasing viscosity, surfactant (for lowering surface tension), antioxidant and the like, in addition to the above-described polymer compound and solvent.

The proportion of the polymer compound of the present invention in the solution of the present invention is usually 1 to 99.9 parts by weight, preferably 60 to 99.5 parts by weight, more preferably 80 to 99.0 parts by weight with respect to 100 parts by weight of the solution. The viscosity of the solution of the present invention may be regulated according to the kind of a printing method, and when the solution passes through a discharging apparatus such as in an ink jet print method and the like, the viscosity at 25 °C is preferably in the range of 1 to 20 mpa·s, for preventing clogging and flying curving in discharging.

As the above-described high molecular weight compound used as the thickener, those soluble in the same solvent as for the polymer compound of the preset invention and not disturbing light emission and charge transportation may be permissible, and for example, high molecular weight polystyrene, high molecular weight polymethyl methacrylate and the like can be used. These high molecular weight compounds have a polystyrene-equivalent weight average molecular weight of preferably 500000 or more, more preferably 1000000 or more.

Also a poor solvent can be used as the thickening agent. The viscosity can be enhanced by adding a small amount of poor solvent for solid components in the solution. When a poor solvent is added for this purpose, the kind of the solvent and the addition amount thereof may be advantageously selected in a range not causing deposition of solid components in the solution. When also stability in preservation is taken into consideration, the amount of the poor solvent is preferably 50 parts by weight or less, further preferably 30 parts by weight or less with respect to 100 parts by weight of the whole solution.

The above-described antioxidant is used for improving the preservation stability of the solution of the present invention. As the above-described antioxidant, those soluble in the same solvent as for the polymer compound of the present invention and not disturbing light emission and charge transportation may be permissible, and exemplified are phenol antioxidants, phosphorus antioxidants and the like.

As the solvent in the solution of the present invention, those capable of dissolving or uniformly dispersing solid components in the solution are preferable. Exemplified as the solvent are chlorine-based solvents such as chloroform, methylene chloride, 1,2-dichloroethane, 1,1,2-trichloroethane, chlorobenzene, o-dichlorobenzene and the like, aromatic hydrocarbon solvents such as tetrahydrofuran, dioxane, anisole and the like ether solvents, toluene, xylene and the like, aliphatic hydrocarbon solvents such as cyclohexane, methylcyclohexane, n-pentane, n-hexane, n-heptane, n-octane, n-nonane, n-decane and the like, ketone solvents such as acetone, methyl ethyl ketone, cyclohexanone, benzophenone, acetophenone and the like, ester solvents such as ethyl acetate, butyl acetate, ethyl cellosolve acetate, methyl benzoate, phenyl acetate and the like, poly-hydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, 1,2-hexanediol and the like and derivatives thereof, alcohol solvents such as methanol, ethanol, propanol, isopropanol, cyclohexanol and the like, sulfoxide solvents such as dimethyl sulfoxide and the like, amide solvents such as N-methyl-2-pyrrolidone, N,N-dimethylformamide and the like. These solvents may be used singly or in combination with another or more. Of them, aromatic hydrocarbon solvents, ether solvents, aliphatic hydrocarbon solvents, ester solvents and ketone solvents are preferable, and toluene, xylene, ethylbenzene, diethylbenzene, trimethylbenzene, n-propylbenzene, isopropylbenzene, n-butylbenzene, isobutylbenzene, s-butylbenzene, n-hexylbenzene, cyclohexylbenzene, 1-methylnaphthalene, tetralin, anisole, ethoxybenzene, cyclohexane, bicyclohexyl, cyclohexenyl cyclohexanone, n-heptylcyclohexane, n-hexylcyclohexane, decalin, methyl benzoate, cyclohexanone, 2-propylcyclohexanone, 2-heptanone, 3-heptanone, 4-heptanone, 2-octanone, 2-nonanone, 2-decanone, dicyclohexyl ketone, acetophenone and benzophenone are more preferable, from the standpoint of the solubility of the polymer compound of the present invention, uniformity in film formation, viscosity property, and the like.

Two or more of the above-described solvents are preferably used in combination, two to three of the above-described solvents are more preferably used in combination, and two of the above-described solvents are particularly preferably used in combination, from the standpoint of film formability, device properties and the like.

When two solvents are contained in the solution of the present invention, one of them may be solid at 25°C. From the standpoint of film formability, it is preferable that one solvent has a boiling point of 180°C or higher, and it is more preferable that one solvent has a boiling point of 200°C or higher. From the standpoint of viscosity, it is preferable that 1 wt% or more of an aromatic polymer is dissolved at 60°C in both of two solvents, and it is preferable that 1 wt% or more of an aromatic polymer is dissolved at 25°C in one of two solvents.

When two or more solvents are contained in the solution of the present invention, the proportion of a solvent having highest boiling point is preferably 40 to 90 wt%, more preferably 50 to 90 wt%, further preferably 65 to 85 wt% with respect to the weight of all solvents in the solution, from the standpoint of viscosity and film formability.

One or two or more kinds of polymer compounds of the present invention may be contained in the solution of the present invention, and a compound of high molecular weight other than the polymer compound may also be contained in a range not deteriorating device properties and the like.

In the solution of the present invention, water, metals and salts thereof may also be contained in a range of 1 to 1000 ppm by weight. The above-described metals include lithium, sodium, calcium, potassium, iron, copper, nickel, aluminum, zinc, chromium, manganese, cobalt, platinum, iridium and the like. In the solution of the present invention, silicon, phosphorus, fluorine, chlorine, bromine and the like may be contained in a range of 1 to 1000 ppm by weight.

### <Thin film>

The thin film of the present invention comprises a polymer compound of the present invention, and for example, is a luminous thin film, electric conductive thin film, organic semiconductor thin film or the like.

The thin film of the present invention can be manufactured, for example, by a spin coat method, casting method, micro gravure coat method, gravure coat method, bar coat method, roll coat method, wire bar coat method, dip coat method, spray coat method, screen printing method, flexo printing method, offset printing method, inkjet print method, capillary coat method, nozzle coat method and the like, by a screen printing method, flexo printing method, offset printing method, inkjet print method, more preferably by an inkjet method.

When a thin film is fabricated using a solution of the present invention, baking at temperatures of 100°C or higher is possible since the polymer compound of the present invention contained in the solution has high glass transition temperature, and even if baking is performed at a temperature of 130°C, lowering of device properties is small. Depending on the kind of the polymer compound, baking at temperatures of 160°C or higher is also possible.

The luminous thin film has a quantum yield of light emission of preferably 30% or more, more preferably 50% or more, further preferably 60% or more, particularly preferably 70% or more, from the standpoint of luminance of a device, light emission voltage and the like.

The electric conductive thin film has a surface resistance of preferably 1 KΩ/□ or less, more preferably 100 Ω/□ or less, further preferably 10 Ω/□ or less. The electric conductivity can be enhanced by doping the electric conductive thin film with a Lewis acid, ionic compound or the like.

In the organic semiconductor thin film, one larger parameter of electron mobility or hole mobility is preferably 10⁻⁵ cm²/V/s or more, more preferably 10⁻³ cm²/V/s or more, further preferably 10⁻¹ cm²/V/s or more. By forming the organic semiconductor thin film on a Si substrate carrying a gate electrode and an insulation film of SiO₂ and the like formed thereon, and forming a source electrode and a drain electrode with Au and the like, an organic transistor can be obtained.

### <Light emitting device>

Next, the light emitting device of the present invention will be described.

The light emitting device of the present invention has electrodes composed of an anode and a cathode, and an organic layer provided between the electrodes and containing the above-described polymer compound.

The above-described organic layer is preferably at least one layer selected from the group consisting of a light emitting layer, hole transporting layer, hole injection layer, electron transporting layer, electron injection layer and "interlayer" layer, and it is more preferable that a light emitting layer is included in them or the above-described organic layer is a light emitting layer.

The above-mentioned light emitting layer means a layer having a function of light emission. The hole transporting layer means a layer having a function of transporting holes. The electron transporting layer means a layer having a function of transporting electrons. The "interlayer" layer means a layer which is present adjacent to a light emitting layer between the light emitting layer and an anode, and has a function of insulating a light emitting layer and an anode, or a light emitting layer and a hole injection layer or hole transporting layer. The electron transporting layer and the hole transporting layer are collectively called a charge transporting layer. The electro injection layer and the hole injection layer are collectively called a charge injection layer. The light emitting layer, hole transporting layer, hole injection layer, electron transporting layer, electron injection layer and "interlayer" layer may each be composed of only one layer or two or more layers.

When the organic layer is a light emitting layer, the light emitting layer may further contain a hole transporting material, electron transporting material or light emitting material. Here, the light emitting material means a material showing fluorescence and/or phosphorescence (excluding the polymer compound of the present invention).

When the organic layer contains a polymer compound of the present invention and a hole transporting material, the proportion of the hole transporting material is usually 1 to 80 parts by weight, preferably 5 to 60 parts by weight with respect to 100 parts by weight the sum of the polymer compound of the present invention and the hole transporting material.

When the organic layer contains a polymer compound of the present invention and an electron transporting material, the proportion of the electron transporting material is usually 1 to 80 parts by weight, preferably 5 to 60 parts by weight with respect to 100 parts by weight the sum of the polymer compound of the present invention and the electron transporting material.

When the organic layer contains a polymer compound of the present invention and a light emitting material, the proportion of the light emitting material is usually 1 to 80 parts by weight, preferably 5 to 60 parts by weight with respect to 100 parts by weight the sum of the polymer compound of the present invention and the light emitting material.

When the organic layer contains a polymer compound of the present invention, and two or more selected from the group consisting of a hole transporting material, electron transporting material and light emitting material, the proportion of the light emitting material is usually 1 to 50 parts by weight, preferably 5 to 40 parts by weight with respect to 100 parts by weight the sum of them, and the total proportion of the hole transporting material and electron transporting material is usually 1 to 50 parts by weight, preferably 5 to 40 parts by weight with respect to 100 parts by weight the sum of them.

As the above-described hole transporting material, electron transporting material and light emitting material, known low molecular weight compounds, triplet light emitting complexes or high molecular weight compounds can be used, and it is preferable to use high molecular weight compounds.

Examples of the above-described high molecular weight compounds include polyfluorene, its derivatives and copolymers, polyarylene, its derivatives and copolymers, polyarylenevinylene, its derivatives and copolymers, and (co)polymers of aromatic amine and its derivatives described in WO99/13692, WO99/48160, GB2340304A, WO00/53656, WO01/19834, WO00/55927, GB2348316, WO00/4632 WO00/06665, WO99/54943, WO99/54385 US5777070, WO98/06773, WO97/05184, WO00/35987, WO00/53655, WO01/34722, WO99/24526, WO00/22027, WO00/22026, WO98/27136, US573636, WO98/21262, US5741921, WO97/09394, WO96/29356, WO96/10617, EP0707020, WO95/07955, JP-A No. 2001-181618, JP-A No. 2001-123156, JP-A No. 2001-3045, JP-A No. 2000-351967, JP-A No. 2000-303066, JP-A No. 2000-299189, JP-A No. 2000-252065, JP-A No. 2000-136379, JP-A No. 2000-104057, JP-A No. 2000-80167, JP-A No. 10-324870, JP-A No. 10-114891, JP-A No. 9-111233, JP-A No. 9-45478 and the like.

Examples of the above-described low molecular weight compounds include naphthalene derivatives, anthracene and derivatives thereof, perylene and derivatives thereof, dyes such as polymethine dye, xanthene dye, coumarin dye, cyanine dye and the like, metal complexes of 8-hydroxyquinoline and derivatives thereof, aromatic amines, tetraphenylcyclopentadiene and derivatives thereof, tetraphenylbutadiene and derivatives thereof, and the like, and also compounds described in JP-A Nos. 57-51781, 59-194393, and the like.

The above-described triplet light emitting complexes include, for example, Ir(ppy)₃, Btp₂Ir(acac) containing iridium as a center metal, PtOEP containing platinum as a center metal, Eu(TTA)₃phen containing europium as a center metal, and the like, and described, for example, in Nature, (1998), 395, 151, Appl. Phys. Lett. (1999), 75(1), 4, Proc. SPIE-Int. Soc. Opt. Eng. (2001), 4105(Organic Light-Emitting Materials and DevicesIV), 119, J. Am. Chem. Soc., (2001), 123, 4304, Appl. Phys. Lett., (1997), 71(18), 2596, Syn. Met., (1998), 94(1), 103, Syn. Met., (1999), 99(2), 1361, Adv. Mater., (1999), 11(10), 852 , Jpn.J.Appl.Phys.,34, 1883 (1995) and the like.

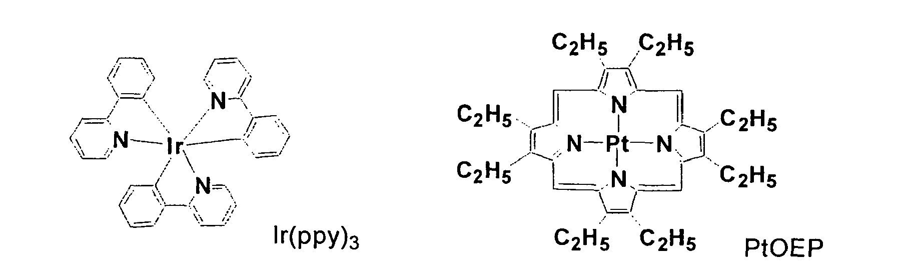

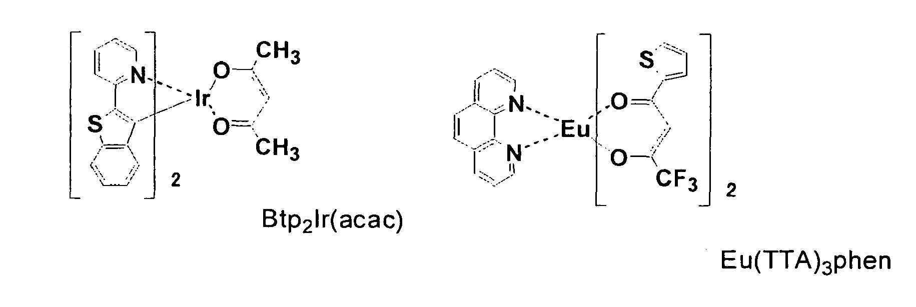

The thickness of the light emitting layer manifests the optimum value varying depending on the material to be used, and may be advantageously selected so as to give optimum driving voltage and light emission efficiency, and it is usually 1 nm to 1 µm, preferably 2 nm to 500 nm, more preferably 5 nm to 200 nm.

As the method for forming the light emitting layer, for example, a method of film formation from a solution is mentioned. As the film formation method from a solution, application methods such as, for example, a spin coat method, casting method, micro gravure coat method, gravure coat method, bar coat method, roll coat method, wire bar coat method, dip coat method, spray coat method, screen printing method, flexo printing method, offset printing method, inkjet print method, capillary coat method, nozzle coat method and the like can be used, and preferable from the standpoint of easiness of pattern formation and multi-color separate painting are printing methods such as a screen printing method, flexo printing method, offset printing method, inkjet print method and the like.

The light emitting device of the present invention shows a maximum external quantum yield of preferably 1% or more, more preferably 1.5% or more when a voltage of 3.5 V or more is applied between an anode and a cathode, from the standpoint of the luminance of the device, and the like.

As the light emitting device of the present invention, mentioned are a light emitting device having an electron transporting layer provided between a cathode and a light emitting layer, a light emitting device having a hole transporting layer provided between an anode and a light emitting layer, a light emitting device having an electron transporting layer provided between a cathode and a light emitting layer and a hole transporting layer provided between an anode and a light emitting layer, and the like, and examples thereof include the following structures a) to d).
a) anode/light emitting layer/cathode
b) anode/hole transporting layer/light emitting layer/cathode
c) anode/light emitting layer/electron transporting layer/cathode
d) anode/hole transporting layer/light emitting layer/electron transporting layer/cathode (Here, / represents adjacent lamination of layers, being applicable also in the following descriptions)

Also exemplified are structures having an "interlayer" layer provided adjacent to a light emitting layer between the light emitting layer and an anode in each of these structures. That is, the following structures a') to d') are exemplified.
a') anode/"interlayer" layer/light emitting layer/cathode
b') anode/hole transporting layer/"interlayer" layer/light emitting layer/cathode
c') anode/"interlayer" layer/light emitting layer/electron transporting layer/cathode
d') anode/hole transporting layer/"interlayer" layer/light emitting layer/electron transporting layer/cathode

When the light emitting device of the present invention has a hole transporting layer, the hole transporting layer contains usually the above-described hole transporting material (high molecular weight compound, low molecular weight compound). Exemplified as the hole transporting material are polyvinylcarbazole and its derivatives, polysilane and its derivatives, polysiloxane derivatives having an aromatic amine on the side chain or main chain, pyrazoline derivatives, arylamine derivatives, stilbene derivatives, triphenyldiamine derivatives, polyaniline and its derivatives, polythiophene and its derivatives, polypyrrole and its derivatives, poly(p-phenylenevinylene) and its derivatives, poly(2,5-thienylenevinylene) and its derivatives, and the like, and compounds described in JP-A Nos. 63-70257, 63-175860, 2-135359, 2-135361, 2-209988, 3-37992 and 3-152184, and the like.

Among them, preferable as the high molecular weight compound are polyvinylcarbazole and its derivatives, polysilane and its derivatives, polysiloxane derivatives having an aromatic amine compound group on the side chain or main chain, polyaniline and its derivatives, polythiophene and its derivatives, poly(p-phenylenevinylene) and its derivatives, poly(2,5-thienylenevinylene) and its derivatives, and the like, and more preferable are polyvinylcarbazole and its derivatives, polysilane and its derivatives, and polysiloxane derivatives having an aromatic amine on the side chain or main chain.

Of them, exemplified as the low molecular weight compound are pyrazoline derivatives, arylamine derivatives, stilbene derivatives, and triphenyldiamine derivatives. These low molecular weight compounds are preferably used in dispersion in a polymer binder.

As the above-described polymer binder, those not extremely disturbing charge transportation and showing no strong absorption against visible ray are preferable. Exemplified as the polymer binder are poly(N-vinylcarbazole), polyaniline and its derivatives, polythiophene and its derivatives, poly(p-phenylenevinylene) and its derivatives, poly(2,5-thienylenevinylene) and its derivatives, polycarbonate, polyacrylate, polymethyl acrylate, polymethyl methacrylate, polystyrene, polyvinyl chloride, polysiloxane and the like.

Polyvinylcarbazole and its derivatives are obtained, for example, from a vinyl monomer by cation polymerization or radical polymerization.

As the polysilane and its derivatives, compounds described in Chem. Rev., vol. 89, p. 1359 (1989), GB Patent No. 2300196 publication, and the like are exemplified. Also as the synthesis method, methods described in them can be used, and particularly, the Kipping method is suitably used.

In the polysiloxane and its derivatives, the siloxane skeleton structure shows little hole transporting property, thus, those having a structure of the above-described low molecular weight hole transporting material on the side chain or main chain are preferable, and those having an aromatic amine showing hole transportability on the side chain or main chain are more preferable.

As the film formation method of a hole transporting layer, a method of film formation from a mixed solution with a polymer binder is exemplified in the case of use of a low molecular weight compound, and a method of film formation from a solution is exemplified in the case.of use of a high molecular weight compound.

As the solvent used for film formation from a solution, those which can dissolve or uniformly disperse a hole transporting material are preferable. Exemplified as the solvent are chlorine-based solvents such as chloroform, methylene chloride, 1,2-dichloroethane, 1,1,2-trichloroethane, chlorobenzene, o-dichlorobenzene and the like, ether solvents such as tetrahydrofuran, dioxane and the like, aromatic hydrocarbon solvents such as toluene, xylene and the like, aliphatic hydrocarbon solvents such as cyclohexane, methylcyclohexane, n-pentane, n-hexane, n-heptane, n-octane, n-nonane, n-decane and the like, ketone solvents such as acetone, methyl ethyl ketone, cyclohexanone and the like, ester solvents such as ethyl acetate, butyl acetate, ethylcellosolve acetate and the like, polyhydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, 1,2-hexanediol and the like and derivatives thereof, alcohol solvents such as methanol, ethanol, propanol, isopropanol, cyclohexanol and the like, sulfoxide solvents such as dimethyl sulfoxide and the like, amide solvents such as N-methyl-2-pyrrolidone, N,N-dimethylformamide and the like. These organic solvents can be used singly or in combination with another or more.

As the method for film formation from a solution, there can be used application methods from a solution such as a spin coat method, casting method, micro gravure coat method, gravure coat method, bar coat method, roll coat method, wire bar coat method, dip coat method, spray coat method, screen printing method, flexo printing method, offset printing method, inkjet print method, capillary coat method, nozzle coat method and the like.

Regarding the thickness of a hole transporting layer, the optimum value varies depending on a material to be used, and it may be advantageously selected so as to give suitable driving voltage and light emission efficiency, and a thickness at least causing no formation of pin holes is necessary, and when the thickness is too large, the driving voltage of a device increases undesirably. Therefore, the thickness of the hole transporting layer is, for example, 1 nm to 1 µm, preferably 2 to 500 nm, more preferably 5 to 200 nm.

When the light emitting device of the present invention has an electron transporting layer, the electron transporting layer contains usually the above-described electron transporting material (high molecular weight compound, low molecular weight compound). As the electron transporting material, known materials can be used, and exemplified are oxadiazole derivatives, anthraquinodimethane and its derivatives, benzoquinone and its derivatives, naphthoquinone and its derivatives, anthraquinone and its derivatives, tetracyanoanthraquinodimethane and its derivatives, fluorenone derivatives, diphenyldicyanoethylene and its derivatives, diphenoquinone derivatives, metal complexes of 8-hydroxyquinoline and its derivatives, polyquinoline and its derivatives, polyquinoxaline and its derivatives, polyfluorene and its derivatives, and the like, and compounds described in JP-A Nos. 63-70257, 63-175860, 2-135359, 2-135361, 2-209988, 3-37992, 3-152184, and the like. Of them, oxadiazole derivatives, benzoquinone and its derivatives, anthraquinone and its derivatives, metal complexes of 8-hydroxyquinoline and its derivatives, polyquinoline and its derivatives, polyquinoxaline and its derivatives, polyfluorene and its derivatives are preferable, and 2-(4-biphenylyl)-5-(4-t-butylphenyl)-1,3,4-oxadiazole, benzoqinone, anthraquinone, tris(8-quinolinol)aluminum and polyquinoline are further preferable.

As the film formation method of an electron transporting layer, a vacuum vapor deposition method from power, or a method of film formation from solution or melted condition is exemplified in the case of use of a low molecular weight compound, and a method of film formation from solution or melted condition is exemplified in the case of use of a high molecular weight compound. In the method of film formation from solution or melted condition, the above-described polymer binder may be used together.

As the solvent to be used in film formation from solution, compounds which can dissolve or uniformly disperse an electron transporting material and/or polymer binder are preferable. Exemplified as the solvent are chlorine-based solvents such as chloroform, methylene chloride, 1,2-dichloroethane, 1,1,2-trichloroethane, chlorobenzene, o-dichlorobenzene and the like, ether solvents such as tetrahydrofuran, dioxane and the like, aromatic hydrocarbon solvents such as toluene, xylene and the like, aliphatic hydrocarbon solvents such as cyclohexane, methylcyclohexane, n-pentane, n-hexane, n-heptane, n-octane, n-nonane, n-decane and the like, ketone solvents such as acetone, methyl ethyl ketone, cyclohexanone and the like, ester solvents such as ethyl acetate, butyl acetate, ethylcellosolve acetate and the like, polyhydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, 1,2-hexanediol and the like and derivatives thereof, alcohol solvents such as methanol, ethanol, propanol, isopropanol, cyclohexanol and the like, sulfoxide solvents such as dimethyl sulfoxide and the like, amide solvents such as N-methyl-2-pyrrolidone, N,N-dimethylformamide and the like. These organic solvents can be used singly or in combination of with another or more.

As the film formation method from solution or melted condition, application methods such as a spin coat method, casting method, micro gravure coat method, gravure coat method, bar coat method, roll coat method, wire bar coat method, dip coat method, spray coat method, screen printing method, flexo printing method, offset printing method, inkjet print method, capillary coat method, nozzle coat method and the like can be used.

Regarding the thickness of an electron transporting layer, the optimum value varies depending on a material to be used, and it may be advantageously selected so as to give suitable driving voltage and light emission efficiency, and a thickness at least causing no formation of pin holes is necessary, and when the thickness is too large, the driving voltage of a device increases undesirably. Therefore, the thickness of the electron transporting layer is usually 1 nm to 1 µm, preferably 2 to 500 nm, more preferably 5 to 200 nm.

The above-described hole injection layer and electron injection layer are those having a function of improving charge injection efficiency from an electrode and having an effect of lowering the driving voltage of a device, among charge transporting layers provided adjacent to an electrode,

For improving close adherence with an electrode and improving charge injection from an electrode, the above-described charge injection layer or an insulation layer (usually, having an average thickness of 0.5 to 4.0 nm, being applicable also in the following descriptions) may be provided adjacent to the electrode, alternatively, for improving close adherence of an interface and preventing mixing, a thin buffer layer may be inserted into an interface of a charge transporting layer and a light emitting layer.

The order and number of layers to be laminated, and thickness of each layer can be appropriately determined in view of light emission efficiency and device life.

In the present invention, as the light emitting (electron injection layer, hole injection layer), mentioned are light emitting devices having a charge injection layer provided adjacent to a cathode and light emitting devices having a charge injection layer provided adjacent to an anode. Examples thereof include the following structures e) to p).
e) anode/charge injection layer/light emitting layer/cathode
f) anode/light emitting layer/charge injection layer/cathode
g) anode/charge injection layer/light emitting layer/charge injection layer/cathode
h) anode/charge injection layer/hole transporting layer/light emitting layer/cathode
i) anode/hole transporting layer/light emitting layer/charge injection layer/cathode
j) anode/charge injection layer/hole transporting layer/light emitting layer/charge injection layer/cathode
k) anode/charge injection layer/light emitting layer/electron transporting layer/cathode
l) anode/light emitting layer/electron transporting layer/charge injection layer/cathode
m) anode/charge injection layer/light emitting layer/electron transporting layer/charge injection layer/cathode
n) anode/charge injection layer/hole transporting layer/light emitting layer/electron transporting layer/cathode
o) anode/hole transporting layer/light emitting layer/electron transporting layer/charge injection layer/cathode
p) anode/charge injection layer/hole transporting layer/light emitting layer/electron transporting layer/charge injection layer/cathode
   Also exemplified are structures having an "interlayer" layer provided adjacent to a light emitting layer between the light emitting layer and an anode in each of these structures. In this case, the "interlayer" layer may also function as a hole injection layer and/or hole transporting layer.
   As the charge injection layer, exemplified are a layer containing an electric conductive polymer, a layer provided between an anode and a hole transporting layer and containing a material having ionization potential of a value between an anode material and a hole transporting material contained in a hole transporting layer, a layer provided between a cathode and an electron transporting layer and containing a material having electron affinity of a value between a cathode material and an electron transporting material contained in an electron transporting layer, and the like.
   When the above-described charge injection layer contains an electric conductive polymer, the electric conductivity of the electric conductive polymer is preferably 10⁻⁵ to 10³ S/cm, and for decreasing leak current between light emission picture elements, more preferably 10⁻⁵ to 10² S/cm, further preferably 10⁻⁵ to 10¹ S/cm.
   When the above-described charge injection layer contains an electric conductive polymer, the electric conductivity of the electric conductive polymer is preferably 10⁻⁵ to 10³ S/cm, and for decreasing leak current between light emission picture elements, more preferably 10⁻⁵ to 10² S/cm, further preferably 10⁻⁵ to 10¹ S/cm. Usually, for adjusting the electric conductivity of the electric conductive polymer to such a range, the electric conductive polymer is doped with a suitable amount of electrons.
   As the kind of ions to be doped, an anion is used in a hole injection layer and a cation is used in an electron injection layer. Examples of the anion include a polystyrenesulfonic ion, alkylbenzenesulfonic ion, camphorsulfonic ion and the like, and examples of the cation include a lithium ion, sodium ion, potassium ion, tetrabutylammonium ion and the like.
   The material used in the charge injection layer may be appropriately selected depending on a relation with materials of an electrode and an adjacent layer, and exemplified are polyaniline and its derivatives, polythiophene and its derivatives, polypyrrole and its derivatives, polyphenylenevinylene and its derivatives, polythienylenevinylene and its derivatives, polyquinoxaline and its derivatives, electric conductive polymers such as polymers containing an aromatic amine structure on the main chain or side chain, metal phthalocyanines (copper phthalocyanine and the like), carbon and the like.
   As the material of the above-described insulation layer, a metal fluoride, metal oxide, organic insulating material and the like are mentioned. As the light emitting device having the above-described insulation layer, there are mentioned light emitting devices in which an insulation layer is provided adjacent to a cathode, and light emitting devices in which an insulation layer is provided adjacent to an anode.
   As the light emitting device having an insulation layer, for example, the following structures q) to ab) are mentioned.
q) anode/insulation layer/light emitting layer/cathode
r) anode/light emitting layer/insulation layer/cathode
s) anode/insulation layer/light emitting layer/insulation layer/cathode
t) anode/insulation layer/hole transporting layer/light emitting layer/cathode
u) anode/hole transporting layer/light emitting layer/insulation layer/cathode
v) anode/insulation layer/hole transporting layer/light emitting layer/insulation layer/cathode
w) anode/insulation layer/light emitting layer/electron transporting layer/cathode
x) anode/light emitting layer/electron transporting layer/insulation layer/cathode
y) anode/insulation layer/light emitting layer/electron transporting layer/insulation layer/cathode
z) anode/insulation layer/hole transporting layer/light emitting layer/electron transporting layer/cathode
aa) anode/hole transporting layer/light emitting layer/electron transporting layer/insulation layer/cathode
ab) anode/insulation layer/hole transporting layer/light emitting layer/electron transporting layer/insulation layer/cathode

Also exemplified are structures having an "interlayer" layer provided adjacent to a light emitting layer between the light emitting layer and an anode in each of these structures. In this case, the "interlayer" layer may also function as a hole injection layer and/or hole transporting layer.

In structures in which an "interlayer" layer is applied to the above-described structures a) to ab), the "interlayer" layer is preferably provided between an anode and a light emitting layer and constituted of a material having intermediate ionization potential between the anode or hole injection layer or hole transporting layer, and a polymer compound constituting the light emitting layer.

As the material to be used in the "interlayer" layer, exemplified are polymers containing an aromatic amine such as polyvinylcarbazole and its derivatives, polyarylene derivatives having an aromatic amine on the side chain or main chain, arylamine derivatives, triphenyldiamine derivatives and the like.

As the method for forming the "interlayer" layer, a method of film formation from solution is exemplified in the case of use of a polymer material.

As the solvent to be used in film formation from solution, compounds which can dissolve or uniformly disperse a material used in an "interlayer" layer are preferable. Exemplified as the solvent are chlorine-based solvents such as chloroform, methylene chloride, 1,2-dichloroethane, 1,1,2-trichloroethane, chlorobenzene, o-dichlorobenzene and the like, ether solvents such as tetrahydrofuran, dioxane and the like, aromatic hydrocarbon solvents such as toluene, xylene and the like, aliphatic hydrocarbon solvents such as cyclohexane, methylcyclohexane, n-pentane, n-hexane, n-heptane, n-octane, n-nonane, n-decane and the like, ketone solvents such as acetone, methyl ethyl ketone, cyclohexanone and the like, ester solvents such as ethyl acetate, butyl acetate, ethylcellosolve acetate and the like, polyhydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, 1,2-hexanediol and the like and derivatives thereof, alcohol solvents such as methanol, ethanol propanol, isopropanol, cyclohexanol and the like, sulfoxide solvents such as dimethyl sulfoxide and the like, amide solvents such as N-methyl-2-pyrrolidone, N,N-dimethylformamide and the like. These organic solvents can be used singly or in combination with another or more.

As the film formation method from solution, application methods such as a spin coat method, casting method, micro gravure coat method, gravure coat method, bar coat method, roll coat method, wire bar coat method, dip coat method, spray coat method, screen printing method, flexo printing method, offset printing method, inkjet printing method, capillary coat method, nozzle coat method and the like can be used.

Regarding the thickness of an "interlayer" layer, the optimum value varies depending on a material to be used, and it may be advantageously selected so as to give suitable driving voltage and light emission efficiency, and it is usually 1 nm to 1 µm, preferably 2 to 500 nm, more preferably 5 to 200 nm.

When the "interlayer" layer is provided adjacent to a light emitting layer, particularly when both the layers are formed by an application method, the two layers may be mixed to exert an undesirable influence on device properties and the like in some cases. When the "interlayer" layer is formed by an application method before formation of a light emitting layer by an application method, there is mentioned a method in which an "interlayer" layer is formed by an application method, then, the "interlayer" layer is heated to be insolubilized in an organic solvent to be used for manufacturing a light emitting layer, then, the light emitting layer is formed, as a method for reducing mixing of materials of the two layers. The above-described heating temperature is usually about 150 to 300°C. The above-described heating time is usually about 1 minute to 1 hour. In this case, for removal of components not insolubilized in solvent by heating, the "interlayer" layer may be rinsed with a solvent to be used for formation of a light emitting layer, after heating and before formation of the light emitting layer. When insolubilization in solvent by heating is carried out sufficiently, rinsing with a solvent can be omitted. For insolubilization in solvent by heating to be carried out sufficiently, it is preferable to use a compound containing at least one polymerizable group in the molecule, as a polymer compound to be used in an "interlayer" layer. Further, the number of polymerizable groups is preferably 5% or more based on the number of repeating units in the molecule.

The substrate which forms a light emitting device of the present invention may be that forming an electrode and which does not change in forming a layer of an organic substance, and exemplified are, for example, substrates of glass, plastic, polymer film, silicon and the like. In the case of an opaque substrate, it is preferable that the opposite electrode is transparent or semi-transparent.

Usually, at least one of an anode and a cathode contained in a polymer light emitting device of the present invention is transparent or semi-transparent, and it is preferable that a cathode is transparent or semi-transparent.

As the material of the cathode, an electric conductive metal oxide film, semi-transparent metal thin film and the like are mentioned. For example, films (NESA and the like) formed using electric conductive glass composed of indium oxide, zinc oxide, tin oxide, and composite thereof: indium•tin•oxide (ITO), indium•zinc•oxide and the like, gold, platinum, silver, copper and the like are used, and ITO, indium•zinc•oxide, tin oxide are preferable. As the manufacturing method, a vacuum vapor-deposition method, sputtering method, ion plating method, plating method and the like are mentioned. As the anode, organic transparent electric conductive films made of polyaniline or its derivative, polythiophene or its derivative, and the like may be used. The anode may take a lamination structure composed of two or more layers.

The thickness of an anode can be appropriately selected in view of light transmission and electric conductivity, and it is, for example, 10 nm to 10 µm, preferably 20 nm to 1 µm, more preferably 50 nm to 500 nm.

For making charge injection easy, a layer made of a phthalocyanine derivative, electric conductive polymer, carbon and the like, an insulation layer made of a metal oxide, metal fluoride, organic insulation material and the like, may be provided on an anode.

As the material of a cathode, materials of small work function are preferable, and for example, metals such as lithium, sodium, potassium, rubidium, cesium, beryllium, magnesium, calcium, strontium, barium, aluminum, scandium, vanadium, zinc, yttrium, indium, cerium, samarium, europium, terbium, ytterbium and the like, alloys of two or more of them, or alloys made of at least one of them and at least one gold, silver, platinum, copper, manganese, titanium, cobalt, nickel, tungsten and tin, or graphite or graphite intercalation compounds and the like are used. The alloy includes magnesium-silver alloy, magnesium-indium alloy, magnesium-aluminum alloy, indium-silver alloy, lithium-aluminum alloy, lithium-magnesium alloy, lithium-indium alloy, calcium-aluminum alloy and the like. The cathode may take a laminated structure including two or more layers.

The thickness of a cathode can be appropriately selected in view of electric conductivity and durability, and it is usually 10 nm to 10 µm, preferably 20 nm to 1 um, further preferably 50 to 500 nm.

As the cathode manufacturing method, a vacuum vapor-deposition method, sputtering method, lamination method of thermally press-fitting a metal thin film, and the like are used. A layer made of an electric conductive polymer, or a layer having an average thickness of 2 nm or less made of a metal oxide, metal fluoride, organic insulation material and the like, may be provided between a cathode and an organic substance layer, and after manufacturing a cathode, a protective layer for protecting the polymer light emitting device may be installed. For use of the polymer light emitting device stably for a long period of time, it is preferable to install a protective layer and/or protective cover, for protecting a device from outside.

As the protective layer, a polymer compound, metal oxide, metal fluoride, metal boride and the like can be used. As the protective cover, a metal plate, a glass plate, and a plastic plate having a surface which has been subjected to low water permeation treatment, and the like can be used, and a method of pasting the cover to a device substrate with a thermosetting resin or photo-curable resin to attain close seal is suitably used. When a space is kept using a spacer, prevention of blemishing of a device is easy. If an inert gas such as nitrogen, argon and the like is filled in this space, oxidation of a cathode can be prevented, further, by placing a drying agent such as barium oxide and the like in this space, it becomes easy to suppress moisture adsorbed in a production process or a trace amount of moisture invaded passing through the hardened resin from imparting damage to the device. It is preferable to adopt one strategy among these methods.

The light emitting device of the present invention can be used as a sheet light source, segment display, dot matrix display, back light of a liquid crystal display, and the like.

For obtaining light emission in the form of sheet using a polymer light emitting device of the present invention, it may be advantages to place a sheet anode and a sheet cathode so as to overlap. For obtaining light emission in the form of pattern, there are a method in which a mask having a window in the form of pattern is placed on the surface of the above-described sheet light emitting device, a method in which an organic substance layer in non-light emitting parts is formed with extremely large thickness to give substantially no light emission, and a method in which either anode or cathode, or both electrodes are formed in the form pattern. By forming a pattern by any of these methods, and placing several electrodes so that on/off is independently possible, a display of segment type is obtained which can display digits, letters, simple marks and the like. Further, for providing a dot matrix device, it may be permissible that both an anode and a cathode are formed in the form of stripe, and placed so as to cross. By using a method in which several polymer fluorescent bodies showing different emission colors are painted separately or a method in which a color filter or a fluorescence conversion filter is used, partial color display and multi-color display are made possible. In the case of a dot matrix device, passive driving is possible, and active driving may be carried out in combination with TFT and the like. These displays can be used as a display of a computer, television, portable terminal, cellular telephone, car navigation, view finder of video camera, and the like.

Further, the above-described sheet light emitting device is of self emitting and thin type, and can be suitably used as a sheet light source for back light of a liquid crystal display, or as a sheet light source for illumination. If a flexible substrate is used, it can also be used as a curved light source or display.

### EXAMPLES

The present invention will be illustrated further in detail by examples below, but the invention is not limited to them.

### (Number average molecular weight and weight average molecular weight)

In examples, the polystyrene-equivalent number average molecular weight and weight average molecular weight were measured by gel permeation chromatography (GPC, manufactured by Shimadzu Corp., trade name: LC-10Avp). A polymer compound to be measured was dissolved in tetrahydrofuran so as to give a concentration of about 0.5 wt%, and the solution was injected in an amount of 30 µL into GPC. Tetrahydrofuran was used as the mobile phase of GPC, and allowed to flow at a flow rate of 0.6 mL/min. In the column, two TSKgel Super HM-H (manufactured by Tosoh Corp.) and one TSKgel Super H2000 (manufactured by Tosoh Corp.) were connected serially. A differential refractive index detector (trade name: RID-10A, manufactured by Shimadzu Corp.) was used as a detector.

### (Fluorescent spectrum)

Fluorescent spectrum was measured according to the following method. A 0.8 wt% toluene solution of a polymer to be measured was spin-coated on quartz to form a thin film of the polymer compound. This thin film was excited at a wavelength of 350 nm, and fluorescent spectrum of the polymer compound was measured using a fluorescence spectrophotometer (trade name: Fluorolog, manufactured by Horiba, Ltd.). For obtaining relative fluorescence intensity in the thin film, fluorescent spectrum plotted against wave number was integrated in the spectrum measuring range utilizing the intensity of Raman line of water as a standard, and measurement was performed using a spectrophotometer (trade name: Cary 5E, manufactured by Varian), obtaining a value allocated to "the absorbance at the excited wavelength".

### <Synthesis Example 1> (Synthesis of dibromopyrene)

Into a solution prepared by dissolving 10.0 g (49.4 mmol) of pyrene in 250 mL of chloroform, a solution composed of 16 g of bromine and 100 mL of chloroform was dropped at 23°C over a period of 7 hours while stirring. The mixture was stirred further for 1 hour, then, the resultant reaction liquid (containing crystal) was filtrated to obtain a crystal. This crystal was washed with chloroform, then, dried under reduced pressure to obtain 11.3 g of dibromopyrene (yield 63%, 1,6-dibromopyrene:1,8-dibromopyrene = 59:41 (hereinafter, "dibromopyrene synthesized in Synthesis Example 1")).

LC-MS (APPI-MS (posi)): 358 [M]⁺

### <Synthesis Example 2> (Synthesis of compound 1)

To dibromopyrene (2.0 g, 5.6 mmol) synthesized in Synthesis Example 1 was added 4-t-butylphenylboric acid (2.2 g, 12.2 mmol), trioctylmethylammonium chloride (trade name: Aliquat 336 (hereinafter, referred to as "Aliquat 336"), manufactured by Aldrich, 0.74 g), palladium acetate (1.3 mg), tris(o-methoxyphenyl)phosphine (13.3 mg), toluene (58 mL) and sodium carbonate aqueous solution (17.8 mmol), and the mixture was stirred at 100°C for 4 hours. The resultant reaction liquid was cooled down to room temperature, then, the reaction liquid (containing crystal) was filtrated to obtain a crystal. This crystal was washed with toluene, water and methanol in this order, then, dried under reduced pressure, to obtain 1.2 g (yield 47%) of a compound 1 of the following formula. 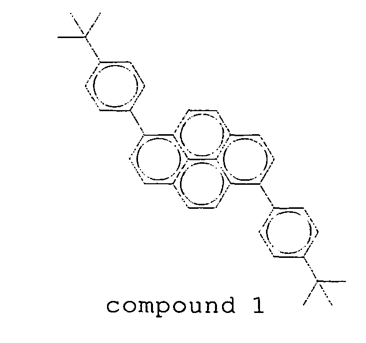 1H-NMR: (299.4 MHz, CDCl₃ ) : 1.45 (s, 18H), 7.59 (s, 8H), 8.00 (m, 4H), 8.22 (m, 4H)
LC-MS (APPI-MS(posi)): 467 [M+H]⁺

### <Synthesis Example 3> (Synthesis of compound 2)

Into a solution composed of the compound 1 (1.15 g, 2.5 mmol) and chloroform (240 mL), a solution composed of bromine (0.9 g) and chloroform (12 mL) was dropped at 20°C over a period of 30 minutes while stirring, and the mixture was further stirred for 6 hours. To this was added methanol (250 mL) and the resultant reaction liquid (containing crystal) was filtrated to obtain a crystal. This crystal was filtrated and washed with methanol, then, dried under reduced pressure to obtain a compound 2 (1.6 g, yield: 100%) of the following formula. 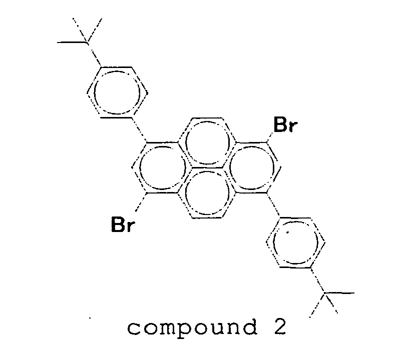 LC-MS (APPI-MS (posi)): 622 [M]⁺

### <Synthesis Example 4> (Synthesis of compound 3)

To the compound 2 (1.0 g, 1.6 mmol) was added (4-t-butylphenyl)phenylamine (0.8 g, 3.4 mmol), sodium t-butoxide (19.4 g, 201 mmol), [tris(dibenzylideneacetone)]dipalladium (0.78 g), tri-t-butylphosphine (0.78 g, 3.9 mmol) and toluene (250 mL), and the mixture was stirred at 100°C for 2.5 hours. The mixture was cooled down to room temperature, then, to the resultant solution was added water (200 mL) and the mixture was stirred, an oil phase was separated from an aqueous phase, and the oil phase was allowed to pass through a silica gel column, and concentrated to dryness. Then, re-crystallization was performed using tetrahydrofuran and methanol to obtain a compound 3 (1.3 g, yield: 84%) of the following formula. 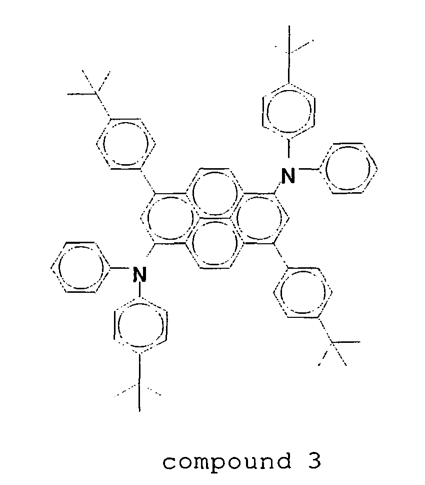 LC-MS (APPI-MS (posi)): 913 [M+H]⁺

### <Synthesis Example 5> (Synthesis of compound 4)

Into a solution composed of the compound 3 (0.61 g, 0.7 mmol) and chloroform (150 g), a solution composed of N-bromosuccinimide (0.24 g, 1.3 mmol) and N,N-dimethylformamide (2.7 mL) was dropped at 25°C over a period of 5 minutes while stirring. The resultant solution was stirred further for 9 hours, then, water (30 mL) was added and the mixture was stirred, and an oil phase was separated from an aqueous phase. The oil phase was dried over anhydrous magnesium sulfate, and concentrated to dryness. Then, chloroform (85 mL) was added to prepare a solution, and methanol (73 g) was dropped into this, to obtain mixed liquid (containing crystal). This mixed liquid (containing crystal) was filtrated, and the resultant crystal was dried under reduced pressure, to obtain a compound 4 (0.7 g, yield: 100%) of the following formula. 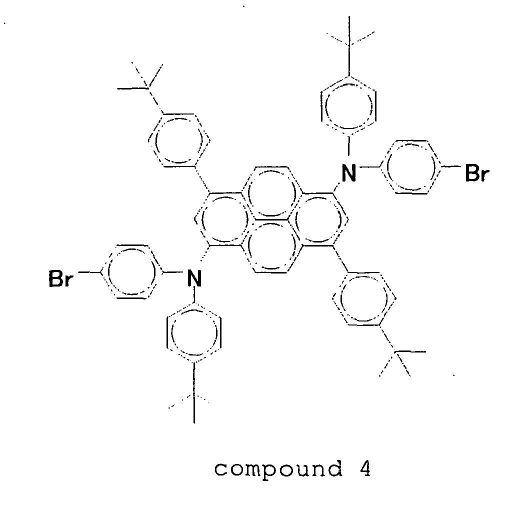 LC-MS (APPI-MS (posi)): 1069 [M+H]⁺

### <Synthesis Example 6> (Synthesis of 1,3,6,8-tetrabromopyrene)

To pyrene (20.0 g, 98.9 mmol) was added nitrobenzene (750 mL), and bromine (70.7 g, 438 mmol) was dropped at 120°C while stirring, and the mixture was stirred further for 2 hours. The mixture was cooled down to room temperature, then, the resultant reaction liquid (containing crystal) was filtrated to obtain a crystal. This crystal was washed with ethanol and toluene in this order and dried under reduced pressure, to obtain 1,3,6,8-tetrabromopyrene (49.6 g, yield: 97%).

### <Synthesis Example 7> (Synthesis of compound 5)

To 1,3,6,8-tetrabromopyrene (1.5 g, 2.9 mmol) synthesized in Synthesis Example 6 was added (4-octylphenyl)phenylamine (3.5 g, 12.5 mmol), sodium t-butoxide (1.4 g, 14.5 mmol), [tris(dibenzylideneacetone)]dipalladium (80 mg), tri-t-butylphosphine (70 mg) and o-xylene(34 mL), and the mixture was stirred at 130°C for 4 hours. The resultant reaction liquid was cooled down to room temperature, then, toluene (60 mL) was added and the mixture was stirred, and filtrated to obtain a filtrate which was then concentrated to dryness. Then, to the resultant solid was added 2-propanol and the mixture was stirred, then, the resultant reaction liquid (containing solid) was filtrated to obtain a solid. This solid was dried under reduced pressure to obtain a compound 5 (0.82 g, yield: 28%) of the following formula. 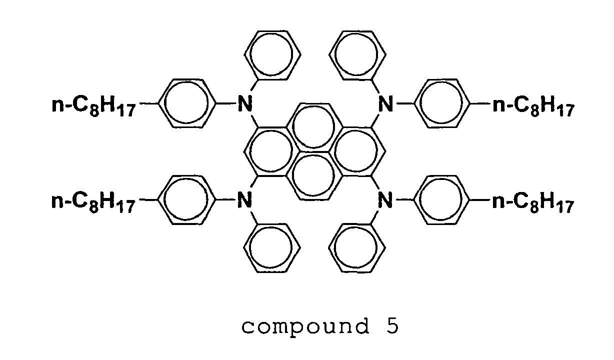 LC-MS (APPI-MS (posi)): 1320 [M+H]⁺

### <Synthesis Example 8> (Synthesis of compound 6)

Into a solution composed of the compound 5 (0.50 g, 0.4 mmol) and 10 g of chloroform, a solution composed of N-bromosuccinimide (0.28 g, 1.5 mmol) and N,N-dimethylformamide (3.2 mL) was dropped at room temperature over a period of 5 minutes while stirring. The resultant mixed liquid was further stirred at room temperature for 15 hours, then, to this was added water (10 mL) and the mixture was stirred and an organic phase was separated from an aqueous phase. Then, the organic phase was washed with water twice and concentrated to dryness, to obtain a solid. The solid was re-crystallized with ethyl acetate to obtain a compound 6 (0.34 g, yield: 55%) of the following formula. 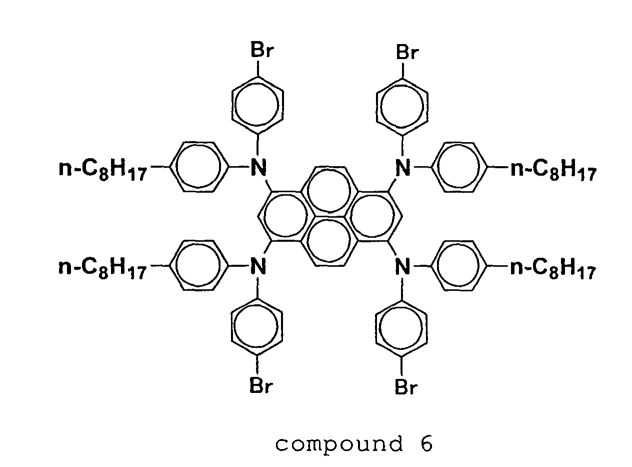 LC-MS (APPI-MS (posi)): 1630 [M+H]⁺

### <Synthesis Example 9>( Synthesis of compound 11)

Under an inert gas atmosphere, diphenylamine (30.0 g, 0.177 mol) was dissolved in 383 mL of chloroform, and a solution composed of tetrabutylammonium tribromide (171.0 g, 0.355 mol) and 766 mL of chloroform was dropped in the range of 0 to 5°C over a period of 4 hours, and the mixture was further stirred in the range of 0 to 5°C for 1 hour. Then, water (30 mL) was added and the mixture was stirred, and a saturated sodium hydrogen carbonate aqueous solution (540 mL) was added and the mixture was stirred, and an organic layer was separated from an aqueous layer, and the organic layer was dried over anhydrous magnesium sulfate and concentrated to dryness. Then, to the resultant solid was added 900 mL of hexane and the mixture was stirred, to obtain mixed liquid (containing crystal). This mixed liquid (containing crystal) was filtrated, and the resultant crystal was dried under reduced pressure to obtain 162.4 g of a white solid. 135.6 g of this white solid was dissolved in 1650 mL of toluene, and washed with 2 L of water five times, and an oil phase was concentrated to dryness and dried under reduced pressure to obtain a compound 11 (39.6 g, yield: 81.5%) of the following formula. 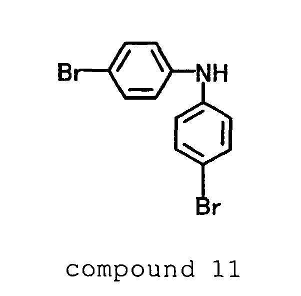 LC-MS (APPI-MS (posi)): 325 M⁺

### <Synthesis Example 10>( Synthesis of compound 12)

Under an inert gas atmosphere, to the compound 11 (26.0 g, 79.5 mmol) was added 2-[4-(1,1-dimethylethyl)phenyl]-4,4,5,5-tetramethyl-1,3,2-dioxaborolane (43.4 g, 167.0 mmol), Aliquat 336 (10.4 g), palladium acetate (180.5 mg), tris(o-methoxyphenyl)phosphine (1936.5 mg) and 436 mL of toluene. Then, 154 mL of a 17.5 wt% sodium carbonate aqueous solution was dropped at 100°C, and the mixture was further stirred at 100°C for 2 hours. Then, an aqueous layer was removed and an organic layer was concentrated to dryness, to obtain a solid. To the solid was added 2 L of toluene and the mixture was stirred for 1.5 hours, and filtrated using a filtering apparatus having silica gel laminated as an upper layer and radiolite laminated as a lower layer. The resultant filtrate was concentrated to 400 mL, and heated to reflux, obtaining a uniform solution, then, the solution was allowed to stand still at room temperature for 2 hours and the deposited crystal was filtrated and dried under reduced pressure to obtain a compound 12 (18.1 g, yield: 62.6%) of the following formula. 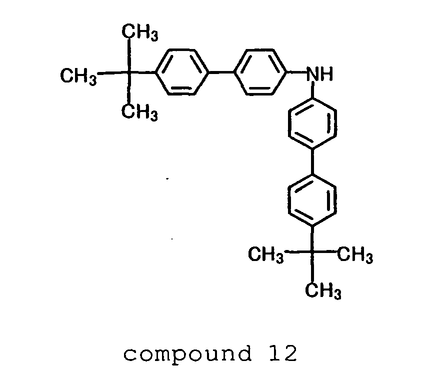 LC-MS (APCI-MS (posi)): 434 [M+H]⁺

### <Synthesis Example 11>(Synthesis of compound 13)

Under an inert gas atmosphere, to the compound 12 (18.3 g, 42.2 mmol), tris(dibenzylideneacetone)dipalladium (0.186 g, 0.2 mmol), tri-tert-butylphosphonium tetrafluoroborate (0.237 g, 0.8 mmol) and sodium tert-butoxide (4.55 g, 47.3 mmol) were added 110 mL of toluene and the mixture was heated up to 80°C while stirring, to cause dissolution. To the resultant solution was added dibromopyrene (7.42 g, 20.6 mmol) at 80°C over a period of 10 minutes, and the mixture was stirred at 100°C for 1 hour. The mixture was cooled down to room temperature, then, to the reaction solution was added 190 mL of toluene and the mixture was stirred, and passed through a filtering apparatus having laminated silica gel and the filtrate was concentrated to dryness. The resultant solid was re-crystallized with ethyl acetate to obtain a compound 13 (16.7 g, yield: 75.9%) of the following formula. 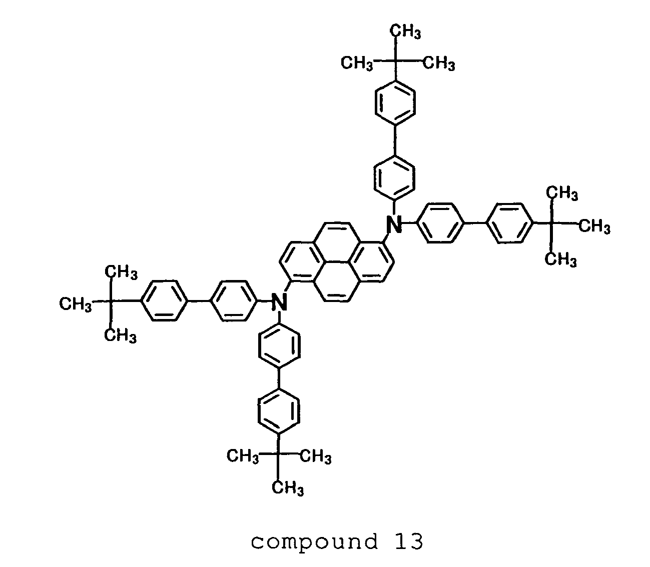 LC-MS (APCI-MS (posi)): 1065 [M+H]⁺

### <Synthesis Example 12> (Synthesis of compound 14)

Under an inert gas atmosphere, the compound 13 (15.0 g, 14.1 mmol) was dissolved in chloroform (270 mL), and a solution composed of tetrabutylammonium tribromide (13.6 g, 28.2 mmol) and chloroform (70 mL) was dropped at 24 to 28°C over a period of 1.5 hours, and the mixture was further stirred at the same temperature for 26 hours. Then, a solution composed of tetrabutylammonium tribromide (1.4 g, 2.8 mmol) and chloroform (6.4 mL) was dropped at 24 to 28°C over a period of 45 minutes, and the mixture was further stirred at the same temperature for 19 hours. Then, a solution composed of tetrabutylammonium tribromide (1.7 g, 3.5 mmol) and chloroform (8 mL) was dropped at 24 to 28°C over a period of 1 hour, and the mixture was further stirred at the same temperature for 3 hours and 20 minutes. Then, a solution composed of tetrabutylammonium tribromide (3.4 g) and chloroform (16 mL) was dropped at 24 to 28°C over a period of 1 hour, and the mixture was further stirred at the same temperature for 5 hours. Then, a 5 wt% sodium sulfite aqueous solution was added, then, an organic layer was separated from an aqueous layer and the organic layer was concentrated to dryness, to obtain a solid. To the solid was added 400 mL of toluene and a uniform solution was prepared, and the organic layer was washed with water five times, and the organic layer was passed through a filtering apparatus having a laminated silica gel column, and the resultant filtrate was concentrated to dryness. The resultant solid was re-crystallized with tetrahydrofuran to obtain a compound 14 (8.5 g, yield: 49%) of the following formula. 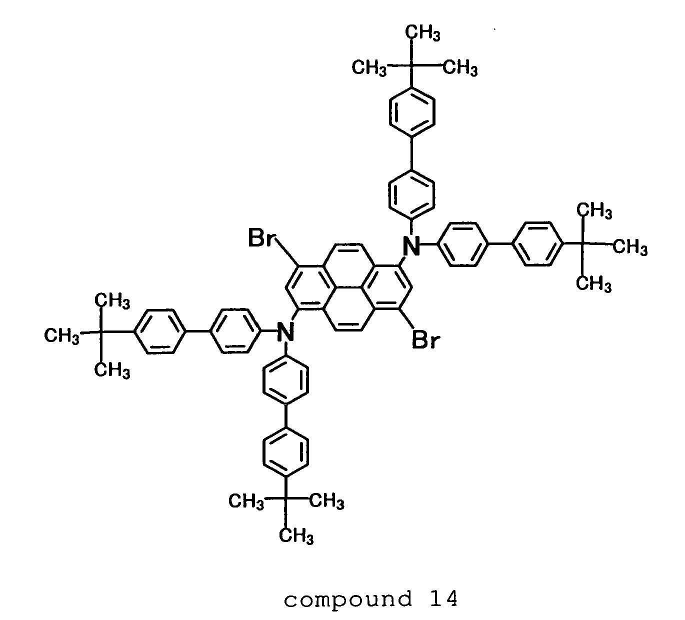 LC-MS (APCI-MS (posi)): 1221 [M+H]⁺

### <Synthesis Example 13> (Synthesis of compound 15)

Under an inert gas atmosphere, to octylphenylphenylamine (1.9 g, 6.7 mmol), tris(dibenzylideneacetone)dipalladium (29.9 mg, 0.03 mmol), tri-tert-butylphosphonium tetrafluoroborate (37.9 mg, 0.13 mmol) and sodium tert-butoxide (1.01 g, 10.5 mmol) were added 40 mL of toluene, and the mixture was heated up to 80°C while stirring, to cause dissolution. To the solution was added the compound 14 (4.0 g, 3.3 mmol) at 80°C and the mixture was stirred at 100°C for 5 hours. The mixture was cooled down to room temperature, then, 40 mL of toluene was added and the mixture was stirred, and passed through a filtering apparatus having laminated silica gel, and concentrated to dryness. The resultant solid was re-crystallized with 1-chlorobutane to obtain a compound 15 (5.0 g, yield: 94%) of the following formula. 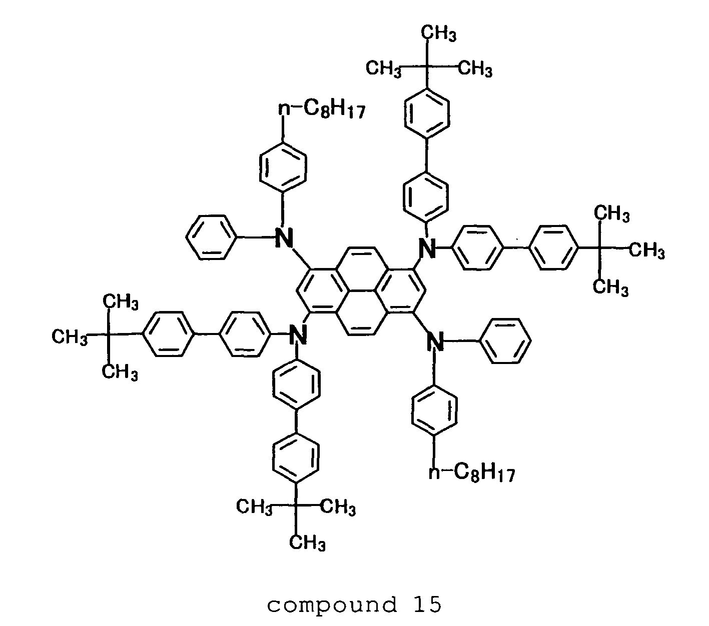 LC-MS (APCI-MS (posi)): 1624 [M+H]⁺

### <Synthesis Example 14> (Synthesis of compound 16)

Under an inert gas atmosphere, the compound 15 (1.2 g, 0.74 mmol) was dissolved in chloroform (182 mL), and a solution composed of N-bromosuccinimide (0.26 g, 1.48 mmol) and N,N-dimethylformamide (10 mL) was dropped at room temperature over a period of 35 minutes, and the mixture was further stirred at the same temperature for 3 hours and 20 minutes. Then, a solution composed of N-bromosuccinimide (9.2 mg, 0.05 mmol) and N,N-dimethylformamide (1 mL) was dropped at room temperature over a period of 5 minutes, and the mixture was further stirred at the same temperature for 2 hours. Then, a solution composed of N-bromosuccinimide (6.0 mg, 0.03 mmol) and N,N-dimethylformamide (1 mL) was dropped at room temperature over a period of 3 minutes, and the mixture was further stirred at the same temperature for 2 hours. Then, a 5 wt% Na₂SO₃ aqueous solution was added, and an organic layer was separated from an aqueous phase, and the organic layer was washed with 100 mL of water three times, and the organic layer was dried over anhydrous magnesium sulfate, and concentrated to dryness and dried under reduced pressure, to obtain a solid. The solid was re-crystallized twice with a mixed solvent of chloroform and hexane to obtain a compound 16 (0.62 g, yield: 49%) of the following formula. 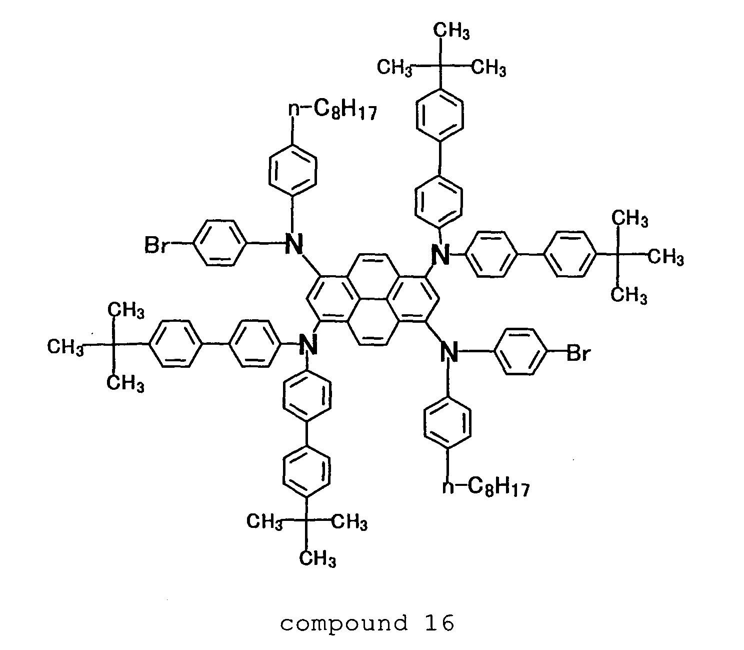 LC-MS (APCI-MS (posi)): 1780 [M+H]⁺

### <Synthesis Example 15> (Synthesis of compound 17)

To dibromopyrene (3.0 g, 8.3 mmol) synthesized in Synthesis Example 1 was added 4-cyanophenylboric acid (4.2 g, 18.3 mmol), Aliquat 336 (1.1 g), palladium acetate (1.9 mg), tris(o-methoxyphenyl)phosphine (20.6 mg), toluene (85 mL) and sodium carbonate aqueous solution (26.7 mmol) and the mixture was stirred at 100°C for 9 hours. The mixture was cooled down to room temperature, then, reaction liquid (containing crystal) was obtained. This reaction liquid (containing crystal) was filtrated, and the resultant crystal was washed with toluene, methanol and water in this order, and dried under reduced pressure to obtain a compound 17 (1.9 g, yield: 85%) of the following formula. 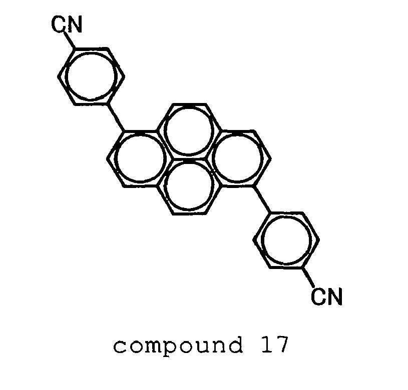 LC-MS (APPI-MS (posi)): 404 [M]⁺

### <Synthesis Example 16> (Synthesis of compound 18)

A suspension composed of 1.6 g (0.4 mmol) of the compound 17, 1.7 g (9.5 mmol) of N-bromosuccinimide and 380 mL of N,N-dimethylformamide was stirred at 50°C. N-bromosuccinimide was added until the area percentage of a compound 18 described later reached 65% according to high performance liquid chromatography. To the resultant mixed liquid was added water and the deposited crystal was filtrated, and dried under reduced pressure, to obtain a solid. Then, to the solid was added N,N-dimethylformamide and the mixture was stirred at 120°C, then, cooled down to room temperature and the deposited crystal was filtrated, and dried under reduced pressure to obtain a compound 18 (1.45 g, yield: 70%) of the following formula. 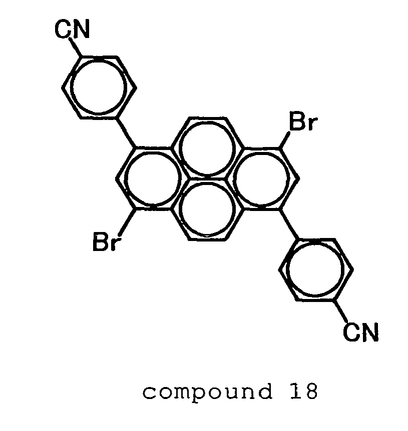 LC-MS (APPI-MS (posi)): 560 [M]⁺

### <Synthesis Example 17> (Synthesis of compound 19)

To the compound 18 (1.1 g, 2.4 mmol) was added (4-octylphenyl)phenylamine (1.4 g, 5.0 mmol), sodium t-butoxide (28.2 g, 292 mmol), [tris(dibenzylideneacetone)]dipalladium (1.1 g), tri-t-butylphosphine (1.1 g) and toluene (340 mL) and the mixture was stirred at 100°C for 6 hours. The resultant reaction liquid was cooled down to room temperature, then, the reaction liquid was filtrated and the resultant filtrate was concentrated to dryness, to obtain a solid. To the solid was added methanol and the mixture was stirred, then, the resultant crystal was filtrated and dried under reduced pressure to obtain a compound 19 (1.5 g, yield: 80%) of the following formula. 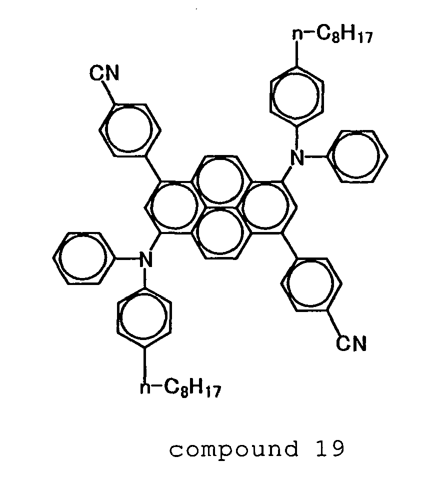 LC-MS (APPI-MS (posi)): 963 [M+H]⁺

### <Synthesis Example 18> (Synthesis of compound 20)

Into a solution composed of the compound 19 (0.57 g, 0.6 mmol) and chloroform (50 g), a solution composed of N-bromosuccinimide (0.21 g, 1.1 mmol) and N,N-dimethylformamide (2 mL) was dropped at room temperature over a period of 5 minutes while stirring, and the mixture was further stirred at room temperature for 7 hours. Then, to the resultant reaction liquid was added 25 mL of water and the mixture was stirred, and to this was added chloroform and water and the mixture was stirred, then, and organic layer was separated from an aqueous layer, and the organic layer was washed with water twice, and concentrated to dryness to obtain a solid. The solid was re-crystallized using toluene and 2-propanol, to obtain a compound 20 (0.44 g, yield: 67%) of the following formula. 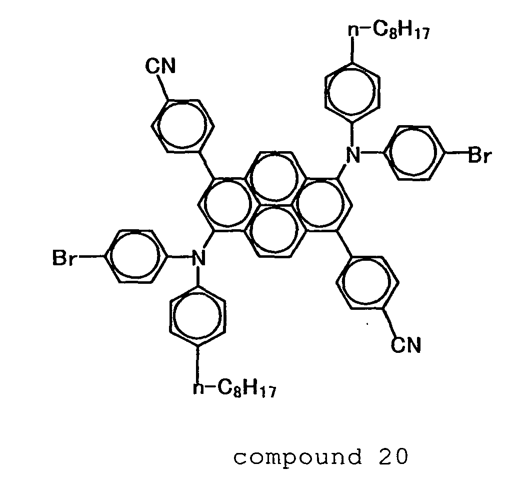 LC-MS (APPI-MS (posi)): 1119 [M+Hl⁺

### <Comparative Example 1> (Synthesis of polymer compound 1)

Under an inert atmosphere, a compound 7 (1.34 g, 2.52 mmol) of the following formula: 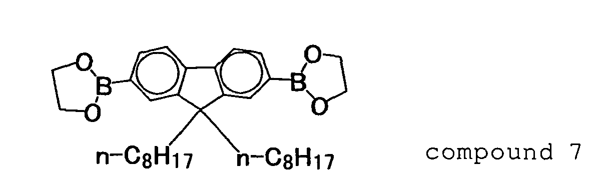 , a compound 8 (1.30 g, 2.37 mmol) of the following formula: 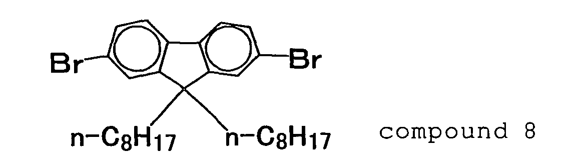 , a compound 9 (0.11 g, 0.15 mmol) of the following formula: 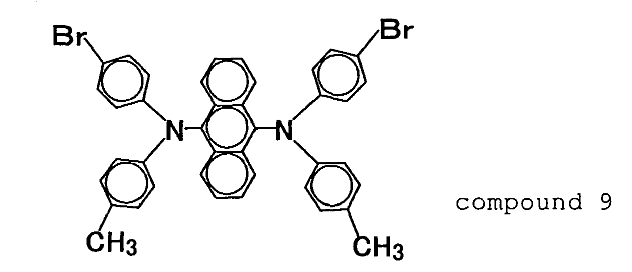 , palladium acetate (1.7 mg), tris(2-methoxyphenyl)phosphine (18.7 mg), Aliquat 336 (0.33 g) and toluene (25 ml) were mixed, and the mixture was heated at 105°C. Into the resultant reaction solution, a 2M sodium carbonate aqueous solution (6.9 ml) was dropped, and the mixture was refluxed for 30 minutes. After the reaction, to the resultant reaction solution was added phenylboric acid (30.8 mg) and the mixture was further refluxed for 2 hours. Then, to this was added a sodium diethyldithiacarbamate aqueous solution and the mixture was stirred at 80°C for 2 hours. After cooling, the resultant reaction liquid was washed with water (33 ml) twice, with a 3 wt% acetic acid aqueous solution (33 ml) twice and with water (33 ml) twice, and the resultant solution was dropped into methanol (390 mL) and filtrated to obtain a precipitate. The precipitate was dissolved in toluene (78 mL), and purified by passing through an alumina column and silica gel column sequentially. The resultant toluene solution was dropped into methanol (390 ml) and stirred, then, the resultant precipitate was filtrated and dried. The yield of this precipitate (hereinafter, referred to as "polymer compound 1") was 1.5 g.

The polymer compound 1 had a polystyrene-equivalent number average molecular weight of 1.4×10⁵ and a polystyrene-equivalent weight average molecular weight of 3.5×10⁵ and showed a fluorescence intensity of 4.6.

The polymer compound 1 is a random copolymer constituted of a repeating unit of the following formula: 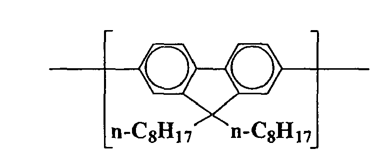 and a repeating unit of the following formula: 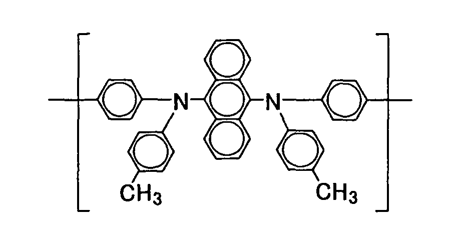 at a molar ratio of 97:3, according to theoretical values calculated from the charged raw materials.

### <Example 1> (Synthesis of polymer compound 2)

Under an inert atmosphere, the compound 7 (1.14 g, 2.16 mmol), the compound 8 (1.11 g, 2.03 mmol), the compound 4 (0.14 g, 0.13 mmol), palladium acetate (1.5 mg), tris(2-methoxyphenyl)phosphine (15.9 mg), Aliquat 336 (0.28 g) and toluene (22 ml) were mixed and heated at 105°C. Into this reaction solution, a 2M sodium carbonate aqueous solution (5.9 ml) was dropped, and the mixture was refluxed for 30 minutes. After the reaction, phenylboric acid (26.3 mg) was added and the mixture was further refluxed for 2 hours. Then, to this was added a sodium diethyldithiacarbamate aqueous solution and the mixture was stirred at 80°C for 2 hours. After cooling, the resultant reaction liquid was washed with water (28 ml) twice, with a 3 wt% acetic acid aqueous solution (28 ml) twice and with water (28 ml) twice, and the resultant solution was dropped into methanol (330 mL) and filtrated to obtain a precipitate. The precipitate was dissolved in toluene (70 mL), and purified by passing through an alumina column and silica gel column. The resultant toluene solution was dropped into methanol (330 ml) and stirred, then, the resultant precipitate was filtrated and dried. The yield of this precipitate (hereinafter, referred to as "polymer compound 2") was 1.4 g.

The polymer compound 2 had a polystyrene-equivalent number average molecular weight of 1.0×10⁵ and a polystyrene-equivalent weight average molecular weight of 2.4×10⁵ and showed a fluorescence intensity of 6.8.

The polymer compound 2 is a random copolymer constituted of a repeating unit of the following formula: 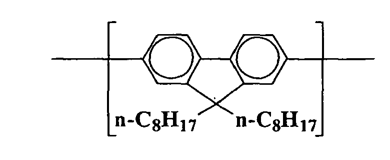 , and a repeating unit of the following formula: 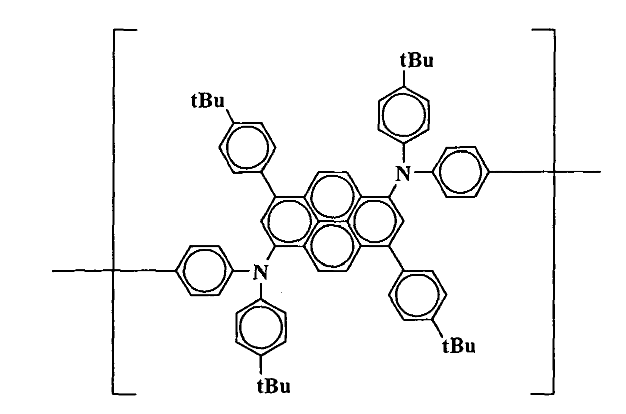 at a molar ratio of 97:3, according to theoretical values calculated from the charged raw materials.

### <Example 2> (Synthesis of polymer compound 3)

Under an inert atmosphere, a compound 10 (1.79 g, 3.05 mmol) of the following formula: 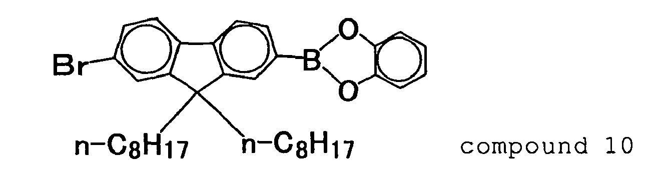 , the compound 6 (0.15 g, 0.09 mmol), palladium acetate (1.3 mg), tris(2-methoxyphenyl)phosphine (14.1 mg), Aliquat 336 (0.25 g) and toluene (18 ml) were mixed and heated at 105°C. Into the resultant reaction solution, a 2M sodium carbonate aqueous solution (5.2 ml) was dropped, and the mixture was refluxed for 1 hour. After the reaction, phenylboric acid (23.2 mg) was added and the resultant reaction solution was further refluxed for 3 hours. Then, to this was added a sodium diethyldithiacarbamate aqueous solution and the mixture was stirred at 80°C for 3 hours. After cooling, the resultant reaction liquid was washed with water (25 ml) twice, with a 3 wt% acetic acid aqueous solution (25 ml) twice and with water (25 ml) twice, and the resultant solution was dropped into methanol (300 mL) and filtrated to obtain a precipitate. The precipitate was dissolved in toluene (60 mL), and purified by passing through an alumina column and silica gel column. The resultant toluene solution was dropped into methanol (300 ml) and stirred, then, the resultant precipitate was filtrated and dried. The yield of this precipitate (hereinafter, referred to as "polymer compound 3") was 0.8 g.

The polymer compound 3 had a polystyrene-equivalent number average molecular weight of 2.3×10⁴ and a polystyrene-equivalent weight average molecular weight of 4.0×10⁴ and showed a fluorescence intensity of 9.1.

The polymer compound 3 is a copolymer constituted of a repeating unit of the following formula: 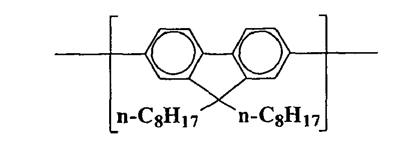 and a repeating unit of the following formula: 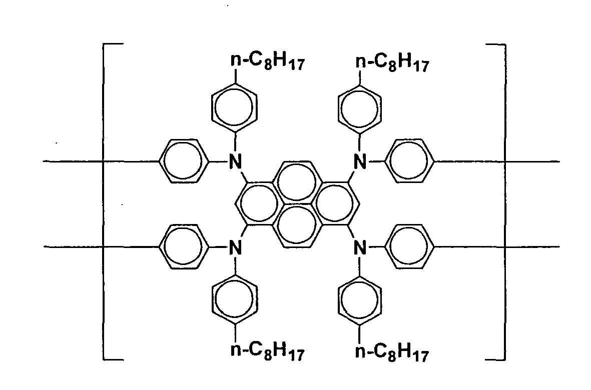 at a molar ratio of 97:3, according to theoretical values calculated from the charged raw materials.

### <Example 3> (Synthesis of polymer compound 4)

Under an inert atmosphere, the compound 7 (1.33 g, 2.52 mmol), the compound 8 (1.30 g, 2.38 mmol), the compound 16 (0.28 g, 0.15 mmol), palladium acetate (1.7 mg), tris(2-methoxyphenyl)phosphine (18.7 mg), Aliquat 33.6 (0.33 g) and toluene (26 ml) were mixed and heated at 105°C. Into this reaction solution, a 2M sodium carbonate aqueous solution (6.9 ml) was dropped, and the mixture was refluxed for 2.5 hours. After the reaction, phenylboric acid (30.8 mg) was added and the solution was further refluxed for 2 hours. Then, to this was added a sodium diethyldithiacarbamate aqueous solution and the mixture was stirred at 80°C for 3.5 hours. After cooling, the resultant reaction liquid was washed with water (33 ml) twice, with a 3 wt% acetic acid aqueous solution (33 ml) twice and with water (33 ml) twice, and the resultant solution was dropped into methanol (390 mL) and filtrated to obtain a precipitate. The precipitate was dissolved in toluene (79 mL), and purified by passing through an alumina column and silica gel column. The resultant toluene solution was dropped into methanol (390 ml) and stirred, then, the resultant precipitate was filtrated and dried. The yield of this precipitate (hereinafter, referred to as "polymer compound 4") was 1.5 g.

The polymer compound 4 had a polystyrene-equivalent number average molecular weight of 9.1×10⁴ and a polystyrene-equivalent weight average molecular weight of 2.2×10⁵ and showed a fluorescence intensity of 5.6.

The polymer compound 4 is a random copolymer constituted of a repeating unit of the following formula: 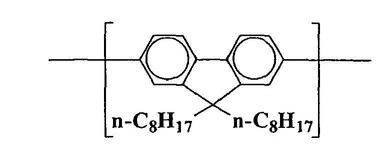 , and a repeating unit of the following formula: 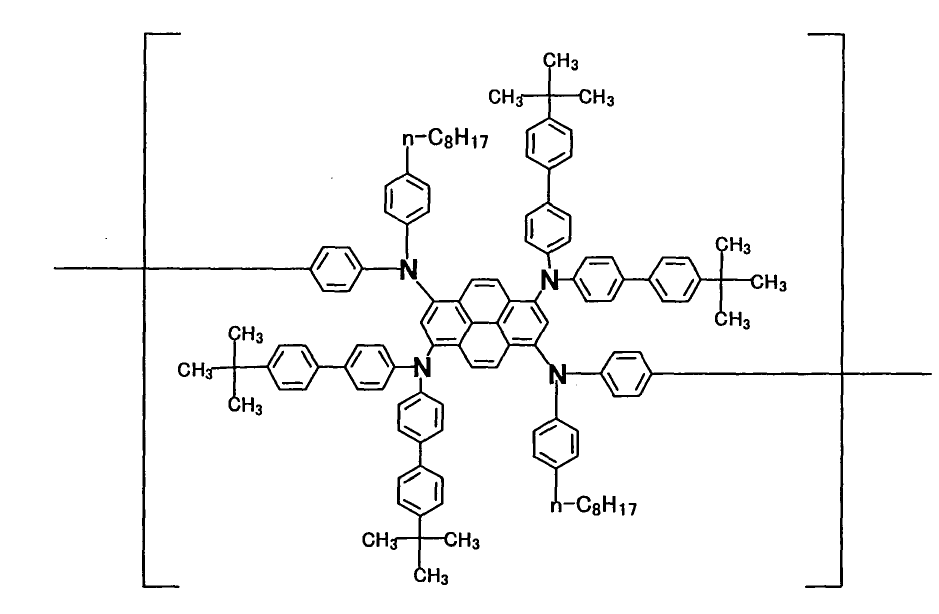 at a molar ratio of 97:3, according to theoretical values calculated from the charged raw materials.

### <Example 4> (Synthesis of polymer compound 5)

Under an inert atmosphere, the compound 7 (1.03 g, 1.94 mmol), the compound 8 (1.00 g, 1.82 mmol), the compound 20 (0.14 g, 0.12 mmol), palladium acetate (1.3 mg), tris(2-methoxyphenyl)phosphine (14.4 mg), Aliquat 336 (0.25 g) and toluene (19 ml) were mixed and heated at 105°C. Into the resultant reaction solution, a 2M sodium carbonate aqueous solution (5.3 ml) was dropped, and the mixture was refluxed for 14 hours. After the reaction, to this was added phenylboric acid (23.7 mg) and the resultant reaction solution was further refluxed for 2 hours. Then, to this was added a sodium diethyldithiacarbamate aqueous solution and the mixture was stirred at 80°C for 2 hours. After cooling, the resultant reaction liquid was washed with water (25 ml) twice, with a 3 wt% acetic acid aqueous solution (25 ml) twice and with water (25 ml) twice, and the resultant solution was dropped into methanol (300 mL) and filtrated to obtain a precipitate. The precipitate was dissolved in toluene (60 mL), and purified by passing through an alumina column and silica gel column sequentially. The resultant toluene solution was dropped into methanol (300 ml) and stirred, then, the resultant precipitate was filtrated and dried. The yield of this precipitate (hereinafter, referred to as "polymer compound 5") was 1.1 g.

The polymer compound 5 had a polystyrene-equivalent number average molecular weight of 1.3×10⁴ and a polystyrene-equivalent weight average molecular weight of 2.7×10⁴ and showed a fluorescence intensity of 4.8.

The polymer compound 5 is a random copolymer constituted of a repeating unit of the following formula: 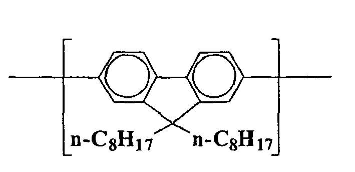 , and a repeating unit of the following formula: 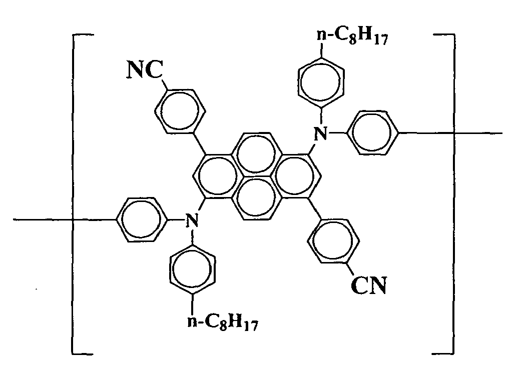 at a molar ratio of 97:3, according to theoretical values calculated from the charged raw materials.

### <Synthesis Example 19> (Synthesis of polymer compound 6)

Under an inert atmosphere, the above-described compound 7 (5.20 g), a compound 21 (5.42 g) of the following formula: 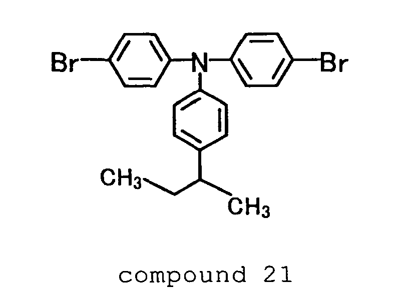 , palladium acetate (2.2 mg), tris(2-methylphenyl)phosphine (15.1 mg), Aliquat 336 (0.91 g) and toluene (70 mL) were mixed and heated at 105°C. Into this reaction solution, a 2M sodium carbonate aqueous solution (19 ml) was dropped, and the mixture was refluxed for 4 hours. After the reaction, phenylboric acid (121 mg) was added and the solution was further refluxed for 3 hours. Then, to this was added a sodium diethyldithiacarbamate aqueous solution and the mixture was stirred at 80°C for 2 hours. After cooling, the resultant reaction liquid was washed with water (60 ml) three times, with a 3 wt% acetic acid aqueous solution (60 ml) four times and with water (60 ml) three times, and the resultant solution was purified by passing through an alumina column and silica gel column. The resultant toluene solution was dropped into methanol (3 L) and stirred, then, the resultant precipitate was filtrated and dried. The yield of this precipitate (hereinafter, referred to as "polymer compound 6") was 5.25 g.

The polymer compound 6 had a polystyrene-equivalent number average molecular weight of 1.2×10⁵ and a polystyrene-equivalent weight average molecular weight of 2.6×10⁵.

The polymer compound 6 is a random copolymer constituted of a repeating unit of the following formula: 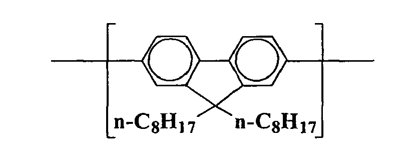 , and a repeating unit of the following formula: 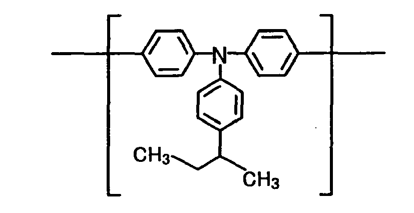 at a molar ratio of 50:50, according to theoretical values calculated from the charged raw materials.

### <Example 5>

On a glass substrate carrying thereon an ITO film with a thickness of 150 nm formed by a sputtering method, a suspension of poly(3,4-ethylenedioxythiophene)/polystyrenesulfonic acid (manufactured by Bayer, trade name: Baytron P; hereinafter, referred to as "Baytron P") was spin-coated to form a film with a thickness of about 65 nm which was then dried on a hot plate at 200°C for 15 minutes. The polymer compound 2 was dissolved in xylene (Kanto Chemical Co., Inc., EL grade) at a concentration of 1.3 wt%, and the resultant xylene solution was spin-coated to form a film having a thickness of about 110 nm. This was dried at 130°C for 1 hour under a nitrogen atmosphere in which the oxygen concentration and water concentration were 10 ppm or less (by weight). After reducing pressure to 1×10⁻⁴ Pa or lower, barium was vapor-deposited with a thickness of about 5 nm, then, aluminum was vapor-deposited with a thickness of about 80 nm, as cathodes. After vapor deposition, sealing was performed using a glass substrate to fabricate a polymer light emitting device A. The device constitution was ITO/BaytronP (about 65 nm)/polymer compound 2 (about 110 nm)/Ba (5 nm)/Al (80 nm).

By applying voltage on the polymer light emitting device A, green light emission showing a peak wavelength of 495 nm was obtained. The polymer light emitting device A showed green light emission, and an external quantum yield at 6.0 V of 2.3%.

### <Example 6>

A polymer light emitting device B was fabricated in the same manner as in Example 5 excepting that the polymer compound 3 was dissolved in xylene (Kanto Chemical Co., Inc., EL grade) at a concentration of 2.0 wt% instead of dissolving the polymer compound 2 in xylene (Kanto Chemica Co., Inc., EL grade) at a concentration of 1.3 wt%, in Example 5. The device constitution was ITO/BaytronP (about 65 nm)/polymer compound 3 (about 110 nm)/Ba (5 nm)/Al (80 nm).

By applying voltage on the polymer light emitting device B, green light emission showing a peak wavelength of 510 nm was obtained. The polymer light emitting device B showed green light emission, and an external quantum yield at 6.0 V of 0.9%.

### <Comparative Example 2>

A polymer light emitting device C was fabricated in the same manner as in Example 5 excepting that the polymer compound 1 was dissolved in xylene (Kanto Chemical Co., Inc., EL grade) at a concentration of 2.0 wt% instead of dissolving the polymer compound 2 in xylene (Kanto Chemica Co., Inc., EL grade) at a concentration of 1.3 wt%, in Example 5. The device constitution was ITO/BaytronP (about 65 nm)/polymer compound 1 (about 110 nm)/Ba (5 nm)/Al (80 nm).

By applying voltage on the polymer light emitting device C, green light emission showing a peak wavelength of 525 nm was obtained. The polymer light emitting device C showed green light emission, and an external quantum yield at 6.0 V of 0.6%.

**Table 1**

| | Polymer compound | Polystyrene-equivalent | | fluorescence intensity |
|---|---|---|---|---|
| | | Number average molecular weight | Weight average molecular weight | |
| Comparative Example 1 | 1 | 1.4×10⁵ | 3.5×10⁵ | 4.6 |
| Example 1 | 2 | 1.0×10⁵ | 2.4×10⁵ | 6.8 |
| Example 2 | 3 | 2.3×10⁴ | 4.0×10⁴ | 9.1 |
| Example 3 | 4 | 9.1×10⁴ | 2.2×10⁵ | 5.6 |
| Example 4 | 5 | 1.3×10⁴ | 2.7×10⁴ | 4.8 |

**Table 2**

| | Polymer compound | Polymer light emitting device | External quantum efficiency (%) |
|---|---|---|---|
| Example 5 | 2 | A | 2.3 |
| Example 6 | 3 | B | 0.9 |
| Comparative Example 2 | 1 | C | 0.6 |

It is recognized from Table 1 and Table 2 that the polymer compounds of the present invention (Examples) show higher external quantum yield (that is, light emission efficiency is higher), show equal fluorescence intensity or more, and excellent in a balance between "fluorescence intensity" and "light emission efficiency of a light emitting device obtained when used for fabrication of a light emitting device", as compared with other polymer compounds (Comparative Examples).

### <Example 7>

On a glass substrate carrying thereon an ITO film with a thickness of 150 nm formed by a sputtering method, a suspension of Baytron P was spin-coated to form a film with a thickness of about 65 nm which was then dried on a hot plate at 200°C for 15 minutes. The polymer compound 4 was dissolved in xylene (Kanto Chemical Co., Inc., EL grade) at a concentration of 1.8 wt%, and the resultant xylene solution was spin-coated to form a film having a thickness of about 140 nm. This was dried at 130°C for 1 hour under a nitrogen atmosphere in which the oxygen concentration and water concentration were 10 ppm or less (by weight). After reducing pressure to 1×10⁻⁴ Pa or lower, barium was vapor-deposited with a thickness of about 5 nm, then, aluminum was vapor-deposited with a thickness of about 80 nm, as cathodes. After vapor deposition, sealing was performed using a glass substrate to fabricate a polymer light emitting device D. The device constitution was ITO/BaytronP (about 65 nm)/polymer compound 4 (about 140 nm)/Ba (5 nm)/Al (80 nm).

By applying voltage on the polymer light emitting device D, green light emission showing a peak wavelength of 520 nm was obtained. The polymer light emitting device D showed green light emission, and an external quantum yield at 6.0 V of 1.3%.

### <Comparative Example 3>

On a glass substrate carrying thereon an ITO film with a thickness of 150 nm formed by a sputtering method, a suspension of Baytron P was spin-coated to form a film with a thickness of about 65 nm which was then dried on a hot plate at 200°C for 15 minutes. The polymer compound 1 was dissolved in xylene (Kanto Chemical Co., Inc., EL grade) at a concentration of 1.4 wt%, and the resultant xylene solution was spin-coated to form a film having a thickness of about 140 nm. This was dried at 130°C for 1 hour under a nitrogen atmosphere in which the oxygen concentration and water concentration were 10 ppm or less (by weight). After reducing pressure to 1×10⁻⁴ Pa or lower, barium was vapor-deposited with a thickness of about 5 nm, then, aluminum was vapor-deposited with a thickness of about 80 nm, as cathodes. After vapor deposition, sealing was performed using a glass substrate to fabricate a polymer light emitting device E. The device constitution was ITO/BaytronP (about 65 nm)/polymer compound 1 (about 140 nm)/Ba (5 nm)/Al (80 nm).

By applying voltage on the polymer light emitting device E, green light emission showing a peak wavelength of 525 nm was obtained. The polymer light emitting device E showed green light emission, and an external quantum yield at 6.0 V of 0.5%.

**Table 3**

| | Polymer compound | Polymer light emitting device | External quantum yield (%) |
|---|---|---|---|
| Example 7 | 4 | D | 1.3 |
| Comparative Example 3 | 1 | E | 0.5 |

It is recognized from Table 1 and Table 3 that the polymer compounds of the present invention (Examples) show higher external quantum yield (that is, light emission efficiency is higher), also show higher fluorescence intensity, and excellent in a balance between "fluorescence intensity" and "light emission efficiency of a light emitting device obtained when used for fabrication of a light emitting device", as compared with other polymer compounds (Comparative Examples).

### <Example 8>

On a glass substrate carrying thereon an ITO film with a thickness of 150 nm formed by a sputtering method, a suspension of Baytron P was spin-coated to form a film with a thickness of about 65 nm which was then dried on a hot plate at 200°C for 15 minutes. The polymer compound 6 was dissolved in xylene (Kanto Chemical Co., Inc., EL grade) at a concentration of 0.5 wt%, and the resultant xylene solution was spin-coated to form a film having a thickness of about 10 nm. This was dried at 180°C for 15 minutes under a nitrogen atmosphere in which the oxygen concentration and water concentration were 10 ppm or less (by weight). The polymer compound 4 was dissolved in xylene (Kanto Chemical Co., Inc., EL grade) at a concentration of 1.8 wt%, and the resultant xylene solution was spin-coated to form a film having a thickness of about 130 nm. This was dried at 130°C for 1 hour under a nitrogen atmosphere in which the oxygen concentration and water concentration were 10 ppm or less (by weight). After reducing pressure to 1×10⁻⁴ Pa or lower, barium was vapor-deposited with a thickness of about 5 nm, then, aluminum was vapor-deposited with a thickness of about 80 nm, as cathodes. After vapor deposition, sealing was performed using a glass substrate to fabricate a polymer light emitting device F. The device constitution was ITO/BaytronP (about 65 nm)/polymer compound 6 (about 10 nm)/polymer compound 4 (about 130 nm)/Ba (5 nm)/Al (80 nm).

By applying voltage on the polymer light emitting device F, green light emission showing a peak wavelength of 520 nm was obtained. The polymer light emitting device F showed green light emission, and an external quantum yield at 8.0 V of 1.1%.

### <Example 9>

On a glass substrate carrying thereon an ITO film with a thickness of 150 nm formed by a sputtering method, a suspension of Baytron P was spin-coated to form a film with a thickness of about 65 nm which was then dried on a hot plate at 200°C for 15 minutes. The polymer compound 6 was dissolved in xylene (Kanto Chemical Co., Inc., EL grade) at a concentration of 0.5 wt%, and the resultant xylene solution was spin-coated to form a film having a thickness of about 10 nm. This was dried at 180°C for 15 minutes under a nitrogen atmosphere in which the oxygen concentration and water concentration were 10 ppm or less (by weight). The polymer compound 5 was dissolved in xylene (Kanto Chemical Co., Inc., EL grade) at a concentration of 2.5 wt%, and the resultant xylene solution was spin-coated to form a film having a thickness of about 130 nm. This was dried at 130°C for 1 hour under a nitrogen atmosphere in which the oxygen concentration and water concentration were 10 ppm or less (by weight). After reducing pressure to 1×10⁻⁴ Pa or lower, barium was vapor-deposited with a thickness of about 5 nm, then, aluminum was vapor-deposited with a thickness of about 80 nm, as cathodes. After vapor deposition, sealing was performed using a glass substrate to fabricate a polymer light emitting device G. The device constitution was ITO/BaytronP (about 65 nm)/polymer compound 6 (about 10 nm)/polymer compound 5 (about 130 nm)/Ba (5 nm)/Al (80 nm).

By applying voltage on the polymer light emitting device G, green light emission showing a peak wavelength of 540 nm was obtained. The polymer light emitting device G showed green light emission, and an external quantum yield at 8.0 V of 1.4%.

### <Comparative Example 4>

On a glass substrate carrying thereon an ITO film with a thickness of 150 nm formed by a sputtering method, a suspension of Baytron P was spin-coated to form a film with a thickness of about 65 nm which was then dried on a hot plate at 200°C for 15 minutes. The polymer compound 6 was dissolved in xylene (Kanto Chemical Co., Inc., EL grade) at a concentration of 0.5 wt%, and the resultant xylene solution was spin-coated to form a film having a thickness of about 10 nm. This was dried at 180°C for 15 minutes under a nitrogen atmosphere in which the oxygen concentration and water concentration were 10 ppm or less (by weight). The polymer compound 1 was dissolved in xylene (Kanto Chemical Co., Inc., EL grade) at a concentration of 1.4 wt%, and the resultant xylene solution was spin-coated to form a film having a thickness of about 130 nm. This was dried at 130°C for 1 hour under a nitrogen atmosphere in which the oxygen concentration and water concentration were 10 ppm or less (by weight). After reducing pressure to 1×10⁻⁴ Pa or lower, barium was vapor-deposited with a thickness of about 5 nm, then, aluminum was vapor-deposited with a thickness of about 80 nm, as cathodes. After vapor deposition, sealing was performed using a glass substrate to fabricate a polymer light emitting device H. The device constitution was ITO/BaytronP (about 65 nm)/polymer compound 6 (about 10 nm)/polymer compound 1 (about 130 nm)/Ba (5 nm)/Al (80 nm).

By applying voltage on the polymer light emitting device H, green light emission showing a peak wavelength of 530 nm was obtained. The polymer light emitting device H showed green light emission, and an external quantum yield at 8.0 V of 0.7%.

**Table 4**

| | Polymer compound | Polymer light emitting device | External quantum yield (%) |
|---|---|---|---|
| Example 8 | 4 | F | 1.1 |
| Example 9 | 5 | G | 1.4 |
| Comparative Example 4 | 1 | H | 0.7 |

It is recognized from Table 1 and Table 4 that the polymer compounds of the present invention (Examples) show higher external quantum yield (that is, light emission efficiency is higher), show equal fluorescence intensity or more, and excellent in a balance between "fluorescence intensity" and "light emission efficiency of a light emitting device obtained when used for fabrication of a light emitting device", as compared with other polymer compounds (Comparative Examples).

### <Comparative Example 5> (Synthesis of polymer compound 7)

Under an inert atmosphere, the compound 7 (1.32 g, 2.50 mmol), the compound 8 (1.15 g, 2.10 mmol), a compound 22 (0.12 g, 0.25 mmol) of the following formula: 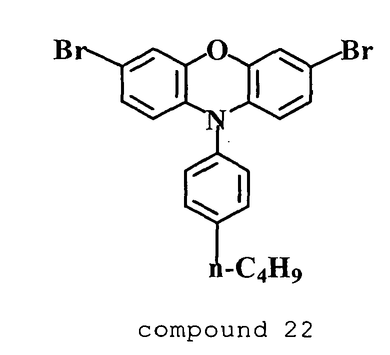 , the compound 9 (0.10 g, 0.15 mmol), palladium acetate (1.7 mg), tris(2-methoxyphenyl)phosphine (18.5 mg), Aliquat 336 (0.32 g) and toluene (41 ml) were mixed and heated at 105°C. Into this reaction solution, a 2M sodium carbonate aqueous solution (6.8 ml) was dropped, and the mixture was refluxed for 1.5 hours. After the reaction, phenylboric acid (30.5 mg) was added and the solution was further refluxed for 2 hours. Then, to this was added a sodium diethyldithiacarbamate aqueous solution and the mixture was stirred at 80°C for 2 hours. After cooling, the resultant reaction liquid was washed with water (33 ml) twice, with a 3 wt% acetic acid aqueous solution (33 ml) twice and with water (33 ml) twice, and the resultant solution was dropped into methanol (500 mL) and filtrated to obtain a precipitate. This precipitate was dissolved in toluene (78 mL), and purified by passing through an alumina column and silica gel column. The resultant toluene solution was dropped into methanol (500 ml) and stirred, then, the resultant precipitate was filtrated and dried. The yield of this precipitate (hereinafter, referred to as "polymer compound 7") was 1.4 g.

The polymer compound 7 had a polystyrene-equivalent number average molecular weight of 1.7×10⁵ and a polystyrene-equivalent weight average molecular weight of 4.2×10⁵, and a fluorescence intensity of 4.1.

The polymer compound 7 is a random copolymer constituted of a repeating unit of the following formula: 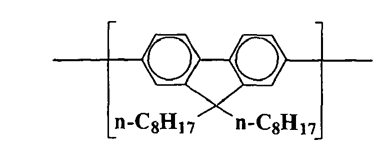 , ad repeating unit of the following formula: 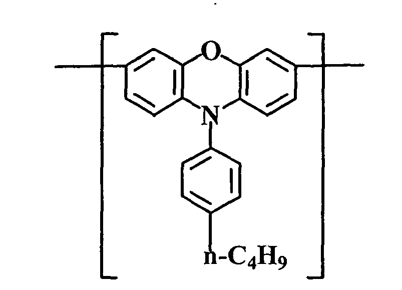 , and a repeating unit of the following formula: 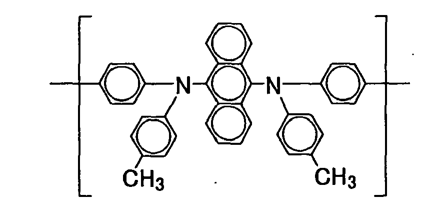 at a molar ratio of 92:5:3, according to theoretical values calculated from the charged raw materials.

### <Example 10> (Synthesis of polymer compound 8)

Under an inert atmosphere, the compound 7 (0.66 g, 1.25 mmol), the compound 8 (0.58 g, 1.05 mmol), the compound 22 (0.06 g, 0.13 mmol), the compound 16 (0.14 g, 0.08 mmol), palladium acetate (0.8 mg), tris(2-methoxyphenyl)phosphine (9.3 mg), Aliquat 336 (0.16 g) and toluene (22 ml) were mixed and heated at 105°C. Into the resultant reaction solution, a 2M sodium carbonate aqueous solution (3.4 ml) was dropped, and the mixture was refluxed for 1.5 hours. After the reaction, phenylboric acid (15.3 mg) was added and the solution was further refluxed for 2 hours. Then, to this was added a sodium diethyldithiacarbamate aqueous solution and the mixture was stirred at 80°C for 2 hours. After cooling, the resultant reaction liquid was washed with water (16 ml) twice, with a 3 wt% acetic acid aqueous solution (15 ml) twice and with water (15 ml) twice, and the resultant solution was dropped into methanol (200 mL) and filtrated to obtain a precipitate. This precipitate was dissolved in toluene (39 mL), and purified by passing through an alumina column and silica gel column. The resultant toluene solution was dropped into methanol (200 ml) and stirred, then, the resultant precipitate was filtrated and dried. The yield of this precipitate (hereinafter, referred to as "polymer compound 8") was 0.7 g.

The polymer compound 8 had a polystyrene-equivalent number average molecular weight of 1.3×10⁵ and a polystyrene-equivalent weight average molecular weight of 3.0×10⁵, and a fluorescence intensity of 4.2.

The polymer compound 8 is a random copolymer constituted of a repeating unit of the following formula: 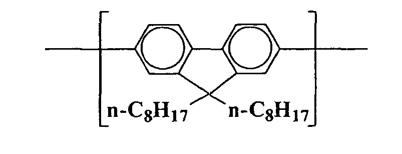 , a repeating unit of the following formula: 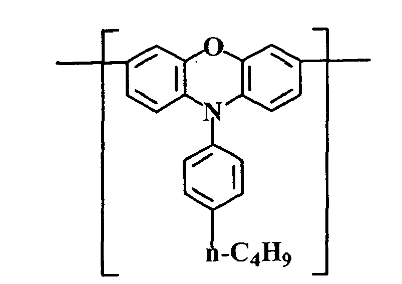 , and a repeating unit of the following formula: 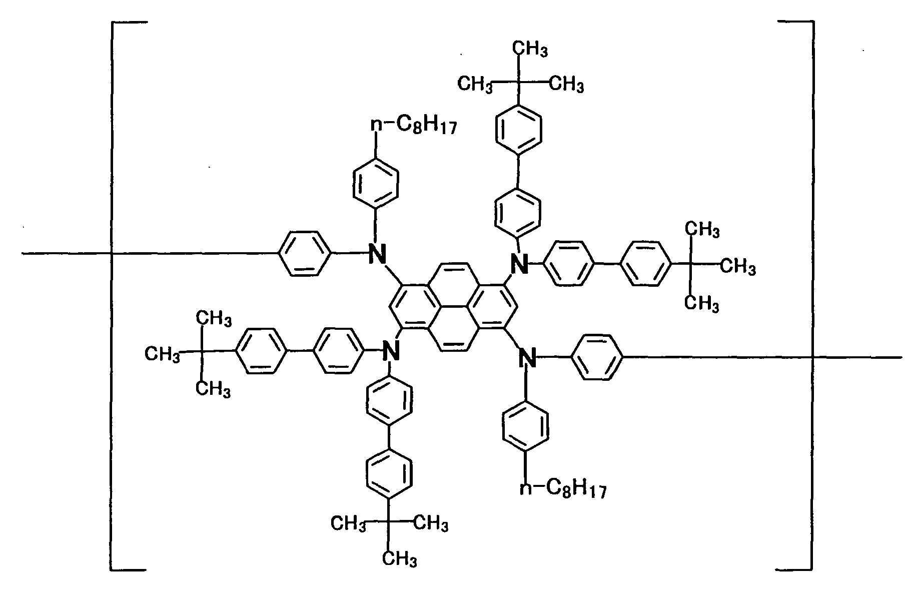 at a molar ratio of 92:5:3, according to theoretical values calculated from the charged raw materials.

### <Example 11>

On a glass substrate carrying thereon an ITO film with a thickness of 150 nm formed by a sputtering method, a suspension of Baytron P was spin-coated to form a film with a thickness of about 65 nm which was then dried on a hot plate at 200°C for 10 minutes. The polymer compound 8 was dissolved in xylene (Kanto Chemical Co., Inc., EL grade) at a concentration of 1.9 wt%, and the resultant xylene solution was spin-coated to form a film having a thickness of about 120 nm. This was dried at 130°C for 1 hour under a nitrogen atmosphere in which the oxygen concentration and water concentration were 10 ppm or less (by weight). After reducing pressure to 1×10⁻⁴ Pa or lower, barium was vapor-deposited with a thickness of about 5 nm, then, aluminum was vapor-deposited with a thickness of about 80 nm, as cathodes. After vapor deposition, sealing was performed using a glass substrate to fabricate a polymer light emitting device I. The device constitution was ITO/BaytronP (about 65 nm)/polymer compound 8 (about 120 nm)/Ba (5 nm)/Al (80 nm).

By applying voltage on the polymer light emitting device I, green light emission showing a peak wavelength of 520 nm was obtained. The polymer light emitting device I showed green light emission, and an external quantum yield at 10.0 V of 0.7%.

### <Comparative Example 6>

On a glass substrate carrying thereon an ITO film with a thickness of 150 nm formed by a sputtering method, a suspension of Baytron P was spin-coated to form a film with a thickness of about 65 nm which was then dried on a hot plate at 200°C for 10 minutes. The polymer compound 7 was dissolved in xylene (Kanto Chemical Co., Inc., EL grade) at a concentration of 1.7 wt%, and the resultant xylene solution was spin-coated to form a film having a thickness of about 120 nm. This was dried at 130°C for 1 hour under a nitrogen atmosphere in which the oxygen concentration and water concentration were 10 ppm or less (by weight). After reducing pressure to 1×10⁻⁴ Pa or lower, barium was vapor-deposited with a thickness of about 5 nm, then, aluminum was vapor-deposited with a thickness of about 80 nm, as cathodes. After vapor deposition, sealing was performed using a glass substrate to fabricate a polymer light emitting device J. The device constitution was ITO/BaytronP (about 65 nm)/polymer compound 7 (about 120 nm)/Ba (5 nm)/Al (80 nm).

By applying voltage on the polymer light emitting device J, green light emission showing a peak wavelength of 525 nm was obtained. The polymer light emitting device J showed green light emission, and an external quantum yield at 10.0 V of 0.3%.

**Table 5**

| | polymer compound | Polystyrene-equivalent | | fluorescence intensity |
|---|---|---|---|---|
| | | Number average molecular weight | Weight average molecular weight | |
| Comparative Example 5 | 7 | 1.7×10⁵ | 4.2×10⁵ | 4.1 |
| Example 10 | 8 | 1.3×10⁵ | 3.0×10⁵ | 4.2 |

**Table 6**

| | polymer compound | Polymer light emitting device | External quantum yield (%) |
|---|---|---|---|
| Example 11 | 8 | I | 0.7 |
| Comparative Example 6 | 7 | J | 0.3 |

It is recognized from Table 5 and Table 6 that the polymer compounds of the present invention (Examples) show higher external quantum yield (that is, light emission efficiency is higher), show equal fluorescence intensity or more, and excellent in a balance between "fluorescence intensity" and "light emission efficiency of a light emitting device obtained when used for fabrication of a light emitting device", as compared with other polymer compounds (Comparative Examples).

### <Comparative Example 7> (Synthesis of polymer compound 9)

Under an inert atmosphere, the compound 7 (0.79 g, 1.50 mmol), the compound 8 (0.69 g, 1.26 mmol), a compound 23 (0.10 g, 0.15 mmol) of the following formula: 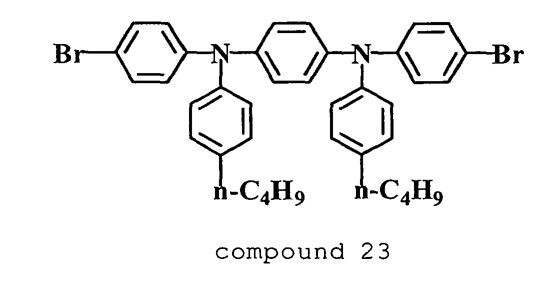 , the compound 9 (0.06 g, 0.09 mmol), palladium acetate (1.0 mg), tris(2-methoxyphenyl)phosphine (11.1 mg), Aliquat 336 (0.19 g) and toluene (26 ml) were mixed and heated at 105°C. Into the resultant reaction solution, a 2M sodium carbonate aqueous solution (4.1 ml) was dropped, and the mixture was refluxed for 2.5 hours. After the reaction, phenylboric acid (18.3 mg) was added and the solution was further refluxed for 2 hours. Then, to this was added a sodium diethyldithiacarbamate aqueous solution and the mixture was stirred at 80°C for 2 hours. After cooling, the resultant reaction liquid was washed with water (20 ml) twice, with a 3 wt% acetic acid aqueous solution (20 ml) twice and with water (20 ml) twice, and the resultant solution was dropped into methanol (233 mL) and filtrated to obtain a precipitate. This precipitate was dissolved in toluene (57 mL), and purified by passing through an alumina column and silica gel column. The resultant toluene solution was dropped into methanol (230 ml) and stirred, then, the resultant precipitate was filtrated and dried. The yield of this precipitate (hereinafter, referred to as "polymer compound 9") was 0.8 g.

The polymer compound 9 had a polystyrene-equivalent number average molecular weight of 1.4×10⁵ and a polystyrene-equivalent weight average molecular weight of 3.3×10⁵, and a fluorescence intensity of 4.5.

The polymer compound 9 is a random copolymer constituted of a repeating unit of the following formula: 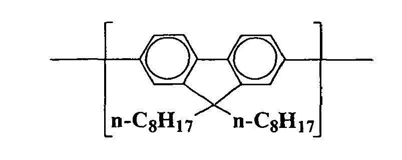 , a repeating unit of the following formula: 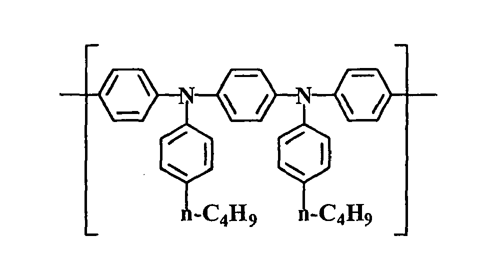 , and a repeating unit of the following formula: 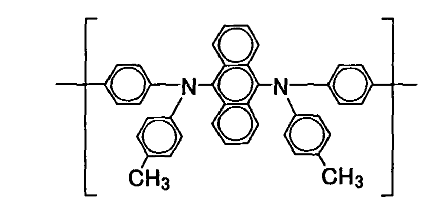 at a molar ratio of 92:5:3, according to theoretical values calculated from the charged raw materials.

### <Example 12> (Synthesis of polymer compound 10)

Under an inert atmosphere, the above-described compound 7 (0.66 g, 1.25 mmol), the compound 8 (0.58 g, 1.05 mmol), the compound 23 (0.09 g, 0.15 mmol), the compound 16 (0.14 g, 0.08 mmol), palladium acetate (0.8 mg), tris(2-methoxyphenyl)phosphine (9.3 mg), Aliquat 336 (0.16 g) and toluene (22 ml) were mixed and heated at 105°C. Into the resultant reaction solution, a 2M sodium carbonate aqueous solution (3.4 ml) was dropped, and the mixture was refluxed for 1.5 hours. After the reaction, phenylboric acid (15.3 mg) was added and the solution was further refluxed for 2 hours. Then, to this was added a sodium diethyldithiacarbamate aqueous solution and the mixture was stirred at 80°C for 2 hours. After cooling, the resultant reaction liquid was washed with water (16 ml) twice, with a 3 wt% acetic acid aqueous solution (16 ml) twice and with water (16 ml) twice, and the resultant solution was dropped into methanol (200 mL) and filtrated to obtain a precipitate. This precipitate was dissolved in toluene (39 mL), and purified by passing through an alumina column and silica gel column. The resultant toluene solution was dropped into methanol (200 ml) and stirred, then, the resultant precipitate was filtrated and dried. The yield of this precipitate (hereinafter, referred to as "polymer compound 10") was 0.8 g.

The polymer compound 10 had a polystyrene-equivalent number average molecular weight of 1.1×10⁵ and a polystyrene-equivalent weight average molecular weight of 2.4×10⁵, and a fluorescence intensity of 4.7.

The polymer compound 10 is a random copolymer constituted of a repeating unit of the following formula: 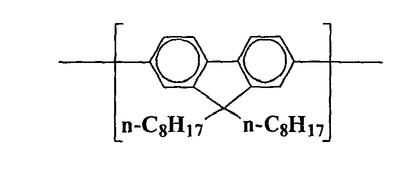 , a repeating unit of the following formula: 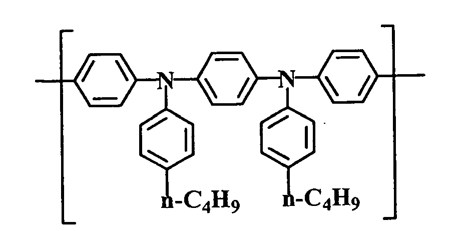 , and a repeating unit of the following formula: 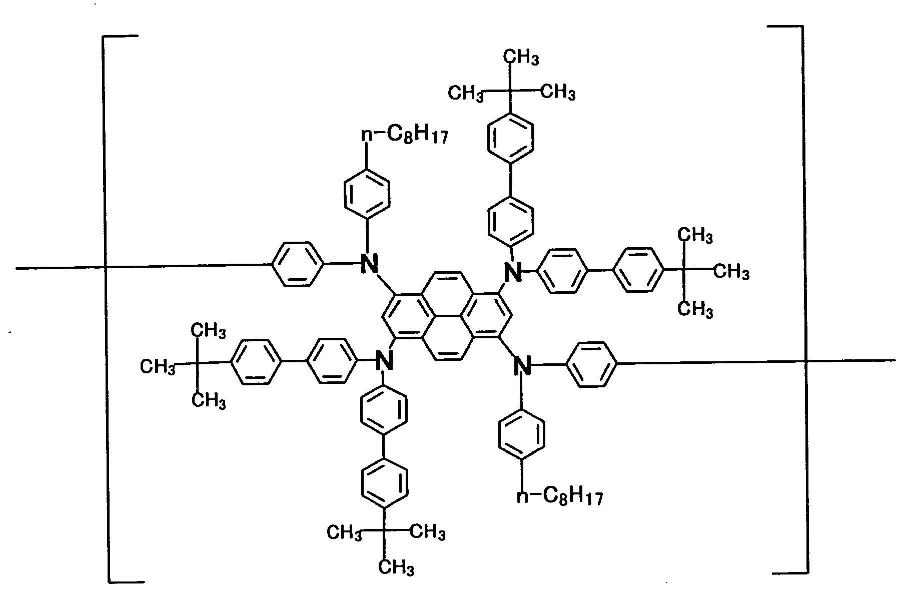 at a molar ratio of 92:5:3, according to theoretical values calculated from the charged raw materials.

### <Example 13>

On a glass substrate carrying thereon an ITO film with a thickness of 150 nm formed by a sputtering method, a suspension of Baytron P was spin-coated to form a film with a thickness of about 65 nm which was then dried on a hot plate at 200°C for 10 minutes. The polymer compound 10 was dissolved in xylene (Kanto Chemical Co., Inc., EL grade) at a concentration of 2.0 wt%, and the resultant xylene solution was spin-coated to form a film having a thickness of about 120 nm. This was dried at 130°C for 1 hour under a nitrogen atmosphere in which the oxygen concentration and water concentration were 10 ppm or less (by weight). After reducing pressure to 1×10⁻⁴ Pa or lower, barium was vapor-deposited with a thickness of about 5 nm, then, aluminum was vapor-deposited with a thickness of about 80 nm, as cathodes. After vapor deposition, sealing was performed using a glass substrate to fabricate a polymer light emitting device K. The device constitution was ITO/BaytronP (about 65 nm)/polymer compound 10 (about 120 nm)/Ba (5 nm)/Al (80 nm).

By applying voltage on the polymer light emitting device K, green light emission showing a peak wavelength of 520 nm was obtained. The polymer light emitting device K showed green light emission, and an external quantum yield at 10.0 V of 1.3%.

### <Comparative Example 8>

On a glass substrate carrying thereon an ITO film with a thickness of 150 nm formed by a sputtering method, a suspension of Baytron P was spin-coated to form a film with a thickness of about 65 nm which was then dried on a hot plate at 200°C for 10 minutes. The polymer compound 9 was dissolved in xylene (Kanto Chemical Co., Inc., EL grade) at a concentration of 1.8 wt%, and the resultant xylene solution was spin-coated to form a film having a thickness of about 120 nm. This was dried at 130°C for 1 hour under a nitrogen atmosphere in which the oxygen concentration and water concentration were 10 ppm or less (by weight). After reducing pressure to 1×10⁻⁴ Pa or lower, barium was vapor-deposited with a thickness of about 5 nm, then, aluminum was vapor-deposited with a thickness of about 80 nm, as cathodes. After vapor deposition, sealing was performed using a glass substrate to fabricate a polymer light emitting device L. The device constitution was ITO/BaytronP (about 65 nm)/polymer compound 9 (about 120 nm)/Ba (5 nm)/Al (80 nm).

By applying voltage on the polymer light emitting device L, green light emission showing a peak wavelength of 525 nm was obtained. The polymer light emitting device L showed green light emission, and an external quantum yield at 10.0 V of 0.4%.

**Table 7**

| | polymer compound | Polystyrene-equivalemt | | fluorescence intensity |
|---|---|---|---|---|
| | | Number average molecular weight | Weight average molecular weight | |
| Comparative Example 7 | 9 | 1.4×10⁵ | 3.3×10⁵ | 4.5 |
| Example 12 | 10 | 1.1×10⁵ | 2.4×10⁵ | 4.7 |

**Table 8**

| | polymer compound | Polymer light emitting device | External quantum yield (%) |
|---|---|---|---|
| Example 13 | 10 | K | 1.3 |
| Comparative Example 8 | 9 | L | 0.4 |

It is recognized from Table 7 and Table 8 that the polymer compounds of the present invention (Examples) show higher external quantum yield (that is, light emission efficiency is higher), show equal fluorescence intensity or more, and excellent in a balance between "fluorescence intensity" and "light emission efficiency of a light emitting device obtained when used for fabrication of a light emitting device", as compared with other polymer compounds (Comparative Examples).

### Industrial Applicability

The polymer compound of the present invention is excellent in a balance between "fluorescence intensity" and "light emission efficiency of a light emitting device obtained when used for fabrication of a light emitting device", and useful, for example, as an electronic part material such as light emitting materials, charge transporting materials and the like. The polymer compound of the present invention is usually excellent also in heat resistance. The light emitting device of the present invention fabricated using such a polymer compound is excellent also in device life. Therefore, the polymer compound and light emitting device of the present invention are, for example, useful for liquid crystal display back light, light sources in the form of curved surface or plat surface for the purpose of illumination, segment type displays, dot matrix type flat panel displays and the like.

## Claims

1. A polymer compound comprising a constitutional unit of the following formula (1), and at least one repeating unit selected from the group consisting of a repeating unit of the following formula (2), a repeating unit of the following formula (3) and a repeating unit of the following formula (4): 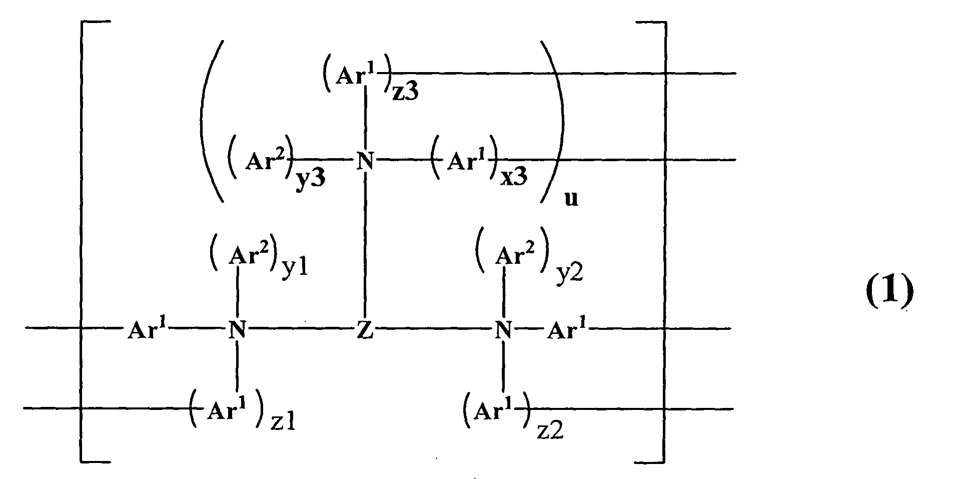 (wherein, x3, y1, y2, z1, z2 and z3 represent each independently 0 or 1. y3 represents an integer of 0 to Here, y1+z1 and y2+z2 are 1, and x3+y3+z3 is 2. u represents an integer of 0 to 8. When u is 2 or more, x3's, y3's and z3's may each be the same or different. Z represents an unsubstituted or substituted (2+u)-valent pyrene residue, Ar¹ represents an unsubstituted or substituted arylene group or an unsubstituted or substituted di-valent heterocyclic group, and Ar² represents an unsubstituted or substituted aryl group or an unsubstituted or substituted mono-valent heterocyclic group. A plurality of Ar¹'s may be the same or different. When there exist a plurality of Ar²'s, they may be the same or different.)
⁅Ar³⁆ (2) 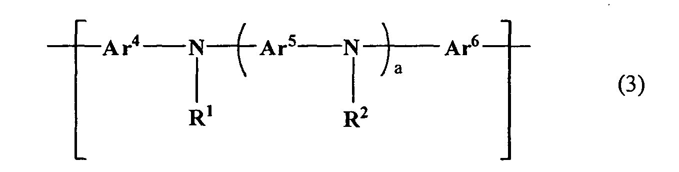
⁅Ar⁷-X¹⁆ (4)
(wherein, Ar³ and Ar⁷ represent each independently an unsubstituted or substituted arylene group, an unsubstituted or substituted di-valent heterocyclic group or a di-valent group having a metal complex structure. Ar⁴, Ar⁵ and Ar⁶ represent each independently an unsubstituted or substituted arylene group, an unsubstituted or substituted di-valent heterocyclic group or an unsubstituted or substituted di-valent group having two aromatic rings connected via a single bond. R¹ and R² represent each independently a hydrogen atom, alkyl group, aryl group, mono-valent heterocyclic group or arylalkyl group. X¹ represents -CR³=CR⁴- or -C≡C-. Here, R³ and R⁴ represent each independently a hydrogen atom, alkyl group, aryl group, mono-valent heterocyclic group, carboxyl group, substituted carboxyl group or cyano group. a represents 0 or 1.).

2. The polymer compound according to Claim 1, wherein said constitutional unit of the formula (1) is a constitutional unit of the following formula (1A) or (1B): 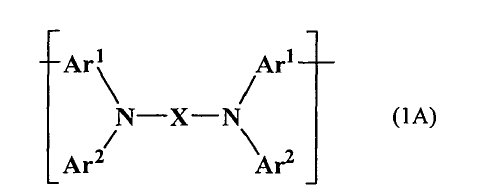 (wherein, X represents an unsubstituted or substituted pyrenediyl group. Ar¹ and Ar² are as described above. Two Ar¹'s may be the same or different. Two Ar²'s may be the same or different.) 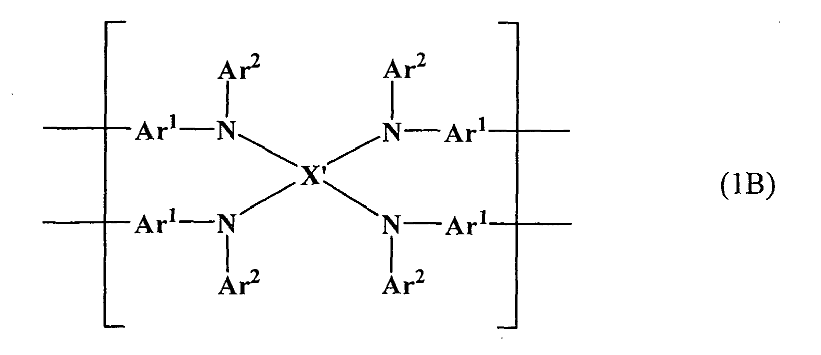 (wherein, X' represents an substituted or substituted tetra-valent pyrene residue. Ar¹ and Ar² are as described above. Four Ar¹'s may be the same or different. Four Ar²'s may be the same or different.).

3. The polymer compound according to Claim 1 or 2, wherein at least one of Ar¹'s is an unsubstituted or substituted arylene group.

4. The polymer compound according to Claim 3, wherein the unsubstituted or substituted arylene group represented by Ar¹ is an unsubstituted or substituted phenylene group.

5. The polymer compound according to any one of Claims 1 to 4, wherein at least one of Ar²'s is an unsubstituted or substituted aryl group.

6. The polymer compound according to Claim 5, wherein the unsubstituted or substituted aryl group represented by Ar² is an unsubstituted or substituted phenyl group.

7. The polymer compound according to any one of Claims 2 to 6, wherein said constitutional unit of the formula (1A) is a constitutional unit of the following formula (5): 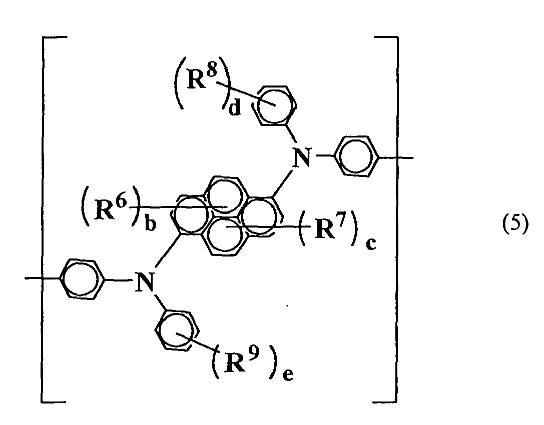 (wherein, R⁶, R⁷, R⁸ and R⁹ represent each independently an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group or cyano group. b and c represent each independently an integer of 0 to 4, and d and e represent each independently an integer of 0 to 5. When there exist a plurality of R⁶'s, R⁷'s, R⁸'s and R⁹'s respectively, they may be mutually the same or different.).

8. The polymer compound according to Claim 7, wherein said constitutional unit of the formula (5) is a constitutional unit of the following formula (5A): 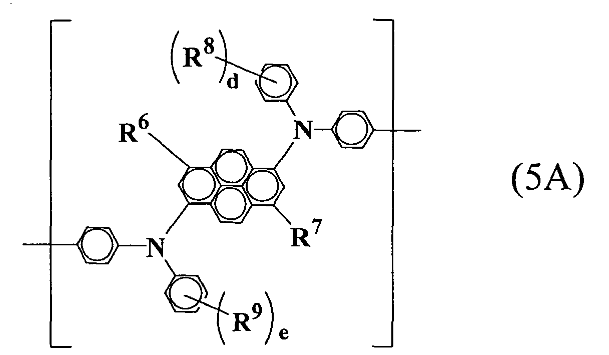 (wherein, R⁶, R⁷, R⁸, R⁹, d and e are as described above. When there exist a plurality of R⁸'s and R⁹'s respectively, they may be mutually the same or different.).

9. The polymer compound according to Claim 7 or 8, wherein R⁶ and R⁷ represent each independently an aryl group substituted with an alkyl group, and R⁸ and R⁹ represent each independently an alkyl group.

10. The polymer compound according to Claim 7 or 8, wherein R⁶ and R⁷ represent each independently a substituted amino group, and R⁸ and R⁹ represent each independently an alkyl group.

11. The polymer compound according to any one of Claims 2 to 6, wherein said constitutional unit of the formula (1B) is a constitutional unit of the following formula (5'): 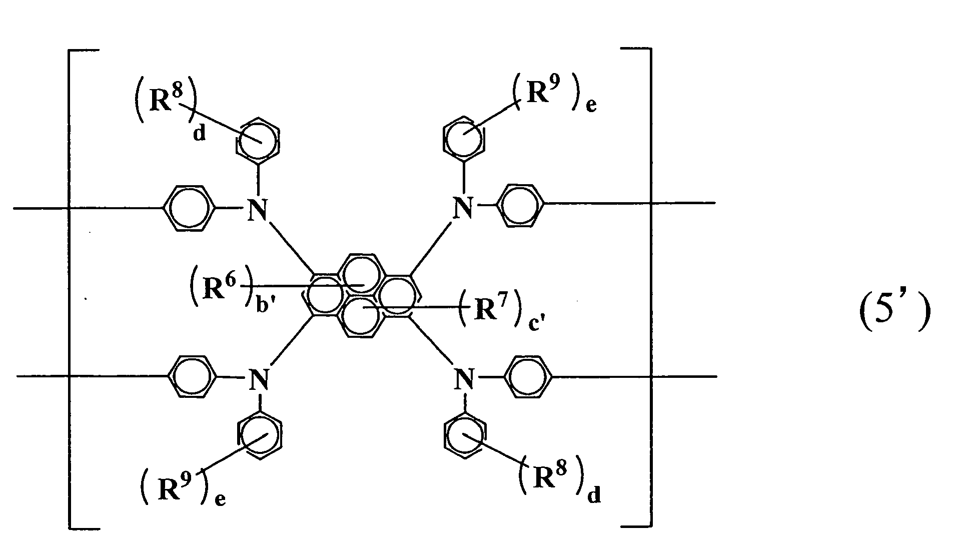 (wherein, R⁶, R⁷, R⁸, R⁹, d and e are as described above. b' and c' represent each independently an integer of 0 to 3. When there exist a plurality of R⁶'s, R^{7's}, R⁸'s and R⁹'s respectively, they may be mutually the same or different. Two d's and two e's may each be the same or different.).

12. The polymer compound according to any one of Claims 1 to 11, wherein Ar³ and Ar⁷ represent each independently an unsubstituted or substituted phenylene group, an unsubstituted or substituted naphthalenediyl group, an unsubstituted or substituted anthracenediyl group, an unsubstituted or substituted phenanthrenediyl group, an unsubstituted or substituted naphthacenediyl group, an unsubstituted or substituted fluorenediyl group, an unsubstituted or substituted pyrenediyl group, an unsubstituted or substituted perylenediyl group, an unsubstituted or substituted pyridinediyl group, an unsubstituted or substituted thiophenediyl group, an unsubstituted or substituted furandiyl group, an unsubstituted or substituted quinolinediyl group, an unsubstituted or substituted isoquinolinediyl group, an unsubstituted or substituted quinoxalinediyl group, an unsubstituted or substituted benzo[1,2,5]thiadiazolediyl group, an unsubstituted or substituted benzothiazolediyl group, an unsubstituted or substituted carbazolediyl group, an unsubstituted or substituted phenoxazinediyl group, an unsubstituted or substituted phenothiazinediyl group, or an unsubstituted or substituted dibenzosilolediyl group.

13. The polymer compound according to Claim 12, wherein Ar³ and Ar⁷ represent each independently an unsubstituted or substituted phenylene group, an unsubstituted or substituted fluorenediyl group, an unsubstituted or substituted benzo[1,2,5]thiadiazolediyl group, an unsubstituted or substituted phenoxazinediyl group, or an unsubstituted or substituted phenothiazinediyl group.

14. The polymer compound according to Claim 13, wherein Ar³ and Ar⁷ represent each independently a di-valent group of the following formula (6), (7), (8), (9) or (10): 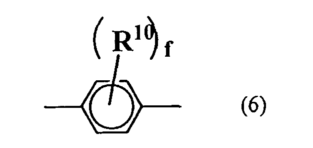 (wherein, R¹⁰ represents an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group or cyano group. f represents an integer of 0 to 4. When there exist a plurality of R¹⁰'s, they may be mutually the same or different.) 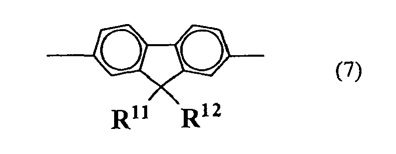 (wherein, R¹¹ and R¹² represent each independently a hydrogen atom, alkyl group, aryl group, arylalkyl group or mono-valent heterocyclic group.) 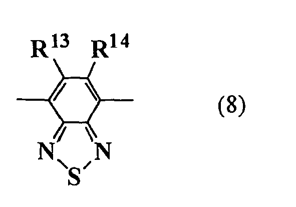 (wherein, R¹³ and R¹⁴ represent each independently a hydrogen atom, alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group or cyano group.) 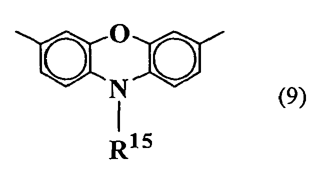 (wherein, R¹⁵ represents a hydrogen atom, alkyl group, aryl group" mono-valent heterocyclic group or arylalkyl group.) 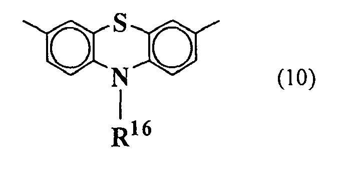 (wherein, R¹⁶ represents a hydrogen atom, alkyl group, aryl group,, mono-valent heterocyclic group or arylalkyl group.).

15. The polymer compound according to any one of Claims 1 to 14, wherein Ar⁴ and Ar⁶ represent each independently an unsubstituted or substituted arylene group.

16. The polymer compound according to any one of Claims 1 to 15, wherein Ar⁵ represents an unsubstituted or substituted 1,3-phenylene group, an unsubstituted or substituted 1,4-phenylene group, an unsubstituted or substituted 1,4-naphthalenediyl group, an unsubstituted or substituted 2,7-fluorenediyl group, an unsubstituted or substituted 2,5-pyridinediyl group, an unsubstituted or substituted 1,4-isoquinolinediyl group, an unsubstituted or substituted 4,7-benzo[1,2,5]thiadiazolediyl group, an unsubstituted or substituted 3,7-phenoxazinediyl group, an unsubstituted or substituted group of the following formula (3A-1): 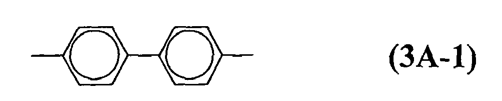 , or an unsubstituted or substituted group of the following formula (3A-4):

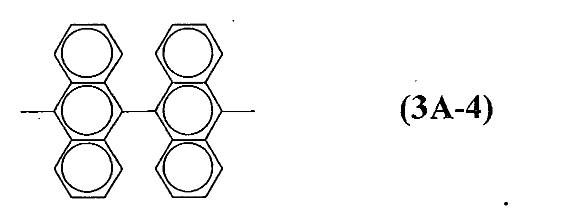

17. The polymer compound according to any one of Claims 1 to 16, wherein the total mole number of said constitutional unit of the formula (1), said repeating unit of the formula (2), said repeating unit of the formula (3) and said repeating unit of the formula (4) with respect to the total mole number of all constitutional units and all repeating units is 90 to 100%.

18. The polymer compound according to any one of Claims 1 to 16, wherein the total mole number of said constitutional unit of the formula (1), said repeating unit of the formula (2) and said repeating unit of the formula (3) with respect to the total mole number of all constitutional units and all repeating units is 90 to 100%.

19. The polymer compound according to Claim 18, wherein the total mole number of said constitutional unit of the formula (1), said repeating unit of the formula (2) and said repeating unit of the formula (3) with respect to the total mole number of all constitutional units and all repeating units is 95 to 100%.

20. A method of producing a polymer compound comprising at least one constitutional unit of the following formula (1), and at least one selected from the group consisting of a repeating unit of the following formula (2), a repeating unit of the following formula (3) and a repeating unit of the following formula (4), comprising condensation-polymerizing at least one compound of the following formula (a), and at least one selected from the group consisting of a compound of the following formula (b-1), a compound of the following formula (b-2) and a compound of the following formula (b-3): 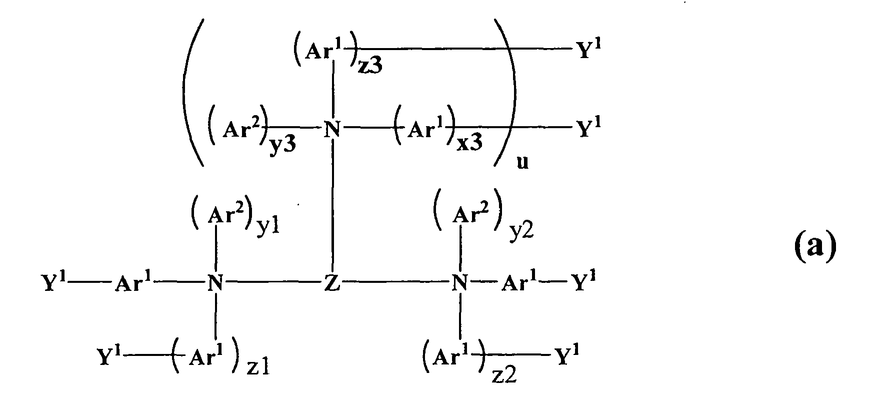 (wherein, x3, y1, y2, z1, z2 and z3 represent each independently 0 or 1. y3 represents an integer of 0 to 2. Here, y1+z1 and y2+z2 are 1, and x3+y3+z3 is 2. u represents an integer of 0 to 8. When u is 2 or more, numerical values of each of x3's, y3's and z3's may be the same or different. Z represents an unsubstituted or substituted (2+u)-valent pyrene residue, Ar¹ represents an unsubstituted or substituted arylene group or an unsubstituted or substituted di-valent heterocyclic group, and Ar² represents an unsubstituted or substituted aryl group or an unsubstituted or substituted mono-valent heterocyclic group. Y¹ represents a halogen atom, sulfonate group of the following formula (a-1), methoxy group, borate residue, boric acid residue, group of the following formula (a-2), group of the following formula (a-3), or group of the following formula (a-4). A plurality of Y¹'s may be the same or different. A plurality of Ar¹'s may be the same or different. When there exist a plurality of Ar²'s, they may be the same or different.)
**Y¹-Ar³-Y¹** **(b-1)**
(wherein, Ar³ represents an unsubstituted or substituted arylene group, an unsubstituted or substituted di-valent heterocyclic group or a di-valent group having a metal complex structure. Y¹ represents the same meaning as described above. A plurality of Y¹'s may be the same or different.) 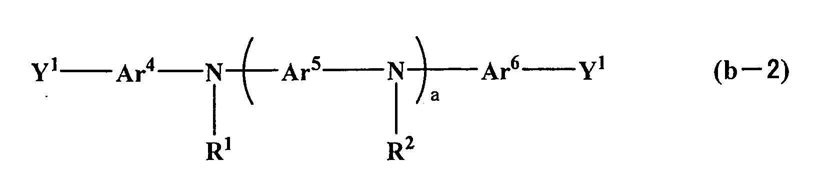 (wherein, Ar⁴, Ar⁵ and Ar⁶ represent each independently an unsubstituted or substituted arylene group, an unsubstituted or substituted di-valent heterocyclic group or an unsubstituted or substituted di-valent group having two aromatic rings connected via a single bond. R¹ and R² represent each independently a hydrogen atom, alkyl group, aryl group, mono-valent heterocyclic group or arylalkyl group. Y¹ has the same meaning as described above. a represents 0 or 1. A plurality of Y¹'s may be the same or different.)
**Y¹-Ar⁷-X¹-Y¹** **(b-3)**
(wherein, Ar⁷ represents an unsubstituted or substituted arylene group, an unsubstituted or substituted di-valent heterocyclic group or a di-valent group having a metal complex structure. X¹ represents -CR³=CR⁴- or -C=C-. Here, R³ and R⁴ represent each independently a hydrogen atom, alkyl group, aryl group, mono-valent heterocyclic group, carboxyl group, substituted carboxyl group or cyano group. Y¹ represents the same meaning as described above. A plurality of Y¹'s may be the same or different.) 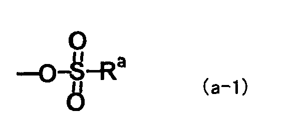 (wherein, R^{a} represents an unsubstituted or substituted alkyl group or an unsubstituted or substituted aryl group.)
**-MgX_{A}** (a-2)
(wherein, X_{A} represents a halogen atom.)
**-ZnX_{A}** (a-3)
(wherein, X_{A} has the same meaning as described above.)
-**Sn(R^{a})₃** **(a-4)**
(wherein, R^{a} has the same meaning as described above. A plurality of R^{a}'s may be mutually the same or different.) 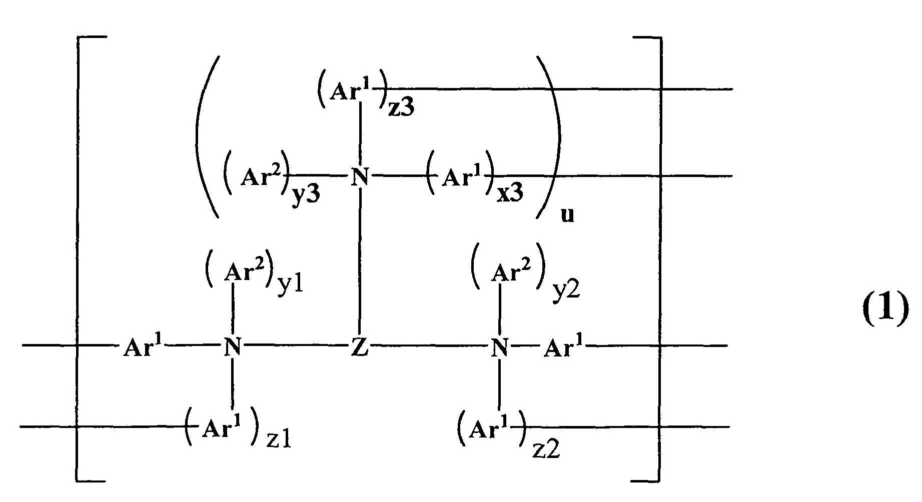 (wherein, x3, y1, y2, y3, z1, z2, z3, u, Z, Ar¹ and Ar² have the same meanings as described above. A plurality of Ar¹'s may be the same or different. When there exist a plurality of Ar²'s, they may be the same or different.)
⁅Ar³⁆ (2) 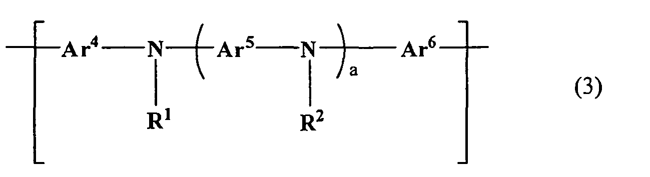
⁅Ar⁷-X¹⁆ (4)
(wherein, Ar³, Ar⁴, Ar⁵, Ar⁶, Ar⁷, R¹, R², X¹ and a have the same meanings as described above.).

21. A compound of the following formula (11): 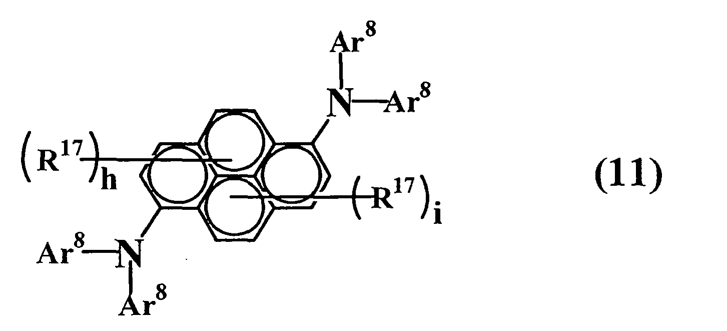 (wherein, R¹⁷ represents a halogen atom. h represents an integer of 1 to 4, and i represents an integer of 0 to 4. Ar⁸ represents an unsubstituted or substituted alkyl group, an unsubstituted or substituted aryl group, or an unsubstituted or substituted mono-valent heterocyclic group. A plurality of Ar⁸'s may be the same or different. When there exist a plurality of R¹⁷'s, they may be mutually the same or different.).

22. The compound according to Claim 21, wherein said compound of the formula (11) is a compound of the following formula (12): 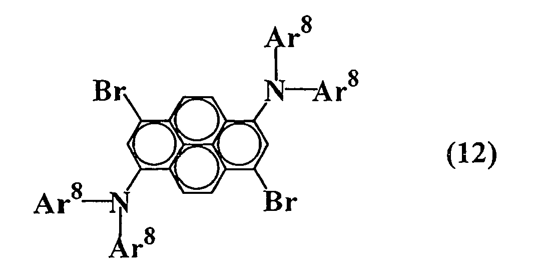 (wherein, Ar⁸ has the same meaning as described above. A plurality of Ar⁸'s may be the same or different.).

23. The compound according to Claim 21, wherein said compound of the formula (11) is a compound of the following formula (13): 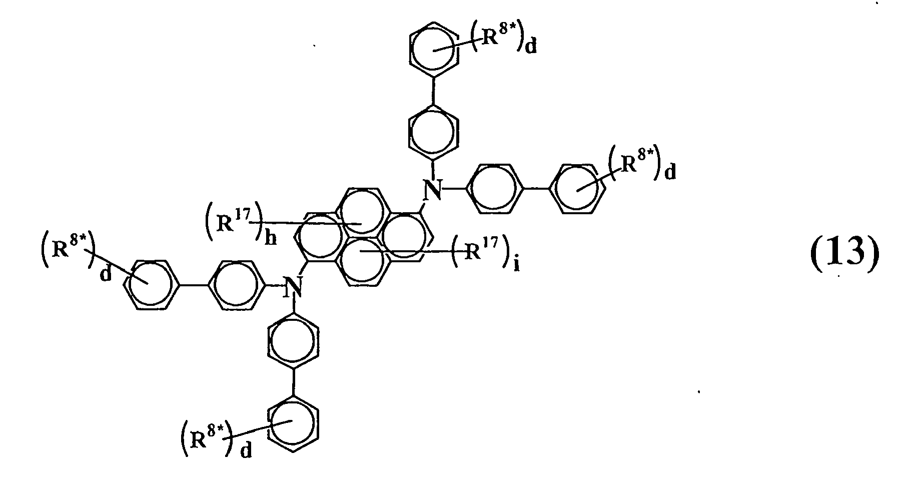 (wherein, R^{8*} represents an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group, cyano group, boric acid residue or borate residue. d represents an integer of 0 to 5. R¹⁷, h and i have the same meanings as described above. When there exist a plurality of R^{8*}'s and R¹⁷'s respectively, they may be mutually the same or different. A plurality of d's may be the same or different.).

24. The compound according to Claim 23, wherein said compound of the formula (13) is a compound of the following formula (14): 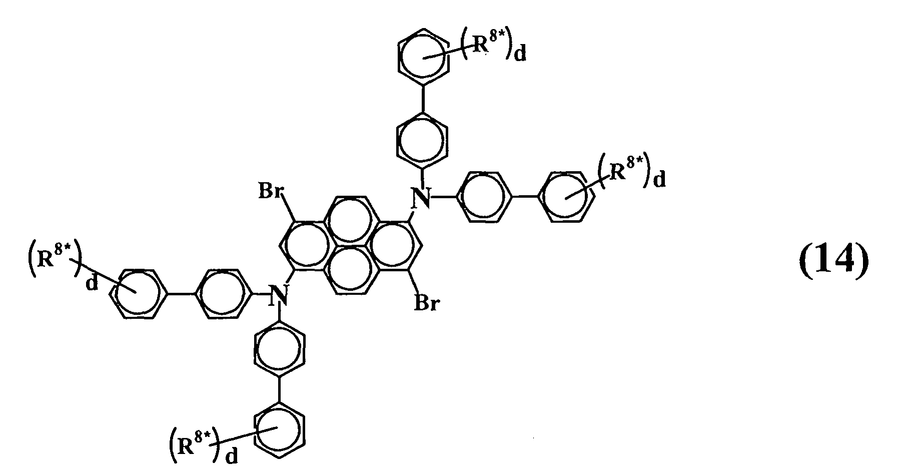 (wherein, R^{8*} and d have the same meanings as described above. When there exist a plurality of R^{8*}'s, they may be mutually the same or different.).

25. A compound of the following formula (15): 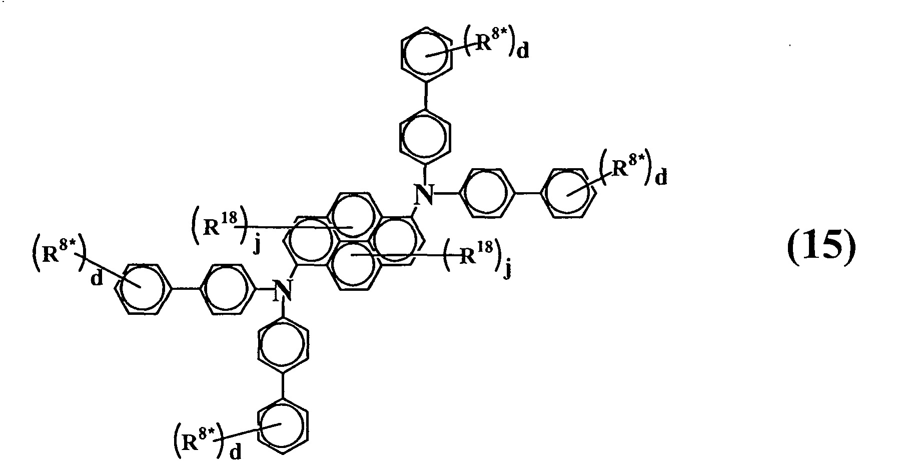 (wherein, R¹⁸ represents an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, acyl group, acyloxy group, imine residue, amide group, acid imide group, mono-valent heterocyclic group, carboxyl group, substituted carboxyl group, nitro group or cyano group. j represents an integer of 0 to 4. R^{8*} represents an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, mono-valent heterocyclic group, carboxyl group, substituted carboxyl group, nitro group, cyano group, boric acid residue or borate residue. d represents an integer of 0 to 5. When there exist a plurality of R^{8*}'s and R¹⁸'s respectively, they may be mutually the same or different. A plurality of d's and j's may be resepctively the same or different.).

26. The compound according to Claim 25, wherein said compound of the formula (15) is a compound of the following formula (16): 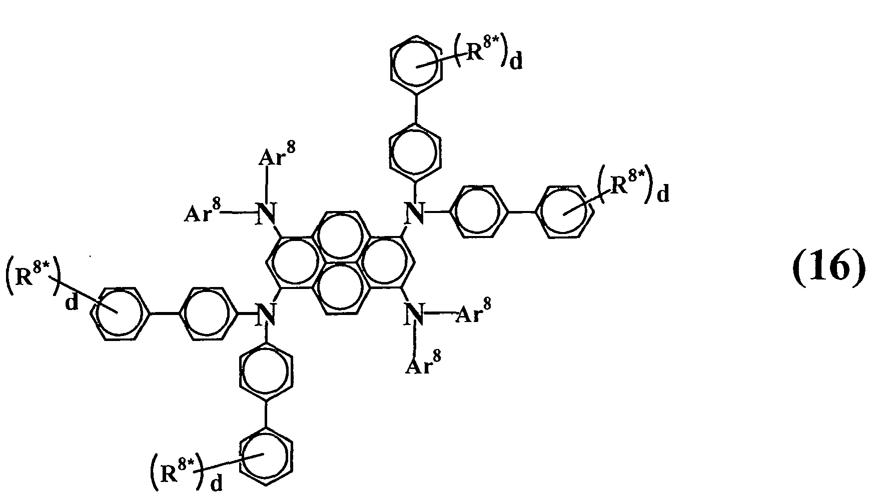 (wherein, Ar⁸, R^{8*} and d are as described above. A plurality of Ar⁸'s and d's may be resepctively the same or different. When there exist a plurality of R^{8*}'s, they may be mutually the same or different.).

27. The compound according to Claim 26, wherein said compound of the formula (16) is a compound of the following formula (17): 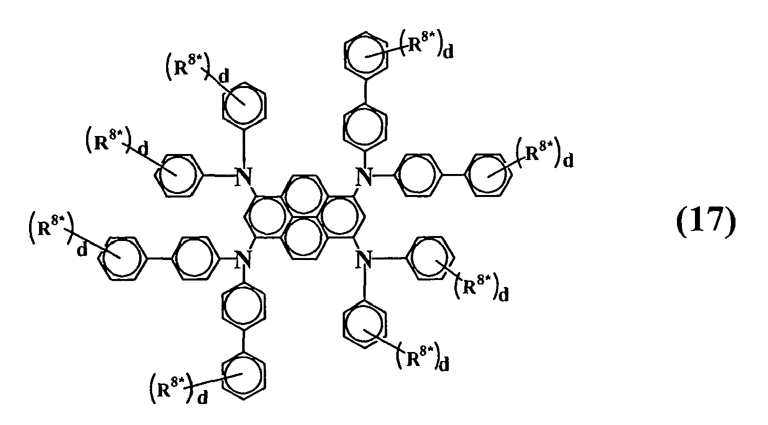 (wherein, R^{8*} and d are as described above. When there exist a plurality of R^{8*}'s, they may be mutually the same or different. A plurality of d's may be the same or different.).

28. The compound according to Claim 27, wherein said compound of the formula (17) is a compound of the following formula (18): 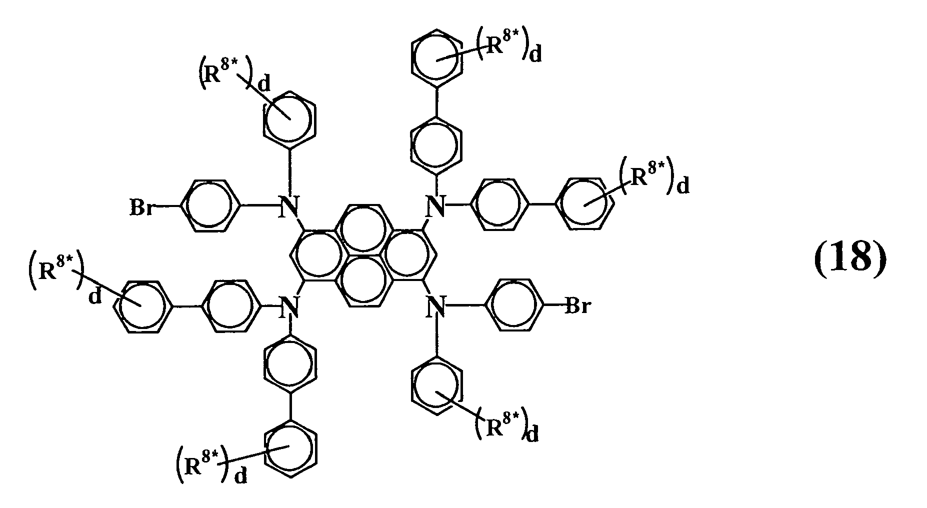 (wherein, R^{8*} and d are as described above. When there exist a plurality of R^{8*}'s, they may be mutually the same or different. A plurality of d's may be the same or different.).

29. A method of producing a compound of the following formula (12): 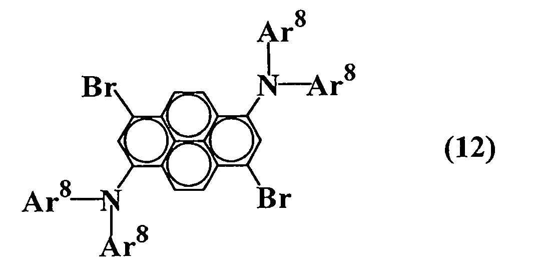 (wherein, Ar⁸ represents an unsubstituted or substituted alkyl group, an unsubstituted or substituted aryl group, or an unsubstituted or substituted mono-valent heterocyclic group. A plurality of Ar⁸'s may be the same or different.) comprising reacting a compound of the following formula (19): 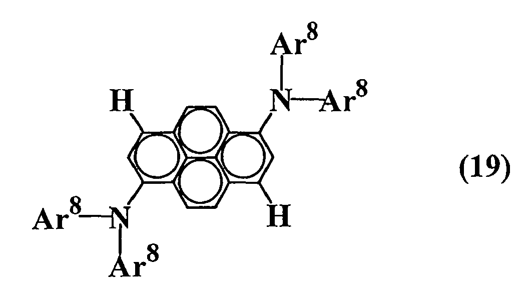 (wherein, Ar⁸ is as described above. A plurality of Ar⁸'s may be mutually the same or different.) with a brominating agent.

30. A composition comprising at least one material selected from the group consisting of hole transporting materials, electron transporting materials and light emitting materials, and the polymer compound as described in any one of Claims 1 to 19.

31. A solution comprising the polymer compound as described in any one of Claims 1 to 19, and a solvent.

32. A thin film comprising the polymer compound as described in any one of Claims 1 to 19.

33. A light emitting device having electrodes composed of an anode and a cathode, and an organic layer provided between the electrodes and containing the polymer compound as described in any one of Claims 1 to 19.
